(19) Europäisches Patentamt
European Patent Office
Office européen des brevets

(11) EP 4 711 361 A1

(12) **EUROPEAN PATENT APPLICATION**
published in accordance with Art. 153(4) EPC

(43) Date of publication:
**18.03.2026 Bulletin 2026/12**

(21) Application number: **24806517.9**

(22) Date of filing: **11.05.2024**

(51) International Patent Classification (IPC):
*C07D 401/04* (2006.01) *C07D 491/052* (2006.01)
*C07D 471/04* (2006.01) *C07D 413/04* (2006.01)
*C07D 519/00* (2006.01) *C07D 498/04* (2006.01)
*A61P 9/12* (2006.01) *A61K 31/438* (2006.01)
*A61K 31/538* (2006.01) *A61K 31/437* (2006.01)
*A61K 31/436* (2006.01) *A61K 31/4375* (2006.01)
*A61K 31/4439* (2006.01)

(52) Cooperative Patent Classification (CPC):
**A61K 31/436; A61K 31/437; A61K 31/4375; A61K 31/438; A61K 31/4439; A61K 31/538; A61P 9/12; C07D 401/04; C07D 413/04; C07D 471/04; C07D 491/052; C07D 498/04; C07D 519/00**

(86) International application number:
**PCT/CN2024/092687**

(87) International publication number:
**WO 2024/235165 (21.11.2024 Gazette 2024/47)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.05.2023 CN 202310537851**
**22.09.2023 CN 202311235509**
**17.11.2023 CN 202311542512**
**08.01.2024 CN 202410027233**
**01.02.2024 CN 202410149461**

(71) Applicant: **Jiangsu Deyuan Pharmaceutical Co.,Ltd.**
**Jiangsu Province, 222047 (CN)**

(72) Inventors:
- **GAN, Lu**
  **Shanghai 200131 (CN)**
- **YU, Tao**
  **Shanghai 200131 (CN)**
- **LIU, Ning**
  **Shanghai 200131 (CN)**
- **ZHANG, Yang**
  **Shanghai 200131 (CN)**
- **CHEN, Shuhui**
  **Shanghai 200131 (CN)**

(74) Representative: **Murgitroyd & Company**
**165-169 Scotland Street**
**Glasgow G5 8PL (GB)**

(54) **NITROGEN-CONTAINING HETEROCYCLIC COMPOUND AND USE THEREOF**

(57) A nitrogen-containing heterocyclic compound and the use thereof, and specifically disclosed are a compound as represented by formula (II), a stereoisomer thereof, and a pharmaceutically acceptable salt thereof.

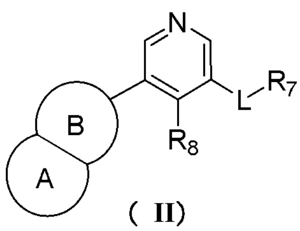

( II)

EP 4 711 361 A1

## Description

**The present application claims the right of the following priorities:**

Application No.: CN202310537851.9, application date: May 12, 2023;
Application No.: CN202311235509.X, application date: September 22, 2023;
Application No.: CN202311542512.6, application date: November 17, 2023;
Application No.: CN202410027233.4, application date: January 08, 2024;
Application No.: CN202410149461.9, application date: February 1, 2024.

TECHNICAL FIELD

The present disclosure relates to a nitrogen-containing heterocyclic compound and a use thereof, and specifically discloses a compound of formula (II), a stereoisomer thereof, and a pharmaceutically acceptable salt thereof.

BACKGROUND

Hypertension is a clinical syndrome characterized primarily by elevated systemic arterial blood pressure (systolic pressure $\geq$140 mmHg and/or diastolic pressure $\geq$90 mmHg), which may be accompanied by functional or organic damage to organs such as the heart, brain, and kidneys. For the treatment of hypertension, commonly used drugs include five categories: calcium channel blockers (CCB), angiotensin-converting enzyme inhibitors (ACEI), angiotensin receptor blockers (ARB), diuretics, and $\beta$-blockers, as well as fixed-dose combination preparations composed of the aforementioned drugs. Additionally, $\alpha$-blockers or other classes of antihypertensive agents (such as renin inhibitors, centrally acting agents, etc.) may sometimes be utilized in certain hypertensive populations.

For adult patients with hypertension requiring pharmacotherapy, initial treatment is recommended with any one of three classes of first-line antihypertensive medications: diuretics, angiotensin-converting enzyme inhibitors or angiotensin receptor blockers, and calcium channel blockers. For hypertensive patients whose blood pressure remains uncontrolled with monotherapy, the combination use of antihypertensive drugs has become a fundamental approach to blood pressure management. To achieve the target blood pressure level, most patients with hypertension require the use of two or more antihypertensive drugs. Resistant hypertension is defined when a patient’s blood pressure remains above 140 mmHg despite the concurrent use of at least three antihypertensive drugs of different classes, one of which is a diuretic. For patients with resistant hypertension, a fourth drug such as a $\beta$-blocker, aldosterone receptor antagonist, triamterene, clonidine, or $\alpha$-blocker should be added to the three-drug combination therapy.

Since 2007, no new antihypertensive drugs have been approved for market release, leaving a large number of patients with resistant hypertension still inadequately treated. Both resistant hypertension and hyperaldosteronism are characterized by water and sodium retention. In clinical studies, Baxdrostat produced a dose-dependent reduction in blood pressure and aldosterone secretion levels, indicating that aldosterone is a significant factor in drug resistance among patients with resistant hypertension. The primary pharmacological targets for treating resistant hypertension by reducing aldosterone levels include blocking the mineralocorticoid receptor and inhibiting aldosterone synthase.

Aldosterone is encoded by the CYP11B2 gene, which possesses 11-$\beta$-hydroxylase, 18-hydroxylase, and 18-dehydrogenase activities. Its primary action occurs at the final step of aldosterone synthesis, where 11-deoxycorticosterone (11-DOC) serves as the substrate to produce aldosterone. CYP11B1 and CYP11B2 share 93% similarity in their amino acid sequences, and inhibition of CYP11B1 activity may lead to adrenal cortical insufficiency. Highly selective inhibition of aldosterone synthase is essential yet challenging to achieve. Baxdrostat, a highly selective small-molecule aldosterone synthase inhibitor, demonstrated four major advantages in phase 2 trials: significant blood pressure reduction, no impact on cortisol levels, rare occurrence of elevated blood potassium, and absence of steroid precursor accumulation. Baxdrostat at a 2 mg dose demonstrated efficacy in reducing office systolic and diastolic blood pressure in patients with resistant hypertension, along with favorable safety and tolerability profiles.

In summary, the development of selective aldosterone synthase inhibitors represents a highly promising new approach for treating diseases associated with elevated aldosterone levels, including resistant hypertension and primary aldosteronism, thereby expanding clinical treatment options.

SUMMARY

The present disclosure provides a compound of formula (II), a stereoisomer thereof, or a pharmaceutically acceptable salt thereof,

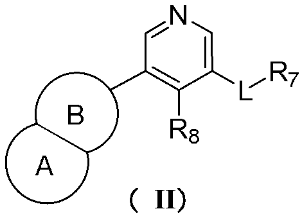

(II) ,

wherein

ring A is selected from the group consisting of ring $A_1$ and ring $A_2$;
ring $A_1$ is

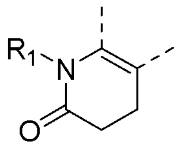;

ring $A_2$ is selected from the group consisting of

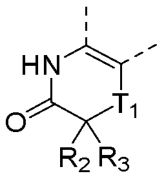, 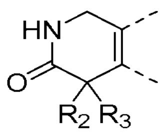,

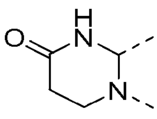, 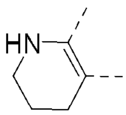, 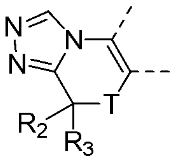, 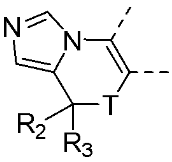, 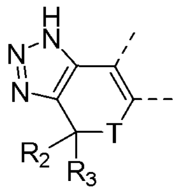, and 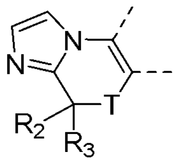,

wherein the

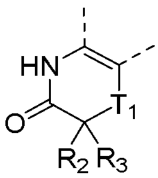, 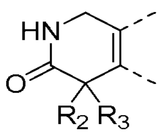, 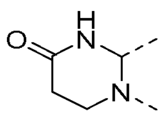, 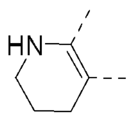, 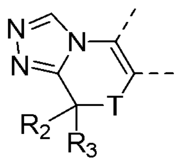,

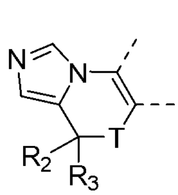, 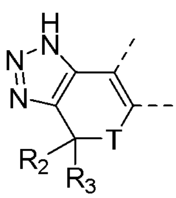, and 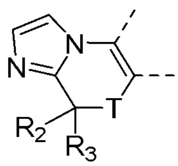

are each independently and optionally substituted by 1, 2, or 3 $R_1$;
ring B is selected from the group consisting of ring $B_1$ and ring $B_2$;
ring $B_1$ is selected from the group consisting of phenyl, pyridyl, and pyridazinyl, wherein the phenyl, pyridyl, and pyridazinyl are each independently and optionally substituted by 1, 2, or 3 $R_6$;
ring $B_2$ is 5-membered heteroaryl, wherein the 5-membered heteroaryl is optionally substituted by 1, 2, or 3 $R_6$;

T is selected from the group consisting of a single bond, $CH_2$, NH, and O;
$T_1$ is selected from the group consisting of O and $CR_4R_5$;
$R_1$ is selected from the group consisting of H, =NR, $C_{1-3}$ alkyl, and $C_{1-3}$ haloalkyl;
each $R_2$, $R_3$, $R_4$, and $R_5$ is independently selected from the group consisting of H, F, Cl, Br, I, $C_{1-3}$ alkyl, $C_{1-3}$ alkoxy, $C_{1-3}$ haloalkyl, and $C_{1-3}$ haloalkoxy, wherein not all four of $R_2$, $R_3$, $R_4$, and $R_5$ are simultaneously H;
alternatively, $R_2$ and $R_3$ together with the carbon atom to which they are attached form a cyclopropyl;
alternatively, $R_4$ and $R_5$ together with the carbon atom to which they are attached form a cyclopropyl;
alternatively, $R_3$ and $R_4$ together with the carbon atom to which they are attached form a double bond or a cyclopropyl;
each $R_6$ is independently selected from the group consisting of H, F, Cl, Br, I, and $C_{1-3}$ alkyl;
the structural moiety

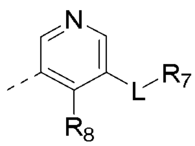

is selected from the group consisting of

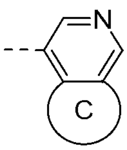 and 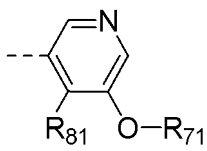;

$R_{71}$ is 4- to 6-membered heterocycloalkyl, wherein the 4- to 6-membered heterocycloalkyl is optionally substituted by 1, 2, or 3 $R_a$;
$R_{81}$ is selected from the group consisting of H, F, Cl, Br, I, and $C_{1-3}$ alkyl;
ring C is selected from the group consisting of ring $C_1$ and ring $C_2$;
ring $C_1$ is $C_{5-6}$ cycloalkyl substituted by 1 $R_9$, wherein the $R_9$ is selected from the group consisting of -NHC(=O)-$C_{1-3}$ alkyl, -NH-C(=O)-5- to 6-membered heteroaryl, -NH-4- to 6-membered heterocycloalkyl, 4- to 6-membered heterocycloalkyl, and

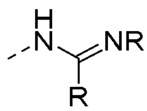,

wherein the -NHC(=O)-$C_{1-3}$ alkyl, -NH-C(=O)-5- to 6-membered heteroaryl, -NH-4- to 6-membered heterocycloalkyl, and 4- to 6-membered heterocycloalkyl are each independently and optionally substituted by 1, 2, or 3 $R_b$;
ring $C_2$ is selected from the group consisting of

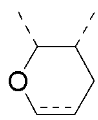, 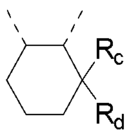, and 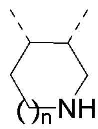,

wherein the

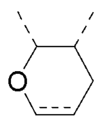, 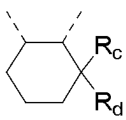, and 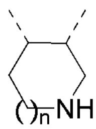

are each independently and optionally substituted by 1, 2, or 3 $R_e$;
each $R_a$ is independently selected from the group consisting of F, Cl, Br, I, $C_{1-3}$ alkyl, and -S(=O)$_2$-$C_{1-3}$ alkyl;
each $R_b$ is independently selected from the group consisting of F, Cl, Br, I, $C_{1-3}$ alkyl, -C(=O)-$C_{1-3}$ alkyl, and -NH-

$C(=O)-C_{1-3}$ alkyl;

$R_c$ and $R_d$ together with the carbon atom to which they are attached form a $C_{3-5}$ cycloalkyl or a 5- to 6-membered heterocycloalkyl;

each $R_e$ is independently selected from the group consisting of F, Cl, Br, I, =O, $C_{1-3}$ alkyl, $C_{1-3}$ alkoxy, $C_{1-3}$ alkylamino, $-NHC(=O)-C_{1-3}$ alkyl, $-NHC(=O)-C_{1-3}$ alkoxy, $-NHC(=O)-C_{1-3}$ alkylamino, and =NR;

R is selected from the group consisting of $C_{1-3}$ alkyl and $C_{1-3}$ alkoxy, wherein the $C_{1-3}$ alkyl and $C_{1-3}$ alkoxy are each independently and optionally substituted by 1, 2, or 3 halogens;

n is 0 or 1;

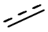 is a single bond or a double bond;

the term "hetero" in the 5-membered heteroaryl, 5- to 6-membered heteroaryl, and 4-to 6-membered heterocycloalkyl represents 1, 2, 3, or 4 heteroatoms or heteroatomic groups, each independently selected from the group consisting of O, S, and N;

provided that

1) when ring A is $A_1$ and ring B is $B_1$, then ring C is $C_2$; or,

2) when ring A is $A_1$, ring B is $B_1$, and ring C is $C_{5-6}$ cycloalkyl substituted by 1 $R_9$, then $R_9$ is

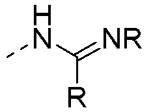

.

The present disclosure provides a compound of formula (II), a stereoisomer thereof, or a pharmaceutically acceptable salt thereof,

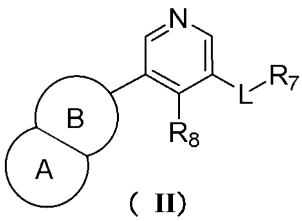

( II)

,

wherein

ring A is selected from the group consisting of ring $A_1$ and ring $A_2$;

ring $A_1$ is

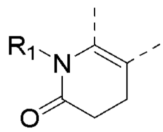

;

ring $A_2$ is selected from the group consisting of

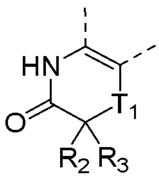

,

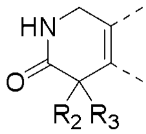

,

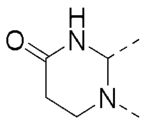, 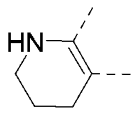, 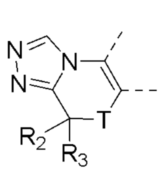, 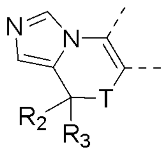, 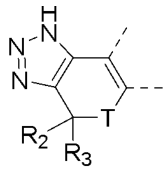, and 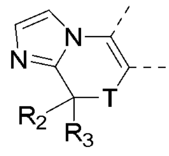,

wherein the

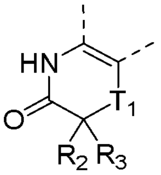, 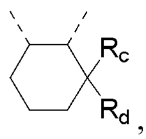, 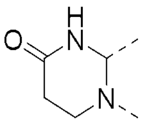, 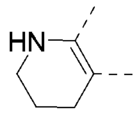, 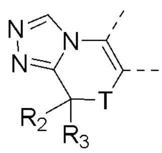, 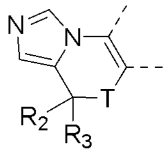,

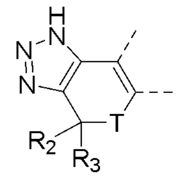, and 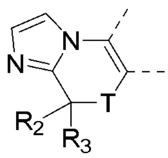;

are each independently and optionally substituted by 1, 2, or 3 $R_1$;
ring B is selected from the group consisting of ring $B_1$ and ring $B_2$;
ring $B_1$ is selected from the group consisting of phenyl, pyridinyl, and pyridazinyl, wherein the phenyl, pyridinyl, and pyridazinyl are each independently and optionally substituted by 1, 2, or 3 $R_6$;
ring $B_2$ is 5-membered heteroaryl, wherein the 5-membered heteroaryl is optionally substituted by 1, 2, or 3 $R_6$;
T is selected from the group consisting of a single bond, $CH_2$, NH, and O;
$T_1$ is selected from the group consisting of O and $CR_4R_5$;
$R_1$ is selected from the group consisting of H, =NR, $C_{1-3}$ alkyl, and $C_{1-3}$ haloalkyl;
each $R_2$, $R_3$, $R_4$, and $R_5$ is independently selected from the group consisting of H, F, Cl, Br, I, $C_{1-3}$ alkyl, $C_{1-3}$ alkoxy, $C_{1-3}$ haloalkyl, and $C_{1-3}$ haloalkoxy, wherein not all four of $R_2$, $R_3$, $R_4$, and $R_5$ are simultaneously H;
alternatively, $R_2$ and $R_3$ together with the carbon atom to which they are attached form a cyclopropyl;
alternatively, $R_4$ and $R_5$ together with the carbon atom to which they are attached form a cyclopropyl;
alternatively, $R_3$ and $R_4$ together with the carbon atom to which they are attached form a double bond or a cyclopropyl;
each $R_6$ is independently selected from the group consisting of H, F, Cl, Br, I, and $C_{1-3}$ alkyl;
the structural moiety

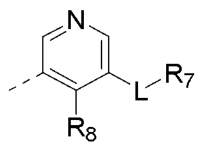

is selected from the group consisting of

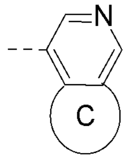 and 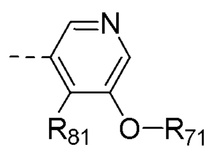;

$R_{71}$ is 4- to 6-membered heterocycloalkyl, wherein the 4- to 6-membered heterocycloalkyl is optionally substituted by 1, 2, or 3 $R_a$;
$R_{81}$ is selected from the group consisting of H, F, Cl, Br, I, and $C_{1-3}$ alkyl;
ring C is selected from the group consisting of ring $C_1$ and ring $C_2$;
ring $C_1$ is cyclohexyl substituted by 1 $R_9$, wherein the $R_9$ is selected from the group consisting of -NHC(=O)-$C_{1-3}$ alkyl,

-NH-C(=O)-5- to 6-membered heteroaryl, -NH-4- to 6-membered heterocycloalkyl, 4- to 6-membered heterocycloalkyl, and

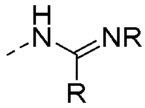,

wherein the -NHC(=O)-$C_{1-3}$ alkyl, -NH-C(=O)-5- to 6-membered heteroaryl, -NH-4- to 6-membered heterocycloalkyl, and 4- to 6-membered heterocycloalkyl are each independently and optionally substituted by 1, 2, or 3 $R_b$;
ring $C_2$ is selected from the group consisting of

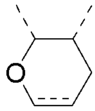, 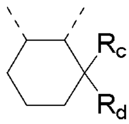, and 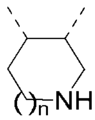,

wherein the

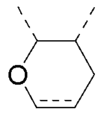, 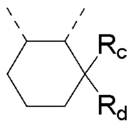, and 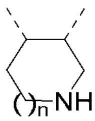

are each independently and optionally substituted by 1, 2, or 3 $R_e$;
each $R_a$ is independently selected from the group consisting of F, Cl, Br, I, $C_{1-3}$ alkyl, and -S(=O)$_2$-$C_{1-3}$ alkyl;
each $R_b$ is independently selected from the group consisting of F, Cl, Br, I, $C_{1-3}$ alkyl, -C(=O)-$C_{1-3}$ alkyl, and -NH-C(=O)-$C_{1-3}$ alkyl;
$R_c$ and $R_d$ together with the carbon atom to which they are attached form a $C_{3-5}$ cycloalkyl or a 5- to 6-membered heterocycloalkyl;
each $R_e$ is independently selected from the group consisting of F, Cl, Br, I, =O, $C_{1-3}$ alkyl, $C_{1-3}$ alkoxy, $C_{1-3}$ alkylamino, -NHC(=O)-$C_{1-3}$ alkyl, -NHC(=O)-$C_{1-3}$ alkoxy, -NHC(=O)-$C_{1-3}$ alkylamino, and =NR;
R is selected from the group consisting of $C_{1-3}$ alkyl and $C_{1-3}$ alkoxy, wherein the $C_{1-3}$ alkyl and $C_{1-3}$ alkoxy are each independently and optionally substituted by 1, 2, or 3 halogens;
n is 0 or 1;
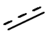 is a single bond or a double bond;
the term "hetero" in the 5-membered heteroaryl, 5- to 6-membered heteroaryl, and 4-to 6-membered heterocycloalkyl represents 1, 2, 3, or 4 heteroatoms or heteroatomic groups, each independently selected from the group consisting of O, S, and N;
provided that

1) when ring A is $A_1$ and ring B is $B_1$, then ring C is $C_2$; or,
2) when ring A is $A_1$, ring B is $B_1$, and ring C is cyclohexyl substituted by 1 $R_9$, then $R_9$ is

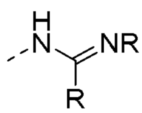.

The present disclosure provides a compound of formula (II), a stereoisomer thereof, or a pharmaceutically acceptable salt thereof,

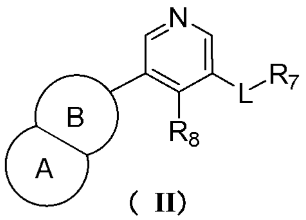

(II) ,

wherein

ring A is selected from the group consisting of ring $A_1$ and ring $A_2$;
ring $A_1$ is

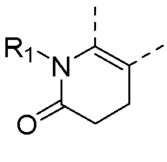

;

ring $A_2$ is selected from the group consisting of

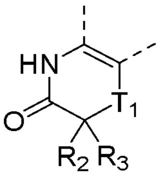 , 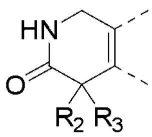 ,

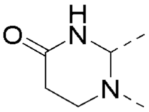 , 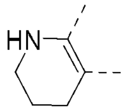 , 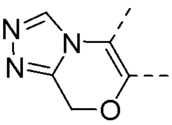 , 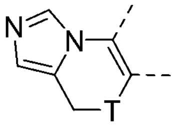 , and 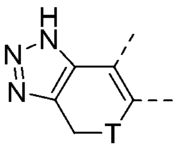 ,

wherein the

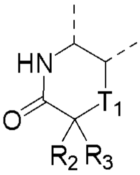 , 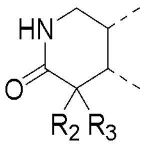 , 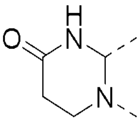 , 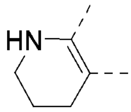 , 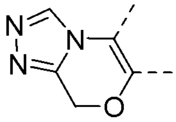 , 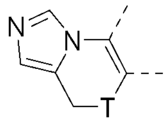 ,

and

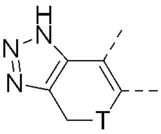

are each independently and optionally substituted by 1, 2, or 3 $R_1$;
ring B is selected from the group consisting of ring $B_1$ and ring $B_2$;
ring $B_1$ is selected from the group consisting of phenyl and pyridyl, wherein the phenyl and pyridyl are each independently and optionally substituted by 1, 2, or 3 $R_6$;
ring $B_2$ is 5-membered heteroaryl, wherein the 5-membered heteroaryl is optionally substituted by 1, 2, or 3 $R_6$;
T is selected from the group consisting of $CH_2$, NH, and O;
$T_1$ is selected from the group consisting of O and $CR_4R_5$;

$R_1$ is selected from the group consisting of H, =NR, $C_{1-3}$ alkyl, and $C_{1-3}$ haloalkyl;
each $R_2$, $R_3$, $R_4$, and $R_5$ is independently selected from the group consisting of H, F, Cl, Br, I, $C_{1-3}$ alkyl, $C_{1-3}$ alkoxy, $C_{1-3}$ haloalkyl, and $C_{1-3}$ haloalkoxy, wherein not all four of $R_2$, $R_3$, $R_4$, and $R_5$ are simultaneously H;
alternatively, $R_2$ and $R_3$ together with the carbon atom to which they are attached form a cyclopropyl;
alternatively, $R_4$ and $R_5$ together with the carbon atom to which they are attached form a cyclopropyl;
alternatively, $R_3$ and $R_4$ together with the carbon atom to which they are attached form a double bond or a cyclopropyl;
each $R_6$ is independently selected from the group consisting of H, F, Cl, Br, I, and $C_{1-3}$ alkyl;
the structural moiety

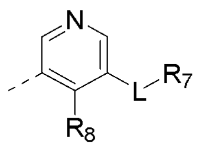

is selected from the group consisting of

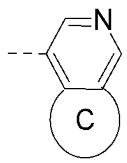 and 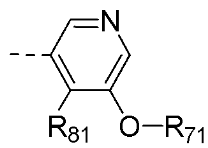;

$R_{71}$ is 4- to 6-membered heterocycloalkyl, wherein the 4- to 6-membered heterocycloalkyl is optionally substituted by 1, 2, or 3 $R_a$;
$R_{81}$ is selected from the group consisting of H, F, Cl, Br, I, and $C_{1-3}$ alkyl;
ring C is selected from the group consisting of ring $C_1$ and ring $C_2$;
ring $C_1$ is cyclohexyl substituted by 1 $R_9$, wherein the $R_9$ is selected from the group consisting of -NHC(=O)-$C_{1-3}$ alkyl, -NH-C(=O)-5- to 6-membered heteroaryl, -NH-4- to 6-membered heterocycloalkyl, 4- to 6-membered heterocycloalkyl, and

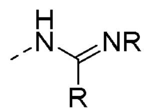,

wherein the -NHC(=O)-$C_{1-3}$ alkyl, -NH-C(=O)-5- to 6-membered heteroaryl, -NH-4- to 6-membered heterocycloalkyl, and 4- to 6-membered heterocycloalkyl are each independently and optionally substituted by 1, 2, or 3 $R_b$;
ring $C_2$ is selected from the group consisting of

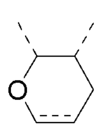, 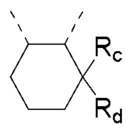, and 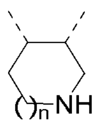,

wherein the

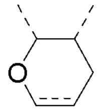, 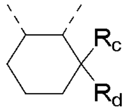, and 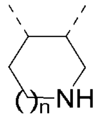

are each independently and optionally substituted by 1, 2, or 3 $R_e$;
each $R_a$ is independently selected from the group consisting of F, Cl, Br, I, $C_{1-3}$ alkyl, and -S(=O)$_2$-$C_{1-3}$ alkyl;
each $R_b$ is independently selected from the group consisting of F, Cl, Br, I, $C_{1-3}$ alkyl, -C(=O)-$C_{1-3}$ alkyl, and -NH-C(=O)-$C_{1-3}$ alkyl;
$R_c$ and $R_d$ together with the carbon atom to which they are attached form a $C_{3-5}$ cycloalkyl or a 5- to 6-membered

heterocycloalkyl;

each $R_e$ is independently selected from the group consisting of F, Cl, Br, I, =O, $C_{1-3}$ alkyl, $C_{1-3}$ alkoxy, $C_{1-3}$ alkylamino, -NHC(=O)-$C_{1-3}$ alkyl, -NHC(=O)-$C_{1-3}$ alkoxy, -NHC(=O)-$C_{1-3}$ alkylamino, and =NR;

R is selected from the group consisting of $C_{1-3}$ alkyl and $C_{1-3}$ alkoxy, wherein the $C_{1-3}$ alkyl and $C_{1-3}$ alkoxy are each independently and optionally substituted by 1, 2, or 3 halogens;

n is 0 or 1;

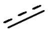 is a single bond or a double bond;

the term "hetero" in the 5-membered heteroaryl, 5- to 6-membered heteroaryl, and 4-to 6-membered heterocycloalkyl represents 1, 2, 3, or 4 heteroatoms or heteroatomic groups, each independently selected from the group consisting of O, S, and N;

provided that

1) when ring A is $A_1$ and ring B is $B_1$, then ring C is $C_2$; or,

2) when ring A is $A_1$, ring B is $B_1$, and ring C is cyclohexyl substituted by 1 $R_9$, then $R_9$ is

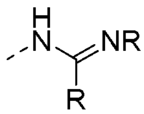 .

The present disclosure provides a compound of formula (I), a stereoisomer thereof, or a pharmaceutically acceptable salt thereof,

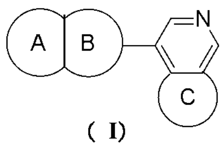

( I) ,

wherein

ring B is selected from the group consisting of ring $B_1$ and ring $B_2$;

ring $B_1$ is phenyl, wherein the phenyl is optionally substituted by 1, 2, or 3 $R_6$;

ring $B_2$ is 5-membered heteroaryl, wherein the 5-membered heteroaryl is optionally substituted by 1, 2, or 3 $R_6$;

ring A is selected from the group consisting of ring $A_1$ and ring $A_2$;

ring $A_1$ is

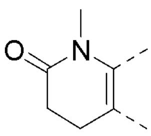 ;

ring $A_2$ is selected from the group consisting of

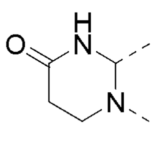 , 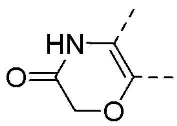 ,

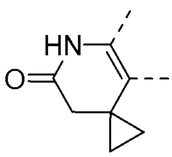 , 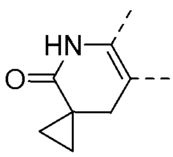 , 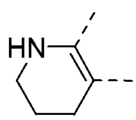 , 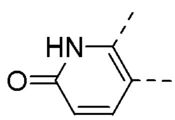 , 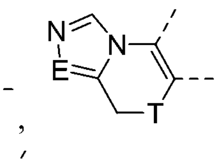 , and 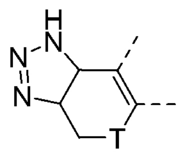 ,

wherein the

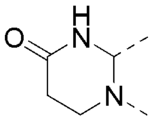, 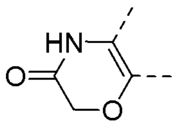, 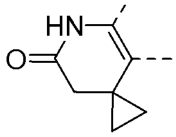, 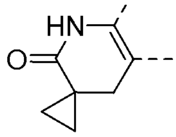, 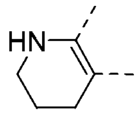,

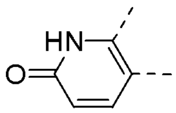, 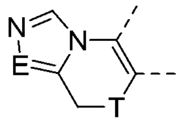, and 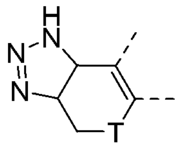

are each independently and optionally substituted by 1, 2, or 3 $R_1$;
E is selected from the group consisting of CH and N;
T is selected from the group consisting of $CH_2$, NH, and O;
ring C is selected from the group consisting of ring $C_1$ and ring $C_2$;
ring $C_1$ is cyclohexyl substituted by 1 $R_9$, wherein the $R_9$ is $-NHC(=O)CH_2CH_3$;
ring $C_2$ is selected from the group consisting of

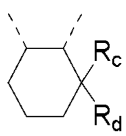, 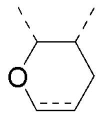, 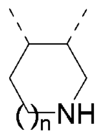,

and

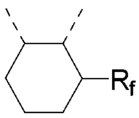,

wherein the

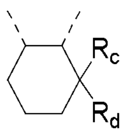, 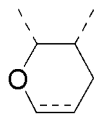, 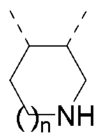, and 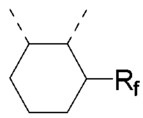

are each independently and optionally substituted by 1, 2, or 3 $R_e$;
each $R_1$ is independently selected from the group consisting of F, Cl, Br, I, $C_{1-3}$ alkyl, $C_{1-3}$ alkoxy, and =NR;
each $R_6$ is independently selected from the group consisting of F, Cl, Br, I, and $C_{1-3}$ alkyl;
$R_c$ and $R_d$ together with the carbon atom to which they are attached form a $C_{3-5}$ cycloalkyl or a 5- to 6-membered heterocycloalkyl;
each $R_e$ is independently selected from the group consisting of F, Cl, Br, I, $C_{1-3}$ alkyl, $C_{1-3}$ alkoxy, $C_{1-3}$ alkylamino, $-NHC(=O)-C_{1-3}$ alkyl, $-NHC(=O)-C_{1-3}$ alkoxy, and $-NHC(=O)-C_{1-3}$ alkylamino;
$R_f$ is selected from the group consisting of =NR,

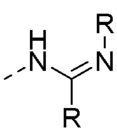, and 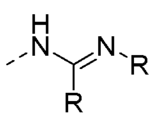;

R is selected from the group consisting of $C_{1-3}$ alkyl and $C_{1-3}$ alkoxy, wherein the $C_{1-3}$ alkyl and $C_{1-3}$ alkoxy are optionally substituted by 1, 2, or 3 halogens;

n is 0 or 1;

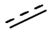 is a single bond or a double bond;

the term "hetero" in the 5-membered heteroaryl, 5- to 6-membered heteroaryl, and 5-to 6-membered heterocycloalkyl represents 1, 2, 3, or 4 heteroatoms or heteroatomic groups, each independently selected from the group consisting of O, S, N, and C(=O);

provided that

when ring A is $A_1$ and ring B is $B_1$, then ring C is $C_2$.

In some embodiments of the present disclosure, the above R is selected from the group consisting of $CH_3$, $CH_2CH_3$, $CF_3$, $OCH_3$, and $OCF_3$, with the other variables as defined in the present disclosure.

In some embodiments of the present disclosure, the above $R_a$ is $-S(=O)_2-CH_2CH_3$, with other variables as defined in the present disclosure.

In some embodiments of the present disclosure, each of the above $R_b$ is independently selected from the group consisting of F, Cl, Br, I, $CH_3$, $-C(=O)-CH_2CH_3$, and $-NHC(=O)-CH_2CH_3$, with other variables as defined in the present disclosure.

In some embodiments of the present disclosure, the above $R_c$ and $R_d$ together with the carbon atom to which they are attached form

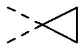 and 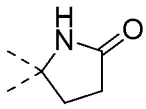 ,

with other variables as defined in the present disclosure.

In some embodiments of the present disclosure, each of the above $R_e$ is selected from the group consisting of $-NHC(=O)-CH_2CH_3$, $=NCH_3$, and $=N-OCH_3$, with other variables as defined in the present disclosure.

In some embodiments of the present disclosure, each of the above $R_e$ is independently selected from the group consisting of F, Cl, Br, I, $CH_3$, $CH_2CH_3$, $OCH_3$, $OCH_2CH_3$, $NCH_3$, $NCH_2CH_3$, $-NHC(=O)-CH_3$, $-NHC(=O)-CH_2CH_3$, $-NHC(=O)-OCH_3$, and $-NHC(=O)-NCH_3$, with other variables as defined in the present disclosure.

In some embodiments of the present disclosure, the above $R_f$ is selected from the group consisting of $=NCH_3$, $=N-OCH_3$,

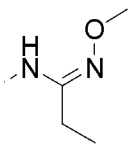 , and 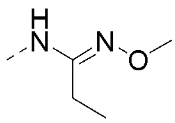 ,

with other variables as defined in the present disclosure.

In some embodiments of the present disclosure, the above $R_1$ is $CH_3$, with other variables as defined in the present disclosure.

In some embodiments of the present disclosure, each of the above $R_1$ is independently selected from the group consisting of F, Cl, Br, I, $CH_3$, $OCH_3$, and $=N-OCH_3$, with other variables as defined in the present disclosure.

In some embodiments of the present disclosure, each of the above $R_2$, $R_3$, $R_4$, and $R_5$ is independently selected from the group consisting of H, F, Cl, Br, $CH_3$, $CHF_2$, $CF_3$, $CH_2CH_3$, $OCH_3$, and $OCH_2CH_3$, wherein not all four of $R_2$, $R_3$, $R_4$, and $R_5$ are simultaneously H, with other variables as defined in the present disclosure.

In some embodiments of the present disclosure, the above $R_2$ is selected from the group consisting of H and F, with other variables as defined in the present disclosure.

In some embodiments of the present disclosure, the above $R_5$ is selected from the group consisting of H, F, Cl, Br, $CH_3$, $CHF_2$, $CF_3$, $CH_2CH_3$, and $OCH_3$, with other variables as defined in the present disclosure.

In some embodiments of the present disclosure, each of the above $R_6$ is independently F, with other variables as defined in the present disclosure.

In some embodiments of the present disclosure, each of the above $R_6$ is independently selected from the group consisting of F, Cl, Br, I, and $CH_3$, with other variables as defined in the present disclosure.

In some embodiments of the present disclosure, the above $R_{71}$ is

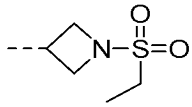,

with other variables as defined in the present disclosure.

In some embodiments of the present disclosure, the above $R_{81}$ is H, with other variables as defined in the present disclosure.

In some embodiments of the present disclosure, the above $R_9$ is selected from the group consisting of -NHC(=O)-$CH_2CH_3$, -NH-C(=O)-pyridyl, -NH-C(=O)-isoxazolyl, -NH-piperidinyl, -NH-pyrrolidinyl, -Nh-azetidinyl, piperidinyl, and

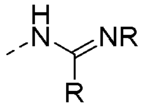,

wherein the - NHC(=O)-$CH_2CH_3$, -NH-C(=O)-pyridyl, -NH-C(=O)-isoxazolyl, -NH-piperidinyl, -NH-pyrrolidinyl, -NH-azetidinyl, and piperidinyl are each independently and optionally substituted by 1, 2, or 3 $R_b$, with other variables as defined in the present disclosure.

In some embodiments of the present disclosure, the above $R_9$ is selected from the group consisting of

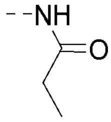, 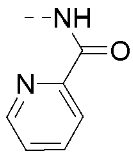, 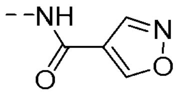, 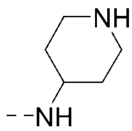, 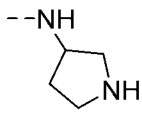,

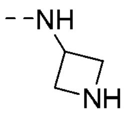, 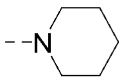, 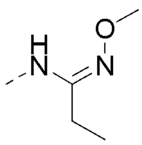, and 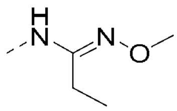, wherein the 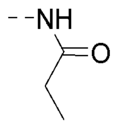, 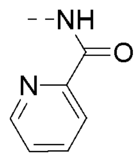,

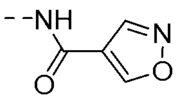, 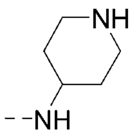, 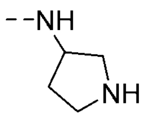, 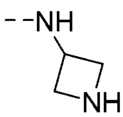, and 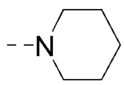

are each independently and optionally substituted by 1, 2, or 3 $R_b$, with other variables as defined in the present disclosure.

In some embodiments of the present disclosure, the above $R_9$ is selected from the group consisting of

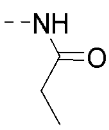, 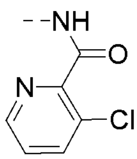, 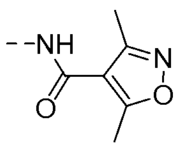, 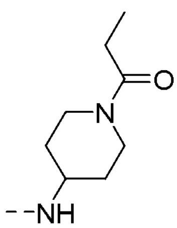, 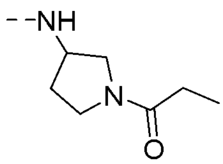,

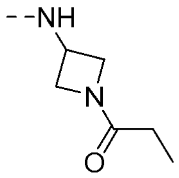, 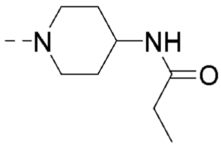, 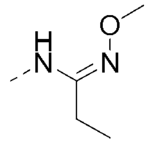, and 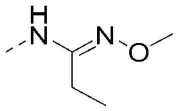,

with other variables as defined in the present disclosure.

In some embodiments of the present disclosure, the above ring $B_1$ is selected from the group consisting of

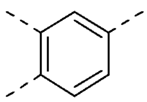, 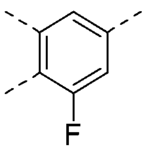, 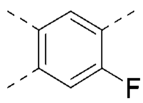, 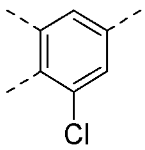, 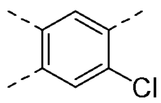,

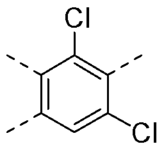, 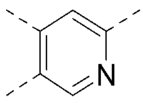, and 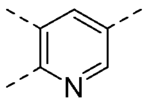,

with other variables as defined in the present disclosure.

In some embodiments of the present disclosure, the above ring $B_1$ is selected from the group consisting of

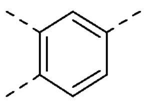, 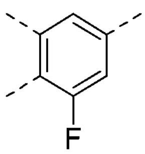, 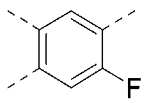, and 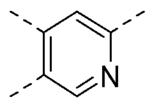,

with other variables as defined in the present disclosure.

In some embodiments of the present disclosure, the above ring $B_1$ is phenyl, with other variables as defined in the present disclosure.

In some embodiments of the present disclosure, the above ring $B_2$ is selected from the group consisting of thienyl, thiazolyl, imidazolyl, and pyrazolyl, wherein the thienyl, thiazolyl, imidazolyl, and pyrazolyl are each independently and optionally substituted by 1, 2, or 3 $R_6$, with other variables as defined in the present disclosure.

In some embodiments of the present disclosure, the above ring $B_2$ is selected from the group consisting of

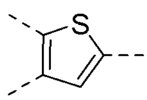, 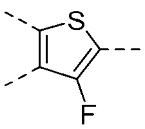, 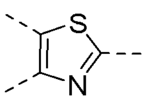, 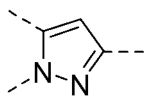, and 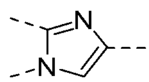,

wherein the

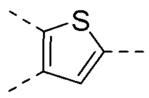, 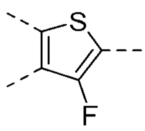, 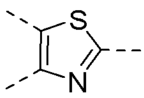, 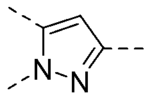, and 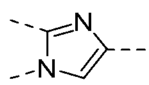

are each independently and optionally substituted by 1, 2, or 3 $R_6$, with other variables as defined in the present disclosure.

In some embodiments of the present disclosure, the above ring $B_2$ is selected from the group consisting of

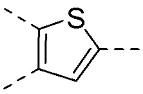, 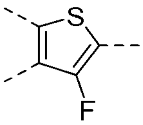, 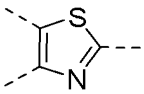, 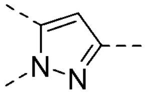, and 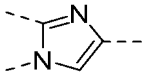,

with other variables as defined in the present disclosure.

In some embodiments of the present disclosure, the above ring $B_2$ is selected from the group consisting of

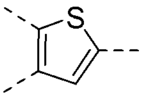, 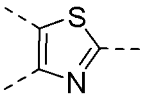, 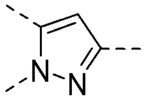, and 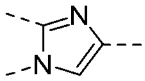,

with other variables as defined in the present disclosure. In some embodiments of the present disclosure, the above ring $A_2$ is selected from the group consisting of

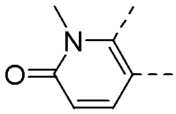, 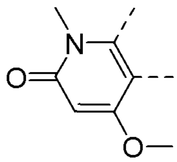,

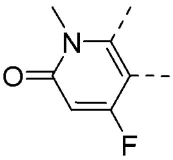, 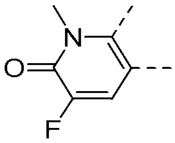, 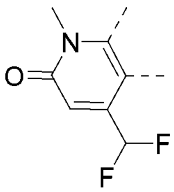, 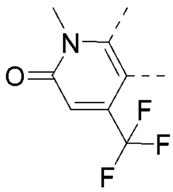, 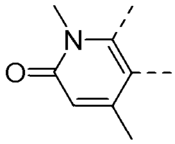, 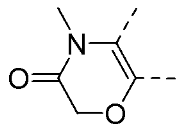,

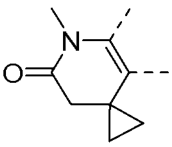, 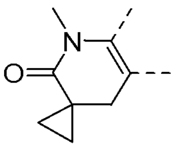, 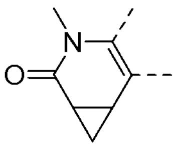, 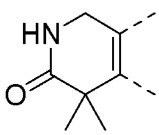, 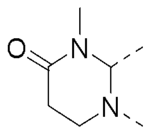, 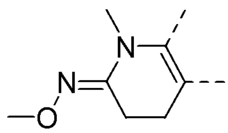,

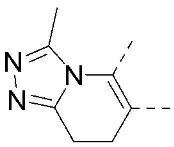, 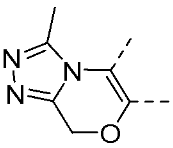, 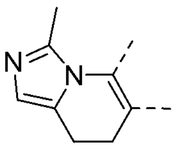, 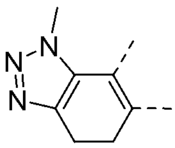, 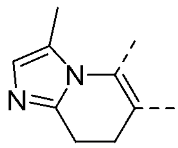,

and

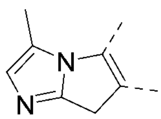,

with other variables as defined in the present disclosure.

In some embodiments of the present disclosure, the above ring $A_2$ is selected from the group consisting of

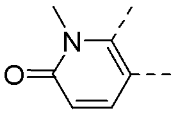, 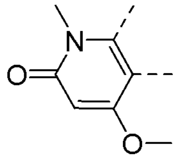, 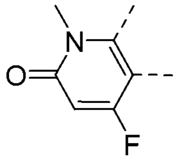, 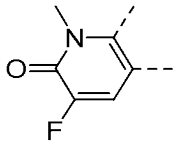,

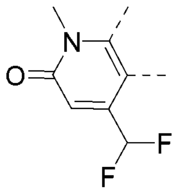, 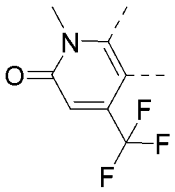, 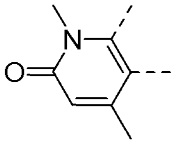, 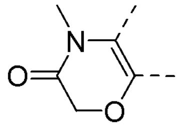, 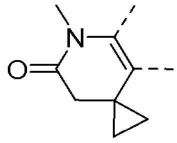, 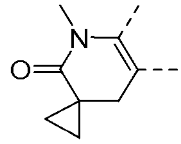,

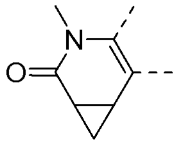, 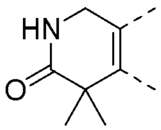, 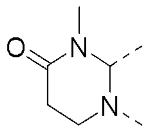, 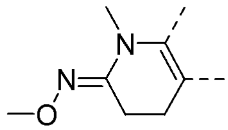, 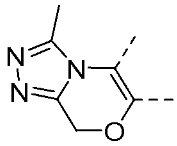, 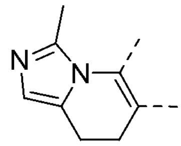,

and

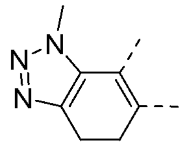,

with other variables as defined in the present disclosure.

In some embodiments of the present disclosure, the above ring $A_2$ is selected from the group consisting of

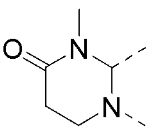, 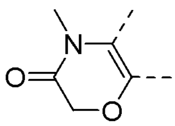, 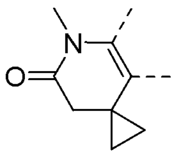, 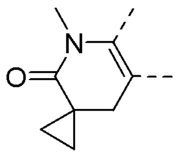,

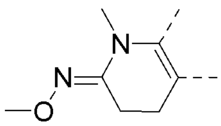, 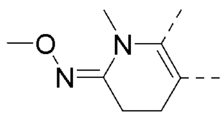, 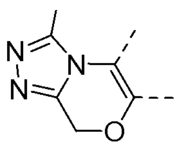, 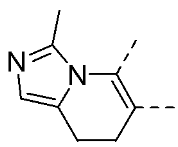, 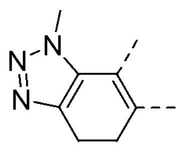,

and

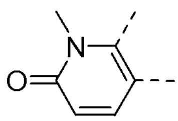,

with other variables as defined in the present disclosure.

In some embodiments of the present disclosure, the above ring $C_1$ is selected from the group consisting of

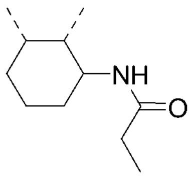, 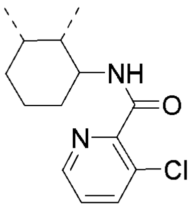, 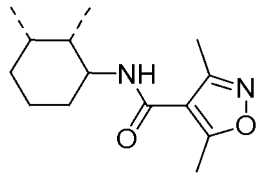, 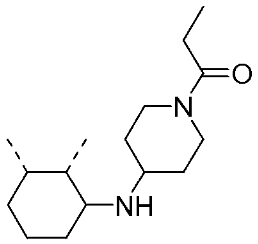, 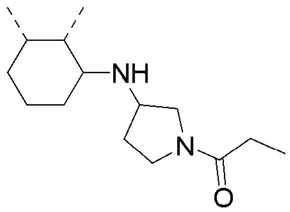, 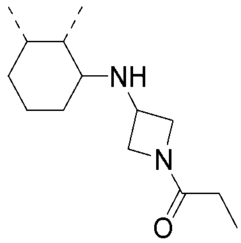, 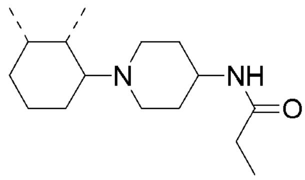, 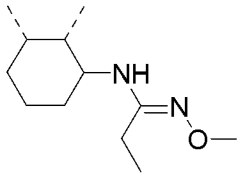, 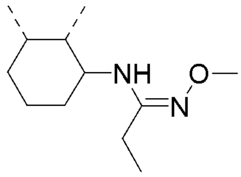, and 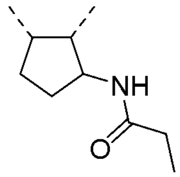,

with other variables as defined in the present disclosure.

In some embodiments of the present disclosure, the above ring $C_1$ is selected from the group consisting of

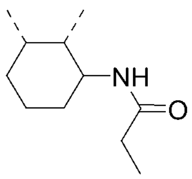, 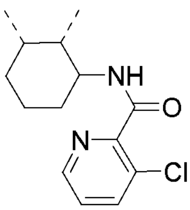, 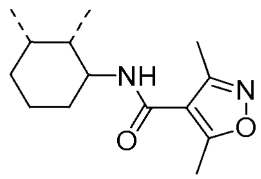, 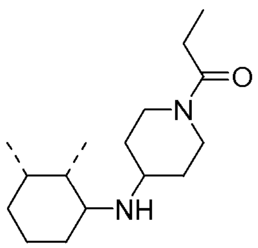, 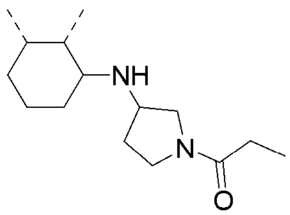, 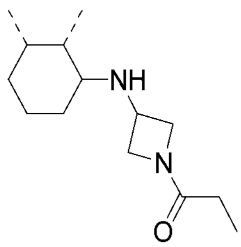, 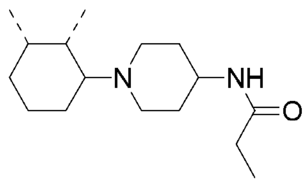, 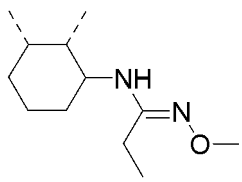, and 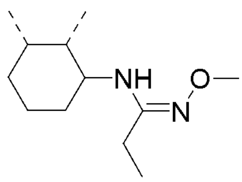,

with other variables as defined in the present disclosure.

In some embodiments of the present disclosure, the above ring $C_1$ is

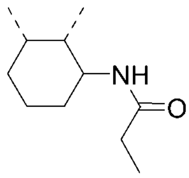,

with other variables as defined in the present disclosure.

In some embodiments of the present disclosure, the above ring $C_1$ is

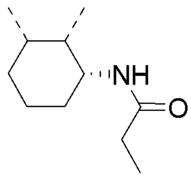,

with other variables as defined in the present disclosure.

In some embodiments of the present disclosure, the above ring $C_1$ is

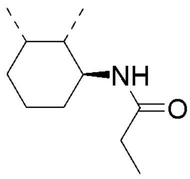,

with other variables as defined in the present disclosure.

In some embodiments of the present disclosure, the above ring $C_2$ is selected from the group consisting of

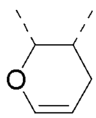, 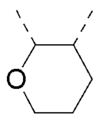, 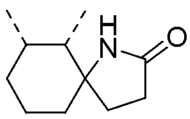, 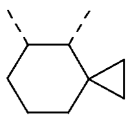, 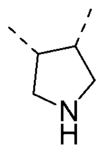, and 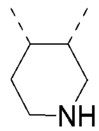,

wherein the

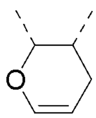, 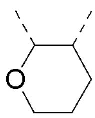, 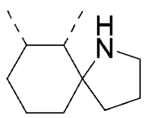, 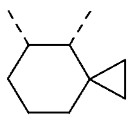, 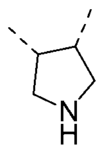, and 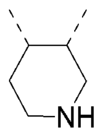

are each independently and optionally substituted by 1, 2, or 3 $R_e$, with other variables as defined in the present disclosure.

In some embodiments of the present disclosure, in the compound of formula (I) described above, the ring $C_2$ is selected from the group consisting of

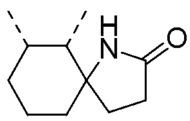, 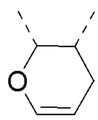,

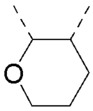, 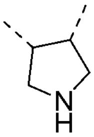, 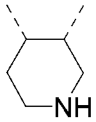, 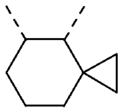, 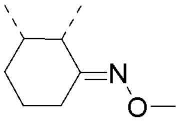, 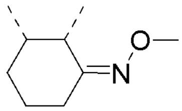, 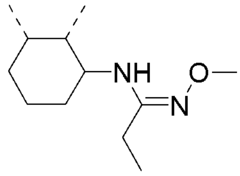,

and

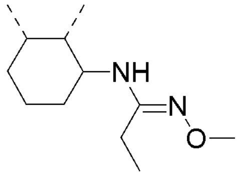,

wherein the

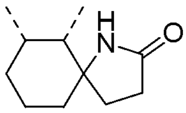, 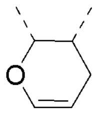, 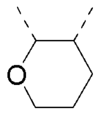, 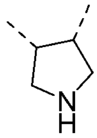, 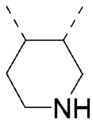,

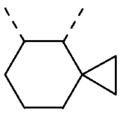, 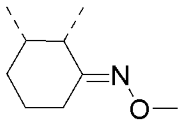, 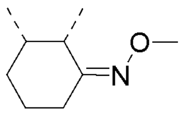, 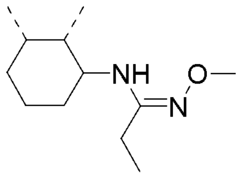, and 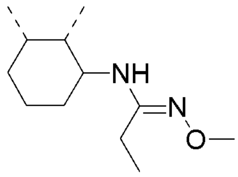 are

each independently and optionally substituted by 1, 2, or 3 $R_g$, with other variables as defined in the present disclosure.

In some embodiments of the present disclosure, the above ring $C_2$ is selected from the group consisting of

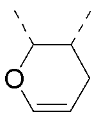, 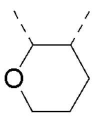, 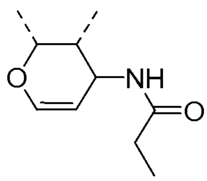, 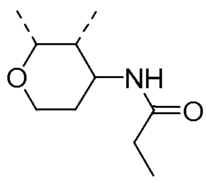, 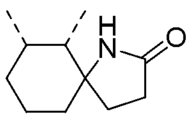,

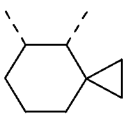, 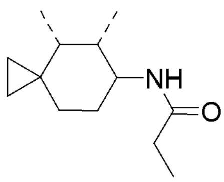, 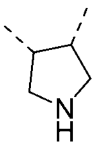, 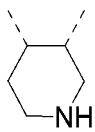, 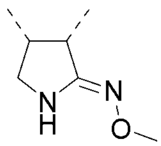, 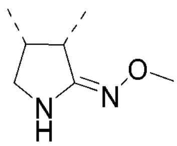,

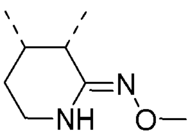, and 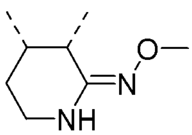,

with other variables as defined in the present disclosure.

In some embodiments of the present disclosure, in the compound of formula (I) described above, the ring $C_2$ is selected from the group consisting of

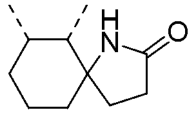, 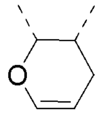,

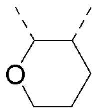, 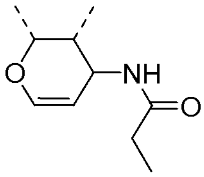, 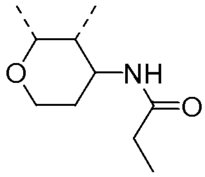, 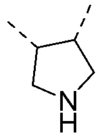, 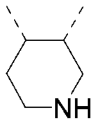, 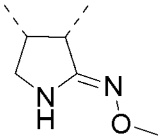, 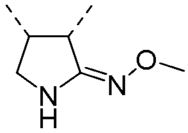,

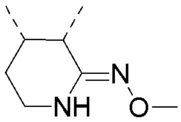, 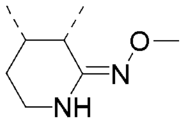, 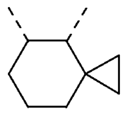, 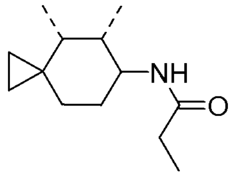, 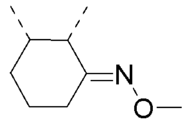, 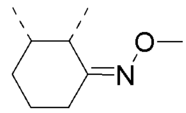,

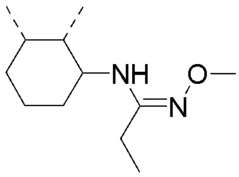, and 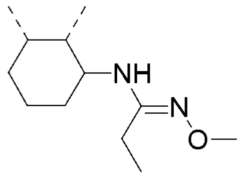,

with other variables as defined in the present disclosure.

In some embodiments of the present disclosure, in the compound of formula (I) described above, the ring $C_2$ is selected from the group consisting of

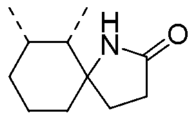, 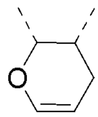,

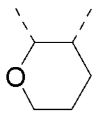, 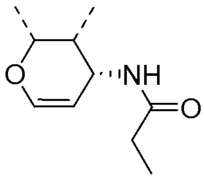, 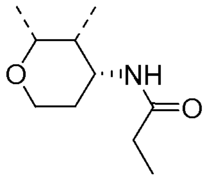, 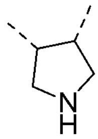, 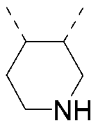, 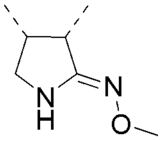, 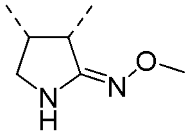,

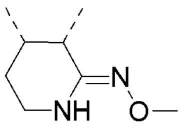, 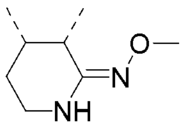, 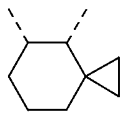, 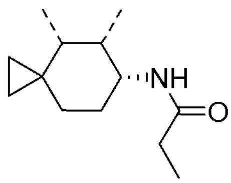, 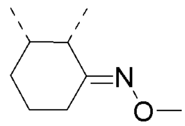, 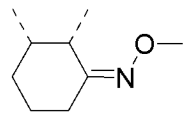,

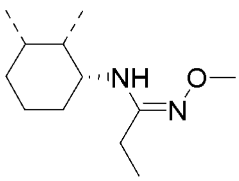, and 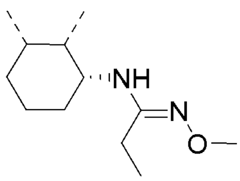,

with other variables as defined in the present disclosure.

In some embodiments of the present disclosure, the structural moiety

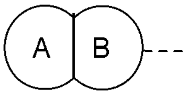

is selected from the group consisting of

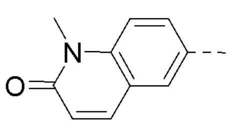 , 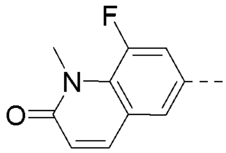 ,

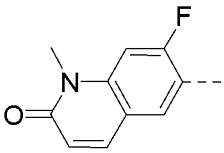 , 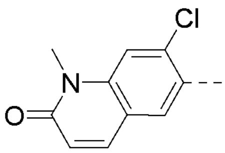 , 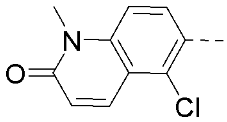 , 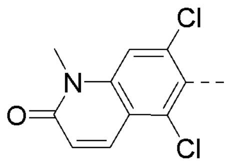 ,

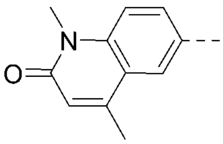 , 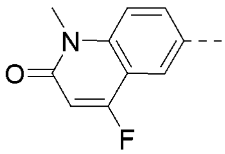 , 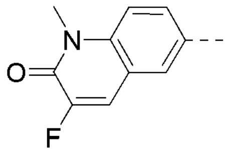 , 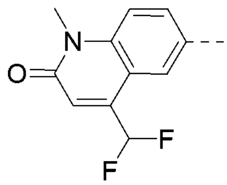 ,

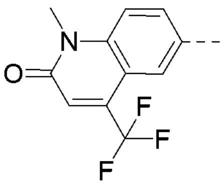 , 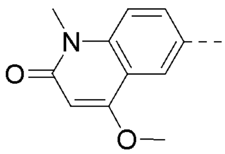 , 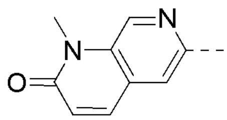 , 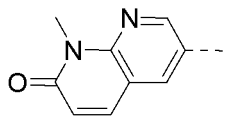 ,

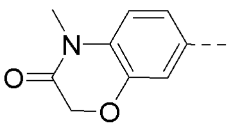 , 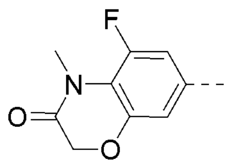 , 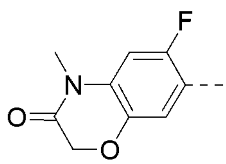 , 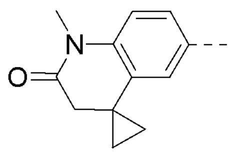 ,

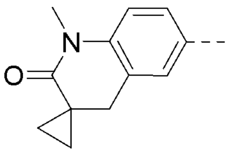 , 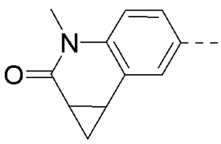 , 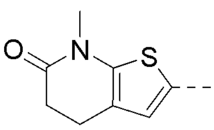 , 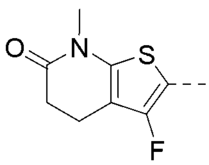 , 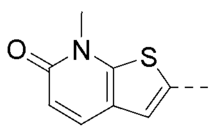 ,

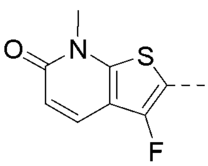 , 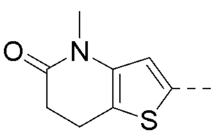 , 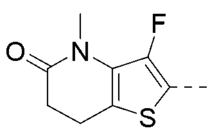 , 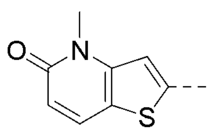 , 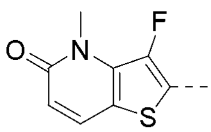 ,

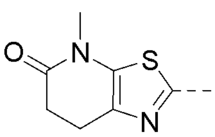, 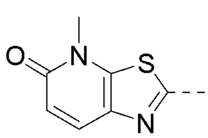, 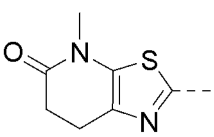, 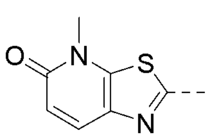, 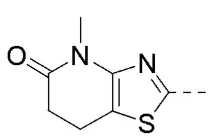,

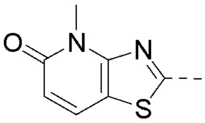, 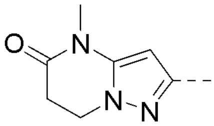, 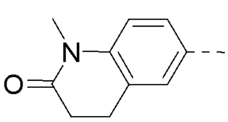, 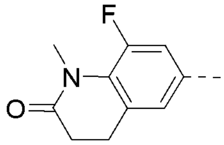, 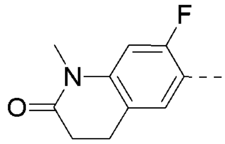,

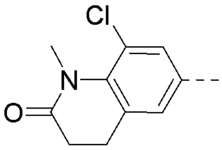, 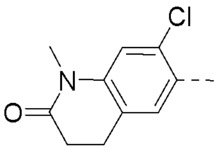, 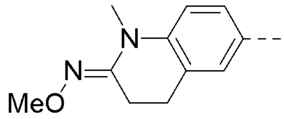, 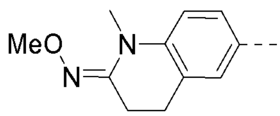,

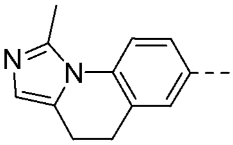, 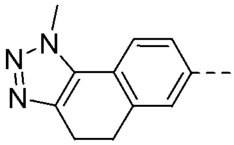, 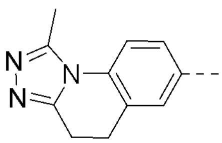, 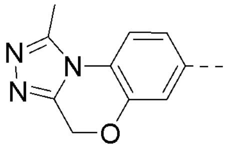,

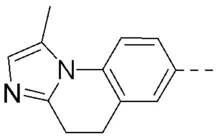, 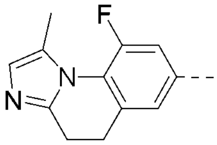, and 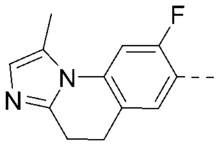,

with other variables as defined in the present disclosure.

In some embodiments of the present disclosure, the structural moiety

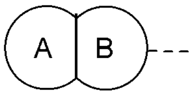

is selected from

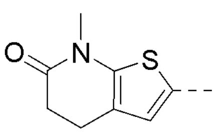, 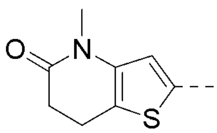, 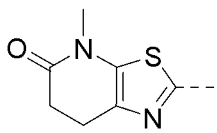, 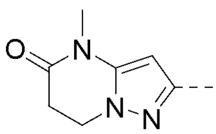,

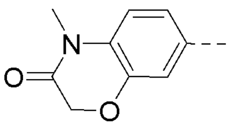, 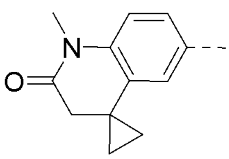, 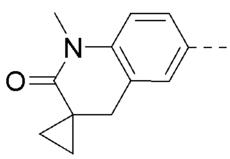, 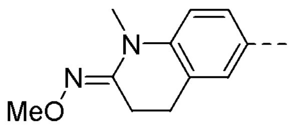,

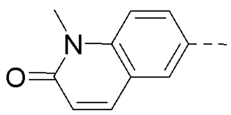, 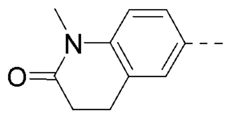, 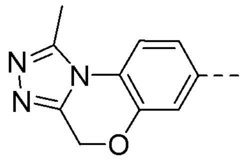, 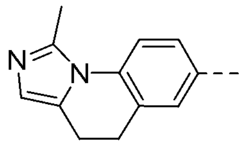,

and

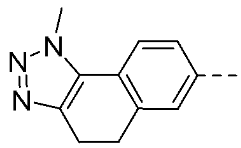,

with other variables as defined in the present disclosure.

In some embodiments of the present disclosure, the above compound, a stereoisomer, or a pharmaceutically acceptable salt thereof is provided, wherein the compound is selected from the group consisting of

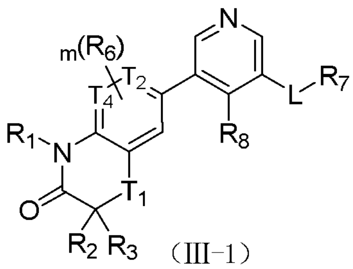
(III-1), 

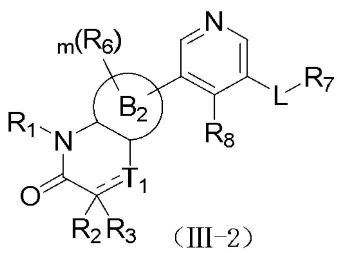
(III-2),

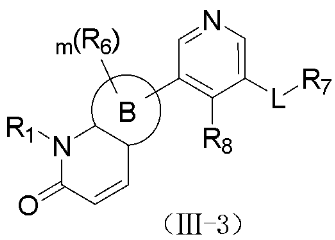
(III-3), and

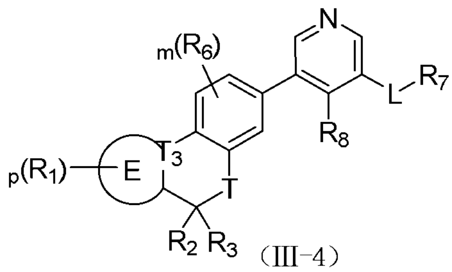
(III-4),

wherein

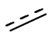 is a single bond or a double bond;
$T_2$ is selected from the group consisting of CH and N;
$T_4$ is selected from the group consisting of CH and N;
$T_3$ is selected from the group consisting of C and N;
ring E is selected from the group consisting of

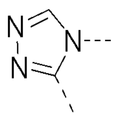, 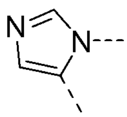, 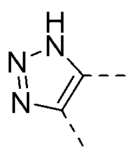,

and

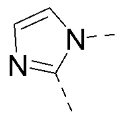

;

m is selected from the group consisting of 0, 1, 2, and 3;
p is selected from the group consisting of 0, 1, and 2;
ring B is selected from the group consisting of ring $B_1$ and ring $B_2$;
ring $B_1$ is selected from the group consisting of phenyl, pyridyl, and pyridazinyl;
ring $B_2$ is 5-membered heteroaryl;
T, $T_1$, $R_1$, $R_2$, $R_3$, $R_6$, L, $R_7$, and $R_8$ are as defined in the present disclosure.

In some embodiments of the present disclosure, the above compound, a stereoisomer, or a pharmaceutically acceptable salt thereof is provided, wherein the compound is

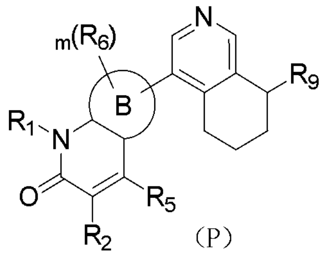

(P)

,

wherein

ring B is selected from the group consisting of phenyl, pyridyl, pyridazinyl, and 5-membered heteroaryl;
$R_1$ is selected from the group consisting of H, $C_{1-3}$ alkyl, and $C_{1-3}$ haloalkyl;
each $R_2$ and $R_5$ is independently selected from the group consisting of H, F, Cl, Br, I, $C_{1-3}$ alkyl, $C_{1-3}$ alkoxy, $C_{1-3}$ haloalkyl, and $C_{1-3}$ haloalkoxy;
each $R_6$ is independently selected from the group consisting of H, F, Cl, Br, I, and $C_{1-3}$ alkyl;
$R_9$ is selected from the group consisting of -NHC(=O)-$C_{1-3}$ alkyl, -NH-C(=O)-5- to 6-membered heteroaryl, -NH-4- to 6- membered heterocycloalkyl, 4- to 6- membered heterocycloalkyl, and

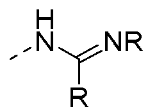

,

wherein the -NHC(=O)-$C_{1-3}$ alkyl, -NH-C(=O)-5- to 6-membered heteroaryl, -NH-4- to 6- membered heterocycloalkyl, and 4- to 6- membered heterocycloalkyl are each independently and optionally substituted by 1, 2, or 3 $R_b$;
each $R_b$ is independently selected from the group consisting of F, Cl, Br, I, $C_{1-3}$ alkyl, -C(=O)-$C_{1-3}$ alkyl, and -NH-C(=O)-$C_{1-3}$ alkyl;
R is selected from the group consisting of $C_{1-3}$ alkyl and $C_{1-3}$ alkoxy, wherein the $C_{1-3}$ alkyl and $C_{1-3}$ alkoxy are each independently and optionally substituted by 1, 2, or 3 halogens;
m is selected from the group consisting of 0, 1, 2, and 3;

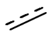 is a single bond or a double bond;
the term "hetero" in the 5-membered heteroaryl, 5- to 6-membered heteroaryl, and 4-to 6-membered heterocycloalkyl represents 1, 2, 3, or 4 heteroatoms or heteroatomic groups, each independently selected from the group consisting of O, S, and N.

In some embodiments of the present disclosure, the above compound, a stereoisomer, or a pharmaceutically acceptable salt thereof is provided, wherein the compound is selected from the group consisting of

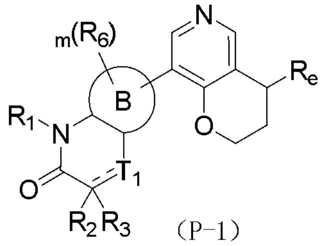
(P-1) ,

wherein

ring B is selected from the group consisting of phenyl, pyridinyl, pyridazinyl, and 5-membered heteroaryl;
$T_1$ is selected from the group consisting of O and $CR_4R_5$;
$R_1$ is selected from the group consisting of H, $C_{1-3}$ alkyl, and $C_{1-3}$ haloalkyl;
each $R_2$, $R_3$, $R_4$, and $R_5$ is independently selected from the group consisting of H, F, Cl, Br, I, $C_{1-3}$ alkyl, $C_{1-3}$ alkoxy, $C_{1-3}$ haloalkyl, and $C_{1-3}$ haloalkoxy;
alternatively, $R_2$ and $R_3$ together with the carbon atom to which they are attached form a cyclopropyl;
alternatively, $R_4$ and $R_5$ together with the carbon atom to which they are attached form a cyclopropyl;
alternatively, $R_3$ and $R_4$ together with the carbon atom to which they are attached form a double bond or a cyclopropyl;
each $R_6$ is independently selected from the group consisting of H, F, Cl, Br, I, and $C_{1-3}$ alkyl;
each $R_e$ is independently selected from the group consisting of F, Cl, Br, I, =O, $C_{1-3}$ alkyl, $C_{1-3}$ alkoxy, $C_{1-3}$ alkylamino, -NHC(=O)-$C_{1-3}$ alkyl, -NHC(=O)-$C_{1-3}$ alkoxy, -NHC(=O)-$C_{1-3}$ alkylamino, and =NR;
R is selected from the group consisting of $C_{1-3}$ alkyl and $C_{1-3}$ alkoxy, wherein the $C_{1-3}$ alkyl and $C_{1-3}$ alkoxy are each independently and optionally substituted by 1, 2, or 3 halogens;
m is selected from the group consisting of 0, 1, 2, and 3;
is a single bond or a double bond;
the term "hetero" in the 5-membered heteroaryl represents 1, 2, 3, or 4 heteroatoms or heteroatomic groups, each independently selected from the group consisting of O, S, and N.

In some embodiments of the present disclosure, in the above formula (P-1), when is a double bond and $T_1$ is O, then $R_3$ and $R_4$ are absent, with other variables as defined in the present disclosure.

In some embodiments of the present disclosure, in the above formula (P-1), the structural moiety

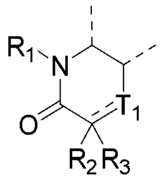

is selected from the group consisting of

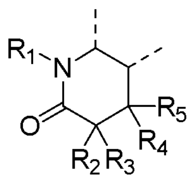
,

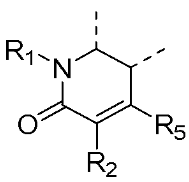
, and
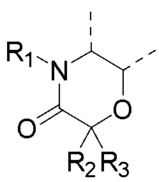
,

with other variables as defined in the present disclosure.

In some embodiments of the present disclosure, in the above formula (P-1), the structural moiety

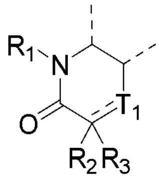

is selected from the group consisting of

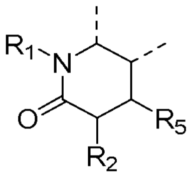

,

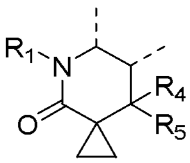

,

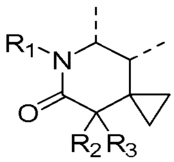

,

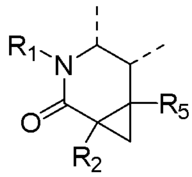

, and

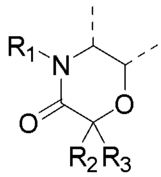

,

with other variables as defined in the present disclosure.

In some embodiments of the present disclosure, in the above formula (III-2), 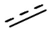 is a double bond and $R_3$ and $R_4$ are absent, with other variables as defined in the present disclosure.

In some embodiments of the present disclosure, in the above formula (III-2), the structural moiety

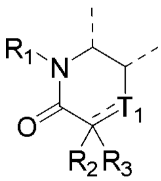

is selected from the group consisting of

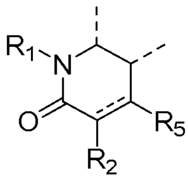

and

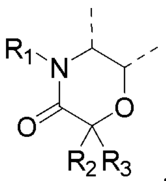

,

with other variables as defined in the present disclosure.

In some embodiments of the present disclosure, in the above formula (III-2), the structural moiety

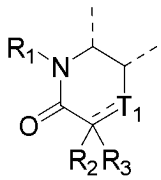

is selected from the group consisting of

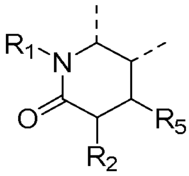

,

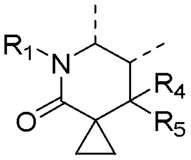

, 

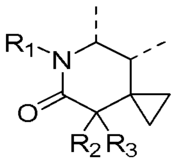

, 

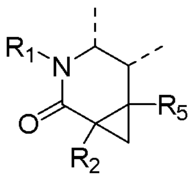

, 

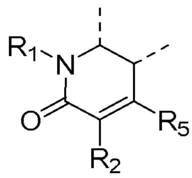

, and 

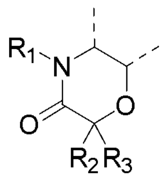

,

with other variables as defined in the present disclosure.

In some embodiments of the present disclosure, in the above formula (III-2), the structural moiety

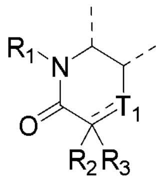

is selected from the group consisting of

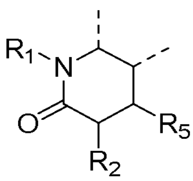

,

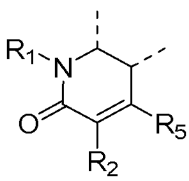

, and 

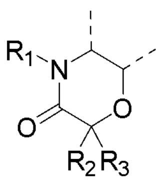

,

with other variables as defined in the present disclosure.

In some embodiments of the present disclosure, the above compound, a stereoisomer, or a pharmaceutically acceptable salt thereof is provided, wherein the compound is selected from the group consisting of

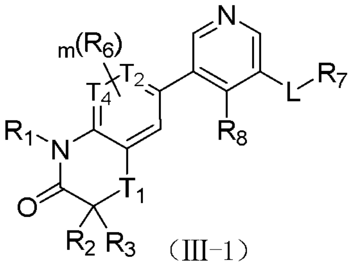

(III-1) ,

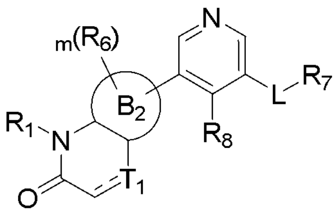

(III'-2) ,

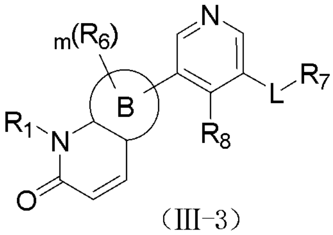

(III-3) , and

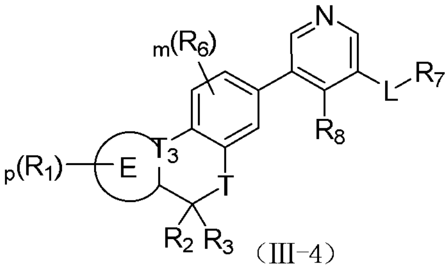

(III-4) ,

wherein

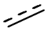 is a single bond or a double bond;
$T_2$ is selected from the group consisting of CH and N;
$T_4$ is selected from the group consisting of CH and N;
$T_3$ is selected from the group consisting of C and N;
ring E is selected from the group consisting of

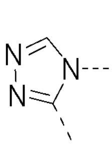 , 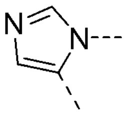 , 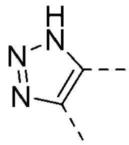 ,

and

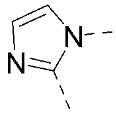 ;

m is selected from the group consisting of 0, 1, 2, and 3;
p is selected from the group consisting of 0, 1, and 2;
ring B
is selected from the group consisting of ring $B_1$ and ring $B_2$;
ring $B_1$ is selected from the group consisting of phenyl, pyridyl, and pyridazinyl;
ring $B_2$ is 5-membered heteroaryl;
T, $T_1$, $R_1$, $R_2$, $R_3$, $R_6$, L, $R_7$, and $R_8$ are as defined in the present disclosure.

In some embodiments of the present disclosure, in the above formula (III'-2), 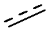 is a double bond and $T_1$ is $CR_5$, with other variables as defined in the present disclosure.

In some embodiments of the present disclosure, the above compound, a stereoisomer, or a pharmaceutically acceptable salt thereof is provided, wherein the compound is selected from the group consisting of

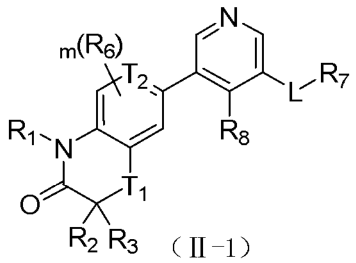
(II-1), 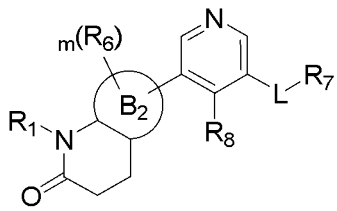
(II-2), and 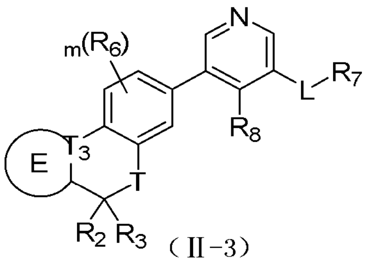
(II-3),

$T_2$ is selected from the group consisting of CH and N;
$T_3$ is selected from the group consisting of C and N;
ring E is selected from the group consisting of

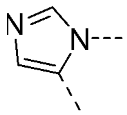
and 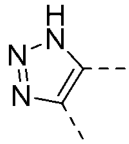
;

m is selected from the group consisting of 0, 1, 2, and 3;
ring $B_2$ is 5-membered heteroaryl;
T, $T_1$, $R_1$, $R_2$, $R_3$, $R_6$, L, $R_7$, and $R_8$ are as defined in the present disclosure.

In some embodiments of the present disclosure, the above compound, a stereoisomer, or a pharmaceutically acceptable salt thereof is provided, wherein the compound is selected from the group consisting of

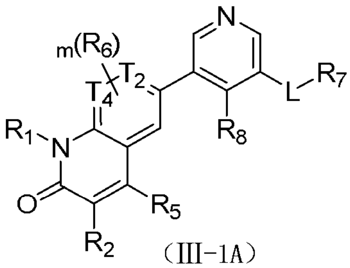
(III-1A), 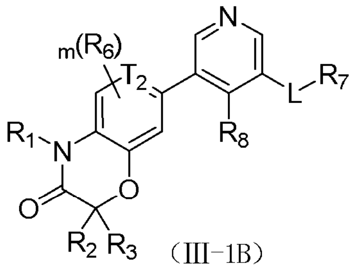
(III-1B), 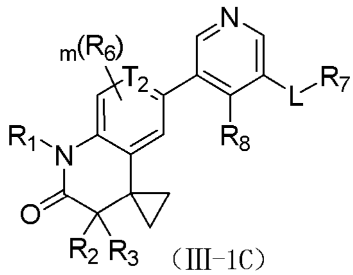
(III-1C),

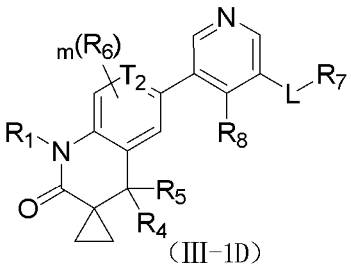
(III-1D), 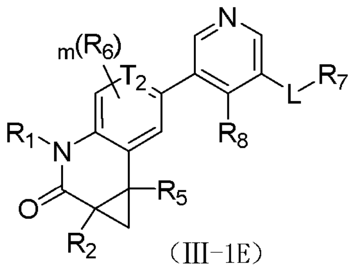
(III-1E), 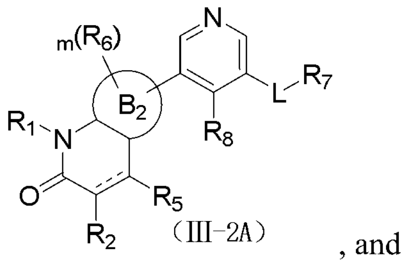
(III-2A), and

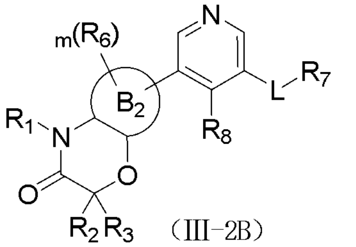
(III-2B),

wherein

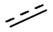 is a single bond or a double bond;
$T_2$ is selected from the group consisting of CH and N;
$T_4$ is selected from the group consisting of CH and N;
ring $B_2$ is 5-membered heteroaryl;
$R_1$, $R_2$, $R_3$, $R_4$, $R_5$, $R_6$, L, $R_7$, $R_8$, and m are as defined in the present disclosure.

In some embodiments of the present disclosure, the above compound, a stereoisomer, or a pharmaceutically acceptable salt thereof is provided, wherein the compound is selected from the group consisting of

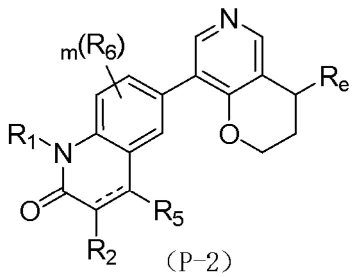
(P-2) ,

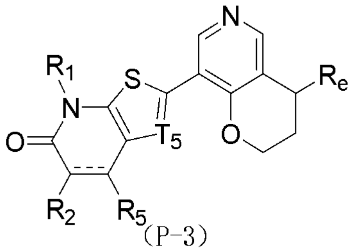
(P-3) , and

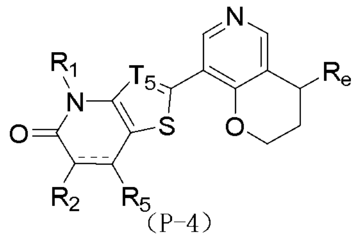
(P-4) ,

wherein

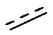 is a single bond or a double bond;
$T_3$ is selected from the group consisting of $CR_{61}$ and N;
$R_{61}$ is selected from the group consisting of H, F, Cl, Br, I, and $C_{1-3}$ alkyl;
$R_1$, $R_2$, $R_5$, $R_6$, $R_e$, and m are as defined in the present disclosure.

In some embodiments of the present disclosure, the above compound, a stereoisomer, or a pharmaceutically acceptable salt thereof is provided, wherein the compound is selected from the group consisting of

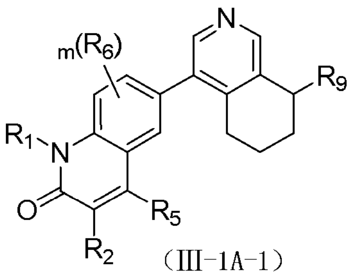
(III-1A-1) ,

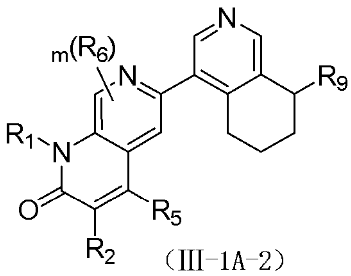
(III-1A-2) ,

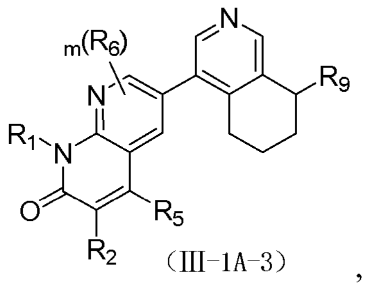
(III-1A-3) ,

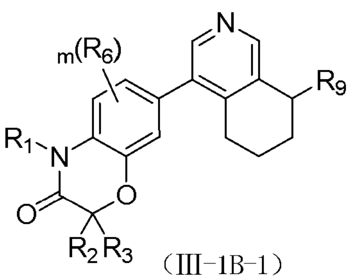
(III-1B-1) ,

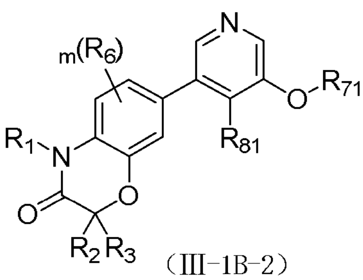
(III-1B-2) ,

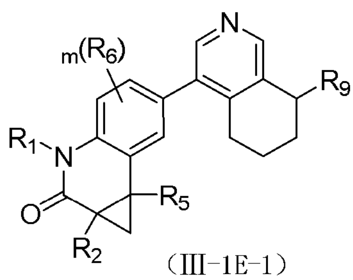
(III-1E-1) ,

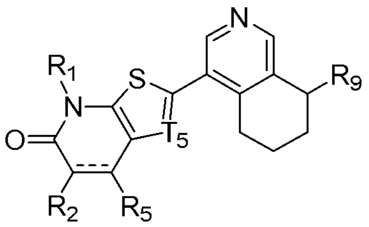

(III-2A-1)

, and

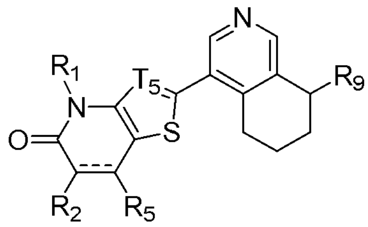

(III-2A-2)

,

wherein

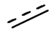 is a single bond or a double bond;

$T_5$ is selected from the group consisting of $CR_{61}$ and N;

$R_{61}$ is selected from the group consisting of H, F, Cl, Br, I, and $C_{1-3}$ alkyl;

$R_1$, $R_2$, $R_3$, $R_5$, $R_6$, $R_9$, $R_{71}$, $R_{81}$, and m are as defined in the present disclosure.

The present disclosure further includes embodiments obtained by any combination of the above variables.

The present disclosure further provides a compound of one of the following formulas, a stereoisomer thereof, or a pharmaceutically acceptable salt thereof,

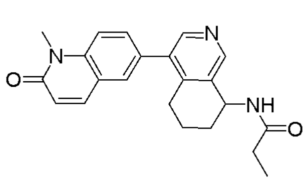 , 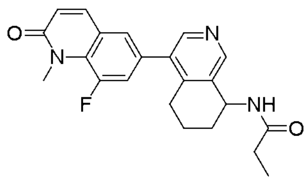 , 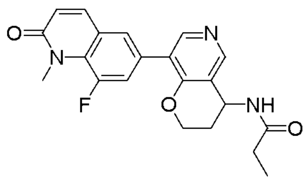 ,

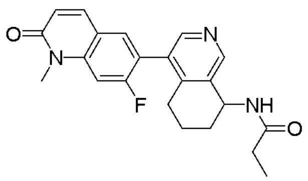 , 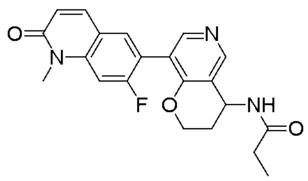 , 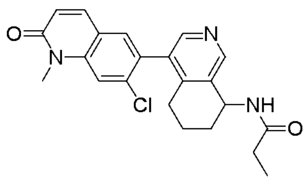 ,

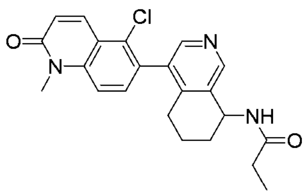 , 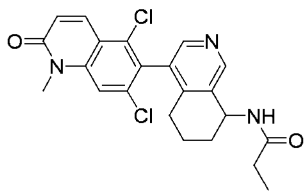 , 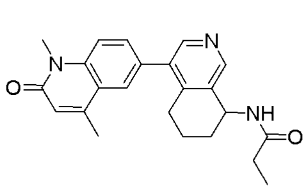 ,

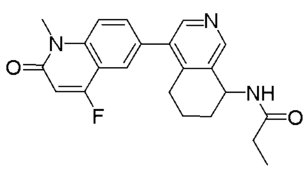 , 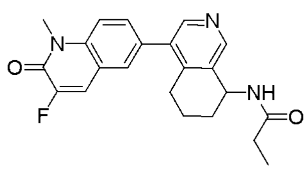 , 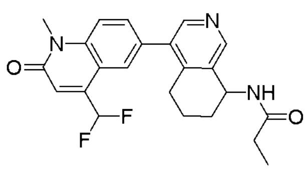 ,

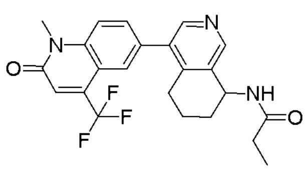 , 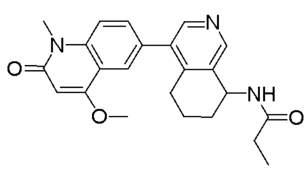 , 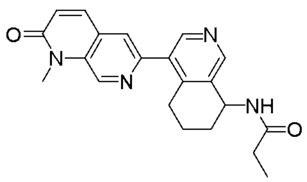 ,

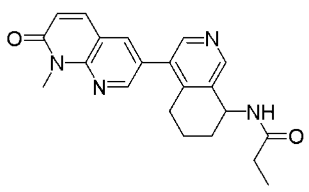 , 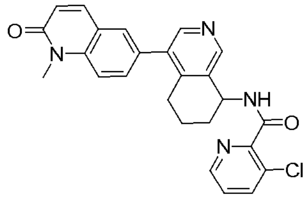 , 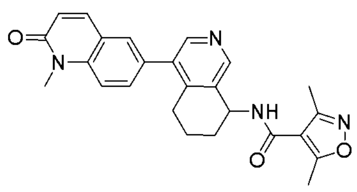 ,

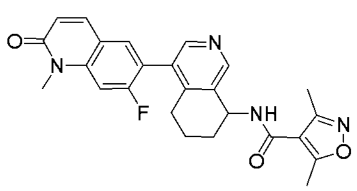 , 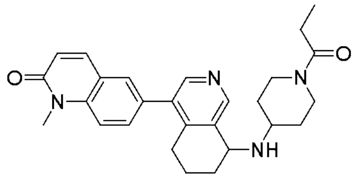 , 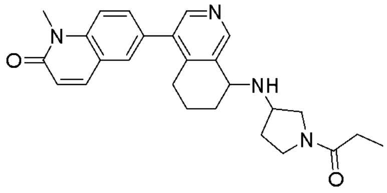 ,

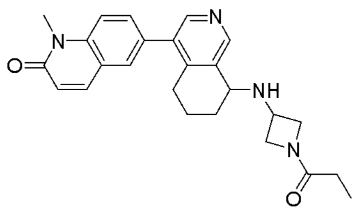 , 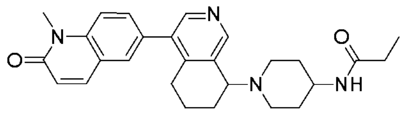 , 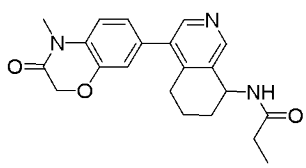 ,

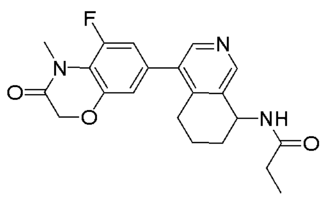 , 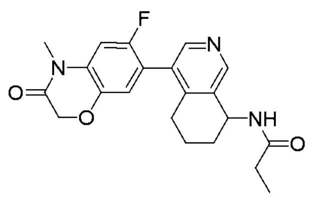 , 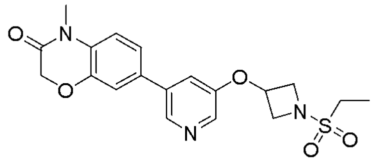 ,

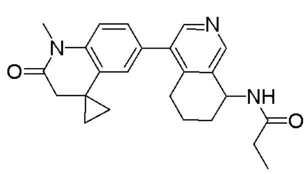 , 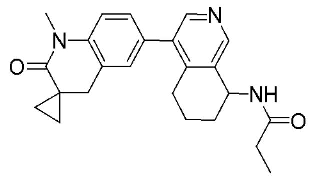 , 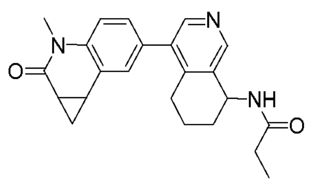 ,

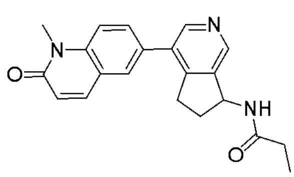 , 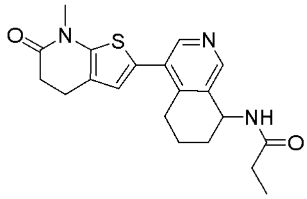 , 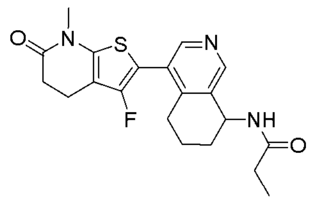 ,

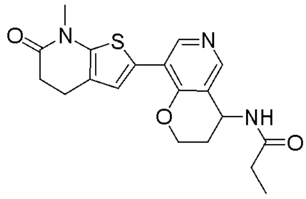 , 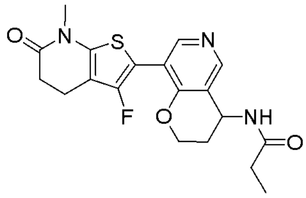 , 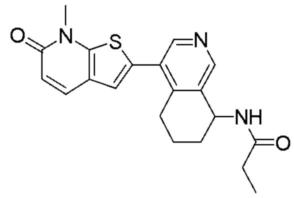 ,

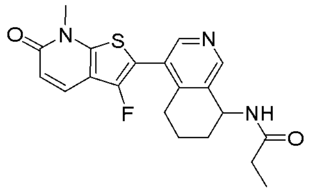,
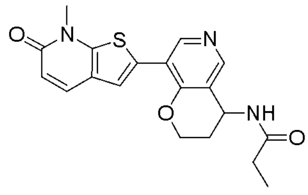,
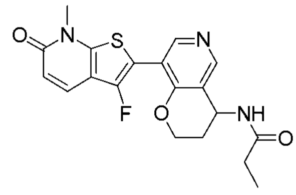,

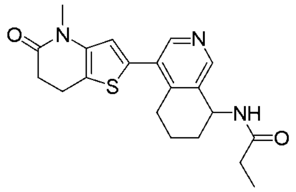,
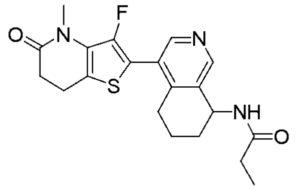,
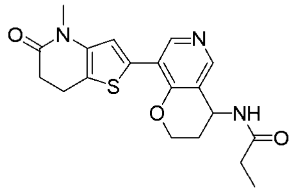,

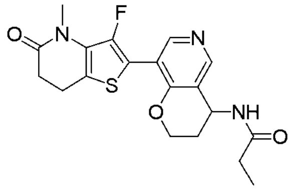,
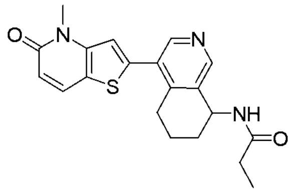,
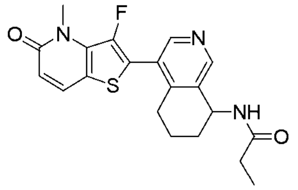,

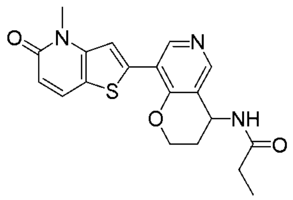,
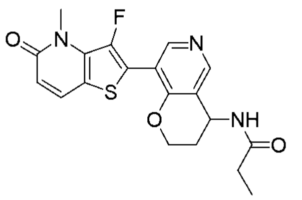,
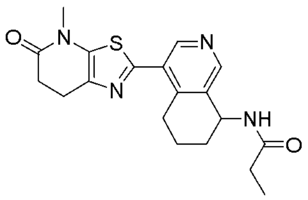,

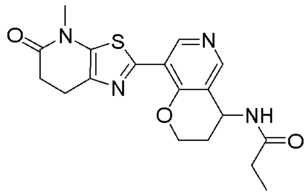,
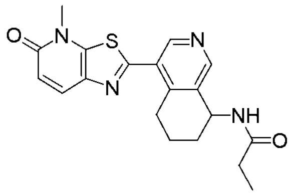,
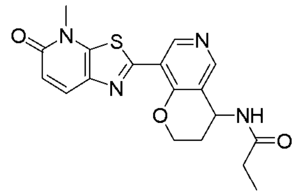,

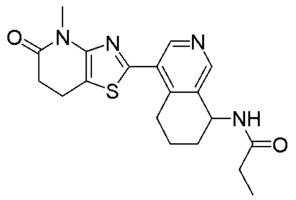,
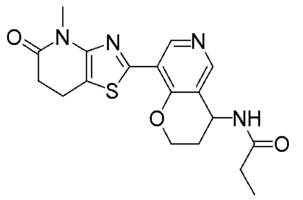,
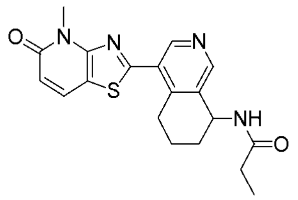,

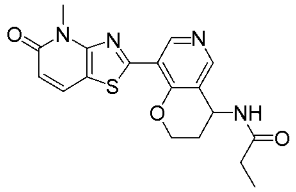,
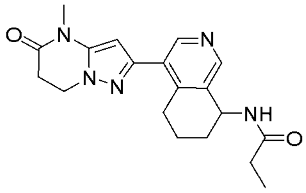,
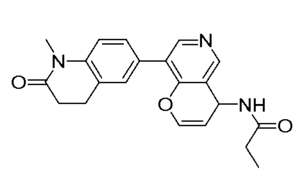,

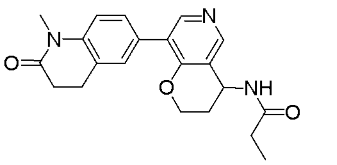,
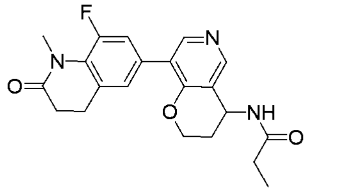,
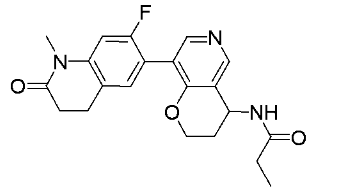,
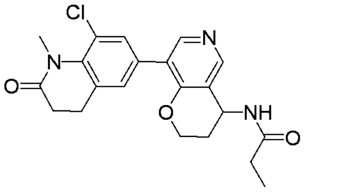,
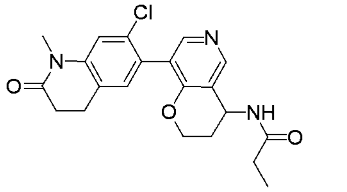,
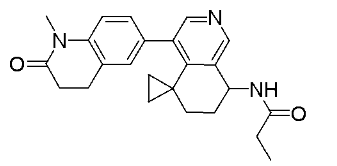,
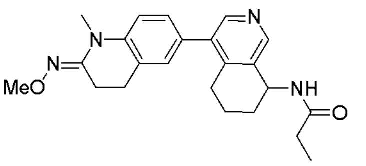,
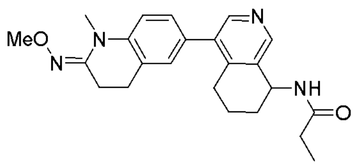,
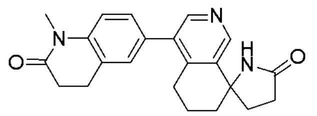,
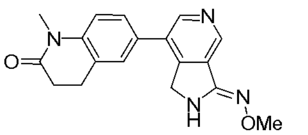,
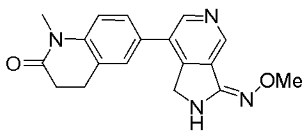,
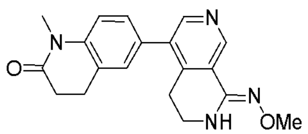,
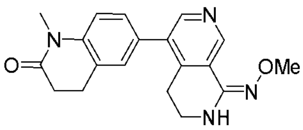,
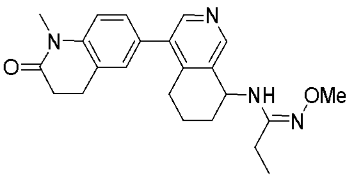,
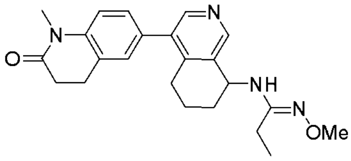,
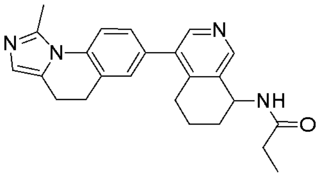,
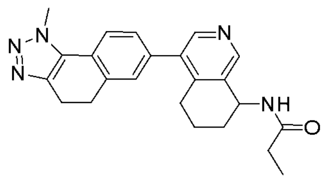,
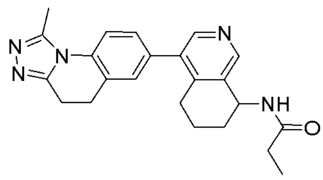,
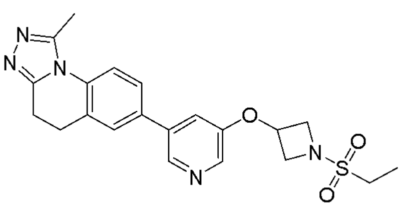,
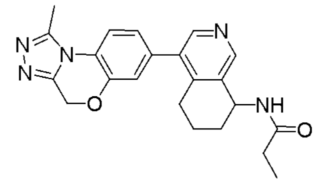,
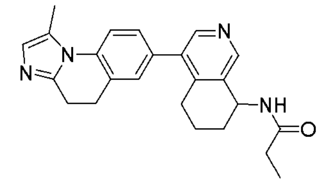,
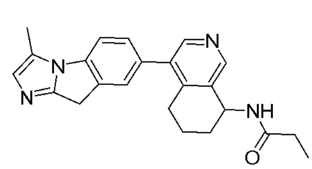,
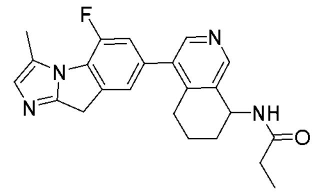, and
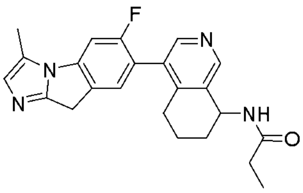.

The present disclosure further provides a compound of one of the following formulas, a stereoisomer thereof, or a pharmaceutically acceptable salt thereof,

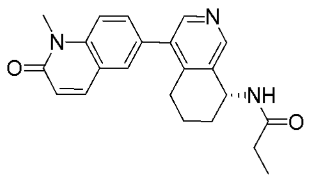
,
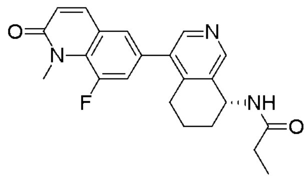
,
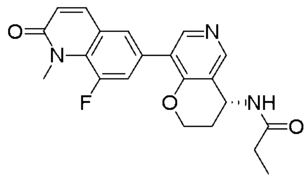
,
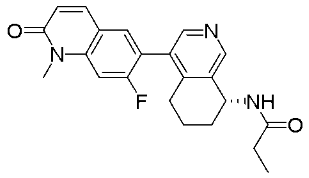
,
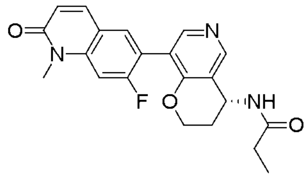
,
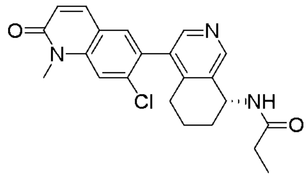
,
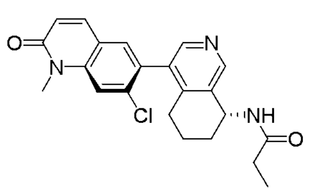
,
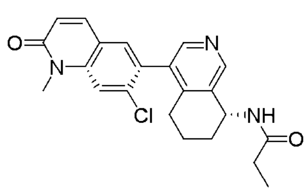
,
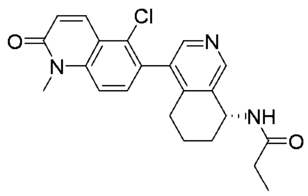
,
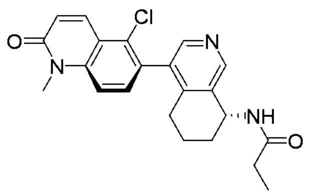
,
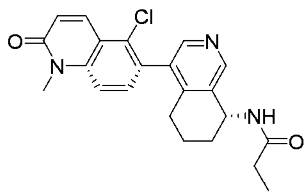
,
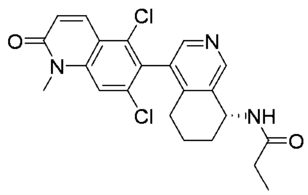
,
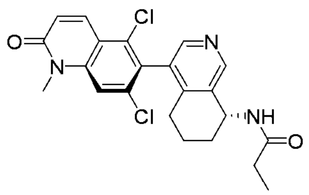
,
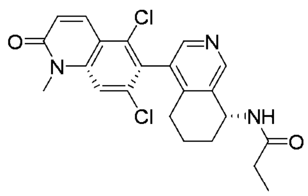
,
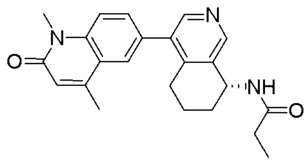
,
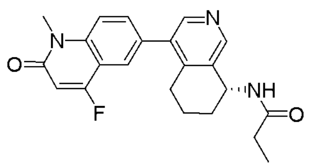
,
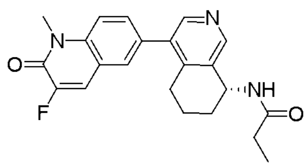
,
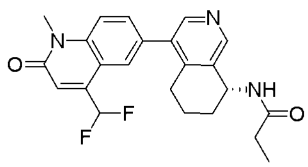
,
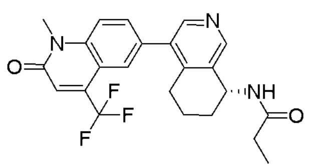
,
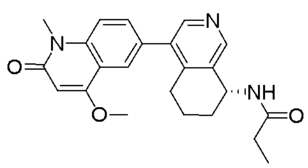
,
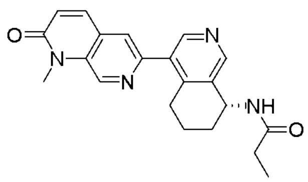
,

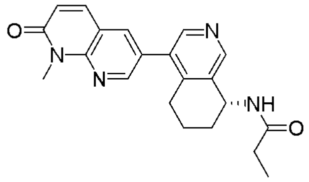,
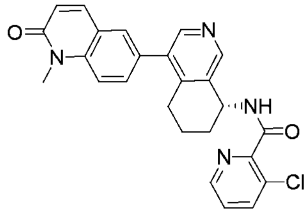,
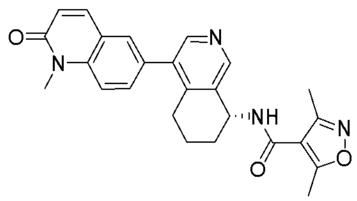,
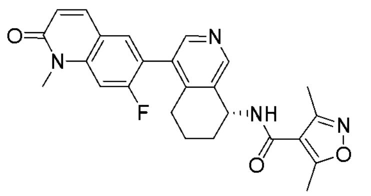,
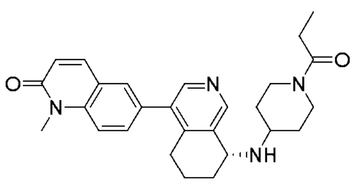,
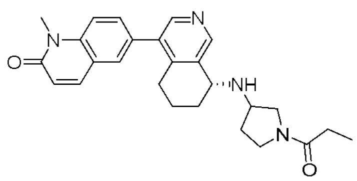,
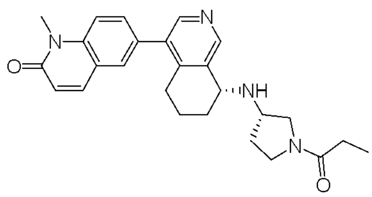,
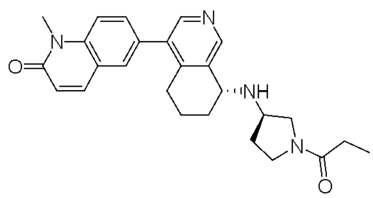,
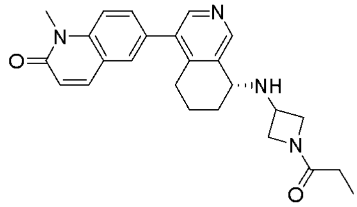,
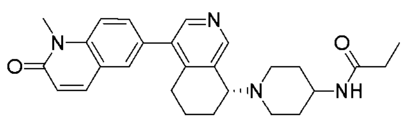,
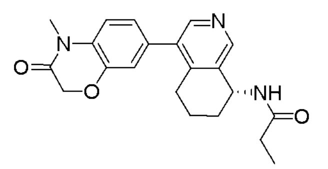,
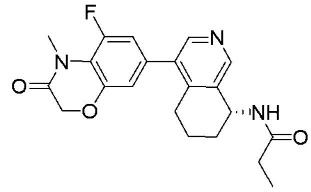,
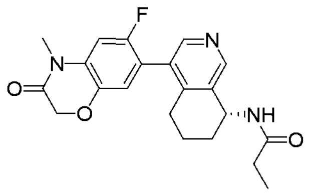,
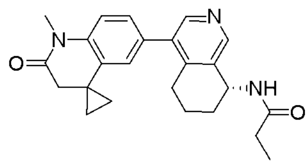,
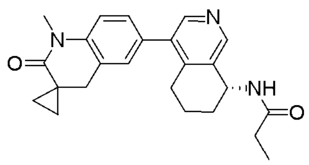,
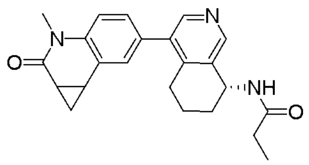,
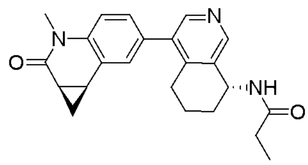,
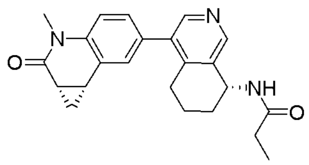,
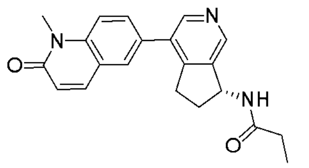,
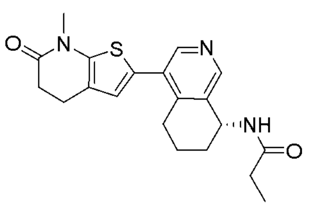,
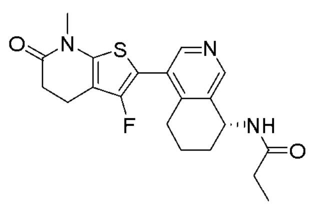,

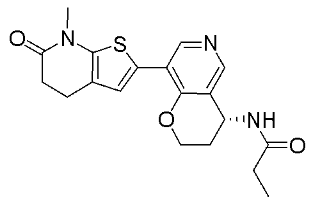,

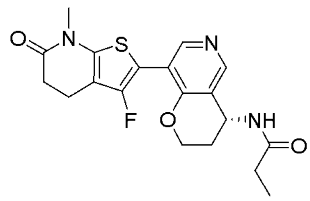,

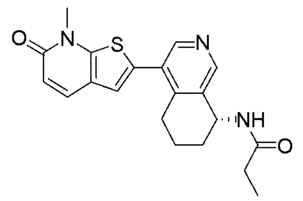,

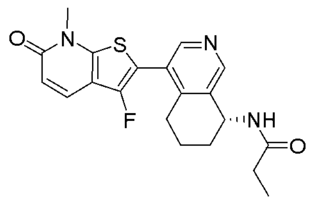,

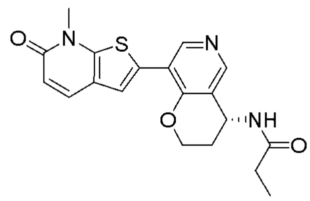,

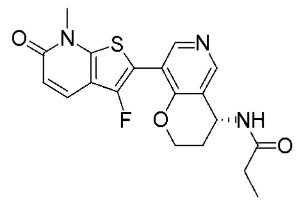,

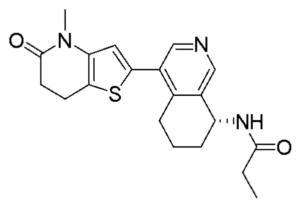,

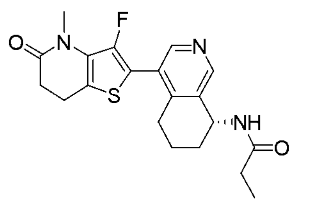,

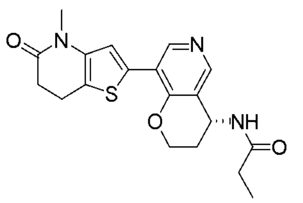,

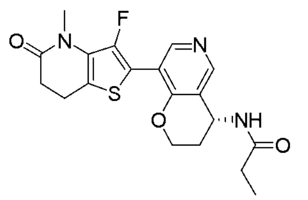,

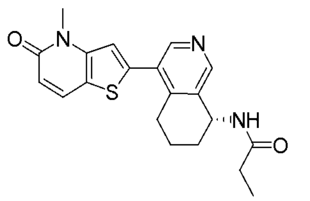,

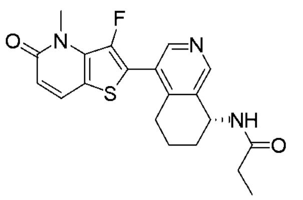,

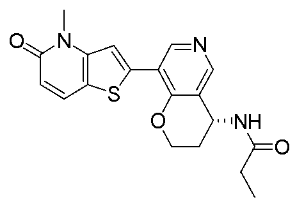,

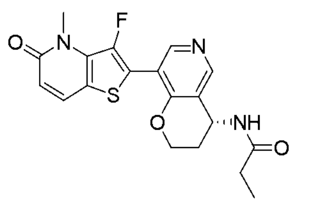,

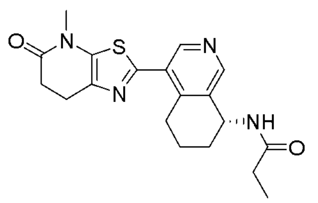,

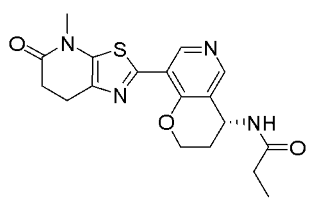,

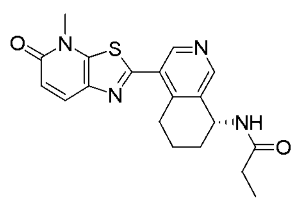,

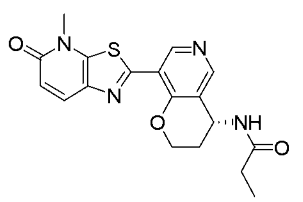,

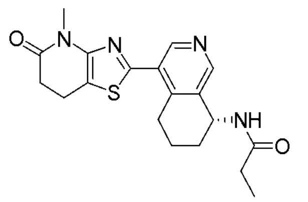,

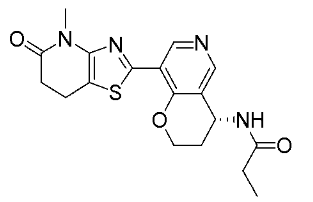,

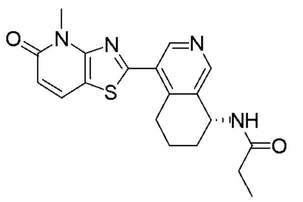,

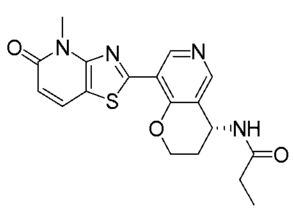,
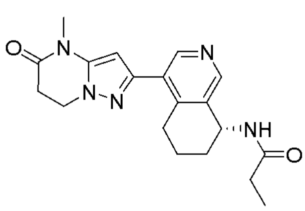,
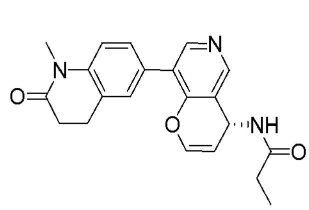,

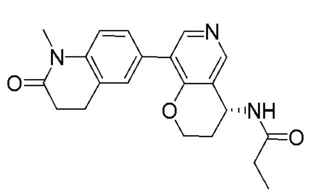,
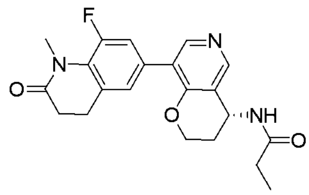,
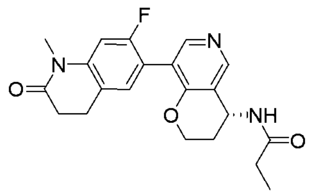,

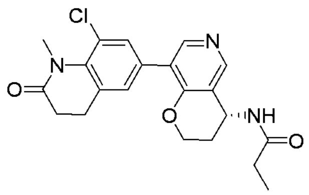,
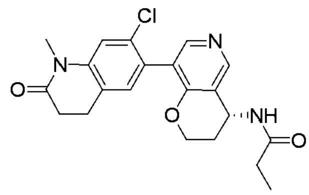,
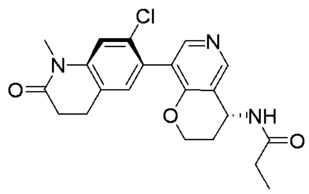,

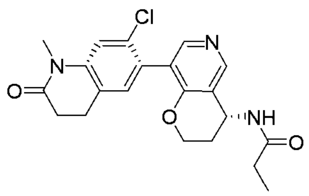,
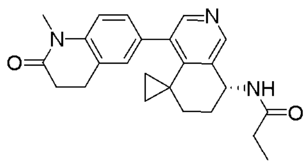,
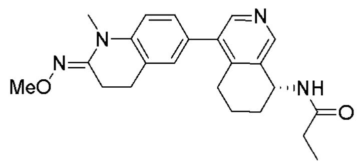,

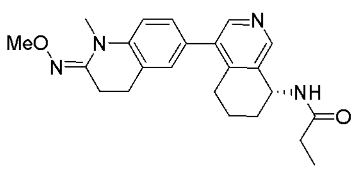,
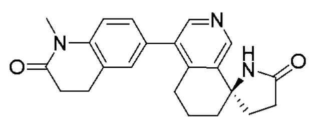,
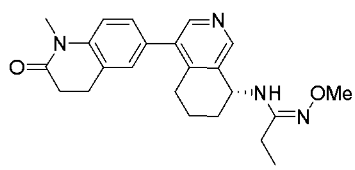,

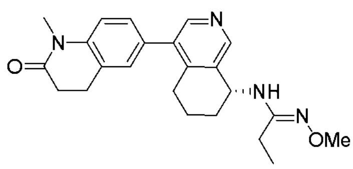,
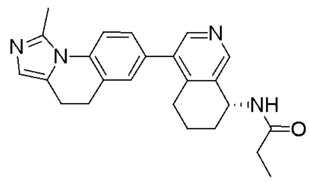,
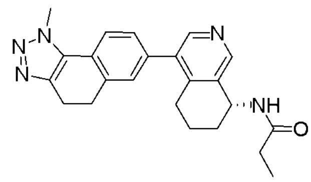,

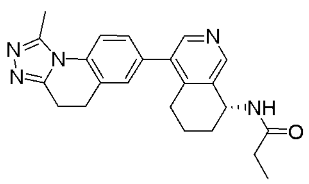,
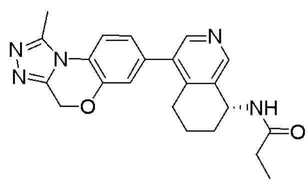,
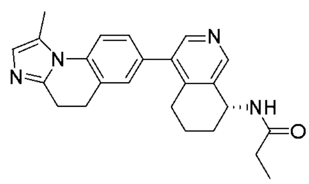,

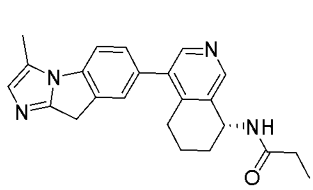 , 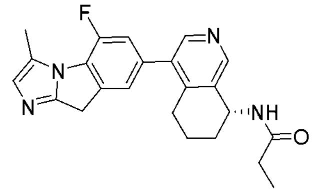 , 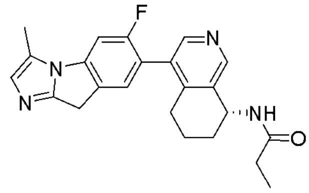 ,

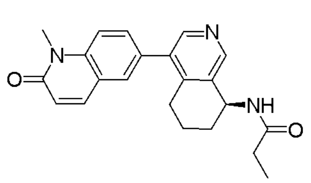 , 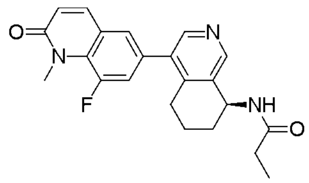 , 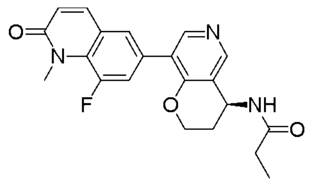 ,

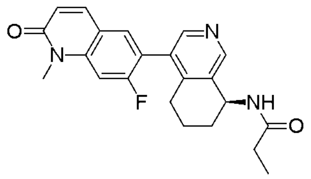 , 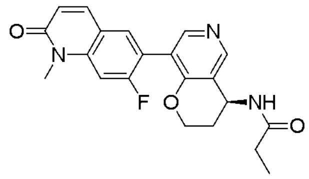 , 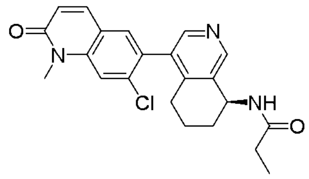 ,

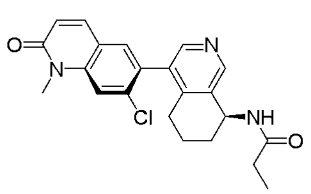 , 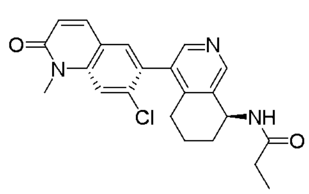 , 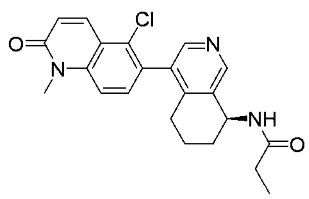 ,

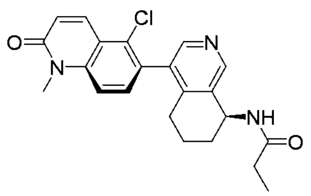 , 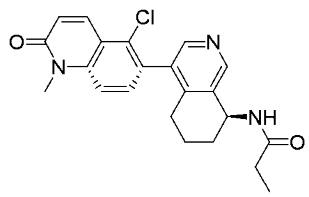 , 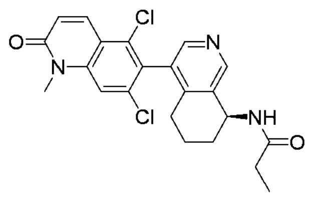 ,

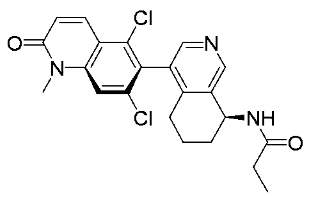 , 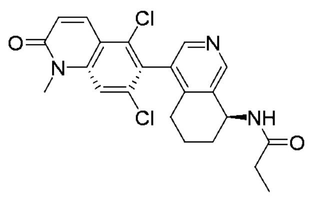 , 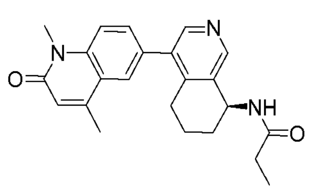 ,

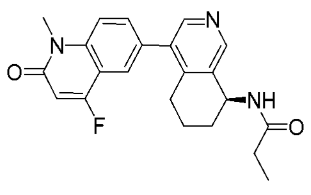 , 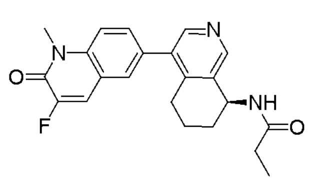 , 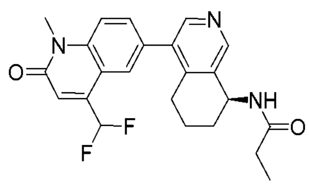 ,

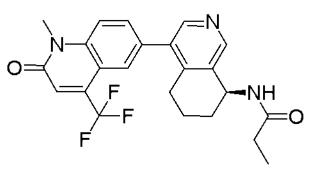,
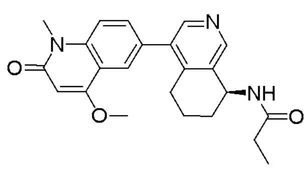,
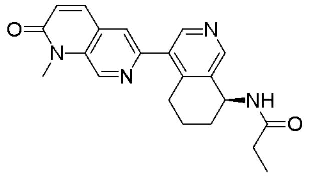,

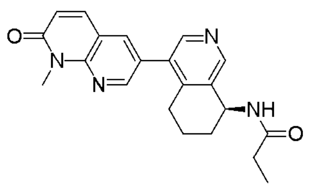,
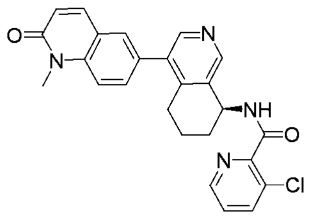,
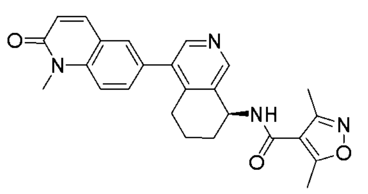,

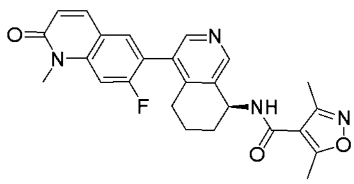,
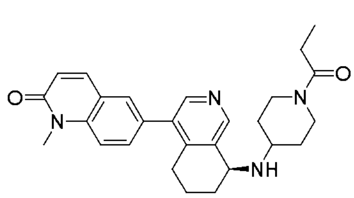,
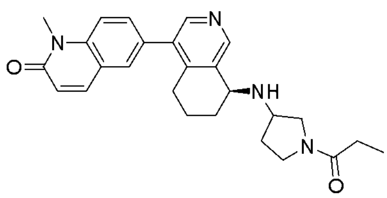,

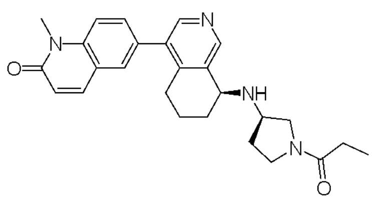,
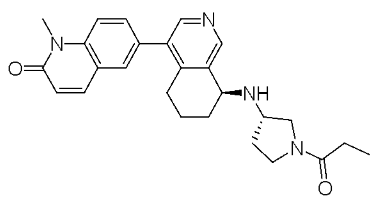,
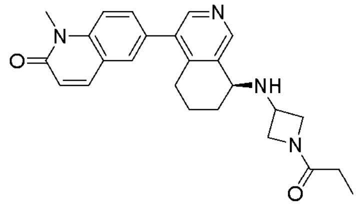,

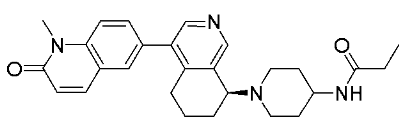,
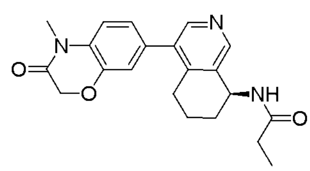,
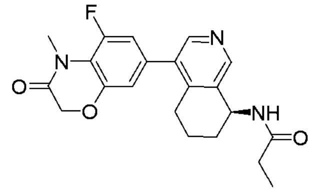,

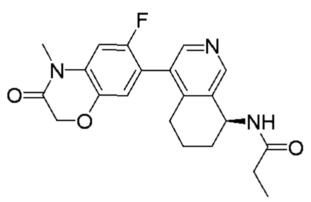,
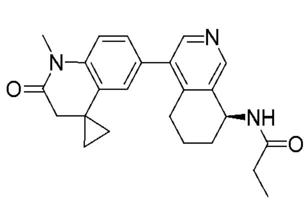,
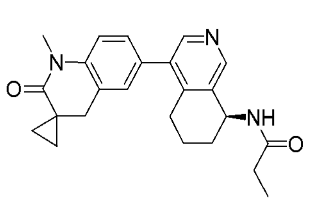,

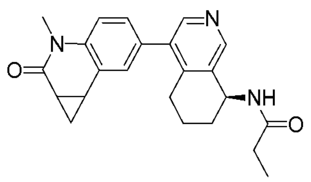,
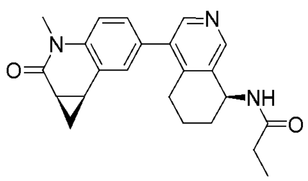,
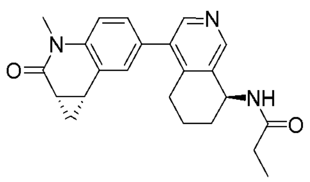,

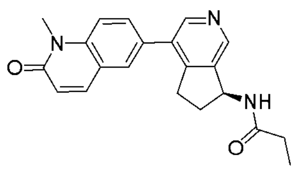, 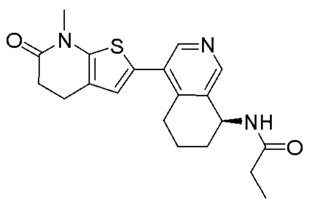, 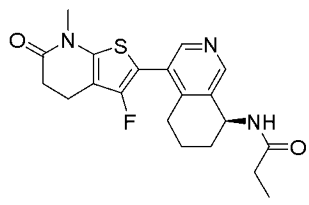,

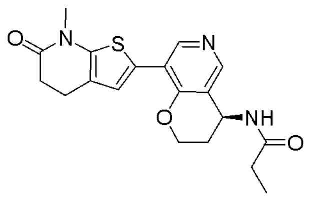, 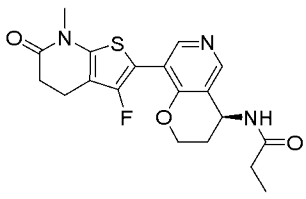, 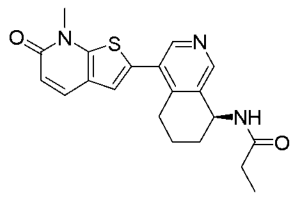,

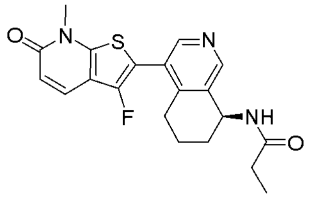, 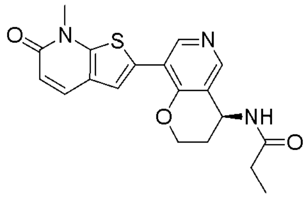, 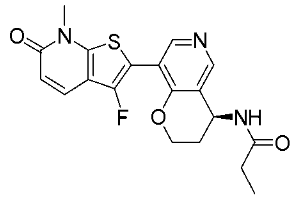,

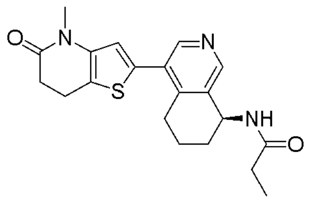, 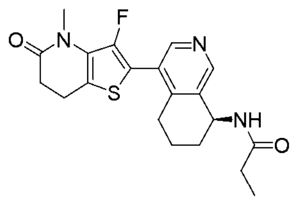, 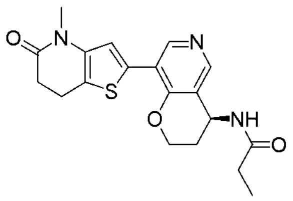,

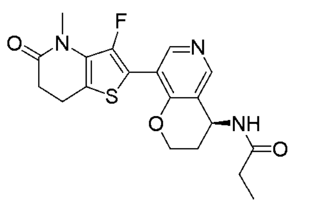, 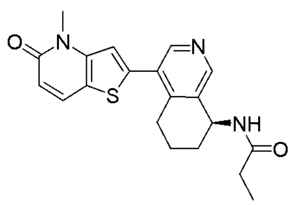, 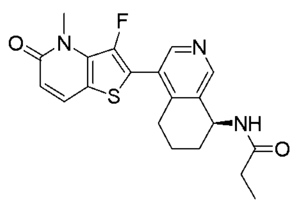,

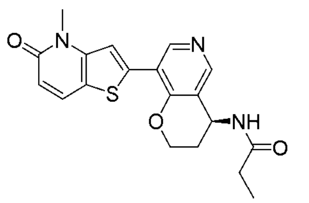, 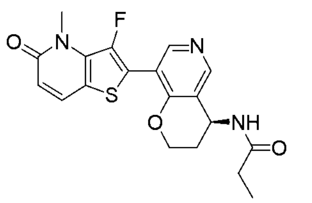, 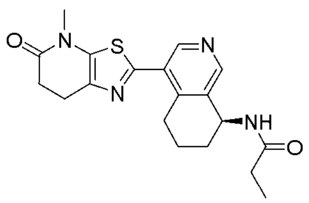,

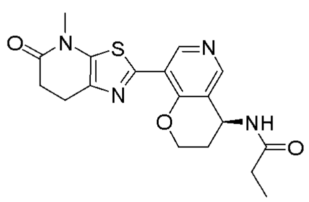, 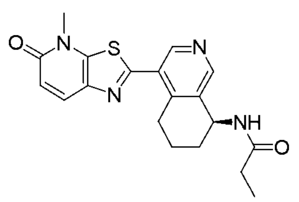, 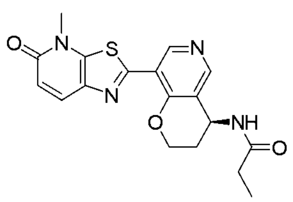,

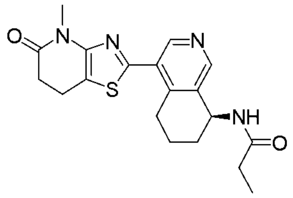 , 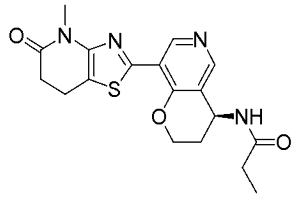 , 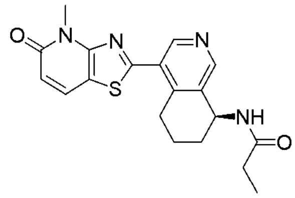 ,

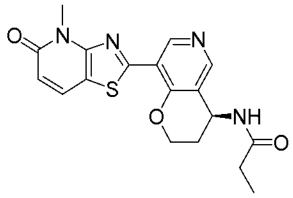 , 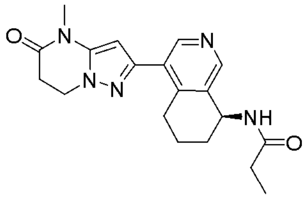 , 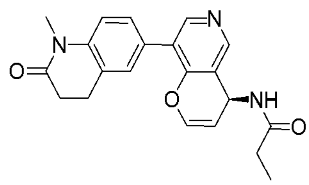 ,

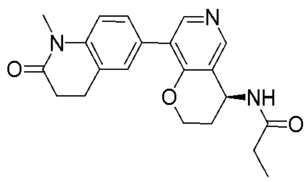 , 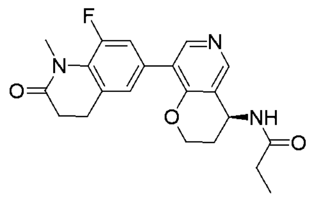 , 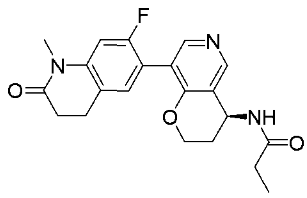 ,

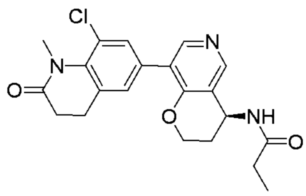 , 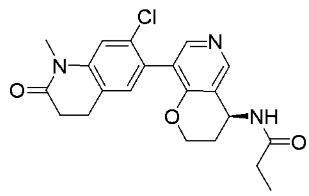 , 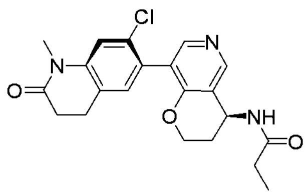 ,

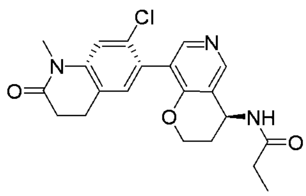 , 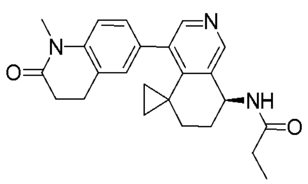 , 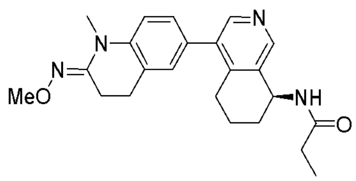 ,

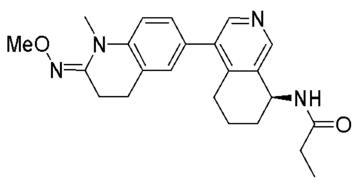 , 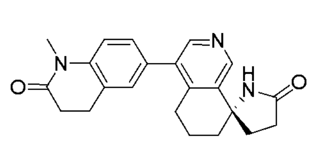 , 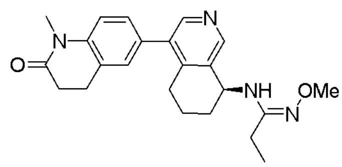 ,

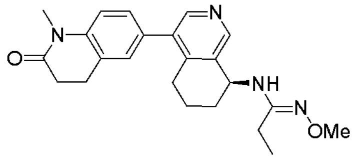 , 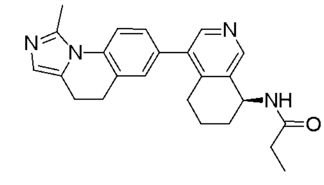 , 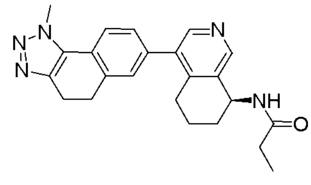 ,

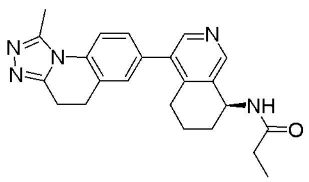,

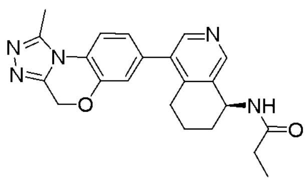,

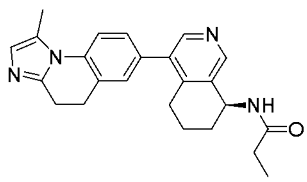,

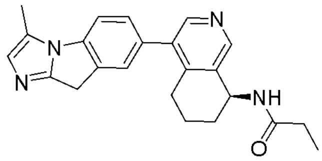,

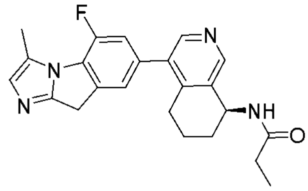, and

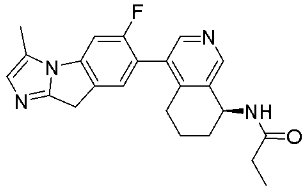.

The present disclosure further provides a use of the above compound, a stereoisomer thereof, or a pharmaceutically acceptable salt thereof in the manufacture of a medicament for treating a disease associated with an aldosterone synthase inhibitor.

The present disclosure further provides a use of the above compound, a stereoisomer thereof, or a pharmaceutically acceptable salt thereof in the manufacture of a medicament for treating resistant hypertension.

**Technical Effect**

The compounds of the present disclosure exhibit high CYP11B2 enzyme inhibitory activity, weak inhibition of CYP11B1, and excellent selectivity, with minimal impact on cortisol synthesis, resulting in superior clinical safety. These compounds also possess favorable in vivo pharmacokinetic properties, including high bioavailability, slow clearance, long half-life, and good oral absorption, making them suitable for once-daily oral administration. They are characterized by high permeability and low efflux, exhibit moderate binding to plasma proteins across different species, and have an appropriate proportion of free plasma drug concentration, indicating good druggability. Furthermore, the compounds of the present disclosure demonstrate low clearance in human liver microsomes and good metabolic stability. They do not inhibit CYP enzymes such as CYP1A2, CYP2C9, CYP2C19, CYP2D6, or CYP3A4, thereby presenting an extremely low risk of drug-drug interactions.

**Definition and explanation**

Unless otherwise specified, the following terms and phrases when used herein have the following meanings. A specific term or phrase should not be considered indefinite or unclear in the absence of a particular definition, but should be understood in the ordinary sense. When a trading name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, an allergic reaction, or other problems or complications, commensurate with a reasonable benefit/risk ratio.

The term "pharmaceutically acceptable salt" refers to a salt of the compound of the present disclosure that is prepared by reacting the compound having a specific substituent of the present disclosure with a relatively non-toxic acid or base. When the compound of the present disclosure contains a relatively acidic functional group, a base addition salt can be obtained by contacting such compounds with a sufficient amount of base in a pure solution or a suitable inert solvent. When the compound of the present disclosure contains a relatively basic functional group, an acid addition salt can be obtained by contacting the compound with a sufficient amount of acid in a pure solution or a suitable inert solvent. Certain specific compounds of the present disclosure contain both basic and acidic functional groups, thus can be converted to any base or acid addition salt.

The pharmaceutically acceptable salt of the present disclosure can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Unless otherwise specified, the term "isomer" is intended to include a geometric isomer, a cis-trans isomer, a stereoisomer, an enantiomer, an optical isomer, a diastereoisomer, and a tautomeric isomer.

The compounds of the present disclosure may exist in specific geometric or stereoisomeric forms. The present

disclosure contemplates all such compounds, including *cis* and *trans* isomers, (-)- and (+)-enantiomers, (R)- and (S)-enantiomers, diastereoisomers, *(D)*-isomers, (L)-isomers, racemic, and other mixtures thereof, such as enantiomers or diastereomer enriched mixtures, all of which are within the scope of the present disclosure. Additional asymmetric carbon atoms may be present in substituents such as alkyl. All these isomers and their mixtures are included within the scope of the present disclosure.

Unless otherwise specified, the term "enantiomer" or "optical isomer" refers to stereoisomers that are mirror images of each other.

Unless otherwise specified, the term *"cis-trans* isomer" or "geometric isomer" is caused by the inability to rotate freely of double bonds or single bonds of ring-forming carbon atoms.

Unless otherwise specified, the term "diastereomer" refers to a stereoisomer in which a molecule has two or more chiral centers and the relationship between the molecules is not mirror images.

Unless otherwise specified, "(+)" refers to dextrorotation, "(-)" refers to levorotation, and "(±)" refers to racemic.

Unless otherwise specified, the absolute configuration of a stereogenic center is represented by a wedged solid bond ( 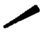 ) and a wedged dashed bond ( 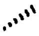 ), and the relative configuration of a stereogenic center is represented by a straight solid bond ( 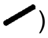 ) and a straight dashed bond ( 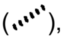 ), a wave line ( 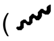 ) is used to represent a wedged solid bond ( 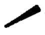 ) or a wedged dashed bond ( 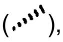 ), or the wave line ( 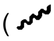 ) is used to represent a straight solid bond ( 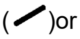 )or a straight dashed bond ( 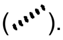 ).

Unless otherwise specified, the terms "enriched in one isomer", "enriched in isomers", "enriched in one enantiomer", or "enriched in enantiomers" refer to the content of one of the isomers or enantiomers is less than 100%, and the content of the isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, the isomer or enantiomer excess (ee value) is 80%.

Optically active (*R*)- and (*S*)-isomers, or *D* and *L* isomers can be prepared using chiral synthesis, chiral reagents, or other conventional techniques. If one kind of enantiomer of a certain compound of the present disclosure is to be obtained, it can be obtained by asymmetric synthesis or derivative action of chiral auxiliary, wherein the resulting diastereomeric mixture is separated and the auxiliary group is cleaved to provide the pure desired enantiomer. Alternatively, when the molecule contains a basic functional group (such as amino) or an acidic functional group (such as carboxyl), a salt of a diastereoisomer is formed with an appropriate optically active acid or base, and then diastereomeric resolution is performed by conventional methods known in the art, and then the pure enantiomer is recovered. In addition, the enantiomer and the diastereoisomer are generally separated through chromatography which uses a chiral stationary phase and optionally combines with a chemical derivative method (such as carbamate generated from amine).

The compound of the present disclosure may contain an unnatural proportion of atomic isotope at one or more than one atom that constitutes the compound. For example, the compound can be radiolabeled with a radioactive isotope, such as tritium ($^3H$), iodine-125 ($^{125}I$), or C-14 ($^{14}C$). For another example, deuterated drugs can be formed by replacing hydrogen with deuterium, the bond formed by deuterium and carbon is stronger than that of ordinary hydrogen and carbon, compared with non-deuterated drugs, deuterated drugs have the advantages of reduced toxic and side effects, increased drug stability, enhanced efficacy, extended biological half-life of drugs, *etc.* All isotopic variations of the compound of the present disclosure, whether radioactive or not, are encompassed within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom on a specific atom is substituted by the substituent, including deuterium and hydrogen variables, as long as the valence of the specific atom is normal and the substituted compound is stable. When the substituent is an oxygen (*i.e.,* =*O*), it means two hydrogen atoms are substituted.

The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted by 0 to -2 R, the group can be optionally substituted with up to two R, wherein the definition of R at each occurrence is independent.

Moreover, a combination of the substituent and/or the variant thereof is allowed only when the combination results in a stable compound.

When the number of a linking group is 0, such as $-(CRR)_0-$, it means that the linking group is a single bond.

When a substituent is 0 in number, it means that the substituent is absent. In the case of $-A-(R)_0$, the structure is actually -A.

When a substituent is vacant, it means that the substituent is absent, for example, when X is vacant in A-X, the structure of A-X is actually A.

When one of the variables is selected from a single bond, it means that the two groups linked by the single bond are connected directly. For example, when L in A-L-Z represents a single bond, the structure of A-L-Z is actually A-Z.

When the enumerative linking group does not indicate the direction for linking, the direction for linking is arbitrary, for example, the linking group L contained in

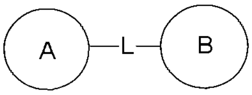

is -M-W-, then -M-W- can link ring A and ring B to form

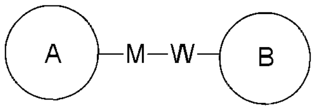

in the direction same as left-to-right reading order, and form

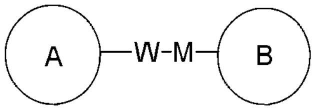

in the direction contrary to left-to-right reading order. A combination of the linking groups, substituents and/or variables thereof is allowed only when such combination can result in a stable compound.

Unless otherwise specified, when a group has one or more than one linkable site, any one or more than one site of the group can be linked to other groups through chemical bonds. When the linking site of the chemical bond is not positioned, and there is an H atom at the linkable site, then the number of H atoms at the site will decrease correspondingly with the number of the chemical bonds linking thereto so as to meet the corresponding valence. The chemical bond between the site and other groups can be represented by a straight solid bond (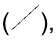), a straight dashed bond (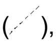), or a wavy line (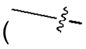). For example, the straight solid bond in $-OCH_3$ means that it is linked to other groups through the oxygen atom in the group; the straight dashed bond in

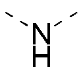

means that it is linked to other groups through the two ends of the nitrogen atom in the group; the wave lines in

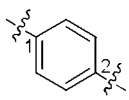

means that the phenyl group is linked to other groups through carbon atoms at position 1 and position 2;

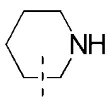

means that it can be linked to other groups through any linkable sites on the piperidinyl by one chemical bond, including at least four types of linkage, including

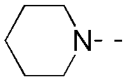, 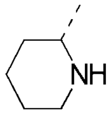, 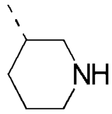,

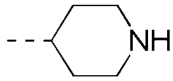.

Even though the H atom is drawn on the -N-,

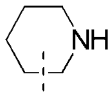

still includes the linkage of

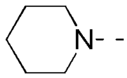,

merely when one chemical bond was connected, the H of this site will be reduced by one to the corresponding monovalent piperidinyl.

Unless otherwise specified, when ring A is fused with ring B, they share a bond and the atoms on said bond may be either C or N atoms. For example, in

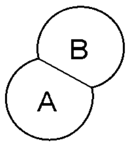,

the atoms on the fused bond between A and B may be C or N, including but not limited to

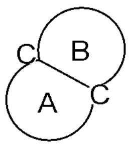,

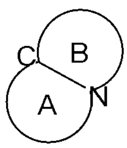, and 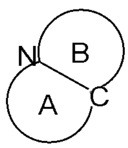.

Unless otherwise specified, the number of atoms in a ring is usually defined as the number of ring members, for example, "5- to 7-membered ring" refers to a "ring" in which 5 to 7 atoms are arranged around.

Unless otherwise specified, the term "halogen element" or "halogen" by itself or as part of another substituent refers to fluorine, chlorine, bromine, or iodine atom.

Unless otherwise specified, the term "$C_{1-3}$ alkyl" refers to a linear or branched saturated hydrocarbon group consisting of 1 to 3 carbon atoms. The $C_{1-3}$ alkyl includes $C_{1-2}$ and $C_{2-3}$ alkyl, *etc.;* it can be monovalent (such as methyl), divalent (such as methylene), or multivalent (such as methine). Examples of $C_{1--3}$ alkyl include, but are not limited to, methyl (Me), ethyl (Et), propyl (including n-propyl and isopropyl), *etc.*

Unless otherwise specified, the term "$C_{1-3}$ alkoxy" refers to an alkyl group containing 1 to 3 carbon atoms that are connected to the rest of the molecule through an oxygen atom. It can be monovalent, divalent, or multivalent. The $C_{1-3}$ alkoxy includes $C_{1-2}$, $C_{2-3}$, $C_3$, $C_2$ alkoxy, *etc.* Examples of $C_{1--3}$ alkoxy include, but are not limited to, methoxy, ethoxy,

propoxy (including n-propoxy and isopropoxy), *etc.*

Unless otherwise specified, the term "$C_{1-3}$ alkylamino" refers to an alkyl group containing 1 to 3 carbon atoms that are connected to the rest of the molecule through a nitrogen atom. The $C_{1-3}$ alkylamino includes $C_{1-2}$, $C_3$, $C_2$ alkylamino, etc. Examples of $C_{1-3}$ alkylamino include, but are not limited to, $-NHCH_3$, $-N(CH_3)_2$, $-NHCH_2CH_3$, $-N(CH_3)CH_2CH_3$, $-NHCH_2CH_2CH_3$, $-NHCH_2(CH_3)_2$, etc.

Unless otherwise specified, the term "$C_{1-3}$ haloalkyl" refers to monohaloalkyl and polyhaloalkyl containing 1 to 3 carbon atoms. The $C_{1-3}$ haloalkyl includes $C_{1-2}$, $C_{2-3}$, $C_3$, $C_2$, $C_1$ haloalkyl, *etc.* Examples of $C_{1-3}$ haloalkyl include, but are not limited to, trifluoromethyl, trichloromethyl, 2,2,2-trifluoroethyl, pentafluoroethyl, pentachloroethyl, 3-bromopropyl, *etc.*

Unless otherwise specified, the term "$C_{1-3}$ haloalkoxy" refers to monohaloalkoxy and polyhaloalkoxy containing 1 to 3 carbon atoms. The $C_{1-3}$ haloalkoxy includes $C_{1-2}$, $C_{2-3}$, $C_3$, $C_2$, $C_1$ haloalkoxy, *etc.* Examples of $C_{1-3}$ haloalkoxy include, but are not limited to, trifluoromethoxy, trichloromethoxy, 2,2,2-trifluoroethoxy, pentafluoroethoxy, pentachloroethoxy, 3-bromopropoxy, *etc.*

Unless otherwise specified, "$C_{5-6}$ cycloalkyl" refers to a saturated or partially saturated cyclic hydrocarbon group consisting of 5 to 6 carbon atoms, which is a monocyclic system, and the $C_{5-6}$ cycloalkyl includes $C_5$, $C_6$ cycloalkyl, etc.; it can be monovalent, divalent, or multivalent. Examples of $C_{5-6}$ cycloalkyl include, but are not limited to, cyclopentyl, cyclohexyl, cyclopentenyl, cyclohexenyl, *etc.*

Unless otherwise specified, "$C_{3-5}$ cycloalkyl" refers to a saturated or partially saturated cyclic hydrocarbon group consisting of 3 to 5 carbon atoms, which is a monocyclic system, and the $C_{3-5}$ cycloalkyl includes $C_3$, $C_4$, and $C_5$ cycloalkyl, *etc.;* it can be monovalent, divalent, or multivalent. Examples of $C_{3-5}$ cycloalkyl include, but are not limited to, cyclopropyl, cyclobutyl, cyclopentyl, cyclopropenyl, cyclobutenyl, cyclopentenyl, *etc.*

Unless otherwise specified, the term "4- to 6-membered heterocycloalkyl" by itself or in combination with other terms refers to a saturated cyclic group consisting of 4 to 6 ring atoms, wherein 1, 2, 3, or 4 ring atoms are heteroatoms independently selected from O, S, and N, and the rest are carbon atoms, wherein carbon atoms are optionally substituted by oxo (i.e., C(O)), nitrogen atoms are optionally quaternized, and nitrogen and sulfur heteroatoms can be optionally oxidized (i.e., NO and $S(O)_p$, p is 1 or 2). It includes monocyclic and bicyclic systems, wherein the bicyclic systems include spirocyclic, fused, and bridged ring systems. In addition, in the case of the "4- to 6-membered heterocycloalkyl", the heteroatom may occupy the position where the heterocycloalkyl is linked to the rest of the molecule. The 4- to 6-membered heterocycloalkyl includes 5- to 6-membered, 4-membered, 5-membered, 6-membered heterocycloalkyl, etc. Examples of 4- to 6-membered heterocycloalkyl include, but are not limited to, azetidinyl, oxetanyl, thietanyl, pyrrolidinyl, pyrazolidinyl, imidazolidinyl, tetrahydrothienyl (including tetrahydrothiophen-2-yl and tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, and 3-piperidinyl, etc.), piperazinyl (including 1-piperazinyl and 2-piperazinyl, etc.), morpholinyl (including 3-morpholinyl and 4-morpholinyl, etc.), dioxinyl, dithianyl, isoxazolidinyl, isothiazolidinyl, 1,2-oxazinyl, 1,2-thiazinyl, hexahydropyridazinyl, etc.

Unless otherwise specified, the term "5- to 6-membered heterocycloalkyl" by itself or in combination with other terms refers to a saturated cyclic group consisting of 5 to 6 ring atoms, wherein 1, 2, 3, or 4 ring atoms are heteroatoms independently selected from O, S, and N, and the rest are carbon atoms, wherein carbon atoms are optionally substituted by oxo (i.e., C(O)), nitrogen atoms are optionally quaternized, and nitrogen and sulfur heteroatoms can be optionally oxidized (i.e., NO and $S(O)_p$, p is 1 or 2). It includes monocyclic and bicyclic systems, wherein the bicyclic systems include spirocyclic, fused, and bridged ring systems. In addition, in the case of the "4- to 6-membered heterocycloalkyl", the heteroatom may occupy the position where the heterocycloalkyl is linked to the rest of the molecule. The 5- to 6-membered heterocycloalkyl includes 5-membered heterocycloalkyl, 6-membered heterocycloalkyl, and the like. Examples of 5- to 6-membered heterocycloalkyl include, but are not limited to, pyrrolidinyl, pyrazolidinyl, imidazolidinyl, tetrahydrothiophenyl (including tetrahydrothiophen-2-yl, tetrahydrothiophen-3-yl, etc.), tetrahydrofuranyl (including tetrahydrofuran-2-yl, etc.), tetrahydropyranyl, piperidinyl (including 1-piperidinyl, 2-piperidinyl, 3-piperidinyl, etc.), piperazinyl (including 1-piperazinyl, 2-piperazinyl, etc.), morpholinyl (including 3-morpholinyl, 4-morpholinyl, etc.), dioxinyl, dithianyl, isoxazolidinyl, isothiazolidinyl, 1,2-oxazinyl, 1,2-thiazinyl, hexahydropyridazinyl, etc.

Unless otherwise specified, the terms "5- to 6-membered heteroaryl ring" and "5- to 6-membered heteroaryl" are used interchangeably. The term "5- to 6-membered heteroaryl" refers to a monocyclic group consisting of 5 to 6 ring atoms with a conjugated $\pi$-electron system, in which 1, 2, 3, or 4 ring atoms are heteroatoms independently selected from O, S, and N, and the rest are carbon atoms, Here, the nitrogen atom is optionally quaternized, and nitrogen and sulfur heteroatoms can be optionally oxidized (i.e., NO and $S(O)_p$, wherein p is 1 or 2). The 5- to 6-membered heteroaryl can be linked to the rest of the molecule through a heteroatom or a carbon atom. The 5- to 6-membered heteroaryl includes 5-membered and 6-membered heteroaryl. Examples of the 5- to 6-membered heteroaryl include, but are not limited to, pyrrolyl (including N-pyrrolyl, 2-pyrrolyl, and 3-pyrrolyl, etc.), pyrazolyl (including 2-pyrazolyl and 3-pyrazolyl, etc.), imidazolyl (including N-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (including 1H-1,2,3-triazolyl, 2H-1,2,3-triazolyl, 1H-1,2,4-triazolyl, and 4H-1,2,4-triazolyl, etc.), tetrazolyl, isoxazolyl (including 3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyll and 3-thienyl, etc.), pyridyl (including 2-pyridyl, 3-pyridyl, and 4-pyridyl, etc.), pyrazinyl, pyrimidinyl (including 2-pyrimidinyl, 4-pyrimidinyl, etc.).

Unless otherwise specified, the terms "5-membered heteroaryl ring" and "5-membered heteroaryl" in the present disclosure can be used interchangeably, and the term "5-membered heteroaryl" refers to a monocyclic group consisting of 5 ring atoms with a conjugated $\pi$-electron system, wherein 1, 2, 3, or 4 ring atoms are heteroatoms independently selected from O, S, and N, and the rest are carbon atoms. Here, the nitrogen atom is optionally quaternized, and nitrogen and sulfur heteroatoms can be optionally oxidized (i.e., NO and $S(O)_p$, wherein p is 1 or 2). The 5-membered heteroaryl can be connected to the rest of the molecule through a heteroatom or a carbon atom. Examples of the 5-membered heteroaryl include, but are not limited to, pyrrolyl (including *N*-pyrrolyl, 2-pyrrolyl, and 3-pyrrolyl, etc.), pyrazolyl (including 2-pyrazolyl and 3-pyrazolyl, etc.), imidazolyl (including *N*-imidazolyl, 2-imidazolyl, 4-imidazolyl, and 5-imidazolyl, etc.), oxazolyl (including 2-oxazolyl, 4-oxazolyl, and 5-oxazolyl, etc.), triazolyl (including 1*H*-1,2,3-triazolyl, 2*H*-1,2,3-triazolyl, 1*H*-1,2,4-triazolyl, and 4*H*-1,2,4-triazolyl, etc.), tetrazolyl, isoxazolyl (including 3-isoxazolyl, 4-isoxazolyl, and 5-isoxazolyl, etc.), thiazolyl (including 2-thiazolyl, 4-thiazolyl, and 5-thiazolyl, etc.), furyl (including 2-furyl and 3-furyl, etc.), thienyl (including 2-thienyll and 3-thienyl, etc.).

The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure.

The structure of the compounds of the present disclosure can be confirmed by conventional methods known to those skilled in the art, and if the present disclosure involves an absolute configuration of a compound, then the absolute configuration can be confirmed by means of conventional techniques in the art. For example, in the case of single crystal X-ray diffraction (SXRD), diffraction intensity data are collected from the cultured single crystal using a Bruker D8 venture diffractometer with CuK$\alpha$ radiation as the light source and scanning mode: $\varphi/\omega$ scan, and after collecting the relevant data, the crystal structure is further analyzed by direct method (Shelxs97), so that the absolute configuration can be confirmed.

The present disclosure adopts the following abbreviations: *eq* represents equivalent; DCM represents dichloromethane; PE represents petroleum ether; DMSO represents dimethyl sulfoxide; EtOAc represents ethyl acetate; EtOH represents ethanol; MeOH represents methanol; DMF represents N,N-dimethylformamide; Boc represents tert-butoxycarbonyl, an amine protecting group; r.t. represents room temperature; TFA represents trifluoroacetic acid; Eaton's reagent represents a 7.5% solution of phosphorus pentoxide in methanesulfonic acid; PEG represents polyethylene glycol; solutol represents polyoxyl 15 hydroxystearate; $C_{max}$ represents maximum concentration; AUC represents oral exposure; $T_{1/2}$ represents half-life; $V_{dss}$ represents steady-state volume of distribution; CL represents clearance.

The solvents used in the present disclosure are commercially available.

The compounds of the present disclosure are named according to the conventional naming principles in the art or by ChemDraw® software, and the commercially available compounds use the supplier catalog names.

BRIEF DESCRIPTION OF THE DRAWINGS

FIG. 1. Binding mode diagram of compound 1A with CYP11B2 protein.
FIG. 2. Binding mode diagram of compound 2A with CYP11B2 protein.
FIG. 3. Binding mode diagram of compound 3A with CYP11B2 protein.

DETAILED DESCRIPTION OF THE PREFERRED EMBODIMENT

The present disclosure is described in detail by the examples below, but it does not mean that there are any adverse restrictions on the present disclosure. The compounds of the present disclosure can be prepared by a variety of synthetic methods known to those skilled in the art, including the specific embodiments listed below, the embodiments formed by their combination with other chemical synthesis methods, and equivalent alternatives known to those skilled in the art, and preferred embodiments include, but are not limited to, the examples of the present disclosure. It will be apparent to those skilled in the art that various variations and improvements can be made to specific embodiments of the present disclosure without departing from the spirit and scope of the present disclosure.

**Computing example 1**

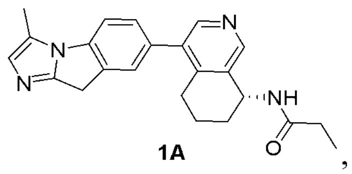

1A,

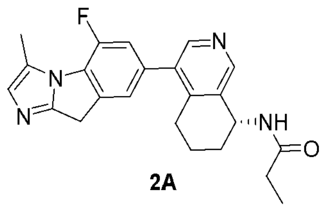

2A,

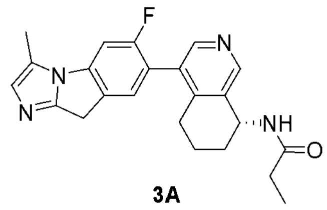

3A

The molecular docking process was conducted by using Glide SP[1] in Maestro (Schrödinger version 2022-1) with default settings. The co-crystal structure of CYP11B2 (PDB ID: 4ZGX) was selected as the docking template. To prepare the protein, hydrogen atoms were added using the Protein Preparation Wizard module of Maestro [2] with the OPLS4 force field. For ligand preparation, the three-dimensional structures of the molecules were generated using the LigPrep module, and energy minimization was performed [3]. Conformational searches for the small molecules were conducted using the ConfGen module [4]. The Receptor Grid Generation module in Glide was used to generate the grid file required for docking, with the ligand from the crystal structure serving as the center of the docking box. The types of interactions between protein receptors and ligands were analyzed, then molecules with high potential were selected for synthesis and testing based on calculated docking scores and binding modes. The binding modes of compounds 1A, 2A, and 3A are shown in FIGs. 1, 2, and 3, respectively.

[1] Glide, Schrödinger, LLC, New York, NY, 2020.
[2] Maestro, Schrödinger, LLC, New York, NY, 2020.
[3] LigPrep, Schrödinger, LLC, New York, NY, 2020.
[4] ConfGen, Schrödinger, LLC, New York, NY, 2020.

Conclusion: The compounds of the present disclosure exhibited favorable binding to th eCYP11B2 protein.

**Reference example 1: Fragment A-1**

Synthetic route:

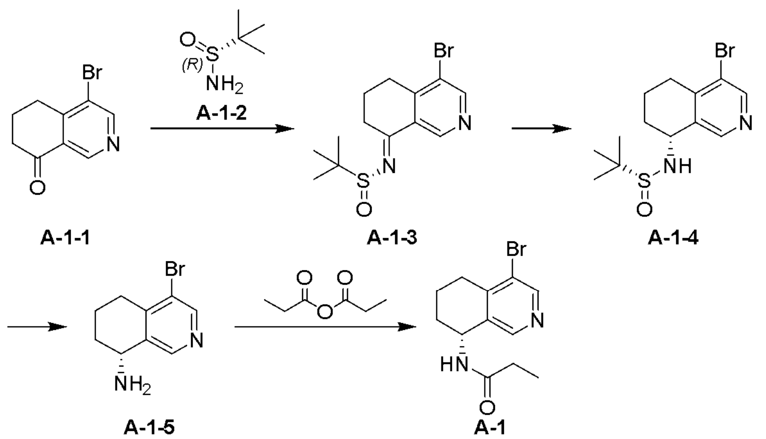

A-1-2, A-1-1, A-1-3, A-1-4, A-1-5, A-1

Step 1: synthesis of compound A-1-3

A-1-1 (19 g, 84.04 mmol) and tetrahydrofuran (250 mL) were added to a reaction flask. After stirring to dissolve, A-1-2 (12.22 g, 100.85 mmol) was added, followed by dropwise addition of tetraethyl titanate (57.51 g, 252.13 mmol). The reaction mixture was stirred at 75°C for 16 hours. 400 mL of ethyl acetate and 800 mL of saturated aqueous sodium bicarbonate solution were added. The mixture was filtered, and the filter cake was washed three times with ethyl acetate (100 mL each time). The filtrate was collected and separated. The aqueous phase was extracted twice with 400 mL of ethyl acetate, and the organic phases were combined. The organic phase was washed with 800 mL of saturated brine, collected, dried over anhydrous sodium sulfate, filtered, and concentrated to obtain crude A-1-3. $^1$H NMR (400 MHz, $CDCl_3$) $\delta$ ppm 9.19 (s, 1 H), 8.71 (s, 1 H), 3.25 - 3.35 (m, 1 H), 3.10 - 3.18 (m, 1 H), 2.83 - 2.99 (m, 2 H), 1.96 - 2.16 (m, 2 H), 1.34 (s, 9 H).

Step 2: synthesis of compound A-1-4

To a reaction flask were added A-1-3 (11.13 g, 33.80 mmol) and tetrahydrofuran (100 mL). The atmosphere was purged with nitrogen. At 0°C, 9-borabicyclo[3.3.1]nonane (0.5 M in tetrahydrofuran, 169.02 mL) was added dropwise, then the mixture was gradually warmed to 25°C and reacted for 16 hours. Saturated aqueous ammonium chloride solution (250 mL) and 250 mL of water were sequentially added to the reaction mixture, and the aqueous phase was extracted 3 times with 500 mL of ethyl acetate. The combined organic phases were washed with saturated brine (1000 mL), dried over anhydrous sodium sulfate, filtered, and concentrated to obtain a crude product. The crude product was purified by silica gel column chromatography (petroleum ether:ethyl acetate = 25% to 100%) to obtain A-1-4. $^1$H NMR (400 MHz, $CDCl_3$) δ ppm 8.58 (d, *J* = 8.78 Hz, 2 H), 4.60 - 4.70 (m, 1 H), 3.18 - 3.31 (m, 1 H), 2.81 - 2.92 (m, 1 H), 2.62 (ddd, *J* = 18.51, 9.60, 6.53 Hz, 1 H), 2.09 - 2.18 (m, 1 H), 1.93 - 2.07 (m, 1 H), 1.80 - 1.91 (m, 2 H), 1.23 (s, 9 H).

Step 3: synthesis of compound A-1-5

A-1-4 (8.31 g, 25.09 mmol) was dissolved in ethyl acetate (50 mL), and hydrogen chloride/ethyl acetate (2 M, 160 mL) was added at 28°C. The mixture was stirred for 16 hours. The reaction mixture was filtered, and the filter cake was washed with 3 mL of ethyl acetate. The filter cake was collected and dried to obtain a hydrochloride salt of the crude A-1-5. ESI-LCMS: *m*/*z* = 226.8, 228.9 $[M+1]^+$.

Step 4: synthesis of compound A-1

To a reaction flask were sequentially added the hydrochloride salt of A-1-5 (7.67 g), dichloromethane (150 mL), and triethylamine (8.83 g, 87.30 mmol). After stirring to dissolve, propionic anhydride (5.68 g, 43.65 mmol) was added, and the mixture was stirred at 28°C for 16 hours. 400 mL of saturated aqueous ammonium chloride solution was added. The resulting phases were separated, and the aqueous phase was extracted three times with dichloromethane (200 mL each time). The organic phases were combind, dried over anhydrous sodium sulfate, filtered, and concentrated to obtain a crude product. The crude product was purified by silica gel column chromatography (dichloromethane/methanol = 10%) to obtain A-1. $^1$H NMR (400 MHz, $CDCl_3$) δ ppm 8.54 (s, 1 H), 8.41 (s, 1 H), 5.74 (br d, *J* = 7.28 Hz, 1 H), 5.24 - 5.32 (m, 1 H), 2.69 - 2.82 (m, 2 H), 2.27 (q, *J* = 7.53 Hz, 2 H), 1.98 - 2.06 (m, 1 H), 1.91 (dt, *J* = 12.05, 6.02 Hz, 2 H), 1.78 - 1.86 (m, 1 H), 1.21 (t, *J* = 7.65 Hz, 3 H). $[\alpha]^{20}_D$ = +5.77 (c 1.0, MeOH).

**Reference example 2: Fragment A-2**

Synthetic route:

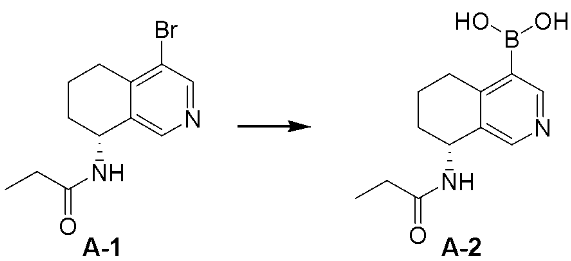

A-1 A-2

Step 1: synthesis of compound A-2

To a reaction flask were sequentially added A-1 (70 mg, 247.21 μmol), triisopropyl borate (155.75 mg, 828.14 μmol), and tetrahydrofuran (3.5 mL). The atmosphere was purged with nitrogen, and at -78°C, and n-butyllithium (2.5 M, 247.21 μL) was added dropwise. The mixture was stirred at -78°C for 2 hours. Methanol (3.5 mL) was added dropwise to the reaction mixture to obtain a solution of A-2 in methanol, which was used immediately after preparation. ESI-LCMS: m/z = 249.3 $[M+H]^+$.

**Example 1**

Synthetic route:

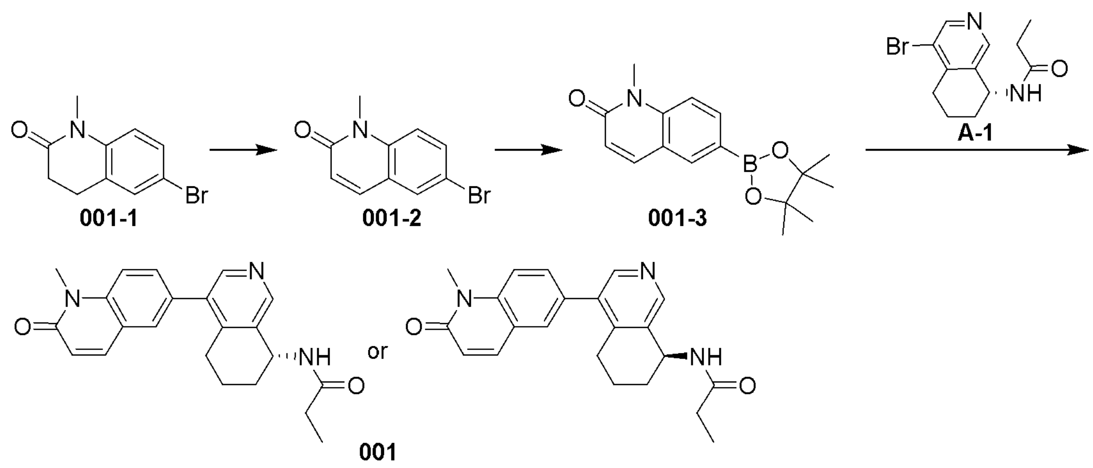

001-1 001-2 001-3 A-1

or

001

Step 1: synthesis of compound 001-2

To a reaction flask were added 001-1 (1 g, 4.16 mmol) and 1,2-dichloroethane (20 mL). After stirring to dissolve, N-bromosuccinimide (1.11 g, 6.25 mmol) and azobisisobutyronitrile (136.79 mg, 833.00 μmol) were added, and the reaction mixture was stirred at 80°C for 4 hours. 20 mL of water was added, and the resulting phases were separated. The aqueous phase was extracted three times with dichloromethane (20 mL each time). The organic phases were combined, dried over anhydrous sodium sulfate, filtered, and concentrated to obtain crude 001-2. $^1$H NMR (400 MHz, $CDCl_3$) δ ppm 7.73 (d, *J* = 2.26 Hz, 1 H), 7.68 (dd, *J* = 9.03, 2.26 Hz, 1 H), 7.62 (d, *J* = 9.54 Hz, 1 H), 7.30 (d, *J* = 2.51 Hz, 1 H), 6.78 (d, *J* = 9.54 Hz, 1 H), 3.73 (s, 3 H).

Step 2: synthesis of compound 001-3

001-2 (1.6 g, 6.72 mmol), bis(pinacolato)diboron (2.56 g, 10.08 mmol), potassium acetate (1.98 g, 20.16 mmol), and 1,4-dioxane (10 mL) were added to a reaction flask. After the atmosphere was purged with nitrogen, [1,1'-bis(diphenylphosphino)ferrocene]dichloropalladium(II) dichloromethane complex (274.41 mg, 336.02 μmol) was added. The reaction mixture was stirred at 80°C for 2 hours. 30 mL of ethyl acetate was added with stirring for dilution, and the mixture was filtered under reduced pressure. The filter cake was washed with 30 mL of ethyl acetate. The organic phase was collected and concentrated to obtain a crude product. The crude product was purified by column chromatography (petroleum ether:ethyl acetate = 0% - 20%) to obtain 001-3. ESI-LCMS: *m*/*z* = 286.1 $[M+H]^+$.

Step 3: synthesis of compound 001

To a reaction flask were added 001-3 (191.33 mg, 670.99 μmol), A-1 (190 mg, 670.99 μmol), and ethanol (2.5 mL). After they were dissolved, sodium carbonate (142.50 mg, 1.34 mmol) and water (0.5 mL) were added. After the atmosphere was purged with nitrogen, tetrakis(triphenylphosphine)palladium (38.77 mg, 33.55 μmol) was added. The reaction mixture was stirred at 80°C for 16 hours. The reaction was quenched by adding 20 mL of half-saturated brine and extracted three times with ethyl acetate (10 mL each time). The combined organic phases were washed with 20 mL of saturated brine, and the organic phase was collected, dried over anhydrous sodium sulfate, filtered, and concentrated to obtain a crude product. The crude product was purified by preparative thin-layer chromatography (dichloromethane/-methanol = 20:1) and then subjected to chiral resolution [chiral column: DAICEL CHIRALCEL OD (250mm*30mm, 10μm); mobile phase: [supercritical carbon dioxide-ethanol (0.1% ammonia water)]; ethanol (0.1% ammonia water): 45%, isocratic elution] to obtain compound 001. $^1$H NMR (400 MHz, $CDCl_3$) δ ppm 8.53 (s, 1 H), 8.31 (s, 1 H), 7.69 (d, *J*=9.29 Hz, 1 H), 7.43 - 7.52 (m, 3 H), 6.77 (d, *J*=9.54 Hz, 1 H), 5.84 (br d, *J*=8.53 Hz, 1 H), 5.32 - 5.40 (m, 1 H), 3.77 (s, 3 H), 2.55 - 2.71 (m, 2 H), 2.29 (q, *J*=7.53 Hz, 2 H), 2.04 - 2.15 (m, 1 H), 1.82 - 1.90 (m, 2 H), 1.23 (t, *J*=7.65 Hz, 3 H); ESI-LCMS: m/z = 362.0 $[M+H]^+$. SFC method: column: Chiralcel OD-3 150×4.6mm I.D., 3μm; mobile phase: A: $CO_2$ B: ethanol (0.05% diethylamine); gradient: B% = 5% to 40% (eluted for 4 min), 40% (eluted for 2 min), 5% (eluted for 2 min); flow rate: 2.5 mL/min; column temperature: 35°C; pressure: 1500 psi. *ee* = 99.52%, retention time: 4.686 minutes.

**Example 2**

Synthetic route:

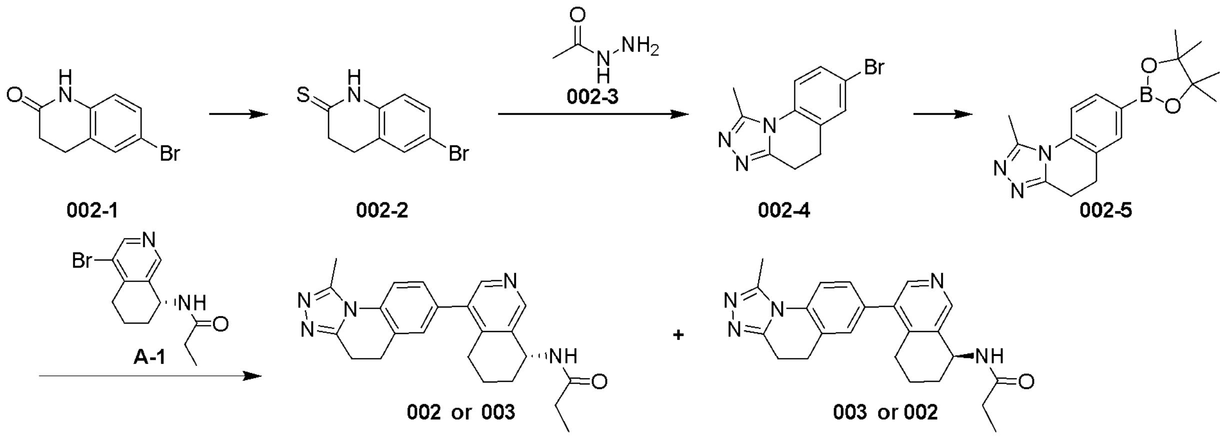

002-3

002-1 002-2 002-4 002-5

A-1

002 or 003 + 003 or 002

Step 1: synthesis of compound 002-2

To a reaction flask was added a solution of 002-1 (6 g, 26.54 mmol) in 1,4-dioxane (60 mL), followed by the addition of Lawesson's reagent (7.51 g, 18.58 mmol). The atmosphere was purged with nitrogen, and the mixture was heated to 110°C and stirred for 2 hours. The reaction mixture was concentrated, and 10 mL of dichloromethane was added. The mixture was then concentrated under reduced pressure to obtain a crude product. To the crude product was added 12 mL of dichloromethane. The mixture was stirred for 0.5 hours, and filtered to obtain 002-2. $^1$H NMR (400 MHz, $CDCl_3$) $\delta$ = 9.84 (br s, 1H), 7.39 - 7.31 (m, 2H), 6.81 - 6.71 (m, 1H), 3.14 - 3.05 (m, 2H), 2.93 - 2.83 (m, 2H).

Step 2: synthesis of compound 002-4

To a reaction flask were added 002-2 (540 mg, 2.23 mmol), 002-3 (198.26 mg, 2.68 mmol), and cyclohexanol (10.4 mL). The atmosphere was purged with nitrogen, and the mixture was heated to 120°C and stirred for 16 hours. To the reaction mixture were added 5 mL of water and 10 mL of ethyl acetate. The mixture was then extracted, and the resulting phases were separated. The organic phase was collected, and the aqueous phase was extracted three times with ethyl acetate (10 mL each time). The organic phases were combined and sequentially washed twice with saturated brine (10 mL each time). The organic phase was collected, dried over anhydrous sodium sulfate, filtered, and concentrated to obtain a crude product. The crude product was purified by column chromatography (petroleum ether : (ethyl acetate/methanol = 5/1) = 1:0 to 0:1) to obtain 002-4. $^1$H NMR (400 MHz, $CDCl_3$) $\delta$ = 7.57 - 7.48 (m, 2H), 7.37 (d, $J$ = 8.5 Hz, 1H), 3.19 - 3.10 (m, 2H), 3.05 - 2.98 (m, 2H), 2.77 (s, 3H).

Step 3: synthesis of compound 002-5

To a reaction flask were added 002-4 (475 mg, 1.80 mmol), bis(pinacolato)diboron (548.02 mg, 2.16 mmol), 1,4-dioxane (10 mL), potassium acetate (529.49 mg, 5.40 mmol), tris(dibenzylideneacetone)dipalladium (164.68 mg, 179.84 $\mu$mol), and tricyclohexylphosphine (100.87 mg, 359.68 $\mu$mol). After the atmosphere was purged with nitrogen, the mixture was heated to 80°C and stirred for 12 hours. To the reaction mixture were added 10 mL of saturated brine and 20 mL of ethyl acetate. The mixture was then extracted, and the resulting phases were separated. The organic phase was collected, and the aqueous phase was extracted three times with ethyl acetate (20 mL each time). The organic phases were combined and sequentially washed three times with saturated brine (20 mL each time). The organic phase was collected, dried over anhydrous sodium sulfate, filtered, and concentrated to obtain a crude product. The crude product was purified by column chromatography [petroleum ether : (ethyl acetate / methanol = 5/1) = 1:0 to 0:1] to obtain 002-5. $^1$H NMR (400 MHz,$CD_3OD$) $\delta$ = 7.84 - 7.78 (m, 2H), 7.68 (d, $J$ = 8.5 Hz, 1H), 3.12 - 3.03 (m, 4H), 2.78 (s, 3H), 1.36 (s, 12H); ESI-LCMS: $m/z$ = 312.2 $[M+H]^+$.

Step 4: synthesis of compounds 002 and 003

To a reaction flask were sequentially added 002-5 (56.92 mg, 127.13 μmol), A-1 (30 mg, 105.95 μmol), ethanol (1.6 mL), and a solution of sodium carbonate (12.35 mg, 116.54 μmol) in water (0.3 mL). The atmosphere was purged with nitrogen, followed by the addition of tetrakis(triphenylphosphine)palladium (3.67 mg, 3.18 μmol). The atmosphere was again purged with nitrogen, and the mixture was heated to 85°C and stirred for 12 hours. The reaction mixture was concentrated to obtain a crude product. The crude product was purified by preparative thin-layer chromatography (dichloromethane:methanol = 10:1) to obtain a crude product. It was then subjected to chiral resolution (chromatographic column: DAICEL CHIRALPAKAD (250mm*30mm, 10μm); mobile phase: [supercritical carbon dioxide-ethanol (0.1% ammonia water)]; ethanol (0.1% ammonia water): 30%, isocratic elution) to obtain 002 and 003. The *ee* value was determined by SFC (chromatographic column: Chiralpak AD-3, 150 × 4.6 mm I.D., 3 μm; mobile phase: A: supercritical $CO_2$, B: ethanol (0.2% 7M ammonia in methanol); gradient: A/B = 90/10 (0 min), 90/10 (0.5 min), 50/50 (3.5 min), 50/50 (4.5 min), 90/10 (5.0 min); flow rate: 2.5 mL/min; column temperature: 35°C; pressure: 2000 psi).

**Compound 002:** ESI-LCMS: *m/z* = 388.3 $[M+H]^+$; $^1H$ NMR (400 MHz, $CDCl_3$) δ = 8.55 (s, 1H), 8.32 (s, 1H), 7.57 (d, *J* = 9.0 Hz, 1H), 7.32 - 7.29 (m, 2H), 5.73 - 5.66 (m, 1H), 5.41 - 5.34 (m, 1H), 3.22 - 3.16 (m, 2H), 3.10 - 3.04 (m, 2H), 2.82 (s, 3H), 2.71 - 2.55 (m, 2H), 2.30 (q, *J* = 7.6 Hz, 2H), 2.10 (br t, *J* = 5.1 Hz, 1H), 1.90 - 1.78 (m, 3H), 1.24 (t, *J* = 7.6 Hz, 3H); *ee* = 100%, retention time: 3.778 minutes.

**Compound 003:** ESI-LCMS: *m/z* = 388.3 $[M+H]^+$; $^1H$ NMR (400 MHz, $CDCl_3$) δ = 8.55 (s, 1H), 8.32 (s, 1H), 7.57 (d, *J* = 9.0 Hz, 1H), 7.32 - 7.29 (m, 2H), 5.73 - 5.66 (m, 1H), 5.41 - 5.34 (m, 1H), 3.22 - 3.16 (m, 2H), 3.10 - 3.04 (m, 2H), 2.82 (s, 3H), 2.71 - 2.55 (m, 2H), 2.30 (q, *J* = 7.6 Hz, 2H), 2.10 (br t, *J* = 5.1 Hz, 1H), 1.90 - 1.78 (m, 3H), 1.24 (t, *J* = 7.6 Hz, 3H); *ee* = 99.86%, retention time: 4.024 minutes.

**Example 3**

Synthetic route:

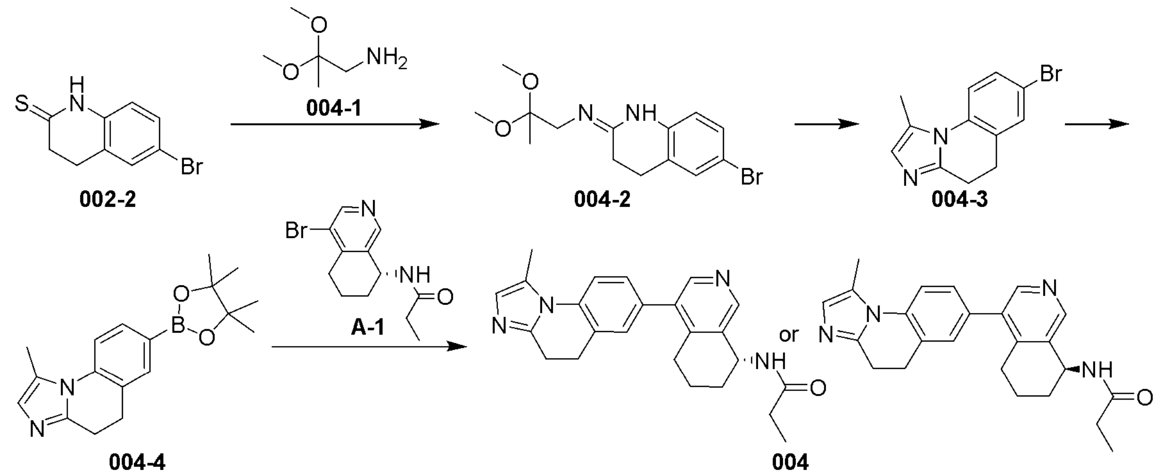
002-2
004-1
004-2
004-3
004-4
A-1
004
or

Step 1: synthesis of compound 004-2

To a reaction flask were added 002-2 (2 g, 8.26 mmol), 004-1 (1.48 g, 12.39 mmol), silver acetate (2.76 g, 16.52 mmol, 846.06 μL), and acetonitrile (20 mL). The mixture was stirred at 25°C for 12 hours. The reaction mixture was filtered, and the filter cake was rinsed with 10 mL of acetonitrile. The filtrate was collected and concentrated to obtain crude 004-2. $^1H$ NMR (400 MHz, $CDCl_3$) δ = 7.35 (br d, *J* = 8.3 Hz, 1H), 7.29 (br s, 1H), 7.13 (d, *J* = 8.4 Hz, 1H), 3.51 (s, 2H), 3.26 (s, 6H), 3.22 (s, 4H), 1.42 (s, 3H).

Step 2: synthesis of compound 004-3

To a reaction flask were added 004-2 (3 g, 9.17 mmol), hydrochloric acid (12 M, 15 mL), and water (5 mL), and the mixture was stirred at 80°C for 5 hours. A saturated aqueous sodium bicarbonate solution was added dropwise to the reaction mixture to adjust the pH to 7-8. The mixture was extracted three times with dichloromethane (10 mL each time)

and separated, and the organic phases were collected. The crude product was obtained by concentration. The crude product was purified by column chromatography (petroleum ether:ethyl acetate = 1:0~0:1) to obtain 004-3. $^1$H NMR (400 MHz, $CDCl_3$) $\delta$ = 7.47 (d, *J* = 2.0 Hz, 1H), 7.46 - 7.41 (m, 1H), 7.38 - 7.33 (m, 1H), 6.79 (s, 1H), 3.07 - 2.91 (m, 4H), 2.51 (s, 3H).

Step 3: synthesis of compound 004-4

To a reaction flask were added 004-3 (230 mg, 874.08 μmol), bis(pinacolato)diboron (443.93 mg, 1.75 mmol), potassium acetate (257.35 mg, 2.62 mmol), tricyclohexylphosphine (49.02 mg, 174.82 μmol), tris(dibenzylideneacetone) dipalladium (80.04 mg, 87.41 μmol), and 1,4-dioxane (7.5 mL). After the atmosphere was purged with nitrogen, the mixture was stirred at 80°C for 16 hours. The reaction mixture was added with water (10 mL) and ethyl acetate (5 mL), and filtered. The filtrate was extracted three times with ethyl acetate (5 mL each time), and the phases were separated. The organic phases were combined and washed with saturated brine (5 mL). The organic phase was collected, dried over anhydrous sodium sulfate, filtered and concentrated to obtain crude 004-4. ESI-LCMS: *m*/*z* = 311.4 [M+H]$^+$.

Step 4: synthesis of compound 004

To a reaction flask were sequentially added 004-4 (65.73 mg, 211.89 μmol), A-1 (40 mg, 141.26 μmol), ethanol (3.2 mL), and a solution of sodium carbonate (16.47 mg, 155.39 μmol) in water (0.52 mL). The atmosphere was purged with nitrogen, followed by the addition of tetrakis(triphenylphosphine)palladium (4.90 mg, 4.24 μmol). The atmosphere was again purged with nitrogen, and the mixture was stirred at 85°C for 12 hours. The reaction mixture was concentrated under reduced pressure to obtain a crude product. The crude product was initially separated by preparative thin-layer chromatography (ethyl acetate:methanol = 1:1), then subjected to chiral resolution (column: DAICEL CHIRALPAK AD (250mm*30 mm, 10 μm); mobile phase: [supercritical carbon dioxide-isopropanol (0.1% ammonia)]; isocratic elution: 30% isopropanol (0.1% ammonia)) to obtain compound 004.

ESI-LCMS: m/z = 387.3[M+H]$^+$; $^1$HNMR (400 MHz, $CDCl_3$) $\delta$ = 8.53 (s, 1H), 8.33 (s, 1H), 7.57 (br d, *J* = 8.5 Hz, 1H), 7.26 - 7.22 (m, 2H), 6.84 (br s, 1H), 5.79 - 5.65 (m, 1H), 5.41 - 5.31 (m, 1H), 3.14 - 2.99 (m, 4H), 2.76 - 2.62 (m, 2H), 2.58 (s, 3H), 2.29 (q, *J*= 7.2 Hz, 2H), 2.14 - 2.06 (m, 1H), 1.86 - 1.78 (m, 3H), 1.23 (br t, *J* = 7.5 Hz, 3H); SFC method: chromatographic column: Chiralpak AD-3, 150 × 4.6 mm I.D., 3 μm; mobile phase: A: $CO_2$, B: ethanol (0.2% 7M ammonia in methanol); gradient: A/B = 90/10 (0 min), 90/10 (0.5 min), 50/50 (3.5 min), 50/50 (4.5 min), 90/10 (5.0 min); flow rate: 2.5 mL/min; column temperature: 35°C; pressure: 2000 psi; *ee* = 100%; retention time: 4.144 minutes.

**Example 4**

Synthetic route:

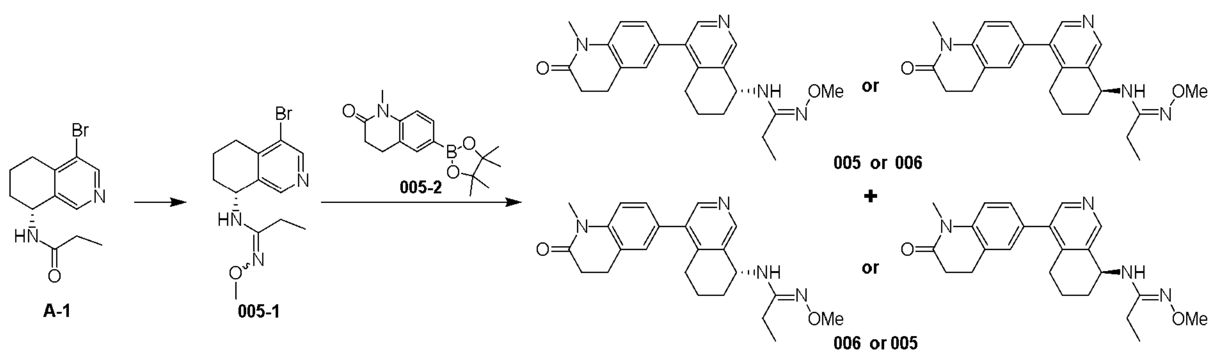


Step 1: synthesis of compound 005-1

To a reaction flask were added A-1 (0.1 g, 353.15 μmol), methoxyamine hydrochloride (38.34 mg, 459.1 μmol), phosphorus oxychloride (119.12 mg, 776.94 μmol), and toluene (1 mL). The mixture was stirred at 90°C for 10 hours. The reaction mixture was concentrated, and the concentrate was slowly poured into water (2 mL). A saturated aqueous sodium carbonate solution was added dropwise to adjust the pH to 9-10. The mixture was extracted three times with dichloromethane (2 mL each time). The organic phases were combined and washed with saturated brine (2 mL). The organic phase was collected, dried over anhydrous sodium sulfate, filtered, and concentrated to obtain 005-1. $^1$H NMR (400 MHz,

$CDCl_3$) δ ppm 8.56 (s, 1 H), 8.49 (s, 1 H), 5.31 (s, 1 H), 4.53 - 4.62 (m, 1 H), 3.75 (s, 3 H), 2.72 - 2.80 (m, 2 H), 2.38 (qd, *J* = 7.48, 3.69 Hz, 2 H), 1.97 - 2.07 (m, 2 H), 1.77 - 1.91 (m, 2 H), 1.28 (t, *J* = 7.50 Hz, 3 H).

Step 2: synthesis of compounds 005 and 006

To a reaction flask were sequentially added 005-1 (100 mg, 320.30 μmol), 005- 2 (110.37 mg, 384.36 μmol), ethanol (4.5 mL), water (1.5 mL), and sodium carbonate (37.34 mg, 352.33 μmol). The atmosphere was purged with nitrogen, followed by the addition of tetrakis(triphenylphosphine)palladium (11.10 mg, 9.61 μmol). The atmosphere was again purged with nitrogen, and the mixture was stirred at 85°C for 12 hours. The reaction mixture was filtered, and the filter cake was washed with dichloromethane (5 mL). The filtrate was collected and concentrated to obtain a crude product. The crude product was purified by preparative thin-layer chromatography (petroleum ether:ethyl acetate = 0:1), preparative high-performance liquid chromatography (column: Waters Xbridge BEH C18 100*30mm*10μm; mobile phase: [water (10 mM ammonium bicarbonate)-acetonitrile]; gradient: acetonitrile%: 20%-50%), and chiral resolution (column: DAICEL CHIRALPAK AD (250mm*30mm, 10μm); mobile phase: [A: supercritical carbon dioxide - B: isopropanol (0.1% ammonia)]; isocratic elution: 35% B) to obtain compounds 005 and 006. SFC method was subsequently performed: chromatographic column: Chiralpak AD-3, 50×4.6 mm I.D., 3 μm; mobile phase: A: $CO_2$, B: methanol [0.2% 7M ammonia in methanol]; gradient: A:B=60:40; flow rate: 4 mL/min; column temperature: 35°C; pressure: 1800 psi.

**Compound 005:** $^1H$ NMR (400 MHz, $CDCl_3$) δ ppm 8.62 (s, 1 H), 8.33 (s, 1 H), 7.14 - 7.25 (m, 1 H), 7.00 - 7.14 (m, 2 H), 5.34 (br d, *J* = 10.13 Hz, 1 H), 4.66 (br s, 1 H), 3.73 - 3.81 (m, 3 H), 3.41 (s, 3 H), 2.91 - 3.00 (m, 2 H), 2.72 (br t, *J* = 7.32 Hz, 3 H), 2.33 - 2.45 (m, 2 H), 2.11 (br s, 1 H), 1.89 (br d, *J* = 7.00 Hz, 1 H), 1.74 - 1.87 (m, 3 H), 1.30 (t, *J* = 7.44 Hz, 3 H). ESI-LCMS: *m/z* = 393.2 $[M+H]^+$; *ee* = 99.98%, retention time: 0.805 minutes.

**Compound 006:** $^1H$ NMR (400 MHz, $CDCl_3$) δ ppm 8.59 (br s, 1 H), 8.22 - 8.37 (m, 1 H), 7.17 - 7.21 (m, 1 H), 7.05 - 7.11 (m, 2 H), 5.34 (br d, *J* = 10.39 Hz, 1 H), 4.60 - 4.66 (m, 1 H), 3.76 (s, 3 H), 3.41 (s, 3 H), 2.96 (t, *J* = 7.42 Hz, 2 H), 2.69 - 2.75 (m, 3 H), 2.36 - 2.42 (m, 2 H), 2.08 (br dd, *J* = 6.99, 1.92 Hz, 1 H), 1.85 - 1.89 (m, 1 H), 1.74 - 1.83 (m, 3 H), 1.29 - 1.32 (m, 3 H); ESI-LCMS: *m/z* = 393.2 $[M+H]^+$; *ee* = 100%, retention time: 0.510 minutes.

**Example 5**

Synthetic route:

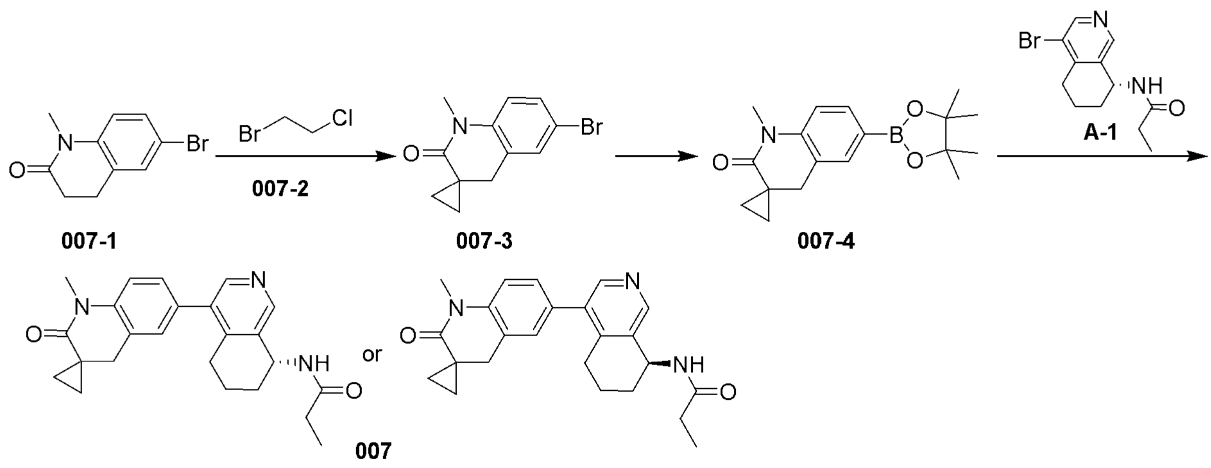

007-2

A-1

007-1 007-3 007-4

or

007

Step 1: synthesis of compound 007-3

To a reaction flask were added 007-1 (3 g, 12.49 mmol) and tetrahydrofuran (15 mL). The atmosphere was purged with nitrogen, and the mixture was cooled to 0°C. A solution of lithium bis(trimethylsilyl)amide (1 M, 14.99 mL) was added dropwise, and the mixture was stirred at 0°C for 1 hour. A solution of 007-2 (5.38 g, 37.48 mmol) in tetrahydrofuran (9 mL) was slowly added dropwise, and the mixture was stirred at 0°C for 1 hour. The mixture was then heated to 25°C and stirred for 1 hour. The reaction mixture was added with saturated aqueous ammonium chloride solution (30 mL), extracted with ethyl acetate three times (20 mL each). The organic phases were combined, washed with saturated brine (20 mL). The organic phase was collected, dried over anhydrous sodium sulfate, filtered, and concentrated to obtain a crude product. The crude product was purified by column chromatography (petroleum ether:ethyl acetate = 1:0 to 6:1) to obtain 007-3. $^1H$

NMR (400 MHz, $CDCl_3$) δ = 7.39 (dd, *J* = 2.2, 8.6 Hz, 1H), 7.25 - 7.21 (m, 1H), 6.85 (d, *J* = 8.6 Hz, 1H), 3.34 (s, 3H), 2.79 (s, 2H), 1.33 - 1.28 (m, 2H), 0.76 - 0.68 (m, 2H).

Step 2: synthesis of compound 007-4

To a reaction flask were sequentially added 007-3 (190 mg, 713.93 μmol), bis(pinacolato)diboron (271.94 mg, 1.07 mmol), 1,4-dioxane (4 mL), and potassium acetate (140.13 mg, 1.43 mmol). The atmosphere was purged with nitrogen, followed by the addition of [1,1'-bis(diphenylphosphino)ferrocene]dichloropalladium(II) dichloromethane complex (58.30 mg, 71.39 μmol). The mixture was stirred at 80°C for 16 hours. The reaction mixture was added with water (2 mL) and extracted three times with ethyl acetate (2 mL each time). The organic phases were combined and washed with saturated brine (2 mL). The organic phase was collected, dried over anhydrous sodium sulfate, filtered, and concentrated to obtain a crude product. The crude product was purified by preparative thin-layer chromatography (petroleum ether: ethyl acetate = 10:1) to obtain 007-4. $^1H$ NMR (400 MHz, $CDCl_3$) δ = 7.73 (d, *J* = 8.1 Hz, 1H), 7.55 (s, 1H), 6.99 (d, *J* = 8.1 Hz, 1H), 3.37 (s, 3H), 2.81 (s, 2H), 1.35 (s, 12H), 1.31 - 1.27 (m, 2H), 0.74 - 0.66 (m, 2H).

Step 3: synthesis of compound 007

To a reaction flask were sequentially added A-1 (100 mg, 353.15 μmol), 007-4 (132.73 mg, 423.78 μmol), ethanol (8.5 mL), and a solution of sodium carbonate (41.17 mg, 388.47 μmol) in water (1.42 mL). The atmosphere was purged with nitrogen, followed by the addition of tetrakis(triphenylphosphine)palladium (12.24 mg, 10.59 μmol). The mixture was stirred at 85°C for 2 hours. The reaction mixture was added with water (2 mL) and extracted three times with ethyl acetate (2 mL each time). The organic phases were combined and washed with saturated brine (2 mL). The organic phase was collected, dried over anhydrous sodium sulfate, filtered, and concentrated to obtain a crude product. The crude product was sequentially separated by preparative thin-layer chromatography (ethyl acetate:methanol = 10:1) and subjected to chiral resolution (chiral column: DAICEL CHIRALPAK AD (250 mm*30 mm, 10 μm); mobile phase: [supercritical carbon dioxide-isopropanol (0.1% ammonia)]; isocratic elution: 40% isopropanol (0.1% ammonia)) to obtain 007. $^1H$ NMR (400 MHz, $CDCl_3$) δ = 8.51 (s, 1H), 8.31 (s, 1H), 7.20 (dd, *J* = 1.6, 8.4 Hz, 1H), 7.08 - 7.01 (m, 2H), 5.85 (br d, *J* = 8.3 Hz, 1H), 5.39 - 5.29 (m, 1H), 3.40 (s, 3H), 2.86 (s, 2H), 2.69 (td, *J* = 6.0, 15.7 Hz, 2H), 2.30 (q, *J* = 7.5 Hz, 2H), 2.16 - 2.05 (m, 1H), 1.92 - 1.83 (m, 3H), 1.38 - 1.31 (m, 2H), 1.23 (t, *J* = 7.6 Hz, 3H), 0.79 - 0.72 (m, 2H). ESI-LCMS: m/z = 390.2 $[M+H]^+$. SFC method: chromatographic column: Chiralpak AD-3, 50×4.6mm I.D., 3 μm; mobile phase: A: $CO_2$, B: isopropanol [0.2% 7M ammonia in methanol]; gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi. *ee* = 100%, retention time: 1.465 minutes.

**Example 6**

Synthetic route:

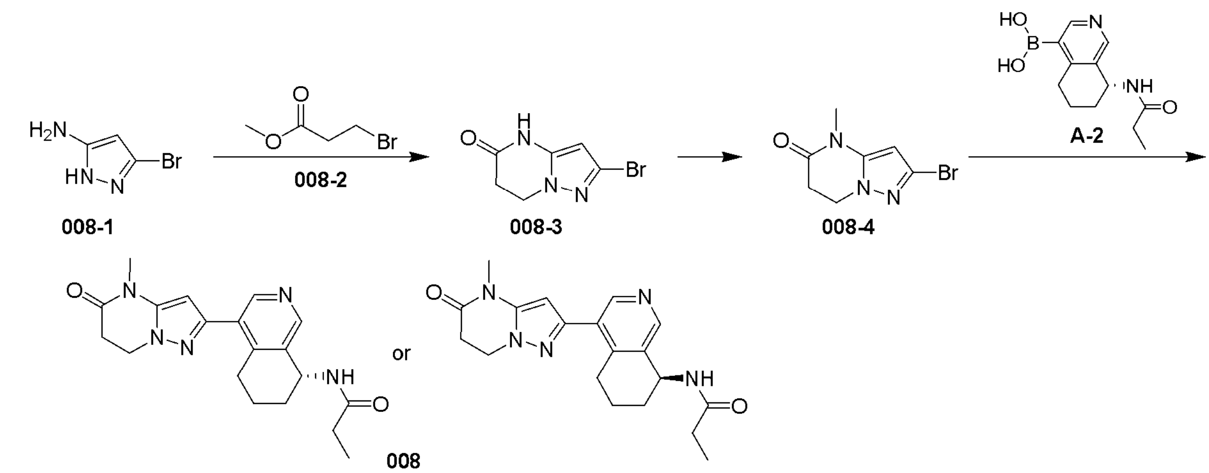


008-1 008-3 008-4

or

008

Step 1: synthesis of compound 008-3

To a reaction flask were sequentially added 008-1 (0.2 g, 1.23 mmol), *N,N*-dimethylformamide (4 mL), cesium

carbonate (1.09 g, 3.33 mmol), and potassium iodide (20.50 mg, 123.47 μmol). Under stirring, 008-2 (206.19 mg, 1.23 mmol) was added dropwise to the reaction flask. The atmosphere was purged with nitrogen, and the mixture was stirred at 20°C for 2 hours. The reaction mixture was added with water (5 mL) and extracted with ethyl acetate (5 mL). The aqueous phase was collected. The aqueous phase was adjusted to pH 3-4 by adding a citric acid aqueous solution, extracted three times with ethyl acetate (5 mL each time). The organic phases were combined and washed with saturated brine (5 mL). The organic phase was collected, dried over anhydrous sodium sulfate, filtered, and concentrated to obtain 008-3. $^1$H NMR (400 MHz, DMSO-$d_6$) δ = 11.02 - 10.68 (m, 1H), 5.68 (s, 1H), 4.20 (t, *J* = 7.1 Hz, 2H), 2.78 (t, *J* = 7.1 Hz, 2H).

Step 2: synthesis of compound 008-4

To a reaction flask were sequentially added 008-3 (180 mg, 833.20 μmol), *N,N*-dimethylformamide (3 mL), cesium carbonate (325.77 mg, 999.84 μmol), and methyl iodide (473.05 mg, 3.33 mmol). The atmosphere was purged with nitrogen and the mixture was stirred at 25°C for 12 hours. The reaction mixture was added with water (5 mL) and ethyl acetate (10 mL). The resulting phases were separated to collect the organic phase. The aqueous phase was extracted three times with ethyl acetate (30 mL each time). The organic phases were combined and washed twice with saturated brine (30 mL each time). The organic phase was collected, dried over anhydrous sodium sulfate, filtered, and concentrated to obtain compound 008-4. $^1$H NMR (400 MHz, $CDCl_3$) δ = 5.78 (s, 1H), 4.29 (t, *J* = 7.1 Hz, 2H), 3.24 (s, 3H), 2.94 (t, *J* = 7.1 Hz, 2H).

Step 3: synthesis of compound 008

To a reaction flask were sequentially added 008-4 (28.28 mg, 122.94 μmol), water (0.7 mL), acetonitrile (2.1 mL), methanol (0.7 mL), potassium carbonate (67.96 mg, 491.77 μmol), and 2-dicyclohexylphosphino-2',6'-dimethoxybiphenyl (7.57 mg, 18.44 μmol). The atmosphere was purged with nitrogen, followed by the addition of bis(triphenylphosphine) palladium(II) chloride (4.31 mg, 6.15 μmol) and palladium acetate (1.38 mg, 6.15 μmol). The atmosphere was purged with nitrogen, and a solution of crude A-2 (prepared immediately prior to use according to the synthesis method of Reference Example 2, added based on a theoretical yield of 61 mg, 245.88 μmol) was added dropwise at 40°C. The mixture was stirred at 75°C for 12 hours. The reaction mixture was filtered. The filtrate was collected, dried over anhydrous sodium sulfate, and concentrated to obtain a crude product. The crude product was sequentially purified by preparative thin-layer chromatography (dichloromethane:methanol = 3:1) and chiral resolution (chromatographic column: DAICEL CHIRALPAK AD (250mm*30mm, 10μm); mobile phase: [A: supercritical carbon dioxide - B: methanol]; isocratic elution: 45% methanol) to obtain 008.

$^1$H NMR (400 MHz, $CDCl_3$) δ = 8.62 - 8.57 (m, 1H), 8.53 - 8.48 (m, 1H), 5.93 (s, 1H), 5.85 - 5.75 (m, 1H), 5.39 - 5.27 (m, 1H), 4.40 (t, *J* = 7.1 Hz, 2H), 3.34 (s, 3H), 3.02 (t, *J* = 7.1 Hz, 3H), 2.30 (br d, *J* = 7.6 Hz, 2H), 2.22 - 2.00 (m, 2H), 1.87 (br s, 2H), 1.22 (br t, *J* = 6.8 Hz, 3H); ESI-LCMS: m/z 354.2 $[M+H]^+$. SFC method: chromatographic column: Chiralpak AD-3, 50×4.6mm I.D., 3 μm; mobile phase: A: supercritical $CO_2$, B: methanol [0.2% 7M ammonia in methanol]; gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi. *ee* = 99.02%, retention time: 1.532 minutes.

**Example 7**

Synthetic route:

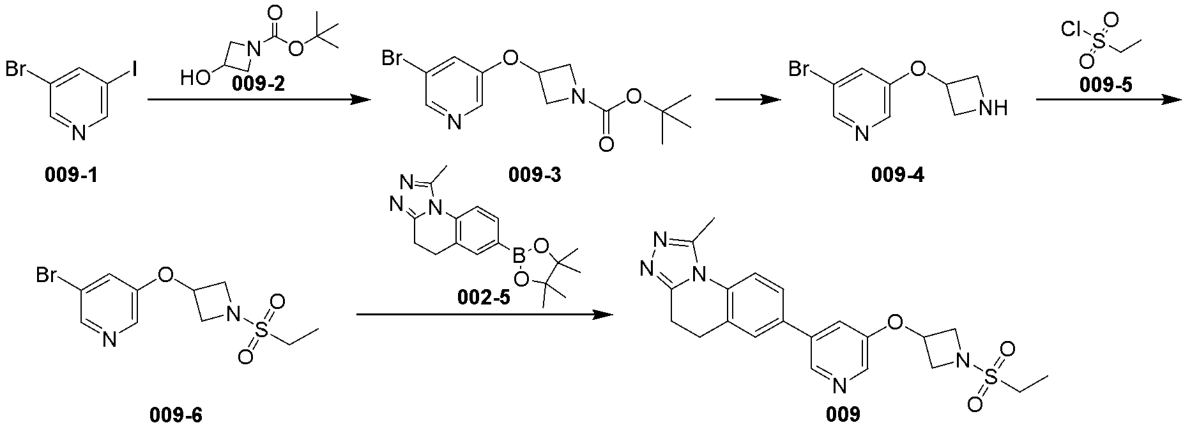


009-1 009-3 009-4 009-6 009

Step 1: synthesis of compound 009-3

To a reaction flask were sequentially added 009-1 (2 g, 7.04 mmol), 009-2 (2.44 g, 14.09 mmol), cesium carbonate (4.59 g, 14.09 mmol), toluene (20 mL), and 1,10-phenanthroline (253.91 mg, 1.41 mmol). The atmosphere was purged with nitrogen, followed by the addition of copper(I) iodide (134.17 mg, 704.49 μmol). The mixture was stirred at 110°C for 24 hours. The reaction mixture was added with 20 mL of water, extracted with ethyl acetate three times (50 mL each time). The organic phases were combined and washed with saturated brine three times (50 mL each time). The organic phase was collected, dried over anhydrous sodium sulfate, filtered, and concentrated to obtain a crude product. The crude product was purified by column chromatography (petroleum ether:ethyl acetate = 10:1 to 1:1) to obtain 009-3. $^1$H NMR (400 MHz, $CDCl_3$) δ = 8.35 (d, *J* = 1.8 Hz, 1H), 8.11 (d, *J* = 2.6 Hz, 1H), 7.23 (t, *J* = 2.2 Hz, 1H), 4.95 - 4.86 (m, 1H), 4.34 (ddd, *J* = 0.8, 6.4, 9.8 Hz, 2H), 4.02 (dd, *J* = 3.9, 10.4 Hz, 2H), 1.46 (s, 9H).

Step 2: synthesis of compound 009-4

To a reaction flask were sequentially added 009-3 (60 mg, 182.27 μmol), dichloromethane (1 mL), and trifluoroacetic acid (5.38 mmol, 0.4 mL). The mixture was stirred at 20°C for 1 hour. The reaction mixture was concentrated to obtain 009-4. $^1$H NMR (400 MHz, $CDCl_3$) δ = 8.56 - 8.33 (m, 2H), 8.04 (br s, 1H), 5.43 (br d, *J* = 1.3 Hz, 1H), 4.71 (br s, 2H), 4.37 (br s, 2H), 3.49 - 3.41 (m, 1H).

Step 3: synthesis of compound 009-6

To a reaction flask were sequentially added 009-4 (60 mg, 261.92 μmol), 009-5 (50.52 mg, 392.89 μmol), dichloromethane (1 mL), and N,N-diisopropylethylamine (67.70 mg, 523.85 μmol). The mixture was stirred at 25°C for 12 hours. The reaction mixture was added with water (1 mL) and dichloromethane (1 mL). The resulting phases were separated to collect the organic phase. The aqueous phase was extracted 3 times with ethyl acetate (1 mL each time). The organic phases were combined and washed twice with saturated brine (1 mL each time). The organic phase was collected, dried over anhydrous sodium sulfate, filtered, and concentrated to obtain a crude product. The crude product was purified by preparative thin-layer chromatography (petroleum ether: ethyl acetate = 1:1) to obtain 009-6. $^1$H NMR (400 MHz, DMSO-$d_6$) δ = 8.36 (d, *J* = 1.5 Hz, 1H), 8.27 (d, *J* = 2.4 Hz, 1H), 7.66 (t, *J* = 2.1 Hz, 1H), 5.23 - 5.13 (m, 1H), 4.33 (dd, *J* = 6.5, 9.7 Hz, 2H), 3.92 (dd, *J* = 4.5, 9.8 Hz, 2H), 3.22 - 3.14 (m, 2H), 1.23 (t, *J* = 7.3 Hz, 3H).

Step 4: synthesis of compound 009

To a reaction flask were sequentially added 002-5 (48.51 mg, 108.35 μmol), 009-6 (29 mg, 90.29 μmol), ethanol (2.4 mL), and a solution of sodium carbonate (10.53 mg, 99.32 μmol) in water (0.39 mL). The atmosphere was purged with nitrogen, followed by the addition of tetrakis(triphenylphosphine)palladium (3.13 mg, 2.71 μmol). The atmosphere was purged with nitrogen, and the mixture was stirred at 85°C for 12 hours. The reaction mixture was concentrated to obtain a crude product. The crude product was purified by preparative thin-layer chromatography (dichloromethane:methanol = 10:1) to obtain 009. $^1$H NMR (400 MHz, $CDCl_3$) δ = 8.54 (s, 1H), 8.18 (d, *J* = 2.3 Hz, 1H), 7.63 - 7.59 (m, 1H), 7.59 - 7.55 (m, 2H), 7.29 - 7.27 (m, 1H), 5.12 - 5.06 (m, 1H), 4.36 (dd, *J* = 6.5, 9.4 Hz, 2H), 4.19 (dd, *J* = 4.8, 9.4 Hz, 2H), 3.24 - 3.17 (m, 2H), 3.14 - 3.08 (m, 2H), 3.04 (q, *J* = 7.4 Hz, 2H), 2.83 (s, 3H), 1.40 (t, *J* = 7.4 Hz, 3H); ESI-LCMS: *m/z* = 426.1 $[M+H]^+$.

**Example 8**

Synthetic route:

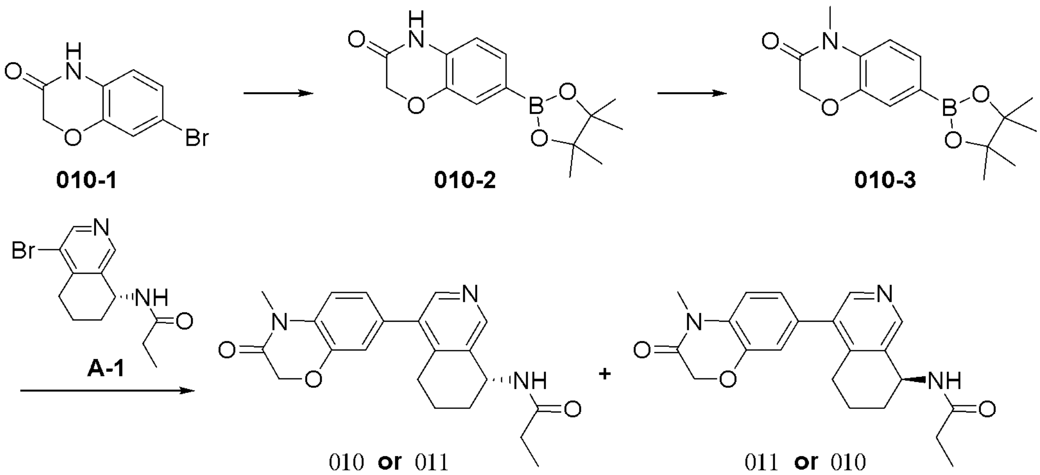

010-1 010-2 010-3

A-1

010 or 011 + 011 or 010

Step 1: synthesis of compound 010-2

To a reaction flask were sequentially added 010-1 (3 g, 13.16 mmol), bis(pinacolato)diboron (5.08 g, 20.02 mmol), potassium acetate (2.01 g, 20.45 mmol), and dioxane (90 mL). The atmosphere was purged with nitrogen, followed by the addition of [1,1'-bis(diphenylphosphino)ferrocene]dichloropalladium(II) dichloromethane complex (512.73 mg, 627.85 μmol). The mixture was stirred at 100°C for 4 hours. The reaction mixture was added with 50 mL of water and 50 mL of dichloromethane and extracted. The resulting phases were separated. The organic phase was collected, dried over anhydrous sodium sulfate and filtered. The filtrate was concentrated to obtain 010-2. ESI-LCMS: *m*/*z* = 276.1 $[M+H]^+$.

Step 2: synthesis of compound 010-3

To a reaction flask were sequentially added 010-2 (2.0 g, 7.27 mmol), *N,N*-dimethylformamide (15 mL), potassium carbonate (3.01 g, 21.81 mmol), and methyl iodide (1.50 g, 10.54 mmol). The mixture was stirred at 25°C for 16 hours. The reaction mixture was added with 10 mL of water and 15 mL of dichloromethane and extracted. The organic phase was collected, dried over anhydrous sodium sulfate and filtered. The filtrate was concentrated to obtain 010-3. ESI-LCMS: *m*/*z* = 290.1 $[M+H]^+$.

Step 3: synthesis of compounds 010 and 011

To a reaction flask were sequentially added a crude product of 010-3 (0.1 g, 345.86 μmol), A-1 (97.94 mg, 345.86 μmol), ethanol (2 mL), water (0.4 mL), and sodium carbonate (36.66 mg, 345.86 μmol). The atmosphere was purged with nitrogen, followed by the addition of tetrakis(triphenylphosphine)palladium (19.98 mg, 17.29 μmol). The mixture was stirred at 80°C for 2 hours. The reaction mixture was added with 5 mL of water and 5 mL of dichloromethane and extracted. The resulting phases were separated. The organic phase was collected, dried over anhydrous sodium sulfate, filtered, and concentrated to obtain a crude product. The crude product was subjected to preparative thin-layer chromatography (dichloromethane:methanol = 20:1) and chiral resolution (chromatographic column: DAICEL CHIRALPAK AD (250 mm*30 mm, 10 μm); mobile phase: [supercritical carbon dioxide-ethanol (0.1% ammonia water)]; isocratic elution: 45% ethanol (0.1% ammonia water)) to obtain 010 and 011. The *ee* value was determined by SFC (chromatographic column: Chiralpak AD-3 (50×4.6 mm I.D., 3 μm); mobile phase: A: supercritical carbon dioxide, B: ethanol (0.05% diethylamine); gradient: 40% B; flow rate: 4 mL/min; column temperature: 35°C; pressure: 1500 psi).

**Compound 010:** $^1H$ NMR (400MHz, $CDCl_3$) δ = 8.43 (s, 1H), 8.21 (s, 1H), 7.03 - 6.93 (m, 1H), 6.92 - 6.82 (m, 2H), 5.65 (br d, *J*=8.5 Hz, 1H), 5.39 - 5.11 (m, 1H), 4.61 (s, 2H), 3.34 (s, 3H), 2.69 - 2.47 (m, 2H), 2.29 - 2.14 (q, *J*=7.7 Hz, 2H), 2.06 - 1.92 (m, 1H), 1.84 - 1.69 (m, 3H), 1.19 - 1.07 (t, *J*=7.7 Hz, 3H); ESI-LCMS: *m*/*z* = 366.1 $[M+H]^+$; *ee* = 96.72%, retention time: 0.582 minutes.

**Compound 011:** $^1H$ NMR (400MHz, $CDCl_3$) δ = 8.41 (br s, 1H),8.17 (br s, 1H), 7.04 - 6.70 (m, 3H), 5.87 (br s, 1H), 5.26 (br s, 1H), 4.59 (br s, 2H), 3.34 ( s, 3H), 2.56 (br s, 2H), 2.20 (br s, 2H), 1.99 (br s, 4H), 1.14 (br s, 3H); ESI-LCMS: *m*/*z* = 366.1 $[M+H]^+$; *ee* = 99.98%, retention time: 2.351 minutes.

**Example 9**

Synthetic route:

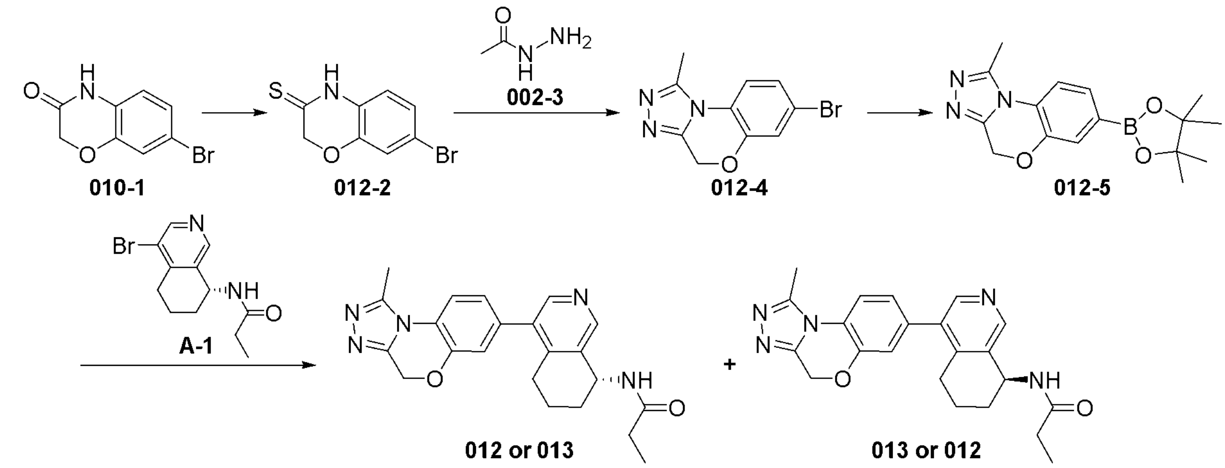

010-1 012-2 012-4 012-5

012 or 013 013 or 012

Step 1: synthesis of compound 012-2

To a reaction flask were sequentially added 010-1 (2.0 g, 8.77 mmol) and toluene (100 mL). The atmosphere was purged with nitrogen, followed by the addition of Lawesson's reagent (7.09 g, 17.54 mmol). The mixture was stirred at 110°C for 16 hours. The reaction mixture was added with 50 mL of water and 50 mL of ethyl acetate and extracted. The resulting phases were separated. The organic phase was collected, dried over anhydrous sodium sulfate, filtered, and concentrated to obtain a crude product. It was purified by column chromatography (petroleum ether:ethyl acetate = 4:1) to obtain 012-2. $^1$H NMR (400MHz, $CDCl_3$) $\delta$ = 9.61 (br s, 1H), 7.19 (s, 1H), 7.13 (dd, *J*=1.9, 8.4 Hz, 1H), 6.77 (d, J=8.5 Hz, 1H), 4.90 (s, 2H).

Step 2: synthesis of compound 012-4

To a reaction flask were sequentially added 012-2 (1.0 g, 4.10 mmol), 002-3 (364.17 mg, 4.92 mmol), and cyclohexanol (5 mL). The mixture was stirred at 120°C for 16 hours. The reaction mixture was concentrated to obtain a crude product. The crude product was purified by column chromatography (dichloromethane:methanol = 20:1) to obtain 012-4.

Step 3: synthesis of compound 012-5

To a reaction flask were sequentially added 012-4 (0.3 g, 1.13 mmol), bis(pinacolato)diboron (429.44 mg, 1.69 mmol), and 1,4-dioxane (10 mL). The atmosphere was purged with nitrogen, followed by the addition of potassium acetate (171.50 mg, 1.75 mmol) and [1,1'-bis(diphenylphosphino)ferrocene]dichloropalladium(II) dichloromethane complex (46.03 mg, 56.37 $\mu$mol). The mixture was stirred at 100°C for 16 hours. The reaction mixture was concentrated to obtain a crude product. The crude product was purified by preparative thin-layer chromatography (dichloromethane:methanol = 20:1) to obtain 012-5. ESI-LCMS: *m/z* = 314.1 $[M+H]^+$.

Step 4: synthesis of compounds 012 and 013

To the reaction mixture were sequentially added 012-5 (0.1 g, 319.33 $\mu$mol), A-1 (90.42 mg, 319.33 $\mu$mol), ethanol (5 mL), water (1 mL), and sodium carbonate (33.85 mg, 319.33 $\mu$mol). The atmosphere was purged with nitrogen, followed by the addition of tetrakis(triphenylphosphine)palladium (18.45 mg, 15.97 $\mu$mol). The mixture was stirred at 80°C for 1 hour. The reaction mixture was concentrated, then added with water (50 mL) and dichloromethane (50 mL), and extracted. The resulting phases were separated. The organic phase was dried over anhydrous sodium sulfate, filtered, and concentrated to obtain a crude product. The crude product was sequentially purified by preparative thin-layer chromatography (dichloromethane:methanol = 20:1) and chiral resolution (chromatographic column: DAICEL CHIRALPAK IF (250 mm*30 mm, 10 $\mu$m); mobile phase: [A: n-hexane, B: isopropanol/acetonitrile = 4:1]; 100% B isocratic

elution) to obtain 012 and 013. The *ee* value was determined by SFC (chromatographic column: Chiralpak IF 100*4.6 mm 3μm; mobile phase: A: n-hexane (0.1% diethylamine), B: isopropanol/acetonitrile = 2/1; elution gradient: A/B = 20/8; flow rate: 1 mL/min; column temperature: 35°C).

**Compound 012:** ESI-LCMS: m/z = 390.1 [M+H]$^+$; $^1$H NMR (400 MHz, $CDCl_3$) δ = 8.46 (s, 1H), 8.22 (s, 1H), 7.53 (d, *J* = 8.3 Hz, 1H), 7.06 (d, *J* = 1.8 Hz, 1H), 7.00 (dd, *J* = 2.0, 8.3 Hz, 1H), 5.77 (br d, *J* = 8.5 Hz, 1H), 5.28 (s, 3H), 2.77 (m, 3H), 2.65 - 2.44 (m, 2H), 2.22 (q, *J* = 7.7 Hz, 2H), 2.08 - 1.96 (m, 1H), 1.84 - 1.76 (m, 3H), 1.15 (t, *J* = 7.5 Hz, 3H); *ee* = 99.87%, retention time: 3.245 minutes.

**Compound 013:** $^1$H NMR (400MHz, $CDCl_3$) δ = 8.48 (s, 1H), 8.24 (s, 1H), 7.53 (d, *J*=8.3 Hz, 1H), 7.07 (d, *J*=1.8 Hz, 1H), 7.03 - 6.94 (m, 1H), 5.65 (br d, *J*=8.0 Hz, 1H), 5.29 (s, 3H), 2.77 (s, 3H), 2.68 - 2.47 (m, 2H), 2.22 (q, *J*=7.5 Hz, 2H), 2.09 - 1.89 (m, 1H), 1.85 - 1.69 (m, 3H), 0.77 (br s, 3H); ESI-LCMS : m/z = 390.1 [M+H]$^+$; *ee* = 98.44%, retention time: 2.328 minutes.

**Example 10**

Synthetic route:

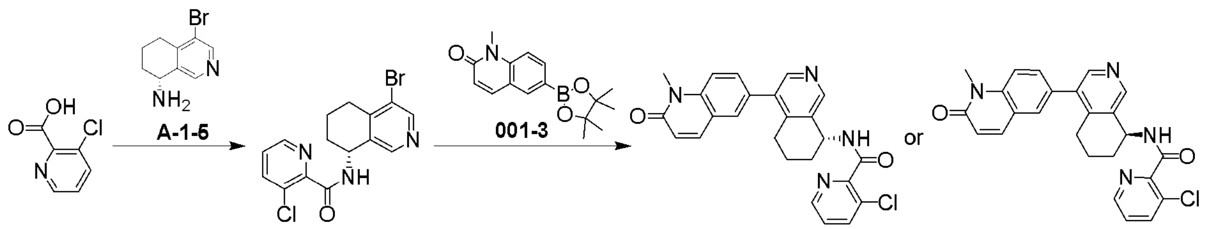


014-1 014-2 014

Step 1: synthesis of compound 014-2

To a reaction flask were added 014-1 (120.00 mg, 761.64 μmol), *N,N*-dimethylformamide (4 mL), *N,N*-diisopropylethylamine (196.15 mg, 1.52 mmol), and 2-(7-azabenzotriazol-1-yl)-*N,N,N',N'*-tetramethyluronium hexafluorophosphate (577.06 mg, 1.52 mmol). After stirring at 25°C for 5 minutes, the mixture was further added with the hydrochloride of A-1-5 (200 mg) and stirred at 25°C for 16 hours. 25 mL of water was added to the reaction mixture, which was then transferred to a separatory funnel and extracted three times with 20 mL of ethyl acetate. The organic phases were combined and washed with saturated brine. The organic phase was collected, dried over anhydrous sodium sulfate, filtered, and concentrated to obtain a crude product. The crude product was purified by preparative thin-layer chromatography (petroleum ether:ethyl acetate = 1:1) to obtain 014-2. $^1$H NMR (400 MHz, $CDCl_3$) δ ppm 8.58 (s, 1 H), 8.53 (s, 1 H), 8.43 (d, *J* = 3.26 Hz, 1 H), 8.18 (br s, 1 H), 7.86 (d, *J* = 8.28 Hz, 1 H), 7.39 (dd, *J* = 8.16, 4.39 Hz, 1 H), 5.40 - 5.48 (m, 1 H), 2.72 - 2.92 (m, 2 H), 1.95 - 2.17 (m, 4 H).

Step 2: synthesis of compound 014

To a reaction flask were sequentially added 014-2 (204 mg, 556.40 μmol), 001-3 (317.32 mg, 1112.8 μmol), ethanol (10 mL), sodium carbonate (117.95 mg, 1.11 mmol), and water (2 mL). The atmosphere was purged with nitrogen, followed by the addition of tetrakis(triphenylphosphine)palladium (64.3 mg, 55.64 μmol). The reaction mixture was stirred at 80°C for 20 hours. The reaction mixture was added with 40 mL of half-saturated brine, then transferred to a separatory funnel, and extracted three times with ethyl acetate (20 mL each time). The organic phases were combined and washed with 40 mL of saturated brine. The organic phase was collected, dried over anhydrous sodium sulfate, filtered, and concentrated to obtain a crude product. The crude product was subjected sequentially to preparative thin-layer chromatography (dichloromethane:methanol = 20:1) and chiral resolution [chromatographic column: DAICEL CHIRALCEL OJ (250 mm*30 mm, 10 μm); mobile phase: [supercritical carbon dioxide-ethanol (0.1% ammonia water)]; isocratic elution: 30% ethanol (0.1% ammonia water)] to obtain 014, which was subsequently analyzed by SFC (chromatographic column: Chiralcel OJ-3 50*4.6 mm I.D., 3 μm; mobile phase: A: supercritical $CO_2$, B: ethanol (0.05% diethylamine); gradient (B%): 5% to 40% (0 to 2 min), 40% (held for 1.2 min), 5% (held for 0.8 min); flow rate: 3 mL/min; column temperature: 35°C; column pressure: 1500 psi): *ee* = 100%, retention time 1.834 minutes. ESI-LCMS: *m*/*z* = 445.0 [M+H]$^+$; $^1$H NMR (400 MHz, $CDCl_3$) δ ppm 8.64 (s, 1 H), 8.44 (dd, *J* =4.52, 1.26 Hz, 1 H), 8.35 (s, 1 H), 8.21 (br d, *J* = 8.53 Hz, 1 H), 7.87 (dd, *J* = 8.16, 1.38 Hz, 1 H), 7.71 (d, *J* = 9.54 Hz, 1 H), 7.50 - 7.55 (m, 2 H), 7.44 - 7.50 (m, 1 H), 7.40

(dd, $J$ = 8.03, 4.52 Hz, 1 H), 6.79 (d, $J$ = 9.54 Hz, 1 H), 5.49 - 5.56 (m, 1 H), 3.78 (s, 3 H), 2.60 - 2.77 (m, 2 H), 2.16 - 2.25 (m, 1 H), 1.99 - 2.08 (m, 1 H), 1.82 - 1.93 (m, 2 H).

**Example 11**

Synthetic route:

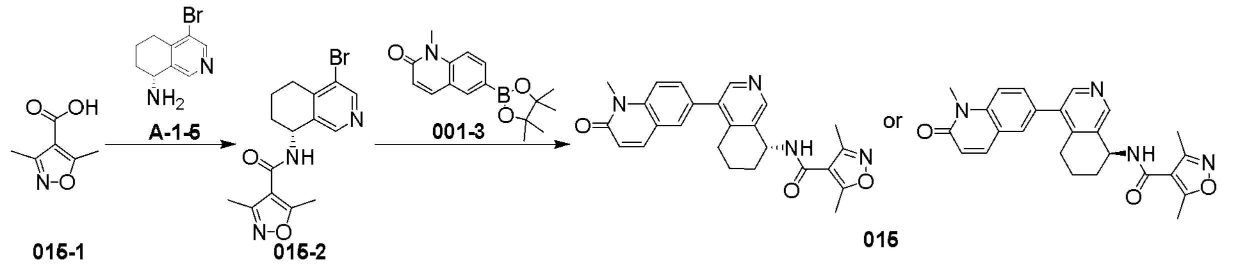


015-1 015-2 015

Step 1: synthesis of compound 015-2

To a reaction flask were sequentially added 015-1 (192.76 mg, 1.37 mmol), *N,N*-dimethylformamide (4 mL), *N,N*-diisopropylethylamine (514.89 mg, 3.98 mmol), 1-hydroxybenzotriazole (76.90 mg, 569.13 μmol), and 1-(3-dimethylaminopropyl)-3-ethylcarbodiimide hydrochloride (109.10 mg, 569.13 μmol). The mixture was stirred at 25°C for 5 minutes, followed by the addition of the hydrochloride salt of A-1-5 (300 mg). The reaction mixture was then stirred at 25°C for 16 hours. The reaction mixture was added with 25 mL of water, then transferred to a separatory funnel, and extracted three times with 20 mL of ethyl acetate. The organic phases were combined and washed with half-saturated brine and saturated brine (40 mL each time). The organic phase was collected, dried over anhydrous sodium sulfate, filtered, and concentrated to obtain a crude product. The crude product was purified by preparative thin-layer chromatography (petroleum ether:ethyl acetate = 1:1) to obtain 015-2.

Step 2: synthesis of compound 015

To a reaction flask were sequentially added 015-2 (124 mg, 354.07 μmol), 001-3 (201.92 mg, 708.15 μmol), ethanol (5 mL), sodium carbonate (112.58 mg, 1.06 mmol), and water (1 mL). The atmosphere was purged with nitrogen, followed by the addition of tetrakis(triphenylphosphine)palladium (40.92 mg, 35.41 μmol). The reaction mixture was stirred at 85°C for 16 hours. The reaction mixture was added with 20 mL of half-saturated brine, then transferred to a separatory funnel, and extracted three times with ethyl acetate (10 mL each time). The organic phases were combined and washed with 20 mL of saturated brine. The organic phase was collected, dried over anhydrous sodium sulfate, filtered, and concentrated to obtain a crude product. The crude product was purified by preparative thin-layer chromatography (dichloromethane:methanol = 20:1) and subjected to chiral resolution [chromatographic column: ChiralPak IH, 250*30 mm, 10 μm; mobile phase: [supercritical carbon dioxide-ethanol (0.1% ammonia water)]; isocratic elution: 45% ethanol (0.1% ammonia water)] to obtain 015, which was subsequently analyzed by SFC (chromatographic column: Chiralpak AS-3 50*4.6 mm I.D., 3 μm; mobile phase: A: $CO_2$, B: ethanol (0.05% diethylamine); gradient (B%): 5% to 40% (0 to 2 min), 40% (held for 1.2 min), 5% (held for 0.8 min); flow rate: 3 mL/min; column temperature: 35°C; column pressure: 1500 psi): *ee* = 100%, retention time 2.383 minutes. ESI-LCMS: $m/z$ = 429.1 $[M+H]^+$; $^1H$ NMR (400 MHz, $CDCl_3$) δ ppm 8.60 (s, 1 H), 8.34 (s, 1 H), 7.69 (d, $J$ = 9.54 Hz, 1 H), 7.42 - 7.56 (m, 3 H), 6.75 (d, $J$ = 9.54 Hz, 1 H), 6.02 (br d, $J$ = 8.28 Hz, 1 H), 5.46 - 5.57 (m, 1 H), 3.76 (s, 3 H), 2.58 - 2.74 (m, 5 H), 2.43 (s, 3 H), 2.15 - 2.25 (m, 1 H), 1.83 - 2.01 (m, 3 H).

**Example 12**

Synthetic route:

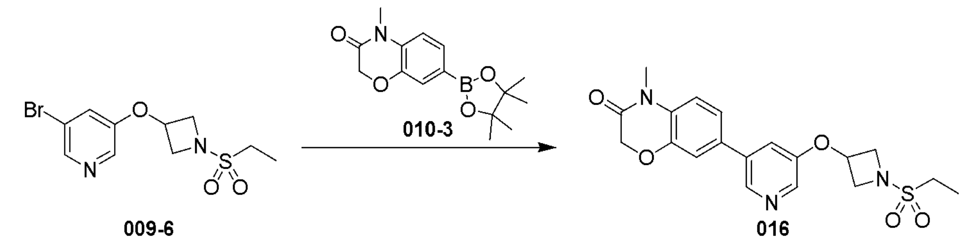

009-6
016

Step 1: synthesis of compound 016

To a reaction flask were sequentially added 009-6 (217 mg, 675.61 μmol), 010-3 (195.34 mg, 675.61 μmol), ethanol (10 mL), sodium carbonate (143.22 mg, 1.35 mmol), and water (2 mL). The atmosphere was purged with nitrogen, followed by the addition of tetrakis(triphenylphosphine)palladium (39.04 mg, 33.78 μmol). The reaction mixture was stirred at 80°C for 16 hours. The reaction mixture was added with 40 mL of half-saturated brine, then transferred to a separatory funnel, and extracted three times with ethyl acetate (20 mL each time). The organic phases were combined and washed with 40 mL of saturated brine. The organic phase was collected, dried over anhydrous sodium sulfate, filtered, and concentrated to obtain a crude product. The crude product was sequentially purified by preparative thin-layer chromatography (dichloromethane:methanol = 20:1) and preparative high-performance liquid chromatography [column: C18 100×40 mm; mobile phase: [water (0.075% trifluoroacetic acid)-acetonitrile]; gradient: 7% to 37% acetonitrile elution] to obtain 016. ESI-LCMS: m/z = 404.0 $[M+H]^+$. $^1H$ NMR (400 MHz, $CDCl_3$) δ ppm 8.55 (s, 1 H), 8.18 (d, *J* = 2.26 Hz, 1 H), 7.22 - 7.35 (m, 3 H), 7.13 (d, *J* = 8.28 Hz, 1 H), 5.11 (quin, *J* = 5.58 Hz, 1 H), 4.73 (s, 2 H), 4.34 - 4.45 (m, 2 H), 4.22 (dd, *J* = 9.16, 4.64 Hz, 2 H), 3.47 (s, 3 H), 3.09 (q, *J* = 7.45 Hz, 2 H), 1.45 (t, *J* = 7.40 Hz, 3 H).

**Example 13**

Synthetic route:

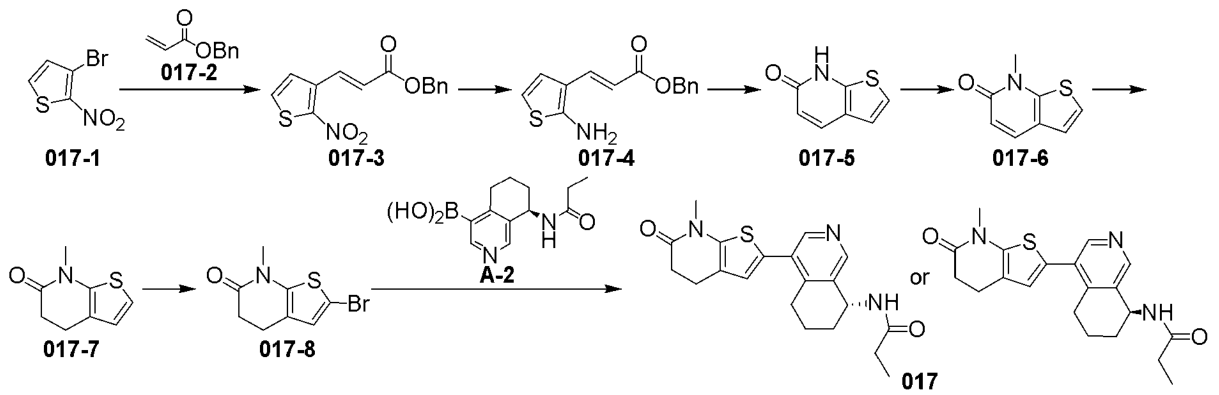

017-1, 017-3, 017-4, 017-5, 017-6
017-7, 017-8
or
017

Step 1: synthesis of compound 017-3

To a reaction flask were sequentially added palladium acetate (480.24 mg, 2.14 mmol), 2-ditert-butylphosphino-2',4',6'-triisopropylbiphenyl (2.04 g, 4.28 mmol), and N,N-dimethylformamide (89 mL). The atmosphere was purged with nitrogen, followed by the addition of 017-1 (8.9 g, 42.78 mmol), triethylamine (34.63 g, 342.25 mmol, 47.64 mL), and 017-2 (41.63 g, 256.69 mmol). The atmosphere was again purged with nitrogen, and the reaction mixture was stirred at 80°C for 12 hours. The reaction mixture was added with 10 mL of water, transferred to a separatory funnel, and extracted with 20 mL of ethyl acetate. The resulting phases were separated. The aqueous phase was further extracted three times with ethyl acetate, 30 mL each time. The organic phases were combined and washed twice with brine, 30 mL each time. The organic phase was collected, dried over anhydrous sodium sulfate, filtered, and concentrated to obtain a crude product. The crude product was purified by column chromatography (petroleum ether:ethyl acetate = 10:1 to 1:1) to obtain 017-3. $^1H$ NMR (400 MHz, $CDCl_3$) δ = 8.49 (d, *J* = 16.1 Hz, 1H), 7.48 (d, *J* = 5.6 Hz, 1H), 7.46 - 7.34 (m, 5H), 7.29 (d, *J*= 5.7 Hz, 1H), 6.52 (d, *J*= 16.1 Hz, 1H), 5.29 (s, 2H).

Step 2: synthesis of compound 017-4

To a reaction flask were sequentially added 017-3 (5 g, 17.28 mmol), methanol (175 mL), ammonium chloride (1.02 g, 19.01 mmol), and water (50 mL). The atmosphere was purged with nitrogen, followed by the slow addition of iron powder (4.83 g, 86.41 mmol). The reaction mixture was stirred at 60°C for 2 hours. The reaction mixture was cooled to room temperature, and added with water (100 mL), then transferred to a separatory funnel, and extracted with 100 mL of dichloromethane. The resulting phases were separated. The organic phase was collected, and the aqueous phase was extracted three times with dichloromethane (100 mL each time). The organic phases were combined, washed twice with saturated brine (100 mL each time), dried over anhydrous sodium sulfate, filtered, and concentrated to obtain crude product 017-4, which was used directly in the next step without further purification. ESI-LCMS: *m*/*z* = 260.1 $[M+H]^+$.

Step 3: synthesis of compound 017-5

To a reaction flask were sequentially added 017-4 (4 g, 15.42 mmol), tributylphosphine (9.36 g, 46.27 mmol), and methanol (70 mL). The atmosphere was purged with nitrogen, and the reaction mixture was stirred at 70°C for 2 hours. The reaction mixture was concentrated to obtain a crude product. The crude product was purified by column chromatography (petroleum ether:ethyl acetate = 10:1 to 1:1) to obtain 017-5. $^1H$ NMR (400 MHz, $CDCl_3$) $\delta$ = 7.88 (d, *J* = 9.3 Hz, 1H), 7.11 - 7.08 (m, 1H), 7.07 - 7.04 (m, 1H), 6.68 (d, *J* = 9.3 Hz, 1H). ESI-LCMS: m/z = 152.1 $[M+H]^+$.

Step 4: synthesis of compound 017-6

To a reaction flask were sequentially added 017-5 (1 g, 6.61 mmol), *N,N*-dimethylformamide (20 mL), cesium carbonate (2.59 g, 7.94 mmol), and methyl iodide (3.76 g, 26.46 mmol). The atmosphere was purged with nitrogen and the mixture was stirred at 25°C for 2 hours. The reaction mixture was added with water (30 mL), then transferred to a separatory funnel, and extracted with ethyl acetate (30 mL). The resulting phases were separated. The organic phase was collected, and the aqueous phase was extracted three times with ethyl acetate (30 mL each time). The organic phases were combined and washed twice with saturated brine (30 mL each time). The organic phase was collected, dried over anhydrous sodium sulfate, filtered, and concentrated to obtain a crude product. The crude product was purified by column chromatography (petroleum ether:ethyl acetate = 10:1 to 1:1) to obtain 017-6. $^1H$ NMR (400 MHz, $CD_3OD$) $\delta$ = 7.91 (d, *J* = 9.3 Hz, 1H), 7.22 (q, *J* = 5.6 Hz, 2H), 6.53 (d, *J* = 9.3 Hz, 1H), 3.72 (s, 3H).

Step 5: synthesis of compound 017-7

To a reaction flask were sequentially added 017-6 (30 mg, 181.58 $\mu$mol) and tetrahydrofuran (0.5 mL). The atmosphere was purged with nitrogen three times, and the mixture was cooled to -78°C before lithium tris(2-butyl) borohydride (1 M in tetrahydrofuran, 297.80 $\mu$L) was added dropwise. The reaction mixture was stirred at -78°C for 1 hour, then slowly warmed to 25°C and stirred for 12 hours. The reaction mixture was added with water (1 mL), then transferred to a separatory funnel, and extracted with 1 mL of dichloromethane. The resulting phases were separated. The aqueous phase was extracted three times with dichloromethane (1 mL each time). The organic phases were combined and washed twice with saturated brine (1 mL each time). The organic phase was collected, dried over anhydrous sodium sulfate, filtered, and concentrated to obtain a crude product. The crude product was purified by preparative thin-layer chromatography (petroleum ether:ethyl acetate = 1:1) to obtain 017-7. $^1H$ NMR (400 MHz, $CDCl_3$) $\delta$ = 6.80 - 6.77 (m, 1H), 6.76 - 6.73 (m, 1H), 3.32 (d, *J* = 2.3 Hz, 3H), 2.87 - 2.81 (m, 2H), 2.75 - 2.67 (m, 2H).

Step 6: synthesis of compound 017-8

To a reaction flask were sequentially added 017-7 (10 mg, 59.80 $\mu$mol) and dichloromethane (0.2 mL). The atmosphere was purged with nitrogen, and the reaction mixture was cooled to 0°C, followed by the addition of *N*-Bromosuccinimide (11.71 mg, 65.78 $\mu$mol). The reaction mixture was stirred at 0°C for 0.5 hours. The reaction mixture was added with water (1 mL), then transferred to a separatory funnel, and extracted with 1 mL of dichloromethane. The resulting phases were separated, and the organic phase was collected. The aqueous phase was extracted three times with dichloromethane (1 mL each time). The organic phases were combined, washed twice with saturated brine (1 mL each time). The organic phase was collected, dried over anhydrous sodium sulfate, filtered, and concentrated to obtain a crude product. The crude product was purified by preparative thin-layer chromatography (petroleum ether:ethyl acetate = 1:1) to obtain 017-8. $^1H$ NMR (400 MHz, $CDCl_3$) $\delta$ = 6.73 (s, 1H), 3.25 (s, 3H), 2.83 - 2.75 (m, 2H), 2.70 (d, *J* = 7.6 Hz, 2H).

Step 7: synthesis of compound 017

To a reaction flask were sequentially added 017-8 (20 mg, 81.26 μmol), potassium carbonate (44.92 mg, 325.04 μmol), 2-dicyclohexylphosphino-2,6-dimethoxybiphenyl (5.00 mg, 12.19 μmol), water (0.4 mL), acetonitrile (1.2 mL), and methanol (0.4 mL). The atmosphere was purged with nitrogen, followed by the addition of bis(triphenylphosphine) palladium dichloride (2.85 mg, 4.06 μmol) and palladium acetate (912.17 μg, 4.06 μmol). The atmosphere was again purged with nitrogen. The reaction mixture was heated to 40°C, and a crude solution of A-2 (prepared immediately prior to use according to the synthesis method of Reference Example 2, added based on a theoretical yield of 40.32 mg, 162.52 μmol) was added dropwise. After completion of the dropwise addition, the reaction mixture was heated to 75°C and stirred for 12 hours. The reaction mixture was filtered through diatomite and anhydrous sodium sulfate, and the filtrate was collected and concentrated to obtain a crude product. The crude product was purified by preparative thin-layer chromatography (eluent: ethyl acetate:dichloromethane:methanol = 3:1:0.3) to obtain 017. $^1H$ NMR (400 MHz, $CDCl_3$) δ = 8.59 - 8.52 (m, 1H), 8.48 - 8.42 (m, 1H), 6.89 (s, 1H), 6.40 - 6.22 (m, 1H), 5.37 - 5.21 (m, 1H), 3.35 (s, 3H), 3.10 - 2.99 (m, 2H), 2.90 (br d, *J* = 8.0 Hz, 2H), 2.79 (br d, *J* = 7.8 Hz, 2H), 2.34 (br d, *J* = 7.4 Hz, 2H), 2.18 (br d, *J* = 2.9 Hz, 2H), 1.94 - 1.90 (m, 2H), 1.23 - 1.20 (m, 3H); ESI-LCMS: m/z = 370.2 $[M+H]^+$. SFC analysis (method: chromatographic column: (S,S)-WHELK-O1, 100×4.6 mm I.D., 3.5 μm; mobile phase: A: $CO_2$, B: ethanol [0.2% 7M ammonia in methanol]; isocratic: A/B = 40:60; flow rate: 4 mL/min; column temperature: 35°C; pressure: 2000 psi), *ee* = 94.22%, retention time: 2.467 minutes.

**Example 14**

Synthetic route:

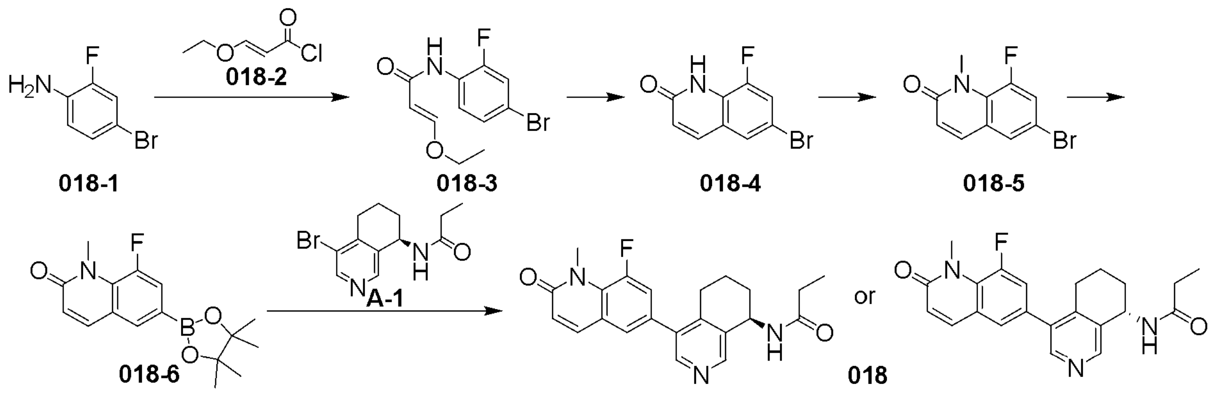
018-1 018-2 018-3 018-4 018-5 018-6 A-1 018 or

Step 1: synthesis of compound 018-3

To a reaction flask were sequentially added 018-1 (10 g, 52.63 mmol), dichloromethane (200 mL), and pyridine (5.00 g, 63.15 mmol). The atmosphere was purged with nitrogen, followed by the addition of 018-2 (7.79 g, 57.89 mmol). The reaction mixture was stirred at 25°C for 3 hours. The reaction mixture was concentrated to obtain a crude product, which was purified by column chromatography (petroleum ether: ethyl acetate = 10:1 to 1:1) to obtain 018-3. $^1H$ NMR (400 MHz, $CDCl_3$) δ = 8.30 (t, *J* = 8.7 Hz, 1H), 7.66 (d, *J* = 12.0 Hz, 1H), 7.27 - 7.23 (m, 2H), 7.10 (br s, 1H), 5.34 (d, *J*= 12.1 Hz, 1H), 3.97 (q, *J* = 7.0 Hz, 2H), 1.37 (t, *J* = 7.1 Hz, 3H).

Step 2: synthesis of compound 018-4

To a reaction flask were added 98% concentrated sulfuric acid (5 mL) and 018-3 (500 mg, 1.74 mmol). The atmosphere was purged with nitrogen. The react
ion mixture was stirred at 25°C for 3 hours, then heated to 80°C and stirred for 1 hour. The reaction mixture was poured into ice water and filtered. The filter cake was washed with water and dried under reduced pressure to obtain crude 018-4. It can be directly used in the next step without further purification.

Step 3: synthesis of compound 018-5

To a reaction flask were sequentially added 018-4 (0.2 g, 826.30 μmol), *N,N*-dimethylformamide (0.5 mL), and

cesium carbonate (538.45 mg, 1.65 mmol). The atmosphere was purged with nitrogen, and the reaction mixture was cooled to 0°C, followed by the slow and dropwise addition of methyl iodide (469.13 mg, 3.31 mmol). The atmosphere was again purged with nitrogen, and the mixture was stirred at 25°C for 2 hours. The reaction mixture was added with 2 mL of water, transferred to a separatory funnel, and extracted three times with ethyl acetate (3 mL each time). The organic phases were combined and washed three times with saturated brine (3 mL each time). The organic phase was collected, dried over anhydrous sodium sulfate, filtered, and concentrated to obtain a crude product. The crude product was purified by preparative thin-layer chromatography (petroleum ether:ethyl acetate = 5:1) to obtain 018-5. $^1$H NMR (400 MHz, $CDCl_3$) $\delta$ = 7.55 (d, *J* = 9.5 Hz, 1H), 7.50 - 7.37 (m, 2H), 6.76 (d, *J* = 9.5 Hz, 1H), 3.90 (d, *J* = 8.4 Hz, 3H).

Step 4: synthesis of compound 018-6

To a reaction flask were sequentially added 018-5 (100 mg, 390.52 μmol), dioxane (2 mL), bis(pinacolato)diboron (148.75 mg, 585.78 μmol), potassium acetate (114.98 mg, 1.17 mmol), and [1,1'-bis(diphenylphosphino)ferrocene] dichloropalladium(II) dichloromethane complex (15.95 mg, 19.53 μmol). The atmosphere was purged with nitrogen, and the reaction mixture was stirred at 80°C for 16 hours. The reaction mixture was filtered and rinsed with ethyl acetate. The filtrate was collected and concentrated to obtain crude 018-6, which was used directly in the next step without purification. ESI-LCMS: *m*/*z* = 304.2 $[M+H]^+$.

Step 5: synthesis of compound 018

To a reaction flask were sequentially added A-1 (0.05 g, 176.58 μmol), potassium carbonate (97.62 mg, 706.31 μmol), 2-dicyclohexylphosphino-2',6'-dimethoxybiphenyl (10.87 mg, 26.49 μmol), bis(triphenylphosphine)palladium dichloride (6.20 mg, 8.83 μmol), palladium acetate (1.98 mg, 8.83 μmol), water (1.2 mL), acetonitrile (3.6 mL), and methanol (1.2 mL). The atmosphere was purged with nitrogen, and the reaction mixture was heated to 40°C, followed by the dropwise addition of a solution of 018-6 (107.05 mg, 353.15 μmol) in tetrahydrofuran (1.2 mL). The reaction mixture was heated to 75°C and stirred for 2 hours. The reaction mixture was allowed to return to room temperature, added with 20 mL of water, transferred to a separatory funnel, and extracted three times with dichloromethane (30 mL each time). The organic phases were combined and washed three times with saturated brine (30 mL each time). The organic phase was collected, dried over anhydrous sodium sulfate, filtered, and concentrated to obtain a crude product. The crude product was sequentially purified by preparative thin-layer chromatography (dichloromethane:ethyl acetate:methanol = 1:1:0.1) and SFC chiral resolution (column: DAICEL CHIRALPAK AD (250 mm*30 mm, 10 μm); mobile phase: [supercritical $CO_2$-methanol (0.1% ammonia)]; isocratic elution: 45% methanol (0.1% ammonia)) to obtain compound 018. $^1$H NMR (400 MHz, $CD_3OD$) $\delta$ = 8.41 (s, 1H), 8.26 (s, 1H), 7.95 (dd, *J* = 1.5, 9.5 Hz, 1H), 7.51 (d, *J*= 1.7 Hz, 1H), 7.46 (dd, *J*= 1.9, 15.3 Hz, 1H), 6.76 (d, *J*= 9.4 Hz, 1H), 5.22 (t, *J*= 5.7 Hz, 1H), 3.96 (d, *J*=8.0 Hz, 3H), 2.83 - 2.63 (m, 2H), 2.29 (dq, *J*= 1.9, 7.6 Hz, 2H), 2.13 - 2.00 (m, 1H), 1.94 - 1.72 (m, 3H), 1.19 (t, *J* = 7.6 Hz, 3H); ESI-LCMS: m/z = 380.2 $[M+H]^+$; SFC analysis was performed (chromatographic column: Chiralpak AD-3, 50 × 4.6 mm I.D., 3 μm; mobile phase: A: $CO_2$, B: methanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.504 minutes.

**Example 15**

Synthetic route:

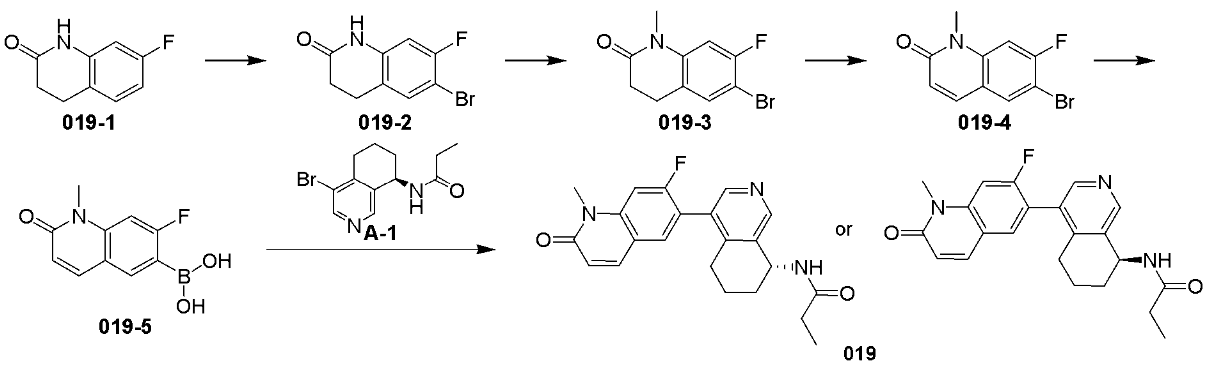
019-1
019-2
019-3
019-4
019-5
A-1
or
019

Step 1: synthesis of compound 019-2

To a reaction flask were added 019-1 (0.96 g, 5.81 mmol) and *N,N*-dimethylformamide (10 mL). The atmosphere was purged with nitrogen, and the reaction mixture was cooled to 0°C, followed by the addition of N-bromosuccinimide (1.24 g, 6.97 mmol). After the addition was completed, the reaction mixture was stirred at 25°C for 8 hours. The reaction mixture was sequentially added with water (20 mL) and a saturated aqueous solution of sodium thiosulfate (2 mL), and stirred for 10 minutes. The mixture was then filtered, and the filter cake was collected. Dichloromethane (20 mL) was added, and the mixture was transferred to a separatory funnel. It was washed with saturated brine (20 mL), and the organic phase was collected. The organic phase was dried over anhydrous sodium sulfate, filtered, and concentrated to obtain crude 019-2, which was used directly in the next step without further purification. $^1$H NMR (400 MHz, $CDCl_3$) $\delta$ ppm 8.32 (br s, 1 H), 7.34 (d, J=7.03 Hz, 1 H), 6.61 (d, *J* = 8.94 Hz, 1 H), 2.95 (t, *J*= 7.57 Hz, 2 H), 2.59 - 2.69 (m, 2 H).

Step 2: synthesis of compound 019-3

To a reaction flask were sequentially added 019-2 (1.3 g, 5.33 mmol), *N,N*-dimethylformamide (13 mL), and cesium carbonate (3.47 g, 10.65 mmol). The atmosphere was purged with nitrogen, and the reaction mixture was cooled to 0°C, followed by the dropwise addition of methyl iodide (2.27 g, 15.98 mmol). The reaction mixture was stirred at 25°C for 1 hour. The reaction mixture was added with water (20 mL), then transferred to a separatory funnel, and extracted three times with ethyl acetate (20 mL each time). The organic phases were combined, washed twice with saturated brine (20 mL each time), dried over anhydrous sodium sulfate, filtered, and concentrated to obtain crude 019-3, which was used directly in the next step without further purification.

Step 3: synthesis of compound 019-4

To a reaction flask were sequentially added 019-3 (1.3 g, 5.04 mmol), dichloroethane (26 mL), *N*-bromosuccinimide (1.34 g, 7.56 mmol), and 2,2'-azobisisobutyronitrile (165.42 mg, 1.01 mmol). The system was purged with nitrogen, and the reaction mixture was stirred at 80°C for 2 hours. The reaction mixture was added with water (30 mL), then transferred to a separatory funnel, and extracted with ethyl acetate (30 mL $\times$ 2). The resulting phases were separated. The organic phases were combined and washed with saturated brine (30 mL). The organic phase was collected, dried over anhydrous sodium sulfate, filtered, and concentrated to obtain a crude product. The crude product was purified by column chromatography (petroleum ether:ethyl acetate = 30:1 to 3:1) to obtain 019-4. $^1$H NMR (400 MHz, $CDCl_3$) $\delta$ ppm 7.76 (d, *J*=7.25 Hz, 1 H), 7.58 (d, *J*=9.51 Hz, 1 H), 7.13 (d, *J*=10.38 Hz, 1 H), 6.69 (d, *J*=9.51 Hz, 1 H), 3.67 (s, 3 H).

Step 4: synthesis of compound 019-5

To a reaction flask were sequentially added 019-4 (500 mg, 1.95 mmol), 1,4-dioxane (20 mL), bis(pinacolato) diboron (743.75 mg, 2.93 mmol), potassium acetate (574.88 mg, 5.86 mmol), and methanesulfonato(2-dicyclohexylphosphino-2',4',6'-tri-i-propyl-1,1'-biphenyl)(2'-amino-1,1'-biphenyl-2-yl)palladium(II) (82.64 mg, 97.63 $\mu$mol). The atmosphere was purged with nitrogen, and the reaction mixture was stirred at 80°C for 16 hours. The reaction mixture was filtered through diatomite, and the filter cake was rinsed with ethyl acetate:methanol = 10:1 (10 mL). The filtrate was collected and concentrated to obtain crude 019-5, which was used directly in the next step without further purification.

Step 5: synthesis of compound 019

To a reaction flask were sequentially added A-1 (100 mg, 353.15 $\mu$mol), water (1.2 mL), acetonitrile (3.6 mL), methanol (1.2 mL), potassium carbonate (195.23 mg, 1.41 mmol), and 2-dicyclohexylphosphino-2,6-dimethoxybiphenyl (21.75 mg, 52.97 $\mu$mol). The atmosphere was purged with nitrogen, followed by the addition of bis(triphenylphosphine) palladium(II) dichloride (12.39 mg, 17.66 $\mu$mol) and palladium(II) acetate (3.96 mg, 17.66 $\mu$mol). The atmosphere was again purged with nitrogen. The reaction mixture was heated to 40°C, and a solution of crude 019-5 (156.09 mg, 706.31 $\mu$mol) in tetrahydrofuran (2 mL) was added dropwise. The reaction mixture was then stirred at 75°C for 2 hours. The reaction mixture was allowed to return to room temperature, and anhydrous sodium sulfate (5 g) was added thereto. The mixture was filtered through diatomite, and the filter cake was rinsed with ethyl acetate (10 mL). The filtrate was collected and concentrated to obtain a crude product. The crude product was sequentially subjected to preparative thin-layer chromatography (dichloromethane:ethyl acetate:methanol = 1:1:0.1) and SFC chiral resolution (column: DAICEL CHIRALPAK AD (250 mm*30 mm, 10 $\mu$m); mobile phase: [supercritical carbon dioxide-methanol (0.1% ammonia)]; isocratic elution: 50% methanol (0.1% ammonia)) to obtain 019. $^1$H NMR (400 MHz, $CD_3OD$) $\delta$ ppm 8.44 (s, 1 H), 8.25 (s, 1 H), 7.95 (d, *J* = 9.51 Hz, 1 H), 7.69 (d, *J* = 7.88 Hz, 1 H), 7.52 (d, *J* = 11.88 Hz, 1 H), 6.69 (d, *J* = 9.38 Hz, 1 H), 5.24 (t, *J* = 5.75 Hz, 1 H), 3.76 (s, 3 H), 2.54 - 2.70 (m, 2 H), 2.29 (qd, *J* = 7.61, 2.19 Hz, 2 H), 2.01 - 2.10 (m, 1 H), 1.78 - 1.95 (m, 3 H), 1.20 (t,

*J*=7.63 Hz, 3 H); ESI-LCMS: m/z = 380.2 $[M+H]^+$. SFC analysis was performed (chromatographic column: ChiralpakAD-3, 150×4.6 mm I.D., 3 μm; mobile phase: A: supercritical carbon dioxide B: methanol (0.2% 7 M ammonia in methanol); gradient: A/B = 50:50; flow rate: 2.5 mL/min; column temperature: 35°C; pressure: 2000 psi), *ee* = 100%, retention time: 1.797 minutes.

**Example 16**

Synthetic route:

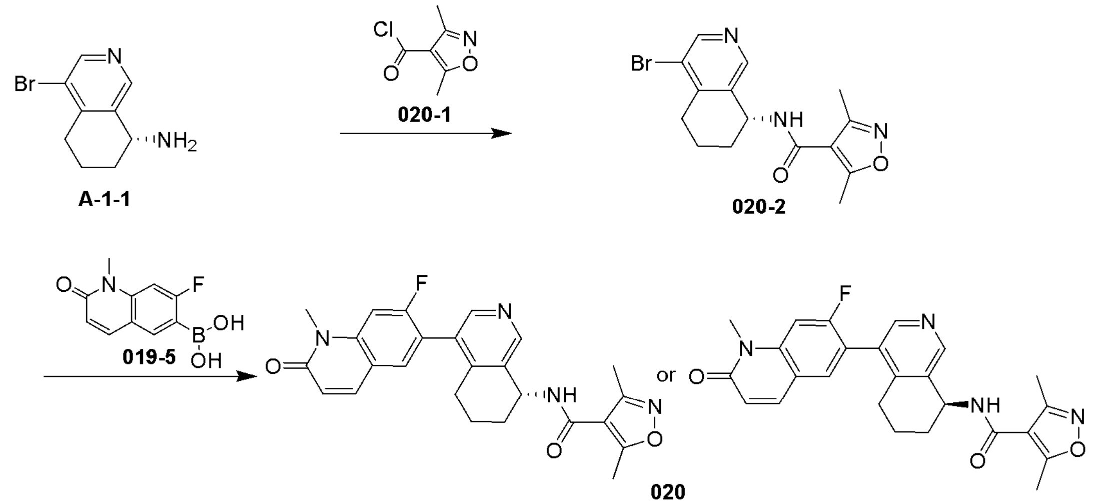

A-1-1

020-1

020-2

019-5

or

020

Step 1: synthesis of compound 020-2

To the reaction mixture were sequentially added A-1-1 (0.2 g, 880.67 μmol), dichloromethane (10 mL), and N,N-diisopropylethylamine (682.92 mg, 5.28 mmol). The atmosphere was purged with nitrogen, followed by the addition of 020-1 (140.53 mg, 880.67 μmol). The reaction mixture was stirred at 25°C for 1 hour. The reaction mixture was added with water (10 mL), then transferred to a separatory funnel, and extracted with dichloromethane twice (10 mL each time). The resulting phases were separated. The organic phases were combined, washed with saturated brine (10 mL), dried over anhydrous sodium sulfate, filtered, and concentrated to obtain a crude product. The crude product was purified by preparative thin-layer chromatography (petroleum ether:ethyl acetate = 3:1) to obtain 020-2. $^1H$ NMR (400 MHz, $CDCl_3$) δ ppm 8.41 - 8.71 (m, 2 H), 5.97 (br d, *J*=6.31 Hz, 1 H), 5.39 - 5.53 (m, 1 H), 2.80 - 2.86 (m, 2 H), 2.63 (s, 3 H), 2.42 (s, 3 H), 2.12 - 2.18 (m, 1 H), 1.90 - 1.96 (m, 3 H).

Step 2: synthesis of compound 020

To a reaction flask were sequentially added compound 020-2 (50 mg, 142.77 μmol), water (0.6 mL), acetonitrile (1.8 mL), methanol (0.6 mL), potassium carbonate (78.93 mg, 571.09 μmol), and 2-dicyclohexylphosphino-2,6-dimethoxybiphenyl (8.79 mg, 21.42 μmol). The atmosphere was purged with nitrogen, followed by the addition of bis(triphenylphosphine)palladium(II) dichloride (5.01 mg, 7.14 μmol) and palladium(II) acetate (1.60 mg, 7.14 μmol). The atmosphere was again purged with nitrogen. The reaction mixture was heated to 40°C, and a solution of 019-5 (63.10 mg, 285.54 μmol) in tetrahydrofuran (1 mL) was added dropwise. The reaction mixture was then heated to 75°C and stirred for 2 hours. The reaction mixture was allowed to return to room temperature, and anhydrous sodium sulfate (5 g) was added thereto. The mixture was filtered through diatomite, and the filter cake was rinsed with ethyl acetate (10 mL). The filtrate was collected and concentrated to obtain a crude product. The crude product was sequentially purified by preparative thin-layer chromatography (dichloromethane:ethyl acetate = 1:1) and SFC chiral resolution (column: DAICEL CHIRALPAK AD (250 mm*30 mm, 10 μm); mobile phase: [supercritical carbon dioxide-methanol (0.1% ammonia)]; isocratic elution: 50% methanol (0.1% ammonia)) to obtain 020. $^1H$ NMR (400 MHz, $CD_3OD$) δ ppm 8.58 (s, 1 H), 8.29 (s, 1 H), 7.95 (d, *J* = 9.51 Hz, 1 H), 7.70 (d, *J* = 7.88 Hz, 1 H), 7.53 (d, *J*= 11.88 Hz, 1 H), 6.69 (d, *J* = 9.51 Hz, 1 H), 5.43 (t, *J* = 6.50 Hz, 1 H), 3.76 (s, 3 H), 2.60 - 2.72 (m, 2 H), 2.54 (s, 3 H), 2.38 (s, 3 H), 2.12 - 2.23 (m, 1 H), 1.85 - 2.02 (m, 3 H); ESI-LCMS: m/z = 447.3 $[M+H]^+$. SFC analysis was performed (chromatographic column: Chiralpak AD-3, 50 × 4.6 mm I.D., 3 μm; mobile phase: A: supercritical carbon dioxide, B: methanol (0.2% 7M ammonia in methanol); gradient: A/B = 95/5 (0 min), 95/5 (0.2 min),

50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.566 minutes.

**Example 17**

Synthetic route:

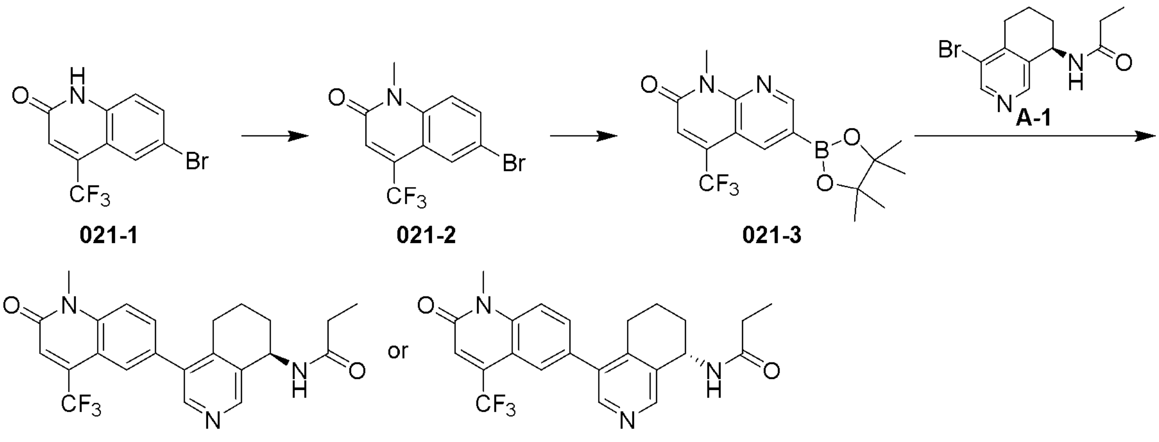

021-1 021-2 021-3 A-1

or

021

Step 1: synthesis of compound 021-2

To a reaction flask were sequentially added 021-1 (0.3 g, 1.03 mmol), potassium carbonate (283.93 mg, 2.05 mmol), and *N*,*N*-dimethylformamide (6 mL). The atmosphere was purged with nitrogen, and the reaction mixture was cooled to 0°C, followed by the addition of methyl iodide (583.21 mg, 4.11 mmol). The reaction mixture was then warmed to 25°C and stirred for 3 hours. The reaction mixture was added with water (30 mL) and extracted three times with ethyl acetate (10 mL each time). The organic phases were combined and washed with saturated brine (20 mL). The organic phase was collected, dried over anhydrous sodium sulfate, filtered, and concentrated to obtain a crude product. The crude product was purified by preparative thin-layer chromatography (petroleum ether:ethyl acetate = 1:1) to obtain 021-2. $^1H$ NMR (400 MHz, $CDCl_3$) $\delta$ = 7.97 (s, 1H), 7.76 (dd, *J* = 1.8, 9.0 Hz, 1H), 7.34 (d, *J* = 9.0 Hz, 1H), 7.14 (s, 1H), 3.74 (s, 3H).

Step 2: synthesis of compound 021-3

To a reaction flask were sequentially added 021-2 (100 mg, 326.71 $\mu$mol), bis(pinacolato)diboron (124.45 mg, 490.07 $\mu$mol), potassium acetate (96.19 mg, 980.14 $\mu$mol), and 1,4-dioxane (2 mL). The atmosphere was purged with nitrogen gas, followed by the addition of [1,1'-bis(diphenylphosphino)ferrocene]dichloropalladium(II) dichloromethane complex (26.68 mg, 32.67 $\mu$mol). The reaction mixture was stirred at 80°C for 3 hours. The reaction mixture was concentrated to obtain crude 021-3, which was used directly in the next step without further purification.

Step 3: synthesis of compound 021

To a reaction flask were sequentially added crude 021-3 (109.75 mg, 310.77 $\mu$mol), A-1 (80 mg, 282.52 $\mu$mol), sodium carbonate (59.89 mg, 565.04 $\mu$mol), water (0.3 mL), and ethanol (1.8 mL). The atmosphere was purged with nitrogen, followed by the addition of tetrakis(triphenylphosphine)palladium (32.65 mg, 28.25 $\mu$mol). The reaction mixture was stirred at 85°C for 2.5 hours. The reaction mixture was concentrated to obtain a crude product, which was sequentially purified by preparative thin-layer chromatography (ethyl acetate:dichloromethane:methanol = 5:5:1) and SFC chiral resolution (chromatographic column: DAICEL CHIRALPAK AD (250mm*30 mm, 10 $\mu$m); mobile phase: [supercritical carbon dioxide-isopropanol (0.1% ammonia)]; isocratic elution: 30% isopropanol (0.1% ammonia)) to obtain 021. $^1H$ NMR (400 MHz, $CD_3OD$) $\delta$ = 8.42 (s, 1H), 8.25 (s, 1H), 7.89 - 7.82 (m, 1H), 7.80 - 7.73 (m, 2H), 7.17 (s, 1H), 5.24 (t, *J*= 5.9 Hz, 1H), 3.84 (s, 3H), 2.78 - 2.60 (m, 2H), 2.28 (dq, *J* = 2.0, 7.6 Hz, 2H), 2.12 - 2.00 (m, 1H), 1.95 - 1.76 (m, 3H), 1.19 (t, *J* = 7.6 Hz, 3H); ESI-LCMS: m/z = 430.2 $[M+H]^+$. SFC analysis was performed (chromatographic column: Chiralpak AD-3, 150 $\times$ 4.6 mm I.D., 3 $\mu$m; mobile phase: A: supercritical carbon dioxide, B: isopropanol (0.2% 7M ammonia in methanol); gradient: A/B = 90/10 (0 min), 90/10 (0.5 min), 50/50 (3.5 min), 50/50 (4.5 min), 90/10 (5.0 min); flow rate: 2.5 mL/min; column

temperature: 35°C; pressure: 2000 psi), *ee* = 100%, retention time: 3.591 minutes.

**Example 18**

Synthetic route:

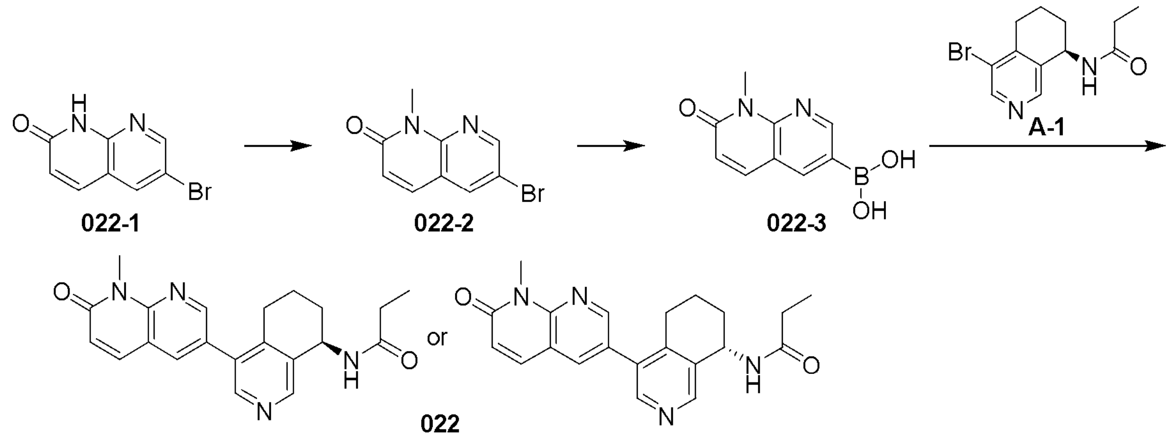

A-1

022-1 022-2 022-3

or

022

Step 1: synthesis of compound 022-2

To a reaction flask were sequentially added 022-1 (0.4 g, 1.78 mmol), *N,N*-dimethylformamide (8 mL), and cesium carbonate (694.95 mg, 2.13 mmol). The atmosphere was purged with nitrogen, and the reaction mixture was cooled to 0°C, followed by the dropwise addition of methyl iodide (1.01 g, 7.11 mmol). The reaction mixture was stirred at 0°C for 1 hour. The reaction mixture was added with water (10 mL), then transferred to a separatory funnel, and extracted with 10 mL of ethyl acetate. The resulting phases were separated. The organic phase was collected, and the aqueous phase was extracted with ethyl acetate three times (15 mL each time). The combined organic phases were washed twice with saturated brine (10 mL each time). The organic phase was collected, dried over anhydrous sodium sulfate, filtered, and concentrated to obtain crude 022-2, which was used directly in the next step without further purification. $^1$H NMR (400 MHz, $CDCl_3$) $\delta$ = 8.63 (d, *J* = 2.4 Hz, 1H), 7.99 (d, *J* = 2.4 Hz, 1H), 7.58 (d, *J* = 9.5 Hz, 1H), 6.81 (d, *J* = 9.5 Hz, 1H), 3.81 (s, 3H).

Step 2: synthesis of compound 022-3

To a reaction flask were sequentially added crude 022-2 (0.32 g, 1.34 mmol), 1,4-dioxane (12.8 mL), bis(pinacolato)diboron (509.85 mg, 2.01 mmol), potassium acetate (394.09 mg, 4.02 mmol), and methanesulfonato(2-dicyclohexylphosphino-2',4',6'-tri-i-propyl-1,1'-biphenyl)(2'-amino-1,1'-biphenyl-2-yl)palladium(II) (56.65 mg, 66.93 μmol). The atmosphere was purged with nitrogen, and the reaction mixture was stirred at 80°C for 16 hours. The reaction mixture was filtered, and the filter cake was rinsed with ethyl acetate. The filtrate was collected and concentrated to obtain crude 022-3, which was used directly in the next step without further purification. ESI-LCMS: m/z = 205.1 $[M+H]^+$.

Step 3: synthesis of compound 022

To a reaction flask were sequentially added A-1 (0.14 g, 494.41 μmol), water (1.6 mL), acetonitrile (4.8 mL), methanol (1.6 mL), potassium carbonate (273.33 mg, 1.98 mmol), and 2-dicyclohexylphosphino-2,6-dimethoxybiphenyl (30.45 mg, 74.16 μmol). The atmosphere was purged with nitrogen, followed by the addition of bis(triphenylphosphine) palladium(II) dichloride (17.35 mg, 24.72 μmol) and palladium(II) acetate (5.55 mg, 24.72 μmol). The atmosphere was again purged with nitrogen. The reaction mixture was heated to 40°C, and a solution of 022-3 (201.71 mg, 988.83 μmol) in tetrahydrofuran (2.8 mL) was added. The reaction mixture was heated to 75°C and stirred for 12 hours. The reaction mixture was allowed to return to room temperature, then anhydrous sodium sulfate was added. The mixture was filtered. The filtrate was collected and concentrated to obtain a crude product. The crude product was sequentially subjected to preparative thin-layer chromatography (ethyl acetate:dichloromethane:methanol = 1:1:0.1) and SFC chiral resolution (chromatographic column: DAICEL CHIRALPAK IG (250mm*30 mm, 10 μm); mobile phase: [supercritical carbon dioxide-isopropanol]; isocratic elution: 55% isopropanol) to obtain 022. $^1$H NMR (400 MHz, $CDCl_3$) $\delta$ = 8.61 (s, 1H), 8.56 (d, *J* = 2.3

Hz, 1H), 8.35 (s, 1H), 7.82 (d, $J$ = 2.3 Hz, 1H), 7.69 (d, $J$ = 9.5 Hz, 1H), 6.85 (d, $J$ = 9.4 Hz, 1H), 5.83 (br d, $J$ = 8.5 Hz, 1H), 5.44 - 5.31 (m, 1H), 3.89 (s, 3H), 2.76 - 2.60 (m, 2H), 2.31 (q, $J$ = 7.5 Hz, 2H), 2.17 - 2.09 (m, 1H), 1.93 (br s, 6H), 1.24 (t, $J$ = 7.6 Hz, 3H); ESI-LCMS: $m/z$ =363.2 $[M+H]^+$; SFC analysis was performed (chromatographic column: Chiralpak IG-3, 50 × 4.6 mm I.D., 3 μm; mobile phase: A: supercritical carbon dioxide, B: isopropanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 2.145 minutes.

**Example 19**

Synthetic route:

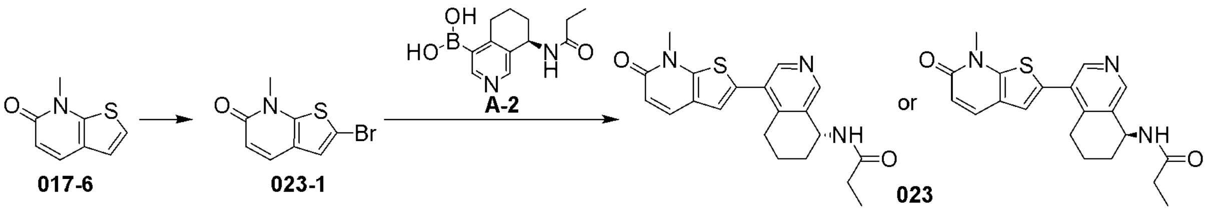

017-6

023-1

A-2

or

023

Step 1: synthesis of compound 023-1

To a reaction flask were sequentially added 017-6 (75 mg, 410.36 μmol) and tetrahydrofuran (3 mL). The atmosphere was purged with nitrogen, and the reaction mixture was cooled to 0°C, followed by the addition ofN-Bromosuccinimide (58.43 mg, 328.29 μmol). The reaction mixture was stirred at 0°C for 0.5 hours. The reaction mixture was added with a half-saturated aqueous sodium thiosulfate solution (3 mL) and stirred at 25°C for 5 minutes. The mixture was transferred to a separatory funnel and extracted three times with ethyl acetate (3 mL each time). The organic phases were combined and washed with saturated brine (3 mL). The organic phase was collected, dried over anhydrous sodium sulfate, filtered, and concentrated to obtain a crude product. The crude product was purified by preparative thin-layer chromatography (petroleum ether:ethyl acetate = 3:1) to obtain 023-1. $^1$H NMR (400 MHz, $CDCl_3$) δ = 7.58 (d, $J$ = 9.4 Hz, 1H), 7.10 (s, 1H), 6.55 (d, $J$ = 9.4 Hz, 1H), 3.64 (s, 3H).

Step 2: synthesis of compound 023

To a reaction flask were sequentially added 023-1 (70 mg, 286.76 μmol), potassium carbonate (158.53 mg, 1.15 mmol), 2-dicyclohexylphosphino-2,6-dimethoxybiphenyl (17.66 mg, 43.01 μmol), water (1.4 mL), acetonitrile (4.2 mL), and methanol (0.4 mL). The atmosphere was purged with nitrogen, followed by the addition of bis(triphenylphosphine) palladium dichloride (10.06 mg, 14.34 μmol) and palladium acetate (3.22 mg, 14.34 μmol). The atmosphere was again purged with nitrogen. The reaction mixture was heated to 40°C, and a crude solution of A-2 (prepared immediately prior to use according to the synthesis method of Reference Example 2, added based on a theoretical yield of 142.28 mg, 573.52 μmol) was added. The reaction mixture was heated to 75°C and stirred for 12 hours. The reaction mixture was filtered through diatomite and anhydrous sodium sulfate, and the filtrate was collected and concentrated to obtain a crude product. The crude product was sequentially purified by preparative thin-layer chromatography (ethyl acetate:dichloromethane:methanol = 3:1:0.3) and SFC chiral resolution (chromatographic column: DAICEL CHIRALPAK IG (250mm*30 mm, 10 μm); mobile phase: [supercritical carbon dioxide-ethanol (0.1% ammonia)]; isocratic elution: 50% ethanol (0.1% ammonia)) to obtain 023. $^1$H NMR (400 MHz, $CDCl_3$) δ = 8.58 - 8.37 (m, 2H), 7.69 (d, $J$ = 9.4 Hz, 1H), 7.05 (s, 1H), 6.61 (d, $J$ = 9.4 Hz, 1H), 5.81 (br d, $J$ = 8.4 Hz, 1H), 5.35 (br d, $J$ = 7.8 Hz, 1H), 3.71 (s, 3H), 2.95 - 2.78 (m, 2H), 2.30 (q, $J$ = 7.5 Hz, 2H), 2.10 (br dd, $J$ = 5.5, 8.6 Hz, 1H), 1.88 (br dd, $J$ = 3.6, 5.8 Hz, 3H), 1.23 (t, $J$ = 7.6 Hz, 3H); ESI-LCMS: $m/z$ = 368.2 $[M+H]^+$. SFC analysis was performed (chromatographic column: Chiralpak IG-3, 100×4.6 mm I.D, 3 μm; mobile phase: A: supercritical carbon dioxide B: ethanol (0.2% 7 M ammonia in methanol); gradient: A/B = 60:40; flow rate: 4 mL/min; column temperature: 35°C; pressure: 1800 psi), *ee* = 100%, retention time: 2.762 minutes.

**Example 20**

Synthetic route:

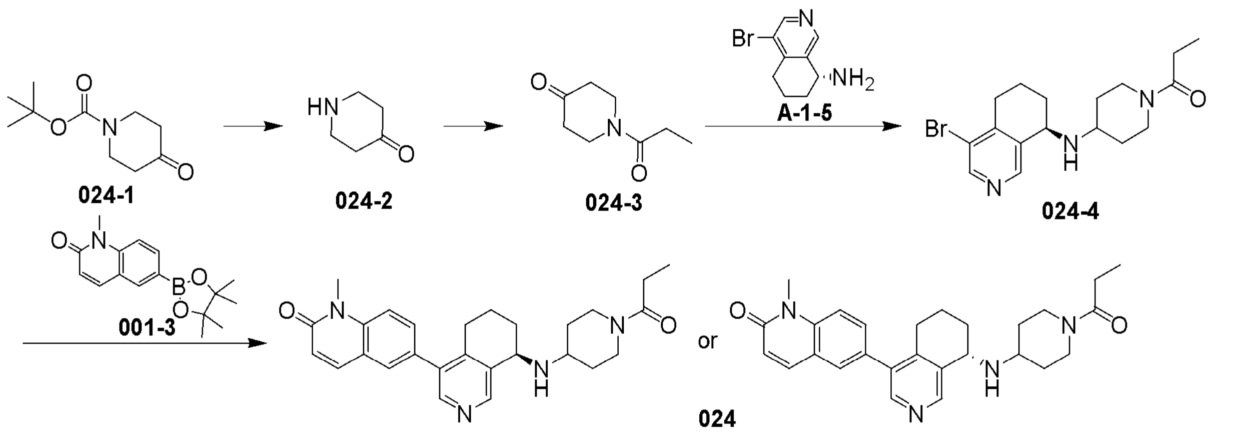

024-1 024-2 024-3 024-4 or 024

Step 1: synthesis of compound 024-2

To a reaction flask were sequentially added 024-1 (500 mg, 2.51 mmol) and hydrogen chloride/ethyl acetate (4 M, 7.50 mL). The reaction mixture was stirred at 25°C for 1 hour. The reaction mixture was concentrated to obtain a hydrochloride of 024-2, which was used directly in the next step without further purification. $^1$H NMR (400 MHz, $CDCl_3$) δ = 3.62 - 3.50 (m, 4H), 2.89 (t, *J*= 6.4 Hz, 4H).

Step 2: synthesis of compound 024-3

To a reaction flask were sequentially added a hydrochloride of 024-2 (260 mg) and dichloromethane (2.6 mL). The reaction mixture was cooled to 0°C, followed by the addition of *N,N*-diisopropylethylamine (1.02 g, 7.87 mmol) and propionyl chloride (364.01 mg, 3.93 mmol). The reaction mixture was stirred at 0°C for 1 hour. The reaction mixture was added with water (2 mL), then transferred to a separatory funnel, and extracted three times with ethyl acetate (2 mL each time). The organic phases were combined and washed with saturated brine (2 mL). The organic phase was collected, dried over anhydrous sodium sulfate, filtered, and concentrated to obtain a crude product. The crude product was purified by column chromatography (petroleum ether:ethyl acetate = 1:0 to 6:1) to obtain 024-3. $^1$H NMR (400 MHz, $CDCl_3$) δ = 3.99 - 3.66 (m, 4H), 2.56 - 2.33 (m, 6H), 1.21 (t, *J* = 7.4 Hz, 3H).

Step 3: synthesis of compound 024-4

To a reaction flask were sequentially added a hydrochloride of A-1-5 (140 mg), triethylamine (161.25 mg, 1.59 mmol), and methanol (1.4 mL). The reaction mixture was stirred at 25°C, followed by the addition of 024-3 (123.66 mg, 796.78 μmol) and acetic acid (76.56 mg, 1.27 mmol). After 10 minutes, sodium cyanoborohydride (100.14 mg, 1.59 mmol) was added. The reaction mixture was stirred at 25°C for 1 hour. The reaction mixture was adjusted to pH 8-9 by adding a saturated sodium bicarbonate solution, transferred to a separatory funnel, and extracted three times with ethyl acetate (2 mL each time). The organic phases were combined and washed with saturated brine (2 mL). The organic phase was collected, dried over anhydrous sodium sulfate, filtered, and concentrated to obtain a crude product. The crude product was purified by column chromatography (dichloromethane:methanol = 1:0 to 15:1) to obtain 024-4. $^1$H NMR (400 MHz, $CDCl_3$) 6 = 8.53 (br s, 2H), 4.59 - 4.36 (m, 1H), 4.18 - 4.03 (m, 1H), 3.75 (br d, *J*= 16.9 Hz, 1H), 3.60 - 3.41 (m, 1H), 3.27 - 3.15 (m, 1H), 2.98 (br s, 1H), 2.85 (br d, *J*= 14.3 Hz, 1H), 2.69 - 2.58 (m, 1H), 2.39 - 2.36 (m, 2H), 2.17 - 1.94 (m, 4H), 1.87 (br d, *J*= 4.4 Hz, 3H), 1.60 (br s, 2H), 1.18 - 1.15 (m, 3H).

Step 4: synthesis of compound 024

To a reaction flask were sequentially added 024-4 (60 mg, 163.80 μmol), water (0.2 mL), acetonitrile (0.6 mL), methanol (0.2 mL), 2-dicyclohexylphosphino-2,6-dimethoxybiphenyl (10.09 mg, 24.57 μmol), and potassium carbonate (90.56 mg, 655.21 μmol). The atmosphere was purged with nitrogen, followed by the addition of bis(triphenylphosphine) palladium(II) dichloride (5.75 mg, 8.19 μmol) and palladium(II) acetate (1.84 mg, 8.19 μmol). The atmosphere was again purged with nitrogen. The reaction mixture was heated to 40°C, and a solution of 001-3 (56.05 mg, 196.56 μmol) in tetrahydrofuran (1.2 mL) was added. The reaction mixture was heated to 75°C and stirred for 12 hours. The reaction mixture was concentrated to obtain a crude product. The crude product was sequentially subjected to preparative thin-layer chromatography (petroleum ether:ethyl acetate:methanol = 1:1:0.1), preparative high-performance liquid chromatography (chromatographic column: Waters Xbridge BEH C18 100*30mm*10μm; mobile phase: [water (10 mM ammonium bicarbonate)-acetonitrile]; gradient: 15%-55% acetonitrile) and SFC chiral resolution (chromatographic column: ChiralPak IH, 250*30 mm, 10 μm; mobile phase: [supercritical carbon dioxide-methanol (0.1% ammonia)]; isocratic elution: 50% methanol (0.1% ammonia)) to obtain 024. $^1$H NMR (400 MHz, $CDCl_3$) δ = 8.62 (br s, 1H), 8.30 (s, 1H), 7.69 (br d, *J* = 9.5 Hz, 1H), 7.55 - 7.41 (m, 3H), 6.78 (br d, *J* = 9.5 Hz, 1H), 4.56 - 4.34 (m, 1H), 4.05 (br d, *J* = 13.0 Hz, 1H), 3.93 - 3.82 (m, 1H), 3.16 (br t, *J* = 11.6 Hz, 1H), 3.06 - 2.97 (m, 1H), 2.96 - 2.85 (m, 1H), 2.76 - 2.63 (m, 1H), 2.61 - 2.50 (m, 1H), 2.44 - 2.33 (m, 2H), 2.05 (br s, 1H), 1.90 (br s, 4H), 1.79 - 1.30 (m, 7H), 1.17 (br t, *J* = 6.6 Hz, 3H); ESI-LCMS: m/z = 445.3 $[M+H]^+$; SFC analysis was performed (chromatographic column: Chiralpak IH-3, 50 × 4.6 mm I.D., 3 μm; mobile phase: A: $CO_2$, B: methanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 2.051 minutes.

**Example 21**

Synthetic route:

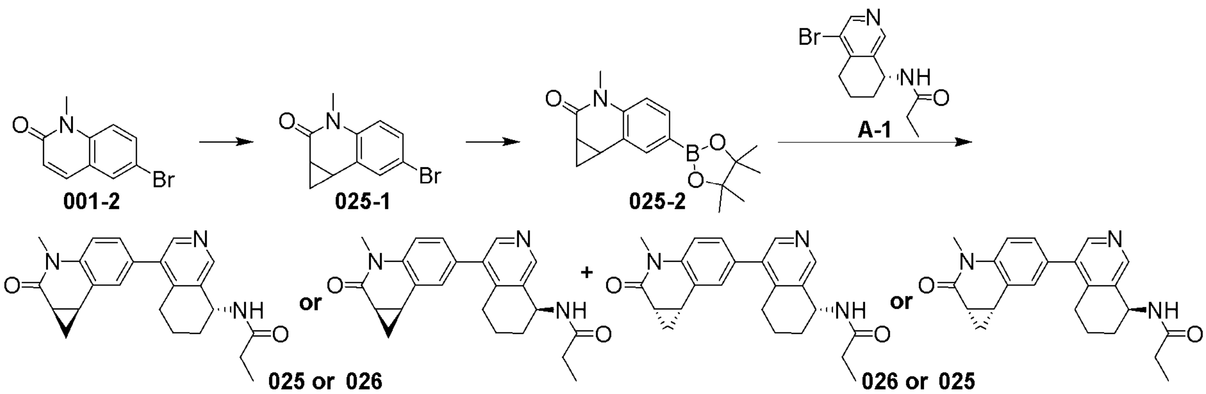

001-2
025-1
025-2
025 or 026
026 or 025

Step 1: synthesis of compound 025-1

To a reaction flask were sequentially added tetrahydrofuran (2 mL) and trimethylsulfoxonium iodide (554.62 mg, 2.52 mmol). The atmosphere was purged with nitrogen, and the reaction mixture was cooled to 0°C, followed by the addition of n-Butyllithium in hexane (2.5 M, 940.86 μL). The reaction mixture was stirred at 0°C for 0.5 hours, and a solution of 001-2 (200 mg, 840.05 μmol) in tetrahydrofuran (1 mL) was added dropwise. The reaction mixture was then warmed to 25°C and stirred for 15.5 hours. The reaction mixture was added with water (2 mL), then transferred to a separatory funnel, and extracted twice with ethyl acetate (2 mL each time). The organic phases were combined and washed with saturated brine (2 mL). The organic phase was collected, dried over anhydrous sodium sulfate, filtered, and concentrated to obtain a crude product. The crude product was purified by preparative thin-layer chromatography (petroleum ether: ethyl acetate = 5:1) to obtain 025-1. $^1$H NMR (400 MHz, $CDCl_3$) δ = 7.49 (d, *J* = 2.4 Hz, 1H), 7.33 (dd, *J* = 2.4, 8.8 Hz, 1H), 6.82 (d, *J* = 8.8 Hz, 1H), 3.31 (s, 3H), 2.49 (dt, *J* = 5.2, 8.0 Hz, 1H), 2.32 (ddd, *J* = 4.8, 7.6, 9.5 Hz, 1H), 1.65 - 1.61 (m, 1H), 0.64 (q, *J* = 4.8 Hz, 1H).

Step 2: synthesis of 025-2

To a reaction flask were sequentially added 025-1 (140 mg, 555.32 μmol), 1,4-dioxane (2.8 mL), bis(pinacolato) diboron (211.53 mg, 832.98 μmol), potassium acetate (163.50 mg, 1.67 mmol), and [1,1-bis(diphenylphosphino) ferrocene]dichloropalladium(II) dichloromethane complex (22.67 mg, 27.77 μmol). The atmosphere was purged with nitrogen, and the reaction mixture was stirred at 80°C for 16 hours. The reaction mixture was allowed to return to room temperature, and 4 mL of water was poured thereto. The mixture was transferred to a separatory funnel and extracted three times with ethyl acetate (3 mL each time). The organic phases were combined and washed three times with saturated brine (3 mL each time). The organic phase was collected, dried over anhydrous sodium sulfate, filtered, and concentrated to obtain a crude product. The crude product was purified by preparative thin-layer chromatography (petroleum ether:ethyl acetate = 5:1) to obtain 025-2. $^1$H NMR (400 MHz, $CDCl_3$) δ = 7.81 (d, *J* = 1.3 Hz, 1H), 7.67 (dd, *J* = 1.4, 8.2 Hz, 1H), 6.95 (d, *J* = 8.2 Hz, 1H), 3.34 (s, 3H), 2.56 (dt, *J* = 5.2, 8.0 Hz, 1H), 2.38 - 2.26 (m, 2H), 1.58 - 1.53 (m, 1H), 1.36 (s, 12H).

Step 3: synthesis of 025 and 026

To a reaction flask were sequentially added A-1 (75 mg, 264.86 μmol), water (1.2 mL), acetonitrile (3.6 mL), methanol (1.2 mL), potassium carbonate (146.43 mg, 1.06 mmol), and 2-dicyclohexylphosphino-2,6-dimethoxybiphenyl (16.31 mg, 39.73 μmol). The atmosphere was purged with nitrogen, followed by the addition of bis(triphenylphosphine) palladium(II) dichloride (9.30 mg, 13.24 μmol) and palladium(II) acetate (2.97 mg, 13.24 μmol). The atmosphere was again purged with nitrogen. The reaction mixture was heated to 40°C, and a solution of 025-2 (95.09 mg, 317.84 μmol) in tetrahydrofuran (1.2 mL) was added. The reaction mixture was heated to 75°C and stirred for 2 hours. The reaction mixture was allowed to return to room temperature, and 2 mL of water was poured thereto. The mixture was transferred to a separatory funnel and extracted three times with dichloromethane (3 mL each time). The organic phases were combined and washed three times with saturated brine (3 mL each time). The organic phase was collected, dried over anhydrous sodium sulfate, filtered, and concentrated to obtain a crude product. The crude product was sequentially purified by preparative thin-layer chromatography (dichloromethane:ethyl acetate:methanol = 1:1:0.3) and SFC chiral resolution (chromatographic column: ChiralPak IH, 250*30 mm, 10 μm; mobile phase: [supercritical carbon dioxide-ethanol (0.1% ammonia)]; isocratic elution: 35% ethanol (0.1% ammonia)) to obtain 025 and 026. The *ee* value was subsequently determined by SFC analysis (chromatographic column: Chiralpak IH-3, 100 × 4.6 mm I.D., 3 μm; mobile phase: A: supercritical $CO_2$, B: ethanol (0.2% 7M ammonia in methanol); gradient: A/B = 90/10 (0 min), 90/10 (0.2 min), 50/50 (2.4 min), 50/50 (3.4 min), 90/10 (4.0 min); flow rate: 3.4 mL/min; column temperature: 35°C; pressure: 2000 psi).

**Compound 025:** $^1H$ NMR (400 MHz, $CD_3OD$) δ ppm 8.35 (s, 1 H) 8.20 (s, 1 H) 7.40 (d, *J*= 1.43 Hz, 1 H) 7.18 - 7.25 (m, 2 H) 5.22 (t, *J* = 5.84 Hz, 1 H) 3.37 (s, 3 H) 2.62 - 2.82 (m, 3 H) 2.22 - 2.37 (m, 3 H) 1.99 - 2.08 (m, 1 H) 1.76 - 1.92 (m, 3 H) 1.69 (td, *J* = 9.00, 4.29 Hz, 1 H) 1.19 (t, *J* = 7.63 Hz, 3 H) 0.62 (q, *J* = 4.65 Hz, 1 H); ESI-LCMS: *m/z* = 376.2 $[M+H]^+$; *ee* = 99.90%, retention time: 2.458 minutes.

**Compound 026:** $^1H$ NMR (400 MHz, $CD_3OD$) δ ppm 8.35 (s, 1 H) 8.20 (s, 1 H) 7.40 (d, *J*= 1.43 Hz, 1 H) 7.18 - 7.26 (m, 2 H) 5.22 (t, *J*= 5.84 Hz, 1 H) 3.37 (s, 3 H) 2.60 - 2.82 (m, 3 H) 2.21 - 2.36 (m, 3 H) 1.98 - 2.10 (m, 1 H) 1.74 - 1.95 (m, 3 H) 1.69 (td, *J* = 9.00, 4.29 Hz, 1 H) 1.19 (t, J=7.63 Hz, 3 H) 0.62 (q, *J*=4.81 Hz, 1 H); ESI-LCMS: m/z = 376.2 $[M+H]^+$; *ee* = 99.88%, retention time: 2.804 minutes.

**Example 22**

Synthetic route:

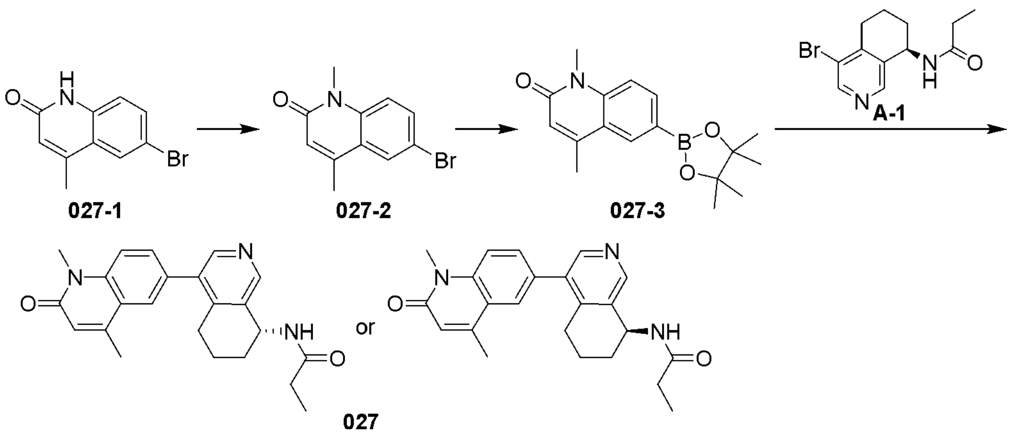


027-1 027-2 027-3

or

027

Step 1: synthesis of compound 027-2

To a reaction flask were sequentially added 027-1 (300 mg, 1.26 mmol), *N,N*-dimethylformamide (3 mL), and cesium carbonate (821.12 mg, 2.52 mmol). The atmosphere was purged with nitrogen, and the reaction mixture was cooled to 0°C, followed by the addition of methyl iodide (715.41 mg, 5.04 mmol). The reaction mixture was stirred at 25°C for 1 hour. The reaction mixture was added with water (15 mL), transferred to a separatory funnel, and extracted three times with ethyl acetate (5 mL each time). The organic phases were combined and washed twice with saturated brine (5 mL each time). The organic phase was collected, dried over anhydrous sodium sulfate, filtered, and concentrated to obtain a crude product. The crude product was purified by column chromatography (petroleum ether:ethyl acetate = 1:0 to 6:1) to obtain 027-2. $^1H$ NMR (400 MHz, $CDCl_3$) δ = 7.81 (d, *J* = 2.1 Hz, 1H), 7.66 (dd, *J* = 2.3, 9.0 Hz, 1H), 7.24 (s, 1H), 6.63 (s, 1H), 3.69

(s, 3H), 2.45 (s, 3H).

Step 2: synthesis of compound 027-3

To a reaction flask were sequentially added 027-2 (210 mg, 832.98 μmol), bis(pinacolato)diboron (423.05 mg, 1.67 mmol), 1,4-dioxane (8.4 mL), and potassium acetate (245.25 mg, 2.50 mmol). The atmosphere was purged with nitrogen, followed by the addition of methanesulfonato(2-dicyclohexylphosphino-2',4',6'-tri-i-propyl-1,1'-biphenyl)(2'-amino-1,1'-biphenyl-2-yl)palladium(II) (35.25 mg, 41.65 μmol). The reaction mixture was stirred at 80°C for 16 hours to obtain a reaction mixture of 027-3, which was used directly in the next step without further purification. ESI-LCMS: *m/z* = 300.0 $[M+H]^+$.

Step 3: synthesis of compound 027

To a reaction flask were sequentially added A-1 (58.68 mg, 207.24 μmol), water (1 mL), acetonitrile (3 mL), methanol (1 mL), potassium carbonate (114.57 mg, 828.96 μmol), and 2-dicyclohexylphosphino-2,6-dimethoxybiphenyl (12.76 mg, 31.09 μmol). The atmosphere was purged with nitrogen, followed by the addition of bis(triphenylphosphine) palladium(II) dichloride (7.27 mg, 10.36 μmol) and palladium(II) acetate (2.33 mg, 10.36 μmol). The atmosphere was again purged with nitrogen. The reaction mixture was heated to 40°C, and a reaction mixture of 027-3 was added. The reaction mixture was heated to 75°C and stirred for 4 hours. The reaction mixture was cooled to room temperature and concentrated to obtain a crude product. The crude product was sequentially subjected to preparative thin-layer chromatography (petroleum ether:ethyl acetate:methanol = 1:1:0.1) and chiral resolution (column: DAICEL CHIRALPAK IG (250 mm*30 mm, 10 μm); mobile phase: [supercritical carbon dioxide-ethanol (0.1% ammonia)]; isocratic elution: 50% ethanol (0.1% ammonia)) to obtain 027. $^1$H NMR (400 MHz, $CD_3OD$) δ = 8.40 (s, 1H), 8.27 (s, 1H), 7.79 (d, *J* = 1.9 Hz, 1H), 7.74 - 7.70 (m, 1H), 7.67 - 7.63 (m, 1H), 6.65 (d, *J* = 1.1 Hz, 1H), 5.23 (t, *J* = 6.0 Hz, 1H), 4.58 (s, 3H), 3.79 (s, 3H), 2.80 - 2.59 (m, 2H), 2.54 (d, *J* = 1.1 Hz, 3H), 2.29 (dq, *J* = 2.0, 7.6 Hz, 2H), 2.12 - 1.98 (m, 1H), 1.91 - 1.87 (m, 1H), 1.20 (t, *J* = 7.6 Hz, 3H); ESI-LCMS: *m/z* = 376.2 $[M+H]^+$; SFC analysis was performed (chromatographic column: Chiralpak IG-3, 50 × 4.6 mm I.D., 3 μm; mobile phase: A: $CO_2$, B: ethanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 2.133 minutes.

**Example 23**

Synthetic route:

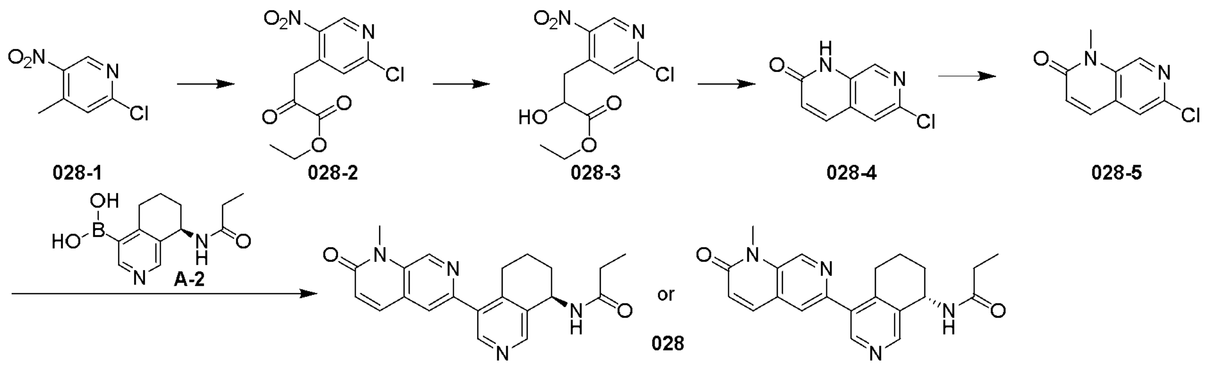

028-1 028-2 028-3 028-4 028-5

A-2

or

028

Step 1: synthesis of compound 028-2

To a reaction flask were sequentially added 028-1 (2 g, 11.59 mmol) and diethyl oxalate (16.94 g, 115.90 mmol). The atmosphere was purged with nitrogen, followed by the addition of 1,8-diazabicyclo[5.4.0]undec-7-ene (2.03 g, 13.33 mmol). The reaction mixture was stirred at 25°C for 15 hours. The reaction mixture was poured into ice water (20 mL), and 1M hydrochloric acid (15 mL) was added. The mixture was filtered, and the filter cake was collected. The filter cake was added to anhydrous methanol (10 mL), stirred for 10 minutes, and filtered. The filter cake was collected to obtain 028-2. $^1$H NMR (400 MHz, $CDCl_3$) δ = 8.96 (s, 1H), 8.26 (s, 1H), 7.26 (d, *J*= 1.3 Hz, 1H), 6.97 (d, *J* = 0.9 Hz, 1H), 4.45 (q, *J* = 7.1 Hz, 2H), 1.43 (t, *J*= 7.1 Hz, 3H).

Step 2: synthesis of compound 028-3

To a reaction flask were sequentially added 028-2 (1.7 g, 6.24 mmol), water (3 mL), and tetrahydrofuran (12 mL). The atmosphere was purged with nitrogen, and the reaction mixture was cooled to 0°C, followed by the portion-wise addition of sodium borohydride (259.49 mg, 6.86 mmol). The reaction mixture was stirred at 0°C for 0.5 hours. The reaction mixture was added with ice water (5 mL) and a saturated aqueous ammonium chloride solution (5 mL), transferred to a separatory funnel, and extracted three times with ethyl acetate (10 mL each time). The organic phases were combined and washed with saturated brine (10 mL). The organic phase was collected, dried over anhydrous sodium sulfate, filtered, and concentrated to obtain a crude product. The crude product was purified by column chromatography (petroleum ether:ethyl acetate=1:0 to 4:1) to obtain 028-3. $^1$H NMR (400 MHz, $CDCl_3$) $\delta$ = 8.97 (s, 1H), 7.48 (s, 1H), 4.50 (td, *J* = 4.2, 8.4 Hz, 1H), 4.30 (dq, *J* = 3.6, 7.1 Hz, 2H), 3.60 (dd, *J* = 3.9, 14.0 Hz, 1H), 3.24 (dd, *J* = 8.4, 13.9 Hz, 1H), 3.01 (d, *J* = 4.8 Hz, 1H), 1.33 (t, *J* = 7.2 Hz, 3H).

Step 3: synthesis of compound 028-4

To a reaction flask were sequentially added 028-3 (0.8 g, 2.91 mmol), iron powder (650.70 mg, 11.65 mmol), and acetic acid (12 mL). The atmosphere was purged with nitrogen, and the reaction mixture was stirred at 70°C for 0.5 hours. Then, 1,4-dioxane (8 mL) and hydrochloric acid (6 M, 5.83 mL) were added. The reaction mixture was heated to 90°C and stirred for 3.5 hours. The mixture was then cooled to 25°C and poured into an aqueous solution of saturated sodium bicarbonate (200 mL). After stirring for 0.5 hours, the mixture was filtered, and the filter cake was collected. 028-4 was obtained and used directly in the next step without purification. ESI-LCMS: m/z = 181.0 $[M+H]^+$.

Step 5: synthesis of compound 028-5

To a reaction flask were sequentially added 028-4 (0.2 g, 1.11 mmol), cesium carbonate (541.25 mg, 1.66 mmol), and *N*,*N*-dimethylformamide (4 mL). The atmosphere was purged with nitrogen, and the reaction mixture was cooled to 0°C, followed by the addition of iodomethane (628.77 mg, 4.43 mmol). The reaction mixture was stirred at 0°C for 1 hour. The reaction mixture was added with water (20 mL), then transferred to a separatory funnel, and extracted three times with ethyl acetate (10 mL each time). The organic phases were combined and washed with saturated brine (20 mL). The organic phase was collected, dried over anhydrous sodium sulfate, filtered, and concentrated to obtain a crude product. The crude product was purified by preparative thin-layer chromatography (petroleum ether:ethyl acetate = 1:1) to obtain 028-5. $^1$H NMR (400 MHz, $CDCl_3$) $\delta$ = 8.58 (s, 1H), 7.60 (d, *J* = 9.6 Hz, 1H), 7.48 (s, 1H), 6.95 (d, *J* = 9.6 Hz, 1H), 3.77 (s, 3H).

Step 4: synthesis of compound 028

To a reaction flask were sequentially added 028-5 (60 mg, 308.30 $\mu$mol), sodium carbonate (98.03 mg, 924.89 $\mu$mol), water (0.3 mL), and 1,4-dioxane (1.8 mL). The atmosphere was purged with nitrogen, followed by the addition of methanesulfonato(2-dicyclohexylphosphino-2',4',6'-tri-i-propyl-1,1'-biphenyl)(2'-amino-1,1'-biphenyl-2-yl)palladium(II) (26.10 mg, 30.83 $\mu$mol) and 2-di-tert-butylphosphino-2',4',6'-triisopropylbiphenyl (29.39 mg, 61.66 $\mu$mol, 0.2 *eq*). The reaction mixture was heated to 85°C, and a crude solution of A-2 (prepared immediately prior to use according to the synthetic method of Reference Example 2, added based on a theoretical yield of 114.73 mg, 462.45 $\mu$mol) was added. The reaction mixture was stirred at 85°C for 0.5 hours. The reaction mixture was cooled to room temperature, added with water (2 mL), transferred to a separatory funnel, and extracted three times with dichloromethane (2 mL each time). The organic phases were combined and washed with saturated brine (2 mL). The organic phase was collected, dried over anhydrous sodium sulfate, filtered, and concentrated to obtain a crude product. The crude product was sequentially purified by preparative thin-layer chromatography (dichloromethane:ethyl acetate:methanol = 5:5:1) and chiral resolution (column: DAICEL CHIRALPAK AD (250 mm*30 mm, 10 $\mu$m); mobile phase: [supercritical carbon dioxide-isopropanol (0.1% ammonia)]; isocratic elution: 50% isopropanol (0.1% ammonia)) to obtain 028. $^1$H NMR (400 MHz, $CD_3OD$) $\delta$ = 9.05 (s, 1H), 8.44 (s, 1H), 8.40 (s, 1H), 8.00 (d, *J* = 9.5 Hz, 1H), 7.83 (s, 1H), 6.97 (d, *J* = 9.5 Hz, 1H), 5.24 (t, *J*= 6.1 Hz, 1H), 3.86 (s, 3H), 2.96 - 2.85 (m, 1H), 2.82 - 2.71 (m, 1H), 2.29 (dq, *J*= 2.1, 7.6 Hz, 2H), 2.13 - 2.00 (m, 1H), 1.96 - 1.76 (m, 3H), 1.20 (t, *J* = 7.6 Hz, 3H); ESI-LCMS: m/z = 363.2 $[M+H]^+$; SFC analysis was performed (chromatographic column: Chiralpak AD-3, 50 $\times$ 4.6 mm I.D., 3 $\mu$m; mobile phase: A: $CO_2$, B: isopropanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%, retention time: 1.827 minutes.

**Example 24**

Synthetic route:

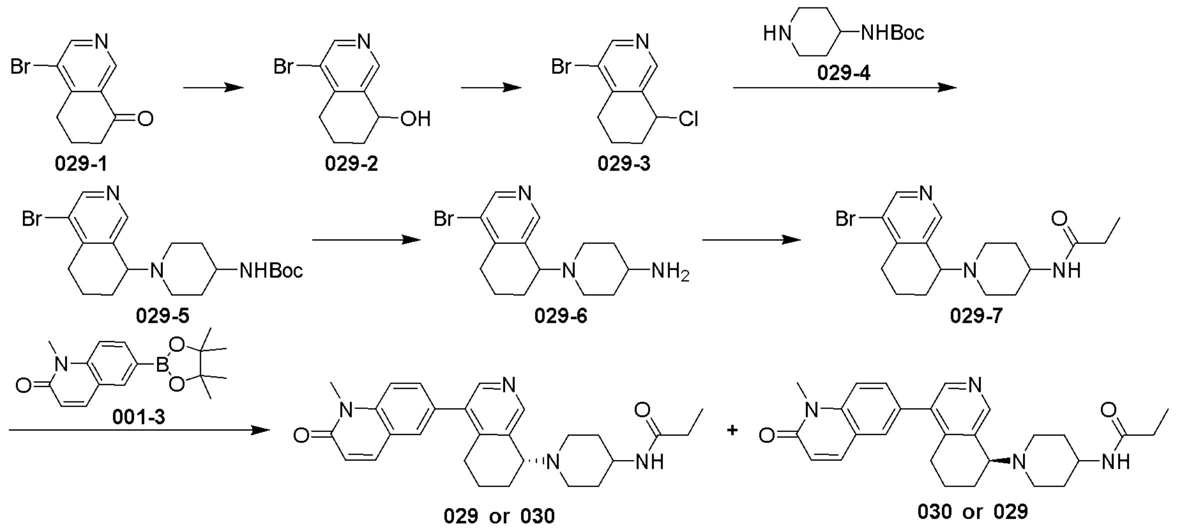

029-1 029-2 029-3 029-4 029-5 029-6 029-7 001-3

029 or 030 + 030 or 029

Step 1: synthesis of compound 029-2

To a reaction flask were sequentially added 029-1 (0.45 g, 1.99 mmol), acetic acid (956.25 mg, 15.92 mmol), and isopropanol (4 mL). The atmosphere was purged with nitrogen, followed by portion-wise addition of sodium cyanoborohydride (375.26 mg, 5.97 mmol). The reaction mixture was stirred at 75°C for 14 hours. The reaction mixture was adjusted to pH 7-8 by adding a saturated aqueous solution of sodium bicarbonate, transferred to a separatory funnel, and extracted three times with dichloromethane (5 mL each time). The organic phases were combined and washed with saturated brine (5 mL). The organic phase was collected, dried over anhydrous sodium sulfate, filtered, and concentrated to obtain crude 029-2. The crude product was used directly in the next step without purification. $^{1}H$ NMR (400 MHz, $CDCl_3$) $\delta$ = 8.60 (s, 1H), 8.57 (s, 1H), 4.88 (t, *J* = 4.9 Hz, 1H), 2.88 - 2.83 (m, 1H), 2.72 - 2.65 (m, 1H), 2.10 - 1.79 (m, 5H).

Step 2: synthesis of compound 029-3

To a reaction flask were sequentially added 029-2 (0.4 g, 1.75 mmol) and dichloromethane (4 mL). The atmosphere was purged with nitrogen, and the reaction mixture was cooled to 0°C, followed by the addition of thionyl chloride (312.96 mg, 2.63 mmol). The reaction mixture was stirred at 0°C for 1 hour, then warmed to 25°C and stirred for 1.5 hours. The reaction mixture was concentrated to obtain crude 029-3, which was used directly in the next step without purification. $^{1}H$ NMR (400 MHz, $CDCl_3$) $\delta$ = 8.83 (s, 1H), 8.74 (s, 1H), 5.52 - 5.36 (m, 1H), 3.15 (td, *J* = 4.5, 19.6 Hz, 1H), 2.93 - 2.80 (m, 1H), 2.40 - 2.22 (m, 3H), 2.15 - 2.05 (m, 1H).

Step 3: synthesis of compound 029-5

To a reaction flask were sequentially added 029-3 (0.36 g, 1.46 mmol), 029-4 (350.95 mg, 1.75 mmol), *N*,*N*-diisopropylethylamine (566.19 mg, 4.38 mmol), potassium iodide (242.41 mg, 1.46 mmol), and acetonitrile (5 mL). The atmosphere was purged with nitrogen, and the mixture was stirred at 40°C for 2 hours. The reaction mixture was subsequently heated to 80°C and stirred for 16 hours. The reaction mixture was cooled to room temperature and concentrated to obtain a crude product. The crude product was purified by column chromatography (petroleum ether:ethyl acetate = 1:0 to 1:1) to obtain 029-5. $^{1}H$ NMR (400 MHz, $CDCl_3$) $\delta$ = 8.79 (s, 1H), 8.51 (s, 1H), 4.49 (br s, 1H), 3.88 (br d, *J* = 2.5 Hz, 1H), 3.47 (br d, *J*= 1.3 Hz, 1H), 2.81 (br d, *J* = 17.3 Hz, 2H), 2.73 - 2.52 (m, 3H), 2.22 (br d, *J* = 7.5 Hz, 1H), 2.11 (br d, *J*= 9.3 Hz, 1H), 1.99 (br dd, *J*= 5.1, 9.3 Hz, 2H), 1.87 (br d, *J*= 9.9 Hz, 1H), 1.59 - 1.32 (m, 13H).

Step 4: synthesis of compound 029-6

To a reaction flask were sequentially added 029-5 (0.2 g, 487.39 $\mu$mol) and hydrogen chloride/ethyl acetate (4 M, 10.00 mL). The atmosphere was purged with nitrogen, and the reaction mixture was stirred at 25°C for 0.5 hours. The reaction mixture was concentrated to obtain a hydrochloride of 029-6, which was used directly in the next step without

purification. $^1H$ NMR (400 MHz, DMSO-$d_6$) δ = 8.98 (s, 1H), 8.75 (s, 1H), 4.69 (br s, 1H), 3.43 - 3.19 (m, 4H), 3.18 - 3.04 (m, 1H), 2.91 - 2.66 (m, 2H), 2.36 - 2.22 (m, 2H), 2.19 - 1.99 (m, 4H), 1.97 - 1.92 (m, 1H), 1.86 - 1.73 (m, 1H).

Step 5: synthesis of compound 029-7

To a reaction flask were sequentially added the hydrochloride salt of 029-6 (220 mg), N,N-diisopropylethylamine (410.07 mg, 3.17 mmol), and dichloromethane (5 mL). The atmosphere was purged with nitrogen, and the reaction mixture was cooled to 0°C, followed by the addition of propionic anhydride (99.10 mg, 761.48 μmol). The reaction mixture was stirred at 0°C for 1 hour. The reaction mixture was added with water (2 mL) and extracted three times with dichloromethane (2 mL each time). The organic phases were combined and washed with saturated brine (2 mL). The organic phase was collected, dried over anhydrous sodium sulfate, filtered, and concentrated to obtain crude 029-7. The crude product was purified by preparative thin-layer chromatography (dichloromethane: ethyl acetate: methanol = 5:5:1) to obtain 029-7. $^1H$ NMR (400 MHz, $CDCl_3$) δ = 8.80 (s, 1H), 8.51 (s, 1H), 5.34 (br d, *J* = 4.6 Hz, 1H), 3.97 - 3.73 (m, 2H), 2.90 - 2.70 (m, 3H), 2.66 - 2.49 (m, 2H), 2.32 - 2.24 (m, 1H), 2.20 (q, *J* = 7.7 Hz, 2H), 2.11 (br d, *J* = 5.0 Hz, 1H), 2.04 - 1.95 (m, 2H), 1.90 - 1.82 (m, 1H), 1.67 (br s, 1H), 1.59 - 1.53 (m, 2H), 1.46 (d, *J* = 6.6 Hz, 1H), 1.17 - 1.12 (m, 3H).

Step 6: synthesis of compounds 029 and 030

To a reaction flask were sequentially added 029-7 (0.1 g, 273.00 μmol), 001-3 (93.41 mg, 327.60 μmol), sodium carbonate (57.87 mg, 546.01 μmol), water (0.7 mL), and ethanol (2 mL). The atmosphere was purged with nitrogen, followed by the addition of tetrakis(triphenylphosphine)palladium (31.55 mg, 27.30 μmol). The reaction mixture was stirred at 85°C for 2 hours. The reaction mixture was allowed to return to room temperature, added with water (5 mL), transferred to a separatory funnel, and extracted three times with dichloromethane (5 mL each time). The organic phases were combined, washed with saturated brine (5 mL), dried over anhydrous sodium sulfate, filtered, and concentrated to obtain a crude product. The crude product was sequentially purified by preparative thin-layer chromatography (dichloromethane:ethyl acetate:methanol = 3:3:1) and SFC chiral resolution (chromatographic column: Chiralpak AD-3, 50×4.6 mm I.D., 3 μm; mobile phase: A: supercritical carbon dioxide, B: ethanol (0.2% 7M ammonia in methanol); flow rate: 3.4 mL/min; column temperature: 35°C; pressure: 1800 psi) to obtain 029 and 030. SFC analysis was subsequently performed (chromatographic column: Chiralpak AD-3, 50 × 4.6 mm I.D., 3 μm; mobile phase: A: carbon dioxide, B: ethanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi).

**Compound 029:** $^1H$ NMR (400 MHz, $CD_3OD$) δ = 8.83 (s, 1H), 8.21 (s, 1H), 7.97 (d, *J* = 9.5 Hz, 1H), 7.73 - 7.68 (m, 2H), 7.67 - 7.63 (m, 1H), 6.74 (d, *J* = 9.5 Hz, 1H), 4.03 (br dd, *J* = 5.5, 9.6 Hz, 1H), 3.80 (s, 3H), 3.73 - 3.61 (m, 1H), 3.00 - 2.90 (m, 1H), 2.80 - 2.68 (m, 2H), 2.64 - 2.52 (m, 2H), 2.38 - 2.28 (m, 1H), 2.19 (q, *J* = 7.5 Hz, 2H), 2.06 - 1.90 (m, 3H), 1.87 - 1.76 (m, 2H), 1.71 - 1.58 (m, 2H), 1.44 (dq, *J* = 3.7, 11.6 Hz, 1H), 1.13 (t, *J* = 7.6 Hz, 3H); ESI-LCMS: *m*/*z* = 445.3 $[M+H]^+$; *ee* = 100%, retention time: 1.489 minutes.

**Compound 030:** $^1H$ NMR (400 MHz, $CD_3OD$) δ = 8.83 (s, 1H), 8.21 (s, 1H), 7.97 (d, *J* = 9.5 Hz, 1H), 7.73 - 7.69 (m, 2H), 7.67 - 7.63 (m, 1H), 6.74 (d, *J* = 9.5 Hz, 1H), 4.03 (br dd, *J* = 5.6, 9.5 Hz, 1H), 3.80 (s, 3H), 3.72 - 3.61 (m, 1H), 2.95 (br d, *J* = 11.3 Hz, 1H), 2.82 - 2.68 (m, 2H), 2.65 - 2.51 (m, 2H), 2.32 (dt, *J* = 2.1, 11.4 Hz, 1H), 2.19 (q, *J* = 7.6 Hz, 2H), 2.09 - 1.90 (m, 3H), 1.88 - 1.76 (m, 2H), 1.72 - 1.57 (m, 2H), 1.44 (dq, *J* = 3.9, 11.6 Hz, 1H), 1.12 (t, *J* = 7.7 Hz, 3H); ESI-LCMS: *m*/*z* = 445.3 $[M+H]^+$; *ee* = 100%, retention time: 1.806 minutes.

**Example 25**

Synthetic route:

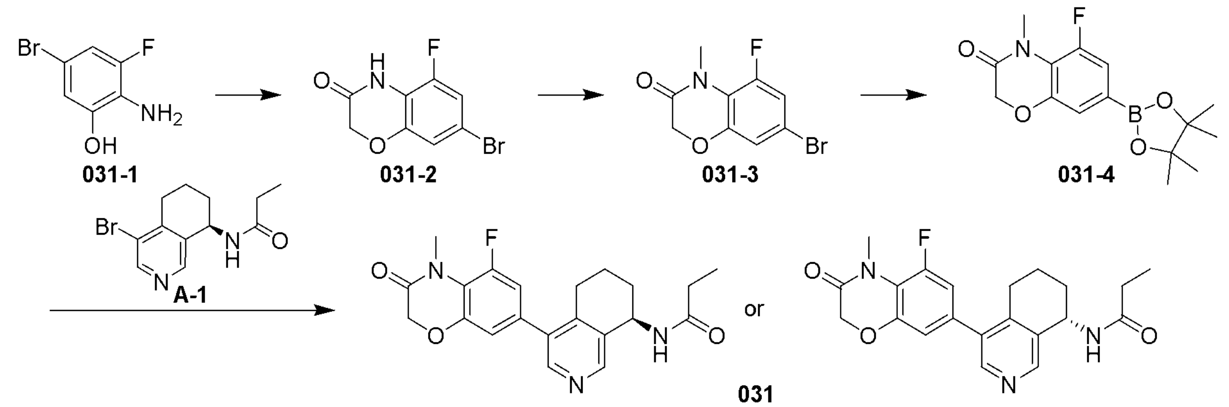

031-1 031-2 031-3 031-4

A-1

or

031

Step 1: synthesis of compound 031-2

To a reaction flask were sequentially added 031-1 (0.3 g, 1.46 mmol), potassium carbonate (402.52 mg, 2.91 mmol), and *N*,*N*-dimethylformamide (3 mL). The atmosphere was purged with nitrogen, followed by the addition of chloroacetyl chloride (180.92 mg, 1.60 mmol). The reaction mixture was stirred at 80°C for 2 hours. The reaction mixture was allowed to return to room temperature, added with water (30 mL), then transferred to a separatory funnel, and extracted three times with ethyl acetate (10 mL each time). The organic phases were combined and washed with saturated brine (20 mL). The organic phase was collected, dried over anhydrous sodium sulfate, filtered, and concentrated to obtain crude 031-2. The crude product was purified by preparative thin-layer chromatography (petroleum ether: ethyl acetate = 4:1) to obtain 031-2. $^1$H NMR (400 MHz, DMSO-$d_6$) $\delta$ = 11.01 (br s, 1H), 7.22 (dd, *J* = 2.0, 9.8 Hz, 1H), 7.08 (t, *J* = 1.7 Hz, 1H), 4.64 (s, 2H).

Step 2: synthesis of compound 031-3

To a reaction flask were sequentially added 031-2 (0.1 g, 406.45 $\mu$mol), cesium carbonate (198.64 mg, 609.67 $\mu$mol), and *N*,*N*-dimethylformamide (2 mL). The atmosphere was purged with nitrogen, and the reaction mixture was cooled to 0°C, followed by the addition of iodomethane (230.76 mg, 1.63 mmol). The reaction mixture was stirred at 0°C for 1 hour. The reaction mixture was added with water (10 mL), then transferred to a separatory funnel, and extracted three times with ethyl acetate (3 mL each time). The organic phases were combined and washed with saturated brine (5 mL). The organic phase was collected, dried over anhydrous sodium sulfate, filtered, and concentrated to obtain a crude product. The crude product was purified by preparative thin-layer chromatography (petroleum ether:ethyl acetate = 5:1) to obtain 031-3. $^1$H NMR (400 MHz, DMSO-$d_6$) $\delta$ = 7.34 (dd, *J* = 1.9, 12.1 Hz, 1H), 7.19 (s, 1H), 4.67 (s, 2H), 3.33 (br s, 3H).

Step 3: synthesis of compound 031-4

To a reaction flask were sequentially added 031-3 (100 mg, 384.53 $\mu$mol), bis(pinacolato)diboron (146.47 mg, 576.79 $\mu$mol), potassium acetate (113.21 mg, 1.15 mmol), and 1,4-dioxane (2 mL). The atmosphere was purged with nitrogen, followed by the addition of [1,1'-bis(diphenylphosphino)ferrocene]dichloropalladium(II) (28.14 mg, 38.45 $\mu$mol). The reaction mixture was stirred at 80°C for 4 hours. The reaction mixture was allowed to return to room temperature and concentrated to obtain crude 031-4, which was used directly in the next step without purification.

Step 4: synthesis of compound 031

To a reaction flask were sequentially added A-1 (100 mg, 353.15 $\mu$mol), 031-4 (108.46 mg, 353.15 $\mu$mol), sodium carbonate (74.86 mg, 706.31 $\mu$mol), water (0.5 mL), and ethanol (3 mL). The atmosphere was purged with nitrogen, followed by the addition of tetrakis(triphenylphosphine)palladium (40.81 mg, 35.32 $\mu$mol). The reaction mixture was stirred at 85°C for 1 hour. The reaction mixture was allowed to return to room temperature, diluted with water (5 mL), and extracted with dichloromethane (5 mL x 3). The organic phases were combined, washed with saturated brine (2 mL), dried over anhydrous sodium sulfate, filtered, and concentrated to obtain a crude product. The crude product was sequentially purified by column chromatography (ethyl acetate:methanol = 1:0 to 10:1) and SFC chiral resolution (chromatographic column: DAICEL CHIRALPAKAD (250 mm*30 mm, 10 $\mu$m); mobile phase: [supercritical carbon dioxide-isopropanol (0.1% ammonia)]; isocratic elution: 50% isopropanol (0.1% ammonia)) to obtain 031. $^1$H NMR (400 MHz, $CD_3OD$) $\delta$ = 8.37 (s, 1H), 8.19 (s, 1H), 6.93 (dd, *J* = 1.8, 13.1 Hz, 1H), 6.88 (d, *J* = 1.3 Hz, 1H), 5.20 (t, *J* = 5.8 Hz, 1H), 4.64 (s, 2H), 3.51 (d, *J* = 5.9 Hz, 3H), 2.78 - 2.62 (m, 2H), 2.27 (dq, *J*= 1.7, 7.6 Hz, 2H), 2.07 - 1.98 (m, 1H), 1.92 - 1.76 (m, 3H), 1.19 (t, *J*= 7.6 Hz, 3H);

ESI-LCMS: m/z = 384.2 [M+H]+. SFC analysis was performed (chromatographic column: Chiralpak AD-3, 50 × 4.6 mm I.D., 3 μm; mobile phase: A: $CO_2$, B: isopropanol (0.2% 7 M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.479 minutes.

**Example 26**

Synthetic route:

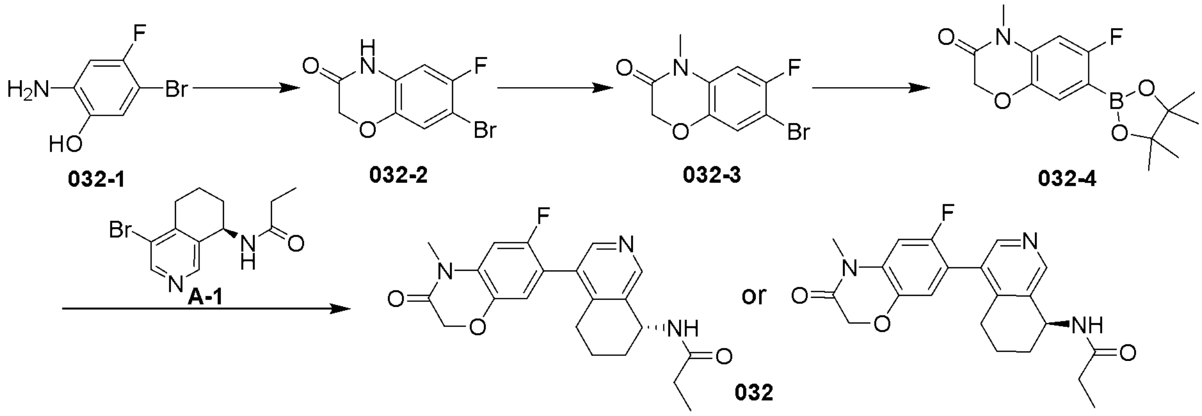

032-1 032-2 032-3 032-4 A-1 032 or

Step 1: synthesis of compound 032-2

To a reaction flask were sequentially added 032-1 (0.3 g, 1.46 mmol), potassium carbonate (402.53 mg, 2.91 mmol), and N,N-dimethylformamide (3 mL). The atmosphere was purged with nitrogen, followed by the addition of chloroacetyl chloride (180.92 mg, 1.60 mmol). The reaction mixture was stirred at 80°C for 2 hours. The reaction mixture was allowed to return to room temperature, added with water (30 mL), then transferred to a separatory funnel, and extracted three times with ethyl acetate (10 mL each time). The organic phases were combined and washed with saturated brine (20 mL). The organic phase was collected, dried over anhydrous sodium sulfate, filtered, and concentrated to obtain a crude product. The crude product was purified by preparative thin-layer chromatography (petroleum ether:ethyl acetate = 4:1) to obtain 032-2. $^1$H NMR (400 MHz, DMSO-$d_6$) δ = 10.88 (br s, 1H), 7.31 (d, *J* = 6.4 Hz, 1H), 6.83 (d, *J*= 9.2 Hz, 1H), 4.59 (s, 2H).

Step 2: synthesis of compound 032-3

To a reaction flask were sequentially added 032-2 (100 mg, 406.45 μmol), cesium carbonate (198.64 mg, 609.67 μmol), and *N*,*N*-dimethylformamide (1.5 mL). The atmosphere was purged with nitrogen, and the reaction mixture was cooled to 0°C, followed by the addition of iodomethane (230.76 mg, 1.63 mmol). The reaction mixture was stirred at 0°C for 1 hour. The reaction mixture was added with water (10 mL), then transferred to a separatory funnel, and extracted three times with ethyl acetate (5 mL each time). The organic phases were combined and washed with saturated brine (10 mL). The organic phase was collected, dried over anhydrous sodium sulfate, filtered, and concentrated to obtain a crude product. The crude product was purified by preparative thin-layer chromatography (petroleum ether:ethyl acetate = 5:1) to obtain 032-3. $^1$H NMR (400 MHz, DMSO-$d_6$) δ = 7.36 (d, *J* = 6.5 Hz, 1H), 7.30 (d, *J*= 10.1 Hz, 1H), 4.67 (s, 2H), 3.24 (s, 3H).

Step 3: synthesis of compound 032-4

To a reaction flask were sequentially added 032-3 (120 mg, 461.43 μmol), bis(pinacolato)diboron (175.76 mg, 692.15 μmol), potassium acetate (135.86 mg, 1.38 mmol), and 1,4-dioxane (3 mL). The atmosphere was purged with nitrogen, followed by the addition of [1,1'-bis(diphenylphosphino)ferrocene]dichloropalladium(II) (33.76 mg, 46.14 μmol). The reaction mixture was stirred at 85°C for 16 hours. The reaction mixture was allowed to return to room temperature and concentrated to obtain crude 032-4, which was used directly in the next step without purification.

Step 4: synthesis of compound 032

To a reaction flask were sequentially added A-1 (120 mg, 423.78 μmol), 032-4 (130.15 mg, 423.78 μmol), sodium carbonate (89.83 mg, 847.57 μmol), water (0.5 mL), and ethanol (3 mL). The atmosphere was purged with nitrogen, followed by the addition of tetrakis(triphenylphosphine)palladium (48.97 mg, 42.38 μmol). The reaction mixture was stirred at 85°C for 1 hour. The reaction mixture was allowed to return to room temperature, added with water (5 mL), and extracted three times with dichloromethane (5 mL each time). The organic phases were combined and washed with saturated brine (2 mL). The organic phase was collected, dried over anhydrous sodium sulfate, filtered, and concentrated to obtain a crude product. The crude product was sequentially subjected to column chromatography (ethyl acetate:methanol = 1:0 to 10:1, 10 SFC chiral resolution (chromatographic column: DAICEL CHIRALPAK AD (250 mm*30 mm, 10 μm); mobile phase: [supercritical carbon dioxide-isopropanol (0.1% ammonia)]; isocratic elution: 50% isopropanol (0.1% ammonia)) to obtain 032. $^1$H NMR (400 MHz, $CD_3OD$) δ = 8.39 (s, 1H), 8.18 (s, 1H), 7.11 (d, *J* = 10.6 Hz, 1H), 6.92 (d, *J* = 6.6 Hz, 1H), 5.22 (br t, *J* = 5.8 Hz, 1H), 4.66 (s, 2H), 3.39 (s, 3H), 2.73 - 2.51 (m, 2H), 2.27 (dq, *J* = 2.0, 7.6 Hz, 2H), 2.11 - 1.97 (m, 1H), 1.94 - 1.75 (m, 3H), 1.19 (t, *J* = 7.6 Hz, 3H); ESI-LCMS: *m*/*z* = 384.2 $[M+H]^+$; SFC analysis was performed (chromatographic column: Chiralpak AD-3, 50 × 4.6 mm I.D., 3 μm; mobile phase: A: $CO_2$, B: isopropanol (0.2% 7 M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.795 minutes.

**Example 27**

Synthetic route:

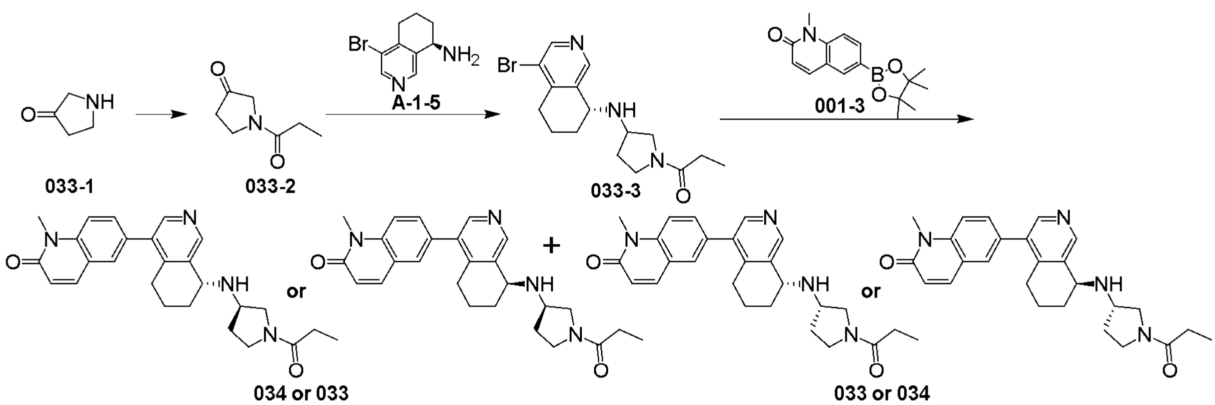


033-1 033-2 033-3

or + or

034 or 033 033 or 034

Step 1: synthesis of compound 033-2

To a reaction flask were sequentially added a hydrochloride of 033-1 (2 g, 16.45 mmol), dichloromethane (20 mL), and N,N-diisopropylethylamine (5.32 g, 41.13 mmol). The atmosphere was purged with nitrogen, and the reaction mixture was cooled to 0°C, followed by the addition of a solution of propionic anhydride (3.00 g, 23.03 mmol) in dichloromethane (10 mL). The reaction mixture was stirred at 0°C for 1 hour. The reaction mixture was added with water (10 mL), transferred to a separatory funnel, and extracted with 10 mL of dichloromethane. The resulting phases were separated and the organic phase was collected. The aqueous phase was extracted three times with a mixed solvent (dichloromethane:methanol=10:1) (10 mL each time). The organic phases were combined and washed twice with saturated brine (10 mL each time). The organic phase was collected, dried over anhydrous sodium sulfate, filtered, and concentrated to obtain a crude product. The crude product was purified by column chromatography (petroleum ether:ethyl acetate = 10:1 to dichloromethane:methanol = 20:1) to obtain 033-2. $^1$H NMR (400 MHz, $CDCl_3$) δ = 3.94 - 3.85 (m, 4H), 2.70 (t, *J* = 7.9 Hz, 1H), 2.62 (t, *J* = 7.9 Hz, 1H), 2.41 - 2.34 (m, 1H), 2.27 (q, *J*= 7.4 Hz, 1H), 1.21 - 1.16 (m, 3H).

Step 2: synthesis of compound 033-3

To a reaction flask were sequentially added a hydrochloride ofA-1-5 (0.7 g), methanol (7 mL), triethylamine (631.53 mg, 6.24 mmol), 033-2 (734.19 mg, 5.20 mmol), and acetic acid (249.85 mg, 4.16 mmol). The mixture was stirred for 15 minutes, followed by the addition of sodium cyanoborohydride (653.64 mg, 10.40 mmol). The reaction mixture was then stirred at 25°C for 1 hour. The reaction mixture was adjusted to pH 8-9 by adding saturated sodium bicarbonate

solution, transferred to a separatory funnel, and extracted three times with ethyl acetate (20 mL each time). The organic phases were combined and washed with saturated brine (20 mL). The organic phase was collected, dried over anhydrous sodium sulfate, filtered, and concentrated to obtain crude 033-3. The crude product was purified by column chromatography (petroleum ether:ethyl acetate = 10:1 to dichloromethane:methanol = 20:1) to obtain 033-3. $^1$H NMR (400 MHz, $CDCl_3$) δ = 8.53 (d, *J* = 3.4 Hz, 1H), 8.51 - 8.47 (m, 1H), 3.98 - 3.83 (m, 1H), 3.73 - 3.57 (m, 3H), 3.52 - 3.44 (m, 1H), 3.12 (s, 2H), 2.88 - 2.78 (m, 1H), 2.70 - 2.59 (m, 1H), 2.31 - 2.25 (m, 2H), 2.19 - 2.10 (m, 1H), 2.02 - 1.96 (m, 1H), 1.92 (br d, *J* = 4.0 Hz, 1H), 1.89 - 1.82 (m, 2H), 1.38 (s, 3H).

Step 3: synthesis of compounds 033 and 034

To a reaction flask were sequentially added 001-3 (553.67 mg, 1.94 mmol), potassium carbonate (894.51 mg, 6.47 mmol), 2-dicyclohexylphosphino-2,6-dimethoxybiphenyl (99.64 mg, 242.71 μmol), bis(triphenylphosphine)palladium dichloride (56.79 mg, 80.90 μmol), palladium acetate (18.16 mg, 80.90 μmol), water (5.5 mL), acetonitrile (16.5 mL), and methanol (5.5 mL). The atmosphere was purged with nitrogen, and the reaction mixture was maintained at 40°C, followed by the addition of a solution of 033-3 (0.57 g, 1.62 mmol) in tetrahydrofuran (5.5 mL). The reaction mixture was then heated to 75°C and stirred for 2 hours. The reaction mixture was allowed to return to room temperature and filtered through anhydrous sodium sulfate. The filtrate was collected and concentrated to obtain a crude product. The crude product was sequentially purified by preparative thin-layer chromatography (ethyl acetate:dichloromethane:methanol = 1:1 :0.3) and SFC chiral resolution (chromatographic column: ChiralPak IH, 250*30 mm, 10 μm); mobile phase: supercritical carbon dioxide-ethanol: acetonitrile =1:1 (0.1% ammonia)]; isocratic elution: 50% ethanol:acetonitrile = 1:1 (0.1% ammonia)) to obtain 033 and 034. The *ee* value was determined by SFC analysis (chromatographic column: Chiralpak IH-3, 50×4.6 mm I.D., 3 μm; mobile phase: A: $CO_2$, B: ethanol:acetonitrile (0.1% 7M ammonia in methanol); gradient: A/B = 65:35; flow rate: 4 mL/min; column temperature: 35°C; pressure: 1800 psi).

**Compound 033:** $^1$H NMR (400 MHz, $CD_3OD$) δ = 8.58 (d, *J* = 10.6 Hz, 1H), 8.21 (d, *J*= 3.1 Hz, 1H), 7.97 (d, *J* = 9.5 Hz, 1H), 7.75 - 7.61 (m, 3H), 6.74 (d, *J* = 9.5 Hz, 1H), 4.03 - 3.95 (m, 1H), 3.80 (s, 3H), 3.79 - 3.50 (m, 4H), 3.49 - 3.33 (m, 2H), 2.81 - 2.71 (m, 1H), 2.68 - 2.57 (m, 1H), 2.43 - 2.31 (m, 2H), 2.28 - 2.13 (m, 1H), 2.02 (br d, *J*= 12.1 Hz, 1H), 1.98 - 1.79 (m, 3H), 1.79 - 1.68 (m, 1H), 1.15 (dt, *J* = 0.8, 7.5 Hz, 3H); ESI-LCMS: m/z = 431.3 $[M+H]^+$; *ee* = 100%, retention time 2.289 minutes.

**Compound 034:** $^1$H NMR (400 MHz, $CD_3OD$) δ = 8.60 (s, 1H), 8.22 (d, *J* = 2.3 Hz, 1H), 7.97 (d, *J* = 9.5 Hz, 1H), 7.72 - 7.60 (m, 3H), 6.74 (d, *J* = 9.4 Hz, 1H), 4.03 (td, *J* = 4.8, 9.6 Hz, 1H), 3.80 (s, 3H), 3.77 (br d, *J* = 3.1 Hz, 4H), 3.45 (td, *J* = 7.5, 12.2 Hz, 1H), 3.29 - 3.22 (m, 1H), 2.79 - 2.71 (m, 1H), 2.68 - 2.58 (m, 1H), 2.36 (qd, *J*= 7.5, 11.7 Hz, 2H), 2.25 - 2.15 (m, 1H), 2.05 - 1.90 (m, 4H), 1.73 (br dd, *J*= 2.1, 5.7 Hz, 1H), 1.13 (dt, *J*= 3.5, 7.5 Hz, 3H); ESI-LCMS: *m*/*z* = 431.2 $[M+H]^+$; *ee* = 100%; retention time 3.580 minutes.

**Example 28**

Synthetic route:

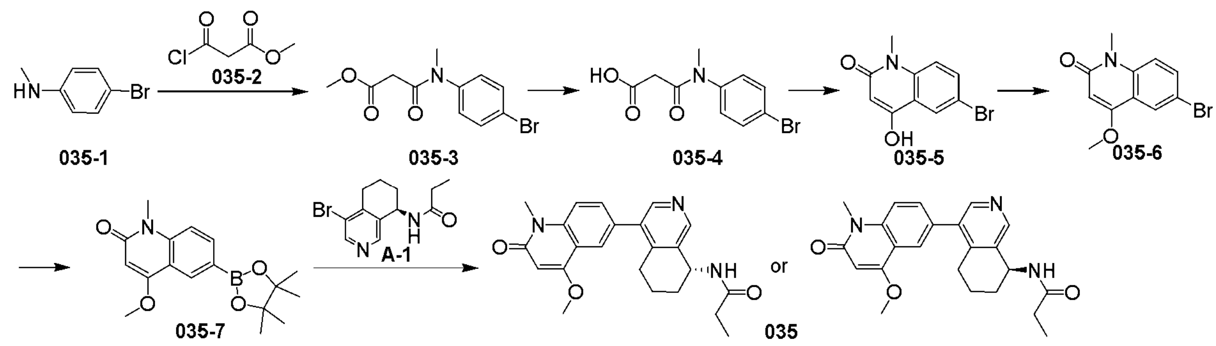


035-1 035-3 035-4 035-5 035-6 035-7 035

or

Step 1: synthesis of compound 035-3

To a reaction flask were sequentially added 035-1 (2.2 g, 11.82 mmol) and dichloromethane (34 mL). The atmosphere was purged with nitrogen, and the reaction mixture was cooled to 0°C, followed by the addition of a solution of 035-2 (1.94 g, 14.19 mmol) in dichloromethane (34 mL). The reaction mixture was then warmed to 25°C and stirred for 12 hours. The reaction mixture was added with water (10 mL), then transferred to a separatory funnel, and extracted three

times with dichloromethane (30 mL each time). The resulting phases were separated. The organic phases were combined and washed twice with saturated brine (20 mL each time). The organic phase was collected, dried over anhydrous sodium sulfate, filtered, and concentrated to obtain a crude product. The crude product was purified by column chromatography (petroleum ether:ethyl acetate = 10:1 to 1:1) to obtain 035-3. $^1$H NMR (400 MHz, $CDCl_3$) δ ppm 7.57 (d, *J* =8.50 Hz, 2 H) 7.13 (d, *J* =8.63 Hz, 2 H) 3.69 (s, 3 H) 3.29 (s, 3 H) 3.23 (s, 2 H).

Step 2: synthesis of compound 035-4

To a reaction flask were sequentially added 035-3 (3.3 g, 11.53 mmol), tetrahydrofuran (33 mL), and an aqueous solution (33 mL) of lithium hydroxide monohydrate (967.98 mg, 23.07 mmol). The reaction mixture was stirred at 25°C for 12 hours. The reaction mixture was adjusted to a pH value of 1 to 2 by adding 12 M hydrochloric acid, further added with water (15 mL), and extracted twice with ethyl acetate (30 mL each time). The organic phases were combined and washed with saturated brine (30 mL). The organic phase was collected, dried over anhydrous sodium sulfate, filtered, and concentrated to obtain a crude product. The crude product was added with 25 mL of a mixed solvent (petroleum ether:ethyl acetate = 10:1), stirred for 0.5 hours, and filtered. The filter cake was collected and dried to obtain 035-4. $^1$H NMR (400 MHz, DMSO-$d_6$) δ ppm 7.66 (br d, *J* =7.63 Hz, 2 H) 7.32 (br d, *J* =7.63 Hz, 2 H) 3.17 (br s, 3 H) 3.11 (br s, 2 H).

Step 3: synthesis of compound 035-5

To a reaction flask were sequentially added 035-4 (2.8 g, 10.29 mmol) and Eaton's reagent (30.30 g, 127.28 mmol). The atmosphere was purged with nitrogen, and the reaction mixture was stirred at 70°C for 12 hours. The reaction mixture was added to ice water (100 mL), stirred at 0°C for 0.5 hours, and filtered to collect the filter cake. The filter cake was then added to acetonitrile (28 mL), stirred at 25°C for 1 hour, and filtered to collect the filter cake, yielding 035-5. $^1$H NMR (400 MHz, DMSO-$d_6$) δ ppm 11.59 (s, 1 H) 7.95 (d, *J* = 2.38 Hz, 1 H) 7.76 (dd, *J* = 9.01, 2.38 Hz, 1 H) 7.44 (d, *J* = 9.01 Hz, 1 H) 5.89 (s, 1 H) 3.51 (s, 3 H).

Step 4: synthesis of compound 035-6

To a reaction flask were sequentially added 035-5 (0.4 g, 1.57 mmol) and *N,N*-dimethylformamide (10 mL). The atmosphere was purged with nitrogen, followed by the addition of sodium carbonate (166.86 mg, 1.57 mmol). The atmosphere was again purged with nitrogen, and the reaction mixture was cooled to 0°C, followed by the addition of iodomethane (335.18 mg, 2.36 mmol). The reaction mixture was stirred at 0°C for 2 hours, then warmed to 25°C and stirred for 10 hours. The reaction mixture was added with 10 mL of water, then transferred to a separatory funnel, and extracted three times with ethyl acetate (30 mL each time). The organic phases were combined and washed twice with saturated brine (30 mL each time). The organic phase was collected, dried over anhydrous sodium sulfate, filtered, and concentrated to obtain a crude product. The crude product was purified by column chromatography (petroleum ether:ethyl acetate = 10:1 to 1:1) to obtain 035-6. $^1$H NMR (400 MHz, DMSO-$d_6$) δ = 7.95 (d, *J*= 2.1 Hz, 1H), 7.79 (dd, *J* = 2.2, 9.1 Hz, 1H), 7.48 (d, *J* = 9.1 Hz, 1H), 6.09 (s, 1H), 3.94 (s, 3H), 3.54 (s, 3H).

Step 5: synthesis of compound 035-7

To a reaction flask were sequentially added 035-6 (0.16 g, 596.78 μmol), 1,4-dioxane (7 mL), bis(pinacolato) diboron (227.32 mg, 895.17 μmol), potassium acetate (175.70 mg, 1.79 mmol), and methanesulfonato(2-dicyclohexylphosphino-2,4,6-tri-i-propyl-1,1'-biphenyl)(2-amino-1,1-biphenyl-2-yl)palladium(II) (25.26 mg, 29.84 μmol). The atmosphere was purged with nitrogen, and the reaction mixture was stirred at 80°C for 6 hours. The reaction mixture was filtered, and the filter cake was rinsed with ethyl acetate. The filtrate was collected and concentrated to obtain crude 035-7. The crude product was used directly in the next step without purification. ESI-LCMS: m/z = 316.2 $[M+H]^+$.

Step 6: synthesis of compound 035

To a reaction flask were sequentially added A-1 (80 mg, 282.52 μmol), water (1.7 mL), acetonitrile (5.1 mL), methanol (1.7 mL), potassium carbonate (156.18 mg, 1.13 mmol), and 2-dicyclohexylphosphino-2,6-dimethoxybiphenyl (17.40 mg, 42.38 μmol). The atmosphere was purged with nitrogen, followed by the addition of bis(triphenylphosphine) palladium(II) dichloride (9.92 mg, 14.13 μmol) and palladium(II) acetate (3.17 mg, 14.13 μmol). The atmosphere was again purged with nitrogen. The reaction mixture was heated to 40°C, and a solution of 035-7 (178.09 mg, 565.04 μmol) in tetrahydrofuran (2.6 mL) was added. The reaction mixture was heated to 75°C and stirred for 12 hours. The reaction mixture was filtered. The filtrate was collected and concentrated to obtain a crude product. The crude product was sequentially subjected to preparative thin-layer chromatography (ethyl acetate:dichloromethane:methanol = 1:1:0.3) and

SFC chiral resolution (chromatographic column: DAICEL CHIRALPAK IG (250 mm*30 mm, 10 μm); mobile phase: [supercritical carbon dioxide-ethanol (0.1% ammonia)]; isocratic elution: 50% ethanol (0.1% ammonia)) to obtain 035. $^{1}H$ NMR (400 MHz, $CDCl_3$) δ = 8.54 (br s, 1H), 8.33 (br s, 1H), 7.91 (d, *J* = 1.9 Hz, 1H), 7.51 (dd, *J* = 2.0, 8.6 Hz, 1H), 7.42 (d, *J* = 8.8 Hz, 1H), 6.09 (s, 1H), 5.81 (br d, *J* = 8.6 Hz, 1H), 5.40 - 5.33 (m, 1H), 3.96 (s, 3H), 3.72 (s, 3H), 2.74 - 2.54 (m, 2H), 2.30 (q, *J* = 7.5 Hz, 2H), 2.15 - 2.07 (m, 1H), 1.87 - 1.80 (m, 3H), 1.23 (t, *J*= 7.6 Hz, 3H); ESI-LCMS: m/z = 392.2 $[M+H]^+$. SFC analysis was performed (chromatographic column: Chiralpak IG-3, 100 × 4.6 mm I.D., 3 μm; mobile phase: A: $CO_2$, B: methanol (0.2% 7 M ammonia in methanol); gradient: A/B = 50:50; flow rate: 4 mL/min; column temperature: 35°C; pressure: 2000 psi), *ee* = 100%, retention time: 2.119 minutes.

**Example 29**

Synthetic route:

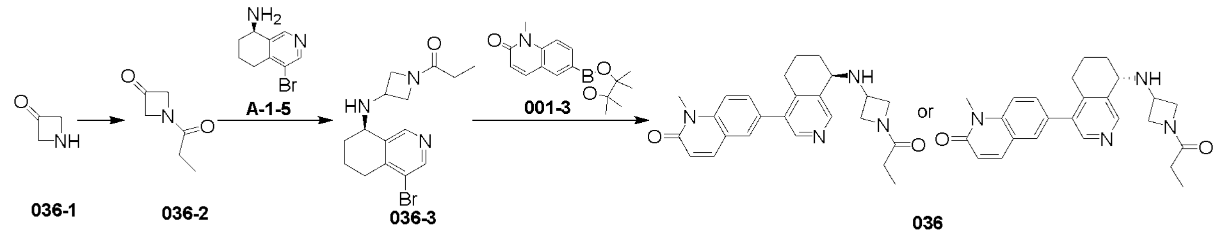

036-1
036-2
036-3
or
036

Step 1: synthesis of compound 036-2

To a reaction flask were sequentially added a hydrochloride of 036-1 (2 g, 18.60 mmol), dichloromethane (20 mL), and N,N-diisopropylethylamine (4.49 g, 34.72 mmol). The atmosphere was purged with nitrogen, and the reaction mixture was cooled to 0°C, followed by the addition of a solution of propionic anhydride (2.53 g, 19.44 mmol) in dichloromethane (5 mL). The reaction mixture was stirred at 0°C for 1 hour. The reaction mixture was poured into 2 mL of water. The resulting phases were separated, and the organic phase was collected and concentrated to obtain crude 036-2, which was used directly in the next step without purification. $^{1}H$ NMR (400 MHz, $CDCl_3$) δ = 4.81 (br d, *J* = 13.8 Hz, 4H), 2.31 (q, *J*= 7.5 Hz, 2H), 1.19 (t, *J*= 7.6 Hz, 3H).

Step 2: synthesis of compound 036-3

To a reaction flask were sequentially added a hydrochloride ofA-1-5 (0.5 g), methanol (5 mL), triethylamine (451.09 mg, 4.46 mmol), 036-2 (472.31 mg, 3.71 mmol), and acetic acid (178.46 mg, 2.97 mmol). The reaction mixture was stirred at 25°C for 0.5 hours, followed by the addition of sodium cyanoborohydride (466.89 mg, 7.43 mmol). The atmosphere was purged with nitrogen, and the mixture was stirred at 25°C for 15.5 hours. The reaction mixture was poured into 5 mL of saturated aqueous sodium bicarbonate solution and extracted three times with dichloromethane (5 mL each time). The organic phases were combined and washed three times with saturated brine (5 mL each time). The organic phase was collected, dried over anhydrous sodium sulfate, filtered, and concentrated to obtain a crude product. The crude product was purified by preparative thin-layer chromatography (dichloromethane: ethyl acetate: methanol = 1:1:0.1) to obtain 036-3. ESI-LCMS: *m*/*z* = 338.0 $[M+H]^+$.

Step 3: synthesis of compound 036

To a reaction flask were sequentially added 001-3 (50.58 mg, 177.39 μmol), potassium carbonate (81.72 mg, 591.29 μmol), 2-dicyclohexylphosphino-2,6-dimethoxybiphenyl (9.10 mg, 22.17 μmol), bis(triphenylphosphine)palladium dichloride (5.19 mg, 7.39 μmol), palladium acetate (1.66 mg, 7.39 μmol), water (0.4 mL), acetonitrile (1.2 mL), and methanol (0.4 mL). The atmosphere was purged with nitrogen, and the reaction mixture was heated to 40°C, followed by the addition of a solution of 036-3 (50 mg, 147.82 μmol) in tetrahydrofuran (0.4 mL). The reaction mixture was heated to 75°C and stirred for 1 hour. The reaction mixture was allowed to return to room temperature, added to 2 mL of water, transferred to a separatory funnel, and extracted three times with dichloromethane (3 mL each time). The organic phases were combined and washed three times with saturated brine (3 mL each time). The organic phase was collected, dried over anhydrous sodium sulfate, filtered, and concentrated to obtain a crude product. The crude product was sequentially purified by preparative thin-layer chromatography (dichloromethane:ethyl acetate:methanol = 1:1:0.3), preparative high-performance liquid chromatography (chromatographic column: Waters Xbridge Prep OBD C18 150*40 mm*10 μm; mobile phase: [water (10 mM ammonium bicarbonate)-acetonitrile]; gradient: 5%-45% 10 mM ammonium bicarbonate), and SFC chiral resolution (chromatographic column: REGIS(S,S)WHELK-O1 (250 mm*25 mm,10 μm); mobile phase: [supercritical carbon dioxide-isopropanol:acetonitrile = 1:1 (0.1% ammonia)]; isocratic elution: 50% isopropanol:acetonitrile = 1:1 (0.1% ammonia)) to obtain 036. $^1$H NMR (400 MHz, $CD_3OD$) δ = 8.56 (d, *J*= 3.8 Hz, 1H), 8.24 (s, 1H), 7.97 (d, *J*= 9.4 Hz, 1H), 7.75 - 7.68 (m, 2H), 7.67 - 7.61 (m, 1H), 6.74 (d, *J* = 9.4 Hz, 1H), 4.47 - 4.33 (m, 1H), 4.27 - 4.13 (m, 1H), 4.01 - 3.87 (m, 3H), 3.78 (br s, 3H), 3.78 - 3.68 (m, 1H), 2.83 - 2.70 (m, 1H), 2.69 - 2.58 (m, 1H), 2.17 (q, *J* = 7.5 Hz, 2H), 2.00 - 1.83 (m, 3H), 1.78 - 1.68 (m, 1H), 1.10 (t, *J* = 7.6 Hz, 3H); ESI-LCMS: *m/z* = 417.3 [M+H]$^+$; SFC analysis was performed (chromatographic column: (S,S)-WHELK-O1, 50×4.6 mm I.D., 3.5 μm; mobile phase: A: $CO_2$ B: 0.1% isopropylamine in (isopropanol:acetonitrile = 1:1); gradient: A/B=55:45; flow rate: 4 mL/min; column temperature: 35°C; pressure: 1800 psi), *ee* = 99.62%, retention time 2.720 minutes.

**Example 30**

Synthetic route:

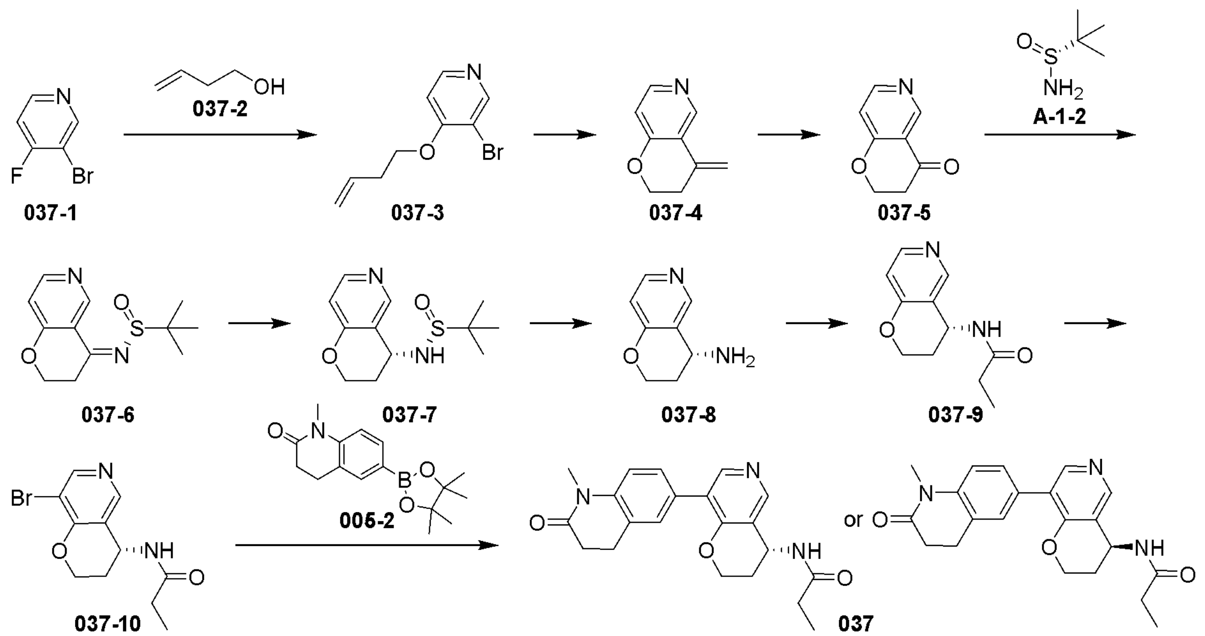

037-1 037-3 037-4 037-5 037-6 037-7 037-8 037-9 037-10 or 037

Step 1: synthesis of compound 037-3

To a reaction flask were sequentially added 037-2 (26.43 g, 366.51 mmol) and tetrahydrofuran (450 mL). The atmosphere was purged with nitrogen, and the reaction mixture was cooled to 0°C, followed by the addition of potassium tert-butoxide (38.38 g, 342.07 mmol). The mixture was stirred at 0°C for 30 minutes. A solution of 037-1 (43 g, 244.34 mmol) in tetrahydrofuran (22.5 mL) was added. The mixture was stirred at 0°C for 10 minutes. The reaction mixture was diluted with an aqueous solution of ammonium chloride (300 mL), then transferred to a separatory funnel, and extracted three times with ethyl acetate (300 mL each time). The organic phases were combined and washed with saturated brine (300 mL). The organic phase was collected, dried over anhydrous sodium sulfate, filtered, and concentrated to obtain a crude product. The crude product was purified by column chromatography (petroleum ether:ethyl acetate=1:0 to 6:1) to obtain 037-3. $^1$H NMR (400 MHz, $CDCl_3$) δ = 8.57 (s, 1H), 8.36 (d, *J* = 5.6 Hz, 1H), 6.79 (d, *J* = 5.6 Hz, 1H), 5.91 (tdd, *J* = 6.8, 10.3, 17.1 Hz, 1H), 5.25 - 5.12 (m, 2H), 4.13 (t, *J*= 6.6 Hz, 2H), 2.62 (q, *J*= 6.6 Hz, 2H).

Step 2: synthesis of compound 037-4

To a reaction flask were sequentially added 037-3 (28.1 g, 123.20 mmol) and *N,N*-dimethylformamide (281 mL). The atmosphere was purged with nitrogen, followed by the addition of acetic acid (60.46 g, 616.00 mmol), 1,2-bis(diphenylphosphino)ethane (9.82 g, 24.64 mmol), and palladium acetate (2.77 g, 12.32 mmol). The atmosphere

was again purged with nitrogen, and the reaction mixture was stirred at 100°C for 2 hours. The reaction mixture was allowed to cool to room temperature, added with water (1.4 L), then transferred to a separatory funnel, and extracted three times with ethyl acetate (500 mL each time). The organic phases were combined and washed twice with saturated brine (500 mL each time). The organic phase was collected, dried over anhydrous sodium sulfate, filtered, and concentrated to obtain a crude product. The crude product was purified by column chromatography (petroleum ether:ethyl acetate = 1:0 to 1:1) to obtain 037-4. $^1$H NMR (400 MHz, $CDCl_3$) δ = 8.72 (s, 1H), 8.26 (d, *J* = 5.7 Hz, 1H), 6.76 (d, *J* = 5.7 Hz, 1H), 5.62 (s, 1H), 5.00 (s, 1H), 4.37 - 4.26 (m, 2H), 2.69 (t, *J* = 5.7 Hz, 2H).

Step 3: synthesis of compound 037-5

To a reaction flask were sequentially added 037-4 (2 g, 13.59 mmol), tert-butanol (10 mL), acetonitrile (10 mL), and water (10 mL). The atmosphere was purged with nitrogen, followed by the addition of osmium tetroxide (345.48 mg, 1.36 mmol). The reaction mixture was stirred at 25°C for 0.5 hours. Sodium periodate (5.81 g, 27.18 mmol) was then added, and the mixture was stirred at 25°C for 4 hours. The reaction mixture was added with water (20 mL), then transferred to a separatory funnel, and extracted with a mixed solvent (ethyl acetate:ethanol = 5:1) twice (20 mL each time). The organic phases were combined and washed with saturated brine (50 mL). The organic phase was collected, dried over anhydrous sodium sulfate, filtered, and concentrated to obtain a crude product. The crude product was purified by column chromatography (petroleum ether:ethyl acetate=1:0 to 2:1) to obtain 037-5. $^1$H NMR (400 MHz, $CDCl_3$) δ = 9.02 (s, 1H), 8.53 (d, *J* = 5.9 Hz, 1H), 6.91 (d, *J* = 5.9 Hz, 1H), 4.70 - 4.56 (m, 2H), 2.93 - 2.79 (m, 2H).

Step 4: synthesis of compound 037-6

To a reaction flask were sequentially added 037-5 (400 mg, 2.68 mmol), tetrahydrofuran (4 mL), A-1-2 (325.05 mg, 2.68 mmol), and tetraethyl titanate (1.84 g, 8.05 mmol). The atmosphere was purged with nitrogen, and the reaction mixture was stirred at 50°C for 16 hours. The reaction mixture was allowed to cool to room temperature, and added with ethyl acetate (4 mL) and an aqueous solution of sodium glycolate (3.4 mL). The mixture was stirred at 25°C for 30 minutes. The resulting phases were separated. The aqueous phase was extracted twice with ethyl acetate (2 mL each time). The organic phases were combined and washed with saturated brine (2 mL). The organic phase was collected, dried over anhydrous sodium sulfate, filtered, and concentrated to obtain a crude product. The crude product was purified by column chromatography (petroleum ether:ethyl acetate=1:0 to 1:1) to obtain 037-6. $^1$H NMR (400 MHz, $CDCl_3$) δ = 9.10 (s, 1H), 8.44 (d, *J* = 5.8 Hz, 1H), 6.86 (d, *J*= 5.8 Hz, 1H), 4.53 - 4.35 (m, 2H), 3.62 - 3.50 (m, 1H), 3.45 - 3.33 (m, 1H), 1.34 (s, 9H).

Step 5: synthesis of compound 037-7

To a reaction flask were sequentially added 037-6 (420 mg, 1.66 mmol) and tetrahydrofuran (4.2 mL). The atmosphere was purged with nitrogen, and the reaction mixture was cooled to 0°C, followed by the addition of 9-borabicyclo[3.3.1]nonane (0.5 M, 9.99 mL). The reaction mixture was warmed to 25°C and stirred for 1 hour. The reaction mixture was added with 20% aqueous solution of citric acid (23 mL), adjusted to a pH value of 8 to 9 by adding a saturated sodium bicarbonate solution, transferred to a separatory funnel, and extracted three times with ethyl acetate (20 mL each time). The organic phases were combined and washed with saturated brine (20 mL). The organic phase was collected, dried over anhydrous sodium sulfate, filtered, and concentrated to obtain a crude product. The crude product was purified by column chromatography (dichloromethane:methanol = 1:0 to 20:1) to obtain 037-7. $^1$H NMR (400 MHz, $CDCl_3$) δ = 8.51 (s, 1H), 8.33 (d, *J* = 5.8 Hz, 1H), 6.79 (d, *J* = 5.8 Hz, 1H), 5.31 (s, 1H), 4.73 - 4.61 (m, 1H), 4.40 - 4.31 (m, 2H), 2.25 - 2.10 (m, 2H), 1.25 (s, 9H).

Step 6: synthesis of compound 037-8

To a reaction flask were sequentially added 037-7 (270 mg, 1.06 mmol) and ethanol (5.4 mL). The atmosphere was purged with nitrogen, and the reaction mixture was cooled to 0°C, followed by the addition of trimethylsilyl chloride (230.66 mg, 2.12 mmol). The reaction mixture was stirred at 25°C for 1 hour. The reaction mixture was concentrated to obtain crude 037-8, which was used directly in the next step without purification. ESI-LCMS: *m*/*z* = 151.3 $[M+H]^+$.

Step 7: synthesis of compound 037-9

To a reaction flask were sequentially added crude 037-8, dichloromethane (2 mL), N,N-diisopropylethylamine (289.16 mg, 2.24 mmol). The atmosphere was purged with nitrogen, and the reaction mixture was cooled to 0°C, followed by the addition of a solution of propionic anhydride (194.11 mg, 1.49 mmol) in dichloromethane (1 mL). The mixture was stirred at 0°C for 1 hour. The reaction mixture was added with water (5 mL), then transferred to a separatory funnel, and

extracted three times with dichloromethane (2 mL each time). The organic phases were combined and washed with saturated brine (2 mL each time). The organic phase was collected, dried over anhydrous sodium sulfate, filtered, and concentrated to obtain a crude product. The crude product was purified by thin-layer chromatography (dichloromethane:ethyl methanol = 10:1) to obtain 037-9. ESI-LCMS: m/z = 207.1 $[M+H]^+$.

Step 8: synthesis of compound 037-10

To a reaction flask were sequentially added 037-9 (95 mg, 460.63 μmol) and sulfuric acid (1 mL). The atmosphere was purged with nitrogen, and the reaction mixture was cooled to 0°C, followed by the addition of N-bromosuccinimide (163.96 mg, 921.25 μmol). The reaction mixture was warmed to 25°C and stirred for 19 hours. The reaction mixture was added to a saturated aqueous sodium bicarbonate solution (ice-water mixture), and the pH was adjusted to 7-8. The mixture was extracted three times with ethyl acetate (5 mL each time). The organic phases were combined and washed with saturated brine (5 mL). The organic phase was collected, dried over anhydrous sodium sulfate, filtered, and concentrated to obtain a crude product. The crude product was purified by preparative thin-layer chromatography (dichloromethane:methanol = 10:1) to obtain 037-10. $^1H$ NMR (400 MHz, $CD_3OD$) δ ppm 8.40 (s, 1 H), 8.21 (s, 1 H), 5.18 (t, *J* =5.63 Hz, 1 H), 4.41 - 4.55 (m, 2 H), 2.23 - 2.32 (m, 2 H), 2.05 - 2.22 (m, 2 H), 1.16 (t, *J*= 7.63 Hz, 3 H).

Step 9: synthesis of compound 037

To a reaction flask were sequentially added 005-2 (58.01 mg, 202.01 μmol), 037-10 (48 mg, 168.34 μmol), ethanol (3 mL), and an aqueous solution (0.5 mL) of sodium carbonate (19.63 mg, 185.17 μmol). The atmosphere was purged with nitrogen, followed by the addition of methanesulfonato(2-dicyclohexylphosphino-2,4,6-triisopropyl-1,1-biphenyl)(2-amino-1,1-biphenyl-2-yl)palladium(II) (4.27 mg, 5.05 μmol). The atmosphere was again purged with nitrogen, and the reaction mixture was stirred at 85°C for 2 hours. The reaction mixture was then concentrated to obtain a crude product. The crude product was purified by preparative thin-layer chromatography (dichloromethane:methanol = 30:1) to obtain 037. $^1H$ NMR (400 MHz, $CD_3OD$) δ = 8.24 (d, *J* = 3.2 Hz, 2H), 7.44 (dd, *J* = 1.6, 8.4 Hz, 1H), 7.40 (s, 1H), 7.19 (d, *J*= 8.3 Hz, 1H), 5.21 (t, *J*= 5.5 Hz, 1H), 4.47 - 4.28 (m, 2H), 3.39 (s, 3H), 2.97 (t, *J*= 7.4 Hz, 2H), 2.66 (t, *J* = 7.4 Hz, 2H), 2.28 (dq, *J* = 2.3, 7.6 Hz, 2H), 2.24 - 2.04 (m, 2H), 1.18 (t, *J* = 7.6 Hz, 3H); ESI-LCMS: *m/z* = 366.2 $[M+H]^+$. SFC analysis was performed (chromatographic column: Chiralpak IG-3, 50 × 4.6 mm I.D., 3 μm; mobile phase: A: $CO_2$, B: ethanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 98.96%; retention time: 2.179 minutes.

**Example 31**

Synthetic route:

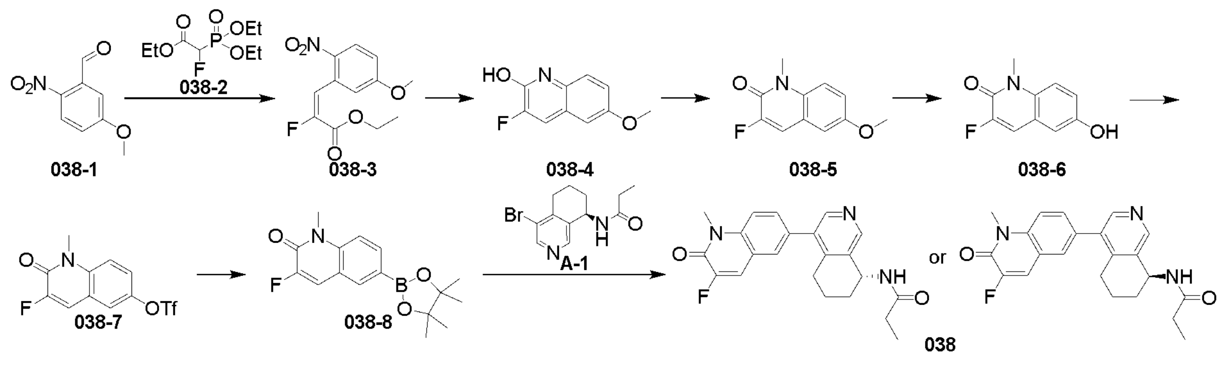

038-1
038-3
038-4
038-5
038-6
038-7
038-8
or
038

Step 1: synthesis of compound 038-3

To a reaction flask were sequentially added 038-2 (18.72 g, 77.29 mmol) and tetrahydrofuran (140 mL). The atmosphere was purged with nitrogen, followed by the addition of triethylamine (15.64 g, 154.57 mmol), magnesium bromide (14.23 g, 77.29 mmol), and a solution of 038-1 (14 g, 77.29 mmol) in tetrahydrofuran (70 mL). The mixture was stirred at 25°C for 2 hours. The reaction mixture was poured into 20 mL of water and extracted three times with

dichloromethane (30 mL each time). The organic phases were combined and washed three times with saturated brine (30 mL each time). The organic phase was collected, dried over anhydrous sodium sulfate, filtered, and concentrated to obtain a crude product. The crude product was purified by silica gel column chromatography (petroleum ether:ethyl acetate = 10:1 to 1:1) to obtain 038-3. $^{1}$H NMR (400 MHz, $CDCl_3$) $\delta$ = 8.22 (d, *J* = 9.2 Hz, 1H), 7.24 (d, *J* = 17.6 Hz, 1H), 6.97 (dd, *J*= 2.8, 9.1 Hz, 1H), 6.84 (d, *J*= 2.6 Hz, 1H), 4.13 (q, *J* = 7.2 Hz, 2H), 3.90 (s, 3H), 1.13 (t, *J* = 7.2 Hz, 3H).

Step 2: synthesis of compound 038-4

To a reaction flask were sequentially added 038-3 (1 g, 3.71 mmol), methanol (35 mL), a solution of ammonium chloride (218.55 mg, 4.09 mmol) in water (10 mL), and iron powder (1.04 g, 18.57 mmol). The reaction mixture was then heated to 60°C and stirred for 2 hours. The reaction mixture was allowed to return to room temperature and filtered. The filter cake was collected to obtain crude 038-4, which was used directly in the next step without purification. ESI-LCMS: m/z = 194.1 $[M+H]^+$.

Step 3: synthesis of compound 038-5

To a reaction flask were sequentially added 038-4 (0.4 g, 2.07 mmol) and N,N-dimethylformamide (5 mL). The atmosphere was purged with nitrogen, followed by the addition of cesium carbonate (1.35 g, 4.14 mmol). The atmosphere was again purged with nitrogen, and the reaction mixture was cooled to 0°C, followed by the addition of iodomethane (1.18 g, 8.28 mmol). The mixture was stirred at 0°C for 1 hour, then warmed to 30°C and stirred for an additional 2 hours. The reaction mixture was added with 5 mL of water and filtered through diatomite. The filtrate was collected, transferred to a separatory funnel, and extracted three times with ethyl acetate (30 mL each time). The organic phases were combined and washed twice with saturated brine (30 mL each time). The organic phase was collected, dried over anhydrous sodium sulfate, filtered, and concentrated to obtain a crude product. The crude product was purified by column chromatography (petroleum ether:ethyl acetate = 10:1 to 1:1) to obtain 038-5. ESI-LCMS: m/z = 208.0 $[M+H]^+$.

Step 4: synthesis of compound 038-6

To a reaction flask were sequentially added 038-5 (564.51 mg, 2.72 mmol) and dichloromethane (4 mL). The atmosphere was purged with nitrogen, and the reaction mixture was cooled to 0°C, followed by the addition of boron tribromide (2 M, 5.45 mL). The mixture was stirred at 0°C for 1 hour. The reaction mixture was added to ice water, concentrated to remove dichloromethane, and filtered. The filter cake was collected to obtain crude 038-6, which was used directly in the next step without purification. $^{1}$H NMR (400 MHz, $CD_3OD$) $\delta$ = 7.59 (d, *J*= 9.9 Hz, 1H), 7.45 (d, *J*= 9.1 Hz, 1H), 7.13 (dd, *J*= 2.6, 9.1 Hz, 1H), 7.03 (d, *J* = 2.7 Hz, 1H), 3.76 (s, 3H).

Step 5: synthesis of compound 038-7

To a reaction flask were sequentially added 038-6 (197.93 mg, 1.02 mmol) and dichloromethane (2 mL). The atmosphere was purged with nitrogen, and the reaction mixture was cooled to -20°C, followed by the addition of pyridine (129.68 mg, 1.64 mmol) and trifluoromethanesulfonic anhydride (346.91 mg, 1.23 mmol). The mixture was stirred at -20°C for 0.5 hours and then warmed to 0°C and stirred for 2 hours. The reaction mixture was added with water (10 mL), then transferred to a separatory funnel, and extracted four times with dichloromethane (10 mL each time). The organic phases were combined, washed twice with saturated brine (10 mL each time). The organic phase was collected, dried over anhydrous sodium sulfate, filtered, and concentrated to obtain a crude product. The crude product was purified by preparative thin-layer chromatography (petroleum ether:ethyl acetate = 1:1) to obtain 038-7. ESI-LCMS: m/z = 325.9 $[M+H]^+$.

Step 6: synthesis of compound 038-8

To a reaction flask were sequentially added 038-7 (90 mg, 276.72 μmol), 1,4-dioxane (1 mL), bis(pinacolato) diboron (105.41 mg, 415.08 μmol), [1,1'-bis(diphenylphosphino)ferrocene]dichloropalladium(II) (30.37 mg, 41.51 μmol), and potassium acetate (81.47 mg, 830.17 μmol, 3 *eq*). The atmosphere was purged with nitrogen, and the reaction mixture was stirred at 100°C for 3 hours. The reaction mixture was filtered. The filtrate was collected and concentrated to obtain crude 038-8, which was used directly in the next step without purification. ESI-LCMS: *m/z* = 304.0 $[M+H]^+$.

Step 7: synthesis of compound 038

To a reaction flask were sequentially added A-1 (50 mg, 176.58 μmol), water (1 mL), acetonitrile (3 mL), methanol

(1 mL), potassium carbonate (97.62 mg, 706.31 μmol), and 2-dicyclohexylphosphino-2,6-dimethoxybiphenyl (10.87 mg, 26.49 μmol). The atmosphere was purged with nitrogen, followed by the addition of [1,1'-bis(diphenylphosphino) ferrocene]dichloropalladium(II) (6.20 mg, 8.83 μmol) and palladium(II) acetate (1.98 mg, 8.83 μmol). The atmosphere was again purged with nitrogen. The reaction mixture was heated to 40°C, and a solution of crude 038-8 (80.29 mg, 264.86 μmol) in tetrahydrofuran (1.5 mL) was added. The reaction mixture was heated to 75°C and stirred for 12 hours. The reaction mixture was allowed to return to room temperature and filtered through anhydrous sodium sulfate. The filtrate was collected and concentrated to obtain a crude product. The crude product was sequentially subjected to preparative thin-layer chromatography (ethyl acetate:dichloromethane:methanol = 1:1:0.5) and SFC chiral resolution (chromatographic column: DAICEL CHIRALPAK IG (250mm*30 mm, 10 μm); mobile phase: [supercritical carbon dioxide-ethanol (0.1% ammonia)]; isocratic elution: 50% ethanol (0.1% ammonia)) to obtain 038. $^1$H NMR (400 MHz, $CD_3OD$) δ = 8.40 (s, 1H), 8.25 (s, 1H), 7.79 (d, *J* = 9.5 Hz, 1H), 7.74 - 7.68 (m, 2H), 7.66 - 7.59 (m, 1H), 5.23 (br t, *J* = 5.8 Hz, 1H), 3.86 (s, 3H), 2.78 - 2.63 (m, 2H), 2.29 (dq, *J* = 2.2, 7.6 Hz, 2H), 2.09 - 2.00 (m, 1H), 1.94 - 1.77 (m, 3H), 1.20 (t, *J* = 7.6 Hz, 3H); ESI-LCMS: m/z = 380.1 $[M+H]^+$. SFC analysis was performed (chromatographic column: Chiralpak IG-3, 50 × 4.6 mm I.D., 3 μm; mobile phase: A: $CO_2$, B: ethanol (0.2% 7 M ammonia in methanol); isocratic elution: 50%; flow rate: 4 mL/min; column temperature: 35°C; pressure: 1800 psi), *ee* = 100%, retention time: 1.345 minutes.

**Example 32**

Synthetic route:

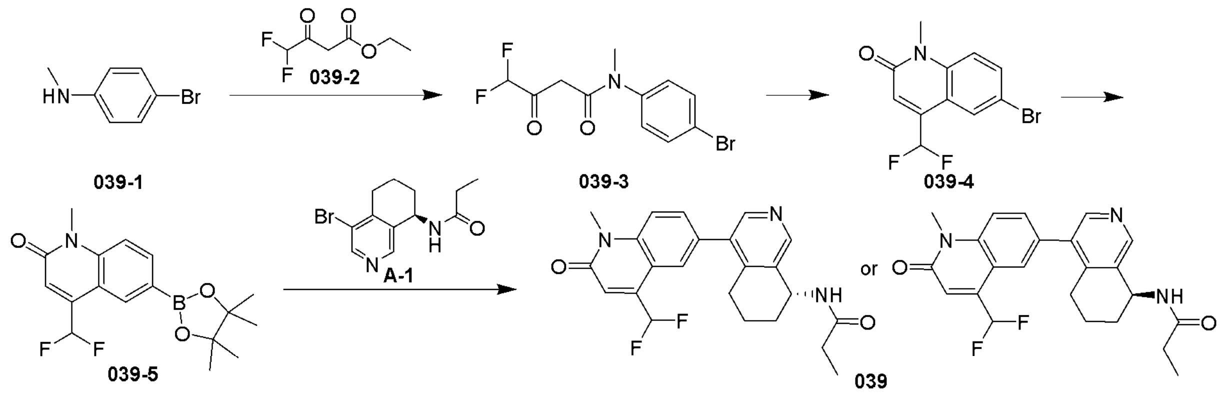

039-1
039-3
039-4
039-5
or
039

Step 1: synthesis of compound 039-3

To a reaction flask were sequentially added 039-1 (0.5 g, 2.69 mmol) and 039-2 (892.90 mg, 5.37 mmol). The atmosphere was purged with nitrogen, and the reaction mixture was heated to 130°C and stirred for 12 hours. The reaction mixture was returned to room temperature and concentrated to obtain crude 039-3. The crude product was used directly in the next step without purification.

Step 2: synthesis of compound 039-4

To a reaction flask were sequentially added 039-3 (822.6 mg, 2.69 mmol) and sulfuric acid (4.60 g, 45.96 mmol). The atmosphere was purged with nitrogen, and the reaction mixture was heated to 95°C and stirred for 1 hour. The reaction mixture was allowed to return to room temperature, added with water (7.5 mL), then transferred to a separatory funnel, and extracted twice with ethyl acetate (7.5 mL each time). The organic phases were combined and washed with saturated brine (7.5 mL). The organic phase was collected, dried over anhydrous sodium sulfate, filtered, and concentrated to obtain a crude product. The crude product was purified by column chromatography (petroleum ether:ethyl acetate = 20:1 to 5:1) to obtain 039-4. $^1$H NMR (400 MHz, $CDCl_3$) δ ppm 7.98 (s, 1 H), 7.74 (dd, *J* = 8.97, 2.17 Hz, 1 H), 7.33 (d, *J* = 9.16 Hz, 1 H), 6.97 (s, 1 H), 6.64 - 6.93 (m, 1 H), 3.73 (s, 3 H).

Step 3: synthesis of compound 039-5

To a reaction flask were sequentially added 039-4 (0.33 g, 1.15 mmol), 1,4-dioxane (6 mL), bis(pinacolato) diboron (436.32 mg, 1.72 mmol), potassium acetate (337.25 mg, 3.44 mmol), and [1,1'-bis(diphenylphosphino)ferrocene]dichloropalladium(II) (83.82 mg, 114.54 μmol). The atmosphere was purged with nitrogen, and the reaction mixture was stirred at 80°C for 18 hours. The reaction mixture was allowed to return to room temperature and filtered through diatomite. The filter cake was rinsed with ethyl acetate (5 mL). The filtrate was collected and concentrated to obtain a crude product. The crude product was purified by column chromatography (petroleum ether:ethyl acetate = 10:1 to 3:1) to obtain 039-5. ESI-LCMS: *m*/*z* =336.0[M+H]+.

Step 4: synthesis of compound 039

To a reaction flask were sequentially added A-1 (100 mg, 353.15 μmol), 039-5 (177.54 mg, 529.73 μmol), ethanol (5 mL), and sodium carbonate (74.86 mg, 706.31 μmol). The atmosphere was purged with nitrogen, followed by the addition of tetrakis(triphenylphosphine)palladium (40.81 mg, 35.32 μmol). The atmosphere was again purged with nitrogen, and the reaction mixture was heated to 85°C and stirred for 2 hours. The reaction mixture was allowed to return to room temperature, and anhydrous sodium sulfate (5 g) was added thereto. The mixture was filtered through diatomite, and the filter cake was rinsed with ethyl acetate (10 mL). The filtrate was collected and concentrated to obtain a crude product. The crude product was sequentially purified by preparative thin-layer chromatography (dichloromethane:ethyl acetate:methanol = 1:1:0.1) and chiral resolution (column: DAICEL CHIRALPAK IG (250 mm*30 mm, 10 μm); mobile phase: [supercritical carbon dioxide-isopropanol (0.1% ammonia)]; isocratic elution: 50% isopropanol (0.1% ammonia)) to obtain 039. $^1$H NMR (400 MHz, $CD_3OD$) δ ppm 8.41 (s, 1 H), 8.26 (s, 1 H), 7.87 (s, 1 H), 7.80 (d, *J* = 8.80 Hz, 1 H), 7.73 (br d, *J* = 8.80 Hz, 1 H), 7.05 - 7.33 (m, 1 H), 6.99 (s, 1 H), 5.24 (br t, *J* = 5.56 Hz, 1 H), 3.83 (s, 3 H), 2.62 - 2.76 (m, 2 H), 2.24 - 2.31 (m, 2 H), 2.03 (br d, *J*= 11.98 Hz, 1 H), 1.78 - 1.91 (m, 3 H), 1.20 (t, *J* = 7.64 Hz, 3 H). ESI-LCMS: *m*/*z* = 412.2 [M+H]+. SFC analysis was performed (chromatographic column: Chiralpak IG-3, 50 × 4.6 mm I.D., 3 μm; mobile phase: A: $CO_2$, B: isopropanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.808 minutes.

**Example 33**

Synthetic route:

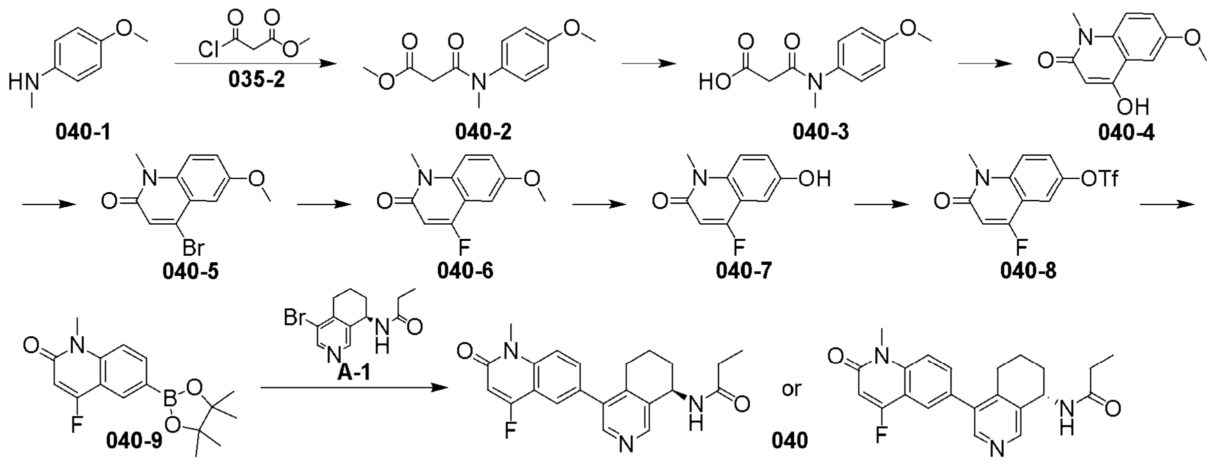


Step 1: synthesis of compound 040-2

To a reaction flask were sequentially added 040-1 (5 g, 36.45 mmol), triethylamine (4.06 g, 40.09 mmol), and dichloromethane (100 mL). The atmosphere was purged with nitrogen, followed by the addition of a solution of 035-2 (5.47 g, 40.09 mmol) in dichloromethane (7.5 mL). The mixture was stirred at 25°C for 10 minutes. The reaction mixture was dried in a rotary evaporator, dissolved in ethyl acetate (100 mL), and sequentially washed once each with 1 N citric acid (50 mL) and saturated brine (50 mL). The organic phase was collected, dried over anhydrous sodium sulfate, filtered, and concentrated to obtain 040-2, which was used directly in the next step without further purification. $^1$H NMR (400 MHz, $CDCl_3$) δ = 7.19 - 7.11 (m, 2H), 6.97 - 6.88 (m, 2H), 3.83 (s, 3H), 3.68 (s, 3H), 3.27 (s, 3H), 3.22 (s, 2H).

Step 2: synthesis of compound 040-3

To a reaction flask were sequentially added crude 040-2 (8.5 g), an aqueous sodium hydroxide solution (1 M, 42.50 mL), and methanol (42.5 mL). The atmosphere was purged with nitrogen, and the mixture was stirred at 25°C for 2 hours. The reaction mixture was concentrated and extracted with ethyl acetate (30 mL) once. The aqueous phase was collected, and its pH was adjusted to 3-4 with 1N hydrochloric acid. The mixture was extracted three times with ethyl acetate (50 mL each time). The organic phases were combined and washed with saturated brine (50 mL). The organic phase was collected, dried over anhydrous sodium sulfate, filtered, and concentrated to obtain crude 040-3, which was directly used in the next step. $^1$H NMR (400 MHz, $CDCl_3$) $\delta$ = 7.14 - 7.07 (m, 2H), 7.00 - 6.94 (m, 2H), 3.85 (s, 3H), 3.32 (s, 3H), 3.13 (s, 2H).

Step 3: synthesis of compound 040-4

To a reaction flask were sequentially added crude 040-3 (8 g) and Eaton's reagent (60.60 g, 254.57 mmol). The atmosphere was purged with nitrogen, and the mixture was stirred at 70°C for 3 hours. The reaction mixture was cooled to 25°C, poured into ice-water (250 mL), and filtered. The filter cake was collected. The mixture was added with acetonitrile (100 mL), stirred for 0.5 hours, and filtered. The filter cake was collected to obtain 040-4. $^1$H NMR (400 MHz, DMSO-$d_6$) $\delta$ = 11.52 (br s, 1H), 7.46 (d, *J* = 9.3 Hz, 1H), 7.34 (d, *J*= 3.0 Hz, 1H), 7.27 (dd, *J* = 3.0, 9.1 Hz, 1H), 5.95 (s, 1H), 3.81 (s, 3H), 3.54 (s, 3H).

Step 4: synthesis of compound 040-5

To a reaction flask were sequentially added phosphorus tribromide (8.59 g, 29.97 mmol) and *N,N*-dimethylformamide (41 mL). The atmosphere was purged with nitrogen, followed by the portionwise addition of compound 040-4 (4.1 g, 19.98 mmol). The mixture was stirred at 80°C for 3 hours. The reaction mixture was cooled to 25 °C, poured into ice water (200 mL), and filtered. The filter cake was collected and dried to obtain 040-5, which was directly used in the next step. $^1$H NMR (400 MHz, $CDCl_3$) $\delta$ = 7.44 (d, *J* = 2.9 Hz, 1H), 7.34 - 7.31 (m, 1H), 7.26 - 7.22 (m, 1H), 7.17 (s, 1H), 3.92 (s, 3H), 3.71 (s, 3H).

Step 5: synthesis of compound 040-6

To a reaction flask were sequentially added 040-5 (1 g, 3.73 mmol), *N*-fluorobenzenesulfonimide (2.35 g, 7.46 mmol), and tetrahydrofuran (30 mL). After the atmosphere was purged with nitrogen, the mixture was cooled to -78°C, and n-butyllithium (2.5 M, 4.48 mL) was added. The mixture was stirred at -78°C for 1 hour. Water (10 mL) was added to dilute the reaction mixture, followed by extraction with ethyl acetate three times (10 mL each). The organic phases were combined, washed with saturated brine (10 mL), and the organic phase was collected. It was dried over anhydrous sodium sulfate, filtered, and concentrated to obtain the crude product. The crude product was purified by silica gel column chromatography (petroleum ether:ethyl acetate = 1:0 to 1:1) to obtain 040-6. $^1$H NMR (400 MHz, $CDCl_3$) $\delta$ = 7.38 - 7.32 (m, 1H), 7.28 (d, *J* = 2.9 Hz, 1H), 7.26 - 7.24 (m, 1H), 6.43 (d, *J* = 11.3 Hz, 1H), 3.90 (s, 3H), 3.70 (s, 3H).

Step 6: synthesis of compound 040-7

To a reaction flask were sequentially added 040-6 (170 mg, 820.46 $\mu$mol) and dichloromethane (4 mL). The atmosphere was purged with nitrogen, and the mixture was cooled to 0°C, followed by the addition of boron tribromide (2 M solution in dichloromethane, 1.64 mL). The mixture was stirred at 0°C for 1 hour. The reaction mixture was concentrated to obtain 040-7, and the crude product was used directly in the next step.

Step 7: synthesis of compound 040-8

To a reaction flask were sequentially added crude 040-7 (0.16 g) and dichloromethane (6 mL). After the atmosphere was purged with nitrogen, the mixture was maintained at 0°C, followed by addition of pyridine (196.55 mg, 2.48 mmol) and trifluoromethanesulfonic anhydride (467.37 mg, 1.66 mmol). The mixture was stirred at 0°C for 0.5 hours. The reaction mixture was added with water (2 mL) and extracted three times with dichloromethane (2 mL each time). The organic phases were combined and washed with saturated brine (5 mL). The organic phase was collected, dried over anhydrous sodium sulfate, filtered, and concentrated to obtain a crude product. The crude product was purified by preparative thin-layer chromatography (petroleum ether:ethyl acetate = 1:1) to obtain 040-8. $^1$H NMR (400 MHz, $CDCl_3$) $\delta$ = 7.75 (d, *J* = 2.8 Hz, 1H), 7.61 - 7.55 (m, 1H), 7.51 - 7.46 (m, 1H), 6.52 (d, *J* = 10.9 Hz, 1H), 3.72 (s, 3H).

Step 8: synthesis of compound 040-9

To a reaction flask were sequentially added 040-8 (150 mg, 461.20 μmol), pinacol boronate (175.68 mg, 691.81 μmol), potassium acetate (135.79 mg, 1.38 mmol), and 1,4-dioxane (3 mL). The atmosphere was purged with nitrogen, followed by the addition of [1,1'-bis(diphenylphosphino)ferrocene]dichloropalladium(II) (33.75 mg, 46.12 μmol). The mixture was stirred at 90°C for 4 hours. The reaction mixture was concentrated to obtain crude 040-9, which was used directly in the next step.

Step 9: synthesis of compound 040

To a reaction flask were sequentially added crude 040-9 (135.42 mg), A-1 (115 mg, 406.13 μmol), sodium carbonate (86.09 mg, 812.25 μmol), water (0.5 mL), and ethanol (3 mL). The atmosphere was purged with nitrogen, followed by the addition of tetrakis(triphenylphosphine)palladium (46.93 mg, 40.61 μmol). The mixture was stirred at 85°C for 1 hour. The reaction mixture was concentrated to obtain a crude product. The crude product was sequentially purified by column chromatography (ethyl acetate:methanol = 1:0 to 20:1), preparative thin-layer chromatography (dichloromethane:ethyl acetate:methanol = 5:5:1), preparative high-performance liquid chromatography (chromatographic column: Waters Xbridge BEH C18 100*30 mm*10 μm; mobile phase: [water (10 mM ammonium bicarbonate)-acetonitrile]; gradient: 20%-50% acetonitrile) and SFC chiral resolution (chromatographic column: DAICEL CHIRALPAK AD, 250 mm*30 mm, 10 μm; mobile phase: [supercritical carbon dioxide-isopropanol (0.1% ammonia)]; isocratic elution: 50% isopropanol (0.1% ammonia)) to obtain 040. $^1$H NMR (400 MHz, $CDCl_3$) δ = 8.56 (s, 1H), 8.33 (s, 1H), 7.76 (d, *J* = 1.7 Hz, 1H), 7.64 - 7.56 (m, 1H), 7.53 - 7.46 (m, 1H), 6.46 (d, *J* = 11.0 Hz, 1H), 5.80 (br d, *J* = 8.7 Hz, 1H), 5.46 - 5.32 (m, 1H), 3.75 (s, 3H), 2.74 - 2.55 (m, 2H), 2.30 (q, *J* = 7.6 Hz, 2H), 2.15 - 2.08 (m, 1H), 1.91 - 1.76 (m, 3H), 1.23 (t, *J* = 7.6 Hz, 3H); ESI-LCMS: *m*/*z* = 380.2 $[M+H]^+$. SFC analysis was performed (chromatographic column: Chiralpak AD-3, 50 × 4.6 mm I.D., 3 μm; mobile phase: A: supercritical carbon dioxide, B: isopropanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.484 minutes.

**Example 34**

Synthetic route:

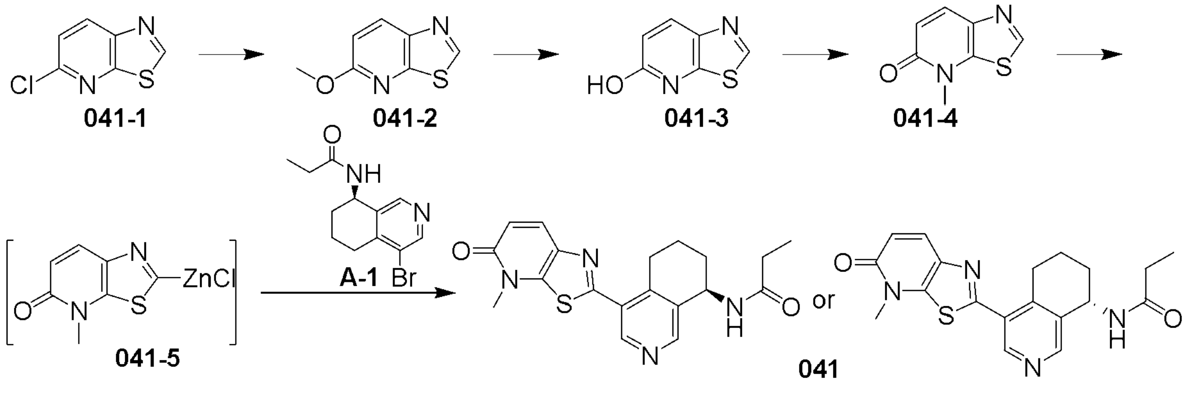
041-1 041-2 041-3 041-4

041-5 A-1 or 041

Step 1: synthesis of compound 041-2

To a reaction flask were sequentially added 041-1 (0.85 g, 4.98 mmol) and sodium methoxide (5.4 M in methanol, 8.5 mL). The mixture was stirred at 80°C for 1 hour. The reaction mixture was allowed to return to 25°C, added with water (5 mL), and extracted with 10 mL of dichloromethane. The organic phase was collected, and the aqueous phase was extracted with dichloromethane (10 mL × 3). The organic phases were combined and washed with saturated brine (10 mL×2). The organic phase was collected, dried over anhydrous sodium sulfate, filtered, and concentrated to obtain crude 041-2, which was directly used in the next step. $^1$H NMR (400 MHz, $CDCl_3$) δ = 8.88 (s, 1H), 8.21 (d, *J* = 8.9 Hz, 1H), 6.91 (d, *J* = 8.9 Hz, 1H), 4.03 (s, 3H).

Step 2: synthesis of compound 041-3

To a reaction flask were sequentially added crude 041-2 (0.26 g) and hydrobromic acid (58.70 g, 239.40 mmol). The atmosphere was purged with nitrogen and the mixture was stirred at 130°C for 2 hours. The reaction mixture was returned to 25°C, concentrated to obtain crude 041-3, which was directly used in the next step. $^1$H NMR (400 MHz, DMSO-$d_6$) δ = 9.10 (s, 1H), 8.21 (d, *J* = 9.0 Hz, 1H), 6.72 (d, *J* = 9.0 Hz, 1H).

Step 3: synthesis of compound 041-4

To a reaction flask were sequentially added crude 041-3 (0.38 g), *N,N*-dimethylformamide (8 mL), and potassium carbonate (690.26 mg, 4.99 mmol). The atmosphere was purged with nitrogen, and the mixture was cooled to 0°C, followed by the addition of methyl iodide (1.42 g, 9.99 mmol). The mixture was stirred at 0°C for 2 hours, then added with 10 mL of water, and extracted with 10 mL of ethyl acetate. The resulting phases were separated. The organic phase was collected and the aqueous phase was extracted with ethyl acetate (30 mL × 3). The organic phases were combined and washed with saturated brine (30 mL × 2). The organic phase was collected, dried over anhydrous sodium sulfate, filtered, and concentrated to obtain a crude product. The crude product was purified by preparative thin-layer chromatography (petroleum ether:ethyl acetate = 1:0) to obtain 041-4. $^1$H NMR (400 MHz, $CDCl_3$) δ = 8.58 (s, 1H), 8.00 (d, *J* = 9.7 Hz, 1H), 6.68 (d, *J* = 9.5 Hz, 1H), 3.74 (s, 3H).

Step 4: synthesis of compound 041-5

To a reaction flask were sequentially added 041-4 (50 mg, 300.84 μmol) and tetrahydrofuran (1 mL). The atmosphere was purged with nitrogen, followed by the addition of zinc chloride (0.7 M in tetrahydrofuran, 429.77 μL). The mixture was cooled to -5°C, and lithium bis(trimethylsilyl)amide (1 M in tetrahydrofuran, 601.68 μL) was added dropwise. The mixture was then warmed to 10°C and stirred for 1 hour. The reaction mixture of 041-5 was used directly in the next step without further treatment.

Step 5: synthesis of compound 041

To a reaction flask were sequentially added A-1 (85.15 mg, 300.71 μmol) and *N,N*-dimethylformamide (3 mL). The atmosphere was purged with nitrogen, followed by the addition of methanesulfonato(2-dicyclohexylphosphino-2',6'-dimethoxy-1,1'-biphenyl)(2'-amino-1,1'-biphenyl-2-yl)palladium(II) (7.04 mg, 9.02 μmol). The atmosphere was again purged with nitrogen. The temperature was raised to 100°C, and the reaction mixture of 041-5 was added. The mixture was stirred at 100°C for 12 hours. The reaction mixture was concentrated to obtain a crude product. The crude product was sequentially purified by preparative thin-layer chromatography (ethyl acetate:dichloromethane:methanol = 1:1:0.3) and SFC chiral resolution (chromatographic column: DAICEL CHIRALPAKAD (250 mm*30 mm, 10 μm); mobile phase: [supercritical carbon dioxide-methanol (0.1% ammonia)]; gradient: 45% to 54% methanol (0.1% ammonia)) to obtain 041. $^1$H NMR (400 MHz, $CD_3OD$) δ = 8.71 (s, 1H), 8.48 (s, 1H), 8.13 (d, *J* = 9.5 Hz, 1H), 6.69 (d, *J* = 9.5 Hz, 1H), 5.24 (br s, 1H), 3.80 (s, 3H), 3.18 (br s, 1H), 3.15 - 3.10 (m, 1H), 2.29 (dq, *J*= 1.5, 7.6 Hz, 2H), 2.09 - 1.84 (m, 5H), 1.19 (t, *J* = 7.7 Hz, 3H); ESI-LCMS: m/z = 369.1 $[M+H]^+$. SFC analysis was performed (chromatographic column: Chiralpak AD-3, 50 × 4.6 mm I.D., 3 μm; mobile phase: A: supercritical carbon dioxide, B: methanol (0.2% 7M ammonia in methanol); gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.968 minutes.

**Example 35**

Synthetic route:

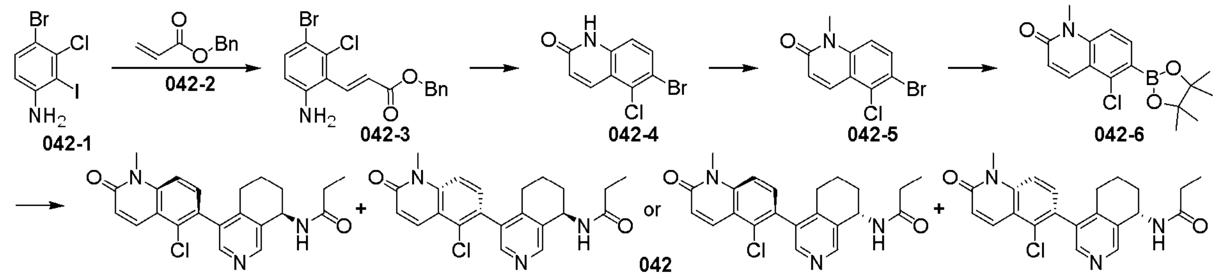

042-1 042-3 042-4 042-5 042-6

or 042

Step 1: synthesis of compound 042-3

To a reaction flask were sequentially added 2-di-tert-butylphosphino-2',4',6'-triisopropylbiphenyl (530.70 mg, 1.11 mmol), palladium acetate (124.97 mg, 556.62 μmol), and *N,N*-dimethylformamide (74 mL). The atmosphere was purged with nitrogen, followed by the addition of 042-1 (3.7 g, 11.13 mmol), triethylamine (9.01 g, 89.06 mmol), and 042-2 (10.83 g, 66.79 mmol). The atmosphere was again purged with nitrogen, and the mixture was stirred at 100°C for 16 hours. The reaction mixture was cooled to 25°C, added with water (370 mL), and extracted with ethyl acetate (50 mL × 3). The organic phases were collected and washed with saturated brine (100 mL × 2). The organic phase was collected, dried over anhydrous sodium sulfate, filtered, and concentrated to obtain a crude product. The crude product was purified by column chromatography (petroleum ether:ethyl acetate = 1:0 to 5:1) to obtain 042-3. $^1$H NMR (400 MHz, DMSO-$d_6$) δ = 7.71 (d, *J* = 16.5 Hz, 1H), 7.47 - 7.33 (m, 6H), 6.68 (d, *J* = 8.9 Hz, 1H), 6.48 (d, *J* = 16.4 Hz, 1H), 5.85 (s, 2H), 5.24 (s, 2H).

Step 2: synthesis of compound 042-4

To a reaction flask were sequentially added 042-3 (2.18 g, 5.95 mmol), methanol (22 mL), and tributylphosphine (3.61 g, 17.84 mmol). The mixture was stirred at 70°C for 1.5 hours. The reaction mixture was directly concentrated to obtain crude 042-4, which was used directly in the next step. $^1$H NMR (400 MHz, DMSO-$d_6$) δ = 12.14 (br s, 1H), 8.10 (d, *J*= 9.9 Hz, 1H), 7.82 (d, *J* = 8.9 Hz, 1H), 7.31 (s, 1H), 6.66 (d, *J* = 9.8 Hz, 1H).

Step 3: synthesis of compound 042-5

To a reaction flask were sequentially added crude 042-4 (5.5 g), *N,N*-dimethylformamide (55 mL), and potassium carbonate (5.88 g, 42.55 mmol). The atmosphere was purged with nitrogen, followed by the addition of methyl iodide (12.08 g, 85.11 mmol). The mixture was stirred at 25°C for 16 hours. The reaction mixture was added with water (50 mL) and filtered. The filter cake was collected to obtain a crude product. The crude product was purified by column chromatography (petroleum ether:ethyl acetate = 1:0 to 3:1) to obtain 042-5. $^1$H NMR (400 MHz, DMSO-$d_6$) δ = 8.14 (d, *J* = 9.9 Hz, 1H), 7.93 (d, *J* = 9.1 Hz, 1H), 7.49 (d, *J* = 9.3 Hz, 1H), 6.78 (d, *J*= 9.9 Hz, 1H), 3.61 (s, 3H).

Step 4: synthesis of compound 042-6

To a reaction flask were sequentially added 042-5 (200 mg, 733.88 μmol), pinacol boronate (279.54 mg, 1.10 mmol), 1,4-dioxane (4 mL), and potassium acetate (144.05 mg, 1.47 mmol). The atmosphere was purged with nitrogen, followed by the addition of [1,1'-bis(diphenylphosphino)ferrocene]dichloropalladium(II) (53.70 mg, 73.39 μmol). The atmosphere was again purged with nitrogen, and the mixture was stirred at 80°C for 16 hours. The reaction mixture was cooled to 25°C and filtered. The filter cake was rinsed with dichloromethane. The filtrate was collected and concentrated under reduced pressure to obtain crude 042-6, which was used directly in the next step.

Step 5: synthesis of compound 042

To a reaction flask were sequentially added A-1 (141.76 mg, 500.64 μmol), water (1.2 mL), acetonitrile (3.6 mL), methanol (1.2 mL), potassium carbonate (276.77 mg, 2.00 mmol), and 2-dicyclohexylphosphino-2,6-dimethoxybiphenyl (30.83 mg, 75.10 μmol). The atmosphere was purged with nitrogen, followed by the addition of bis(triphenylphosphine) palladium(II) chloride (17.57 mg, 25.03 μmol) and palladium acetate (5.62 mg, 25.03 μmol). The temperature was raised to 40°C, and a solution of crude 042-6 (240 mg) in tetrahydrofuran (7.2 mL) was added. The atmosphere was again purged with nitrogen, and the mixture was heated to 75°C and stirred for 2 hours. The reaction mixture was filtered and rinsed with dichloromethane. The filtrate was collected and concentrated under reduced pressure. The resulting product was purified by preparative high-performance liquid chromatography (chromatographic column: 2_Phenomenex Gemini C18, 75*40 mm*3 μm; mobile phase: [water (10 mM ammonium bicarbonate)-acetonitrile]; gradient: 10% to 40% acetonitrile) to obtain

042. $^1$H NMR (400 MHz, DMSO-$d_6$) δ = 8.41 (s, 1H), 8.38 - 8.25 (m, 1H), 8.25 - 8.17 (m, 2H), 7.70 - 7.63 (m, 1H), 7.62 - 7.46 (m, 1H), 6.82 (d, *J*= 9.9 Hz, 1H), 5.11 (br d, *J* = 6.3 Hz, 1H), 3.69 (s, 3H), 2.40 - 2.31 (m, 2H), 2.23 - 2.13 (m, 2H), 1.92 - 1.65 (m, 4H), 1.07 (dt, *J*= 3.8, 7.6 Hz, 3H); ESI-LCMS: m/z = 396.1 [M+H]$^+$.

**Example 36**

Synthetic route:

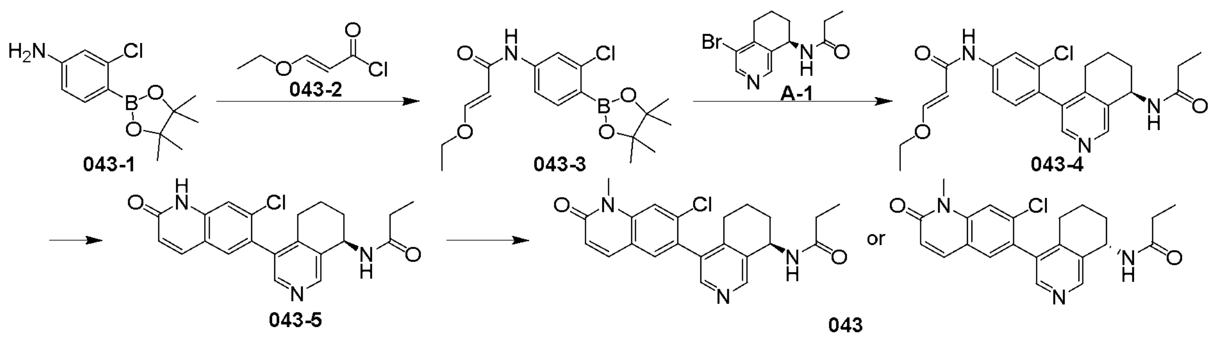


Step 1: synthesis of compound 043-3

To a reaction flask were sequentially added 043-1 (500 mg, 1.97 mmol), dichloromethane (5 mL), and pyridine (187.19 mg, 2.37 mmol). The atmosphere was purged with nitrogen, followed by the addition of 043-2 (291.91 mg, 2.17 mmol). The mixture was stirred at 20°C for 3 hours. The reaction mixture was concentrated to obtain a crude product, which was then purified by silica gel column chromatography (petroleum ether:ethyl acetate = 5:1 to 1:1) to obtain 043-3. $^1$H NMR (400 MHz, $CDCl_3$) δ = 7.74 - 7.57 (m, 3H), 7.42 (dd, *J* = 1.8, 8.2 Hz, 1H), 5.29 (d, *J*= 12.0 Hz, 1H), 3.96 (q, *J*= 7.0 Hz, 2H), 1.36 (s, 15H).

Step 2: synthesis of compound 043-4

To a reaction flask were sequentially added 043-3 (149.02 mg, 423.78 μmol), potassium carbonate (195.24 mg, 1.41 mmol), 2-dicyclohexylphosphino-2,6-dimethoxybiphenyl (21.75 mg, 52.97 μmol), bis(triphenylphosphine)palladium(II) dichloride (12.39 mg, 17.66 μmol), palladium acetate (3.96 mg, 17.66 μmol), water (1.6 mL), acetonitrile (4.8 mL), and methanol (1.6 mL). The atmosphere was purged with nitrogen, and the mixture was heated to 45°C, followed by the addition of a solution of A-1 (100 mg, 353.15 μmol) in tetrahydrofuran (1.6 mL). The reaction mixture was further heated to 75°C and stirred for 2 hours. The reaction mixture was added with 2 mL of water and extracted with dichloromethane (3 mL × 3). The organic phases were combined and washed with saturated brine (3 mL × 3). The organic phase was collected, dried over anhydrous sodium sulfate, filtered, and concentrated to obtain a crude product. The crude product was purified by preparative thin-layer chromatography (dichloromethane: ethyl acetate: methanol = 1:1:0.3) to obtain 043-4. $^1$H NMR (400 MHz, $CDCl_3$) δ = 8.57 (br d, *J* = 2.1 Hz, 1H), 8.41 - 8.09 (m, 1H), 7.69 (dd, *J*= 3.1, 12.0 Hz, 1H), 7.62 - 7.49 (m, 1H), 7.40 - 7.32 (m, 1H), 5.54 - 5.42 (m, 1H), 5.40 - 5.32 (m, 1H), 5.31 - 5.23 (m, 1H), 4.14 - 3.91 (m, 2H), 3.02 - 2.91 (m, 2H), 2.44 - 2.28 (m, 4H), 2.21 - 2.12 (m, 2H), 1.39 (br t, *J* = 6.7 Hz, 3H), 1.23 - 1.19 (m, 3H).

Step 3: synthesis of compound 043-5

To a reaction flask was added concentrated sulfuric acid (1.5 mL). The mixture was cooled to 0°C, followed by the addition of compound 043-4 (90 mg, 210.32 μmol). The atmosphere was purged with nitrogen, and the mixture was stirred at 25°C for 2 hours. The reaction mixture was added with water, and its pH was adjusted to 7-8 with a saturated aqueous sodium bicarbonate solution. It was extracted with ethyl acetate (3 mL × 3). The organic phases were combined and washed with saturated brine (3 mL × 3). The organic phase was collected, then dried over anhydrous sodium sulfate, filtered, and concentrated under reduced pressure to obtain crude 043-5, which was used directly in the next step. $^1$H NMR (400 MHz, $CD_3OD$) δ = 8.44 (s, 1H), 8.25 (d, *J* = 4.5 Hz, 1H), 8.17 (s, 1H), 7.97 (d, *J* = 9.6 Hz, 1H), 7.66 - 7.58 (m, 1H), 7.18 (d, *J*= 5.4 Hz, 1H), 5.31 - 5.18 (m, 1H), 2.88 - 2.79 (m, 2H), 2.56 - 2.47 (m, 2H), 2.27 - 2.22 (m, 2H), 2.05 - 1.99 (m, 2H), 1.18 (s, 3H).

Step 4: synthesis of compound 043

To a reaction flask were sequentially added crude 043-5 (40 mg), *N,N*-dimethylformamide (0.5 mL), and cesium carbonate (68.26 mg, 209.50 μmol). The mixture was cooled to 0°C, followed by the addition of methyl iodide (22.30 mg, 157.13 μmol). The atmosphere was purged with nitrogen, and the mixture was stirred at 0°C for 1 hour. The reaction mixture was poured into 2 mL of water and extracted three times with ethyl acetate (3 mL each time). The organic phases were combined and washed with saturated brine (3 mL each time). The organic phase was collected, then dried over anhydrous sodium sulfate, filtered, and concentrated to obtain a crude product. The crude product was sequentially purified by preparative thin-layer chromatography (dichloromethane:ethyl acetate:methanol = 1:1:0.3) and SFC chiral resolution (chromatographic column: ChiralPak IH, 250*30 mm, 10 μm; mobile phase: [supercritical carbon dioxide-isopropanol (0.1% ammonia)]; isocratic elution: 50% isopropanol (0.1% ammonia)) to obtain 043. $^1H$ NMR (400 MHz, DMSO-$d_6$) δ = 8.41 (s, 1H), 8.39 - 8.25 (m, 1H), 8.21 (s, 1H), 7.93 (dd, *J* = 9.7, 18.6 Hz, 1H), 7.82 - 7.61 (m, 2H), 6.74 - 6.62 (m, 1H), 5.18 - 5.04 (m, 1H), 3.65 (s, 3H), 2.42 - 2.33 (m, 2H), 2.25 - 2.10 (m, 2H), 1.95 - 1.74 (m, 2H), 1.74 - 1.61 (m, 2H), 1.07 (dt, *J* = 3.4, 7.5 Hz, 3H); ESI-LCMS: *m/z* = 396.1 $[M+H]^+$. SFC analysis was performed (chromatographic column: Chiralpak IH-3, 100 × 4.6 mm I.D., 3 μm; mobile phase: A: supercritical carbon dioxide, B: isopropanol (0.2% 7M ammonia in methanol); gradient: A/B = 90/10 (0 min), 90/10 (0.2 min), 50/50 (2.4 min), 50/50 (3.4 min), 90/10 (4.0 min); flow rate: 3.4 mL/min; column temperature: 35°C; pressure: 2000 psi), *ee* = 93.92%, retention time: 3.194 minutes.

**Example 37**

Synthetic route:

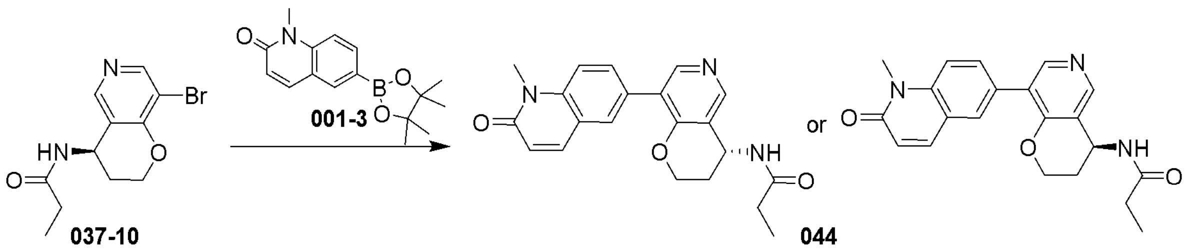

037-10

001-3

or

044

Step 1: synthesis of compound 044

To a reaction flask were sequentially added 001-3 (120.00 mg, 420.85 μmol), water (1 mL), acetonitrile (3 mL), methanol (1 mL), potassium carbonate (193.89 mg, 1.40 mmol), and 2-dicyclohexylphosphino-2,6-dimethoxybiphenyl (21.60 mg, 52.61 μmol). The atmosphere was purged with nitrogen, followed by the addition of bis(triphenylphosphine) palladium(II) dichloride (12.31 mg, 17.54 μmol) and palladium(II) acetate (3.94 mg, 17.54 μmol). The atmosphere was again purged with nitrogen. The reaction mixture was heated to 45°C, and a solution of 037-10 (100 mg, 350.71 μmol) in tetrahydrofuran (6 mL) was added. The reaction mixture was heated to 75°C and stirred for 16 hours. The reaction mixture was concentrated to obtain a crude product, which was sequentially subjected to preparative thin-layer chromatography (petroleum ether:ethyl acetate = 0:1), a second preparative thin-layer chromatography (dichloromethane:methanol = 10:1), and SFC chiral resolution (chromatographic column: DAICEL CHIRALPAK AD (250 mm*30 mm, 10 μm); mobile phase: [supercritical carbon dioxide-isopropanol (0.1% ammonia)]; isocratic elution: 40% isopropanol (0.1% ammonia)) to obtain 044. $^1H$ NMR (400 MHz, $CD_3OD$) δ = 8.31 (d, *J* = 15.0 Hz, 2H), 7.98 (d, *J* = 9.5 Hz, 1H), 7.90 - 7.84 (m, 2H), 7.68 (s, 1H), 6.72 (d, *J* = 9.5 Hz, 1H), 5.24 (s, 1H), 4.53 - 4.28 (m, 2H), 3.79 (s, 3H), 2.33 - 2.26 (m, 2H), 2.25 - 2.07 (m, 2H), 1.19 (t, *J* = 7.6 Hz, 3H); ESI-LCMS: *m/z* = 364.2 $[M+H]^+$. SFC analysis was performed (chromatographic column: Chiralcel AD-3, 150 × 4.6 mm I.D., 3 μm; mobile phase: A: supercritical carbon dioxide, B: isopropanol (0.2% 7M ammonia in methanol); gradient: A/B = 90/10 (0 min), 90/10 (0.5 min), 50/50 (3.5 min), 50/50 (4.5 min), 90/10 (5.0 min); flow rate: 2.5 mL/min; column temperature: 35°C; pressure: 2000 psi), *ee* = 100%, retention time: 3.894 minutes.

**Example 38**

Synthetic route:

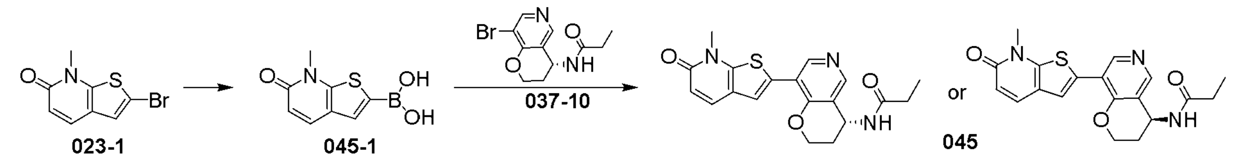


023-1 045-1 or 045

Step 1: synthesis of compound 045-1

To a reaction flask were sequentially added 023-1 (120 mg, 491.59 μmol), triisopropyl borate (309.72 mg, 1.65 mmol), and tetrahydrofuran (1.2 mL). The atmosphere was purged with nitrogen, and the mixture was cooled to -5°C, followed by the addition of isopropylmagnesium chloride-lithium chloride complex (1.3 M, 567.21 μL). The mixture was stirred at -5°C for 0.5 hours. Additional isopropylmagnesium chloride-lithium chloride complex (1.3 M in tetrahydrofuran, 378.14 μL) was added, and the mixture was stirred at -5°C for 1 hour. Additional isopropylmagnesium chloride-lithium chloride complex (1.3 M in tetrahydrofuran, 567.21 μL) and triisopropyl borate (92.45 mg, 491.59 μmol) were added, and the mixture was stirred at 0°C for 0.5 hours. To the reaction mixture was added 0.9 mL of methanol, and the resulting mixture of 045-1 was directly used in the next step.

Step 2: synthesis of compound 045

To a reaction flask were sequentially added 037- 10 (69.57 mg, 243.98 μmol), tetrahedronfuran (0.9 mL), water (0.3 mL), acetonitrile (0.3 mL), potassium carbonate (134.88 mg, 975.94 μmol), and 2-dicyclohexylphosphino-2,6-dimethoxybiphenyl (15.02 mg, 36.60 μmol). The atmosphere was purged with nitrogen, followed by the addition of bis(triphenylphosphine)palladium(II) dichloride (8.56 mg, 12.20 μmol) and palladium(II) acetate (2.74 mg, 12.20 μmol). The atmosphere was again purged with nitrogen. The reaction mixture was added with a reaction mixture of 045-1 at 45°C, then heated to 75°C and stirred for 16 hours. The reaction mixture was concentrated to obtain a crude product. The crude product was sequentially purified by preparative thin-layer chromatography (petroleum ether:ethyl acetate = 0:1), a second preparative thin-layer chromatography (dichloromethane:methanol = 10:1), and preparative high-performance liquid chromatography (chromatographic column: Waters Xbridge BEH C18 100*30 mm*10 μm; mobile phase: [water (10 mM ammonium bicarbonate)-acetonitrile]; gradient: 10% to 43% acetonitrile) to obtain 045. $^1$H NMR (400 MHz, $CD_3OD$) δ = 8.67 (s, 1H), 8.28 - 8.19 (m, 1H), 7.97 - 7.90 (m, 1H), 7.74 (s, 1H), 6.56 (d, *J* = 9.3 Hz, 1H), 5.23 (s, 1H), 4.66 - 4.46 (m, 2H), 3.76 (s, 3H), 2.32 - 2.27 (m, 2H), 2.26 (br s, 2H), 1.19 (t, *J* = 7.6 Hz, 3H); ESI-LCMS : *m/z* = 370.1 $[M+H]^+$. SFC analysis was performed (chromatographic column: Chiralpak AD-3, 50 × 4.6 mm I.D., 3 μm; mobile phase: A: supercritical carbon dioxide, B: 0.2% 7M ammonia in methanol; gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.479 minutes.

**Example 39**

Synthetic route:

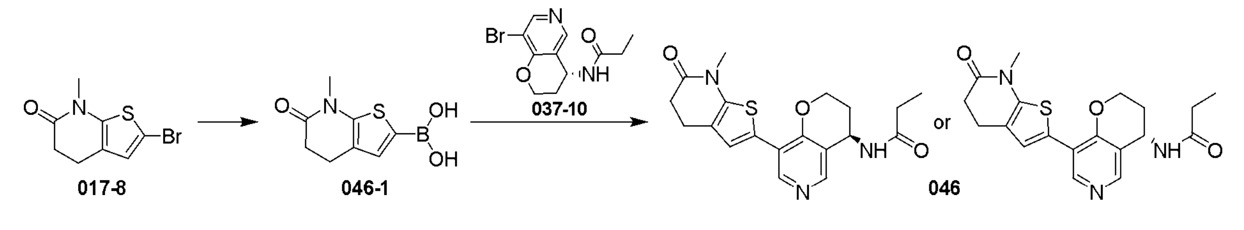


017-8 046-1 or 046

Step 1: synthesis of compound 046-1

To a reaction flask were sequentially added 017-8 (0.25 g, 1.02 mmol) and tetrahydrofuran (6.25 mL). The atmosphere was purged with nitrogen, and the mixture was frozen to -15 to -20°C, followed by the dropwise addition of isopropylmagnesium chloride-lithium chloride complex (1.3 M in tetrahydrofuran, 1.95 mL). The mixture was stirred at - 15 to -20°C for 0.5 hours. Triisopropyl borate (353.59 mg, 3.40 mmol) was added, and the mixture was stirred for an additional 0.5 hour. To the reaction mixture was added 0.5 mL of methanol, and the resulting reaction mixture of 046-1 was directly used in the next step.

Step 2: synthesis of compound 046

To a reaction flask were sequentially added 037-10 (0.13 g, 455.92 μmol), potassium phosphate (193.56 mg, 911.84 μmol), isopropanol (7.2 mL), and water (1.2 mL). The atmosphere was purged with nitrogen, followed by the addition of dichlorodi-tert-butyl-(4-dimethylaminophenyl)phosphine palladium(II) (32.28 mg, 45.59 μmol). The atmosphere was again purged with nitrogen, and the mixture was heated to 45°C, followed by the addition of the reaction mixture of 046-1 described above. Upon completion of the addition, the mixture was heated to 75°C and stirred for 2 hours. The reaction mixture was filtered through anhydrous sodium sulfate. The filtrate was collected and concentrated to obtain a crude product. The crude product was sequentially purified by column chromatography (petroleum ether:ethyl acetate = 10:1 to dichloromethane:methanol = 1:1) and preparative high-performance liquid chromatography (chromatographic column: Waters Xbridge BEH C18 100*30 mm*10 μm; mobile phase: [water (10 mM ammonium bicarbonate)-acetonitrile]; 15% to 50% acetonitrile, isocratic elution) to obtain 046. $^1$H NMR (400 MHz, $CD_3OD$) δ = 8.57 (br s, 1H), 8.14 (br s, 1H), 7.36 (s, 1H), 5.19 (t, *J*= 5.5 Hz, 1H), 4.64 - 4.42 (m, 2H), 3.34 (s, 3H), 2.93 - 2.84 (m, 2H), 2.77 - 2.66 (m, 2H), 2.34 - 2.08 (m, 4H), 1.18 (t, *J* = 7.6 Hz, 3H); ESI-LCMS: m/z =372.2 [M+H] $^+$; SFC analysis was performed (chromatographic column: Chiralpak AD-3, 50 × 4.6 mm I.D., 3 μm; mobile phase: A: supercritical carbon dioxide, B: 0.2% 7M ammonia in methanol; gradient: A/B = 95/5 (0 min), 95/5 (0.2 min), 50/50 (1.2 min), 50/50 (2.2 min), 95/5 (2.6 min), 95/5 (3.0 min); flow rate: 3.4 mL/min; column temperature: 35°C; pressure: 1800 psi), *ee* = 100%; retention time: 1.431 minutes.

**Example 40**

Synthetic route:

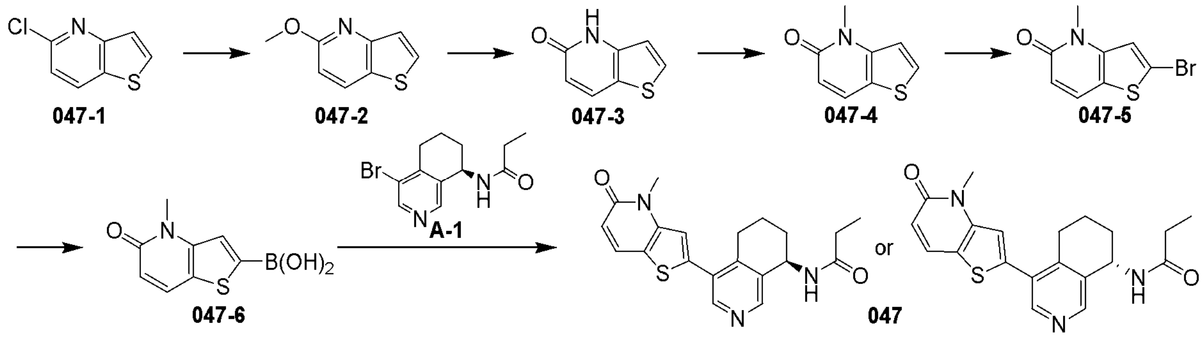
047-1 047-2 047-3 047-4 047-5
A-1
047-6
or
047

Step 1: synthesis of compound 047-2

To a reaction flask were sequentially added 047-1 (350 mg, 2.06 mmol), methanol (7 mL), and sodium methoxide (5.4 M in methanol, 2 mL). The mixture was heated to 100°C in a microwave reactor and stirred for 5 hours. Three batches of the same scale reaction were conducted in parallel and combined for work-up. After combining the reaction mixture and concentrating, 20 mL of water and 20 mL of ethyl acetate were added. The mixture was extracted, and the resulting phases were separated. The aqueous phase was collected and extracted with ethyl acetate (10 mL × 2). The organic phases were combined and washed with saturated brine. The organic phase was collected, dried over anhydrous sodium sulfate, filtered, and concentrated to obtain crude 047-2. $^1$H NMR (400 MHz, $CDCl_3$) δ ppm 8.00 (d, *J* = 8.53 Hz, 1H), 7.67 (d, *J* = 5.52 Hz, 1H), 7.41 (d, *J* = 5.52 Hz, 1H), 6.77 (d, *J* = 8.53 Hz, 1H), 4.02 (s, 3H).

Step 2: synthesis of compound 047-3

To a reaction flask were sequentially added 047-2 (0.9 g), acetic acid (9 mL), and hydrobromic acid (9 mL, 66.29 mmol, 40% concentration). After the atmosphere was purged with nitrogen, the mixture was stirred at 85°C for 18 hours. The reaction mixture was concentrated under reduced pressure to obtain crude 047-3. $^1$H NMR (400 MHz, $CD_3OD$) δ ppm 8.73 (d, *J* = 9.29 Hz, 1H), 8.32 (d, *J*= 5.52 Hz, 1H), 7.44 (d, *J*= 5.52 Hz, 1H), 7.07 (d, *J* = 9.03 Hz, 1H).

Step 3: synthesis of compound 047-4

To a reaction flask were sequentially added 047-3 (1.43 g) and *N,N*-dimethylformamide (30 mL), followed by the addition of potassium tert-butoxide (3.18 g, 28.38 mmol) at 0°C. The mixture was stirred at 0°C for 15 minutes, followed by

the dropwise addition of iodomethane (1.47 mL, 23.65 mmol). The mixture was warmed to 20°C and stirred for 18 hours. The mixture was diluted with 40 mL of ethyl acetate, added with 80 mL of hydrochloric acid (1 M), and extracted. The resulting phases were separated. The aqueous phase was extracted with ethyl acetate (40 mL × 5). The organic phases were combined and washed with saturated brine (140 mL × 3). The organic phase was collected, dried over anhydrous sodium sulfate, filtered, and concentrated under reduced pressure to obtain a crude product. The crude product was purified by column chromatography (ethyl acetate:petroleum ether = 10 to 100%) to obtain 047-4. $^1$H NMR (400 MHz, $CD_3OD$) δ ppm 7.97 (d, *J* = 9.29 Hz, 1H), 7.89 (d, *J* = 5.52 Hz, 1H), 7.32 (d, *J*= 5.52 Hz, 1H), 6.55 (d, *J* = 9.29 Hz, 1H), 3.75 (s, 3H).

Step 4: synthesis of compound 047-5

To a reaction flask were sequentially added 047-4 (213 mg, 1.29 mmol) and tetrahydrofuran (12 mL). The atmosphere was purged with nitrogen, and the mixture was cooled to 0°C, followed by the addition of a solution of N-bromosuccinimide (210 mg, 1.18 mmol) in tetrahydrofuran (12 mL). The mixture was stirred at 0°C for 1 hour. The reaction mixture was added with a half-saturated aqueous solution of sodium thiosulfate (40 mL), stirred at 25°C for 5 minutes, and extracted with ethyl acetate (20 mL × 3). The organic phase was collected, dried over anhydrous sodium sulfate, filtered, and concentrated to obtain a crude product, which was separated and purified by preparative thin-layer chromatography (petroleum ether:ethyl acetate = 1:5) to obtain 047-5. $^1$H NMR (400 MHz, $CDCl_3$) δ ppm 8.14 (s, 1H), 7.62 (d, *J* = 5.52 Hz, 1H), 7.06 (d, *J* = 5.52 Hz, 1H) ,3.81 (s, 3H).

Step 5: synthesis of compound 047-6

To a reaction flask were sequentially added 047-5 (174 mg, 712.80 μmol) and tetrahydrofuran (4 mL). The atmosphere was purged with nitrogen. The mixture was cooled to 0°C and isopropylmagnesium chloride-lithium chloride complex (2 M in tetrahydrofuran, 534.60 μL) was added dropwise. The mixture was stirred at 20°C for 0.5 hours. The mixture was cooled to 0°C, and a solution of trimethyl borate (161.02 μL, 1.43 mmol) in tetrahydrofuran (4 mL) was added dropwise. The mixture was warmed to 20°C and stirred for 0.5 hours. To the reaction mixture was added 1 mL of methanol, and the resulting mixture of 047-6 was directly used in the next step.

Step 6: synthesis of compound 047

To a reaction flask were sequentially added A-1 (222.03 mg, 784.10 μmol), potassium carbonate (394.06 mg, 2.85 mmol), 2-dicyclohexylphosphino-2,6-dimethoxybiphenyl(43.89 mg, 106.92 μmol), acetonitrile (8 mL), and water (1.6 mL). After the atmosphere was purged with nitrogen, bis(triphenylphosphine)palladium(II) chloride (25.02 mg, 35.64 μmol) and palladium(II) acetate (8.00 mg, 35.64 μmol) were added. After the atmosphere was purged with nitrogen, the reaction mixture of 047-6 described above was added dropwise. Upon completion of the addition, the mixture was heated to 75°C and stirred for 1 hour. The reaction mixture was filtered through a funnel containing anhydrous sodium sulfate, and the filtrate was collected and concentrated to obtain a crude product. The crude product was sequentially purified by preparative thin-layer chromatography (dichloromethane:methanol = 15:1) and SFC chiral resolution [chromatographic column: DAICEL CHIRALCEL OJ (250 mm*30 mm, 10 μm); mobile phase: [supercritical carbon dioxide-ethanol (0.1% ammonia)]; isocratic elution: 25% ethanol (0.1% ammonia)], preparative high-performance liquid chromatography [chromatographic column: Welch Xtimate C18 150*30 mm*5 μm; mobile phase: [water (0.225% formic acid)-acetonitrile]; gradient: 5% to 35% acetonitrile] to obtain 047. $^1$H NMR (400 MHz, $CDCl_3$) δ ppm 8.51 (br s, 1H), 8.28 (s, 1H), 7.72 (s, 1H), 7.65 - 7.70 (m, 1H), 7.13 (d, *J*= 5.52 Hz, 1H), 5.92 (br s, 1H), 5.30 - 5.37 (m, 1H), 3.80 (s, 3H), 2.41 (br s, 2H), 2.27 (q, *J*= 7.53 Hz, 2H), 2.00 - 2.10 (m, 1H), 1.73 - 1.93 (m, 3H), 1.21 (t, *J* = 7.53 Hz, 3H); ESI-LCMS: m/z = 368.1 $[M+H]^+$. SFC analysis was performed (chromatographic column: Chiralcel OJ-3 50×4.6 mm I.D., 3 μm; mobile phase: A: supercritical carbon dioxide B: ethanol (0.05% diethylamine); gradient: 5% to 40% B (2 min), 40% B (held for 1.2 min), 5% of B (held for 0.5 min); flow rate: 3.0 mL/min; column temperature: 35°C; pressure: 1500 psi), *ee* = 98.58%, retention time 1.394 minutes.

**Example 41**

Synthetic route:

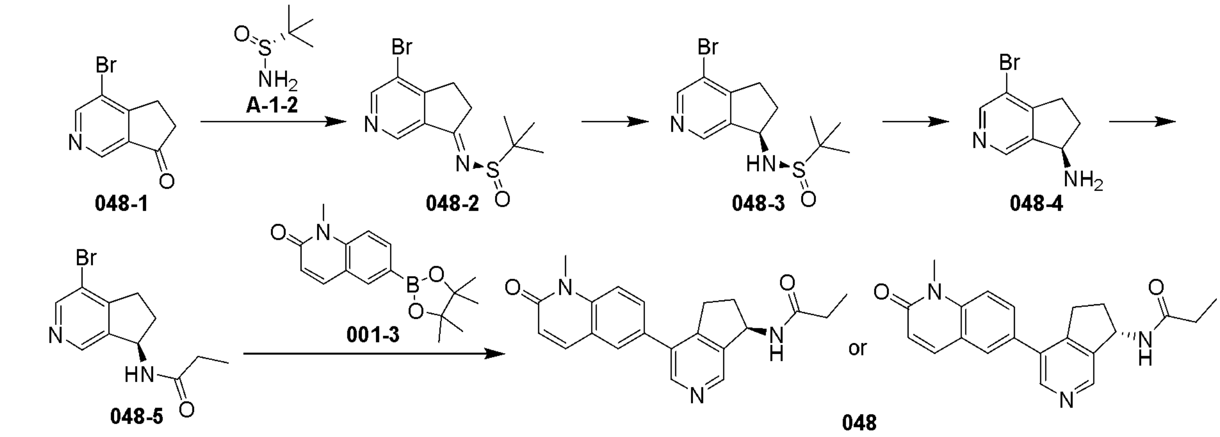
A-1-2

048-1 048-2 048-3 048-4

001-3

048-5

048-5 048 or

Step 1: synthesis of compound 048-2

To a reaction flask were sequentially added 048-1 (0.819 g, 3.86 mmol) and tetrahydrofuran (10 mL). After stirring to dissolve, tetraethyl titanate (1.60 mL, 7.72 mmol) was added, followed by dropwise addition of a tetrahydrofuran (10 mL) solution of A-1-2 (468.13 mg, 3.86 mmol). The mixture was stirred at 75°C for 1 hour. The reaction mixture was allowed to return to room temperature and sequentially added with 40 mL of ethyl acetate and 40 mL of saturated aqueous sodium bicarbonate solution. The mixture was filtered through diatomite, and the filter cake was rinsed sequentially with ethyl acetate, water, and ethyl acetate (40 mL × 2). The filtrate was collected, and the resulting phases were separated. The organic phase was collected and washed sequentially with saturated aqueous sodium bicarbonate solution and saturated brine (100 mL each time). The organic phase was collected, dried over anhydrous sodium sulfate, filtered, and concentrated to obtain a crude product. The crude product was purified by column chromatography (ethyl acetate:petroleum ether = 10 to 30%) to obtain 048-2. $^1H$ NMR (400 MHz, $CDCl_3$) δ ppm 8.90 (s, 1H), 8.73 (s, 1H), 3.50 - 3.63 (m, 2H), 3.10 - 3.22 (m, 2H), 1.34 (s, 9H).

Step 2: synthesis of compound 048-3

To a reaction flask were sequentially added 048-2 (255 mg, 808.94 μmol) and tetrahydrofuran (10 mL). The atmosphere was purged with nitrogen. A solution of 9-borabicyclo[3.3.1]nonane (0.5 M in tetrahydrofuran, 4.04 mL) was added dropwise at 0°C, and the mixture was warmed to 20°C and stirred for 16 hours. The reaction mixture was added with a saturated aqueous ammonium chloride solution (30 mL) and extracted with ethyl acetate (20 mL × 3). The organic phase was collected and washed with saturated brine (30 mL). The organic phase was collected, dried over anhydrous sodium sulfate, filtered, and concentrated to obtain a crude product. The crude product was purified by preparative thin-layer chromatography (dichloromethane:methanol = 10:1) to obtain 048-3.

Step 3: synthesis of compound 048-4

To a reaction flask were sequentially added 048-3 (170 mg, 535.86 μmol) and ethanol (9 mL). The mixture was placed at 0°C, followed by the addition of trimethylsilyl chloride (136.02 μL, 1.07 mmol). The mixture was warmed to 20°C and stirred for 2 hours. The reaction mixture was concentrated to obtain crude 048-4, which was directly used in the next step without purification. ESI-LCMS: m/z = 213.0 $[M+H]^+$.

Step 4: synthesis of compound 048-5

To a reaction flask were sequentially added 048-4 (115 mg, 539.72 μmol), dichloromethane (9 mL), and *N,N*-diisopropylethylamine (159.23 μL, 914.17 μmol). The mixture was maintained at 0°C, and a solution of propionic anhydride (72 μL, 558.78 μmol) in dichloromethane (9 mL) was added. The mixture was stirred at 0°C for 1 hour. 10 mL of water was added, and the resulting phases were separated. The aqueous phase was extracted with dichloromethane (10 mL × 3). The organic phases were combined and washed with 20 mL of saturated brine. The organic phase was collected, dried over anhydrous sodium sulfate, filtered, and concentrated to obtain a crude product. The crude product was purified by preparative thin-layer chromatography (dichloromethane:methanol = 15:1, with 1% triethylamine) to obtain 048-5. ESI-LCMS: m/z = 269.1 $[M+H]^+$.

Step 5: synthesis of compound 048

To a reaction flask were sequentially added 048-5 (58 mg, 215.50 μmol), 1,4-dioxane (5 mL), 001-3 (155.08 mg, 431.01 μmol), potassium carbonate (119.14 mg, 862.01 μmol),2-dicyclohexylphosphino-2,6-dimethoxybiphenyl (13.27 mg, 32.33 μmol), and water (1 mL). The atmosphere was purged with nitrogen, and palladium acetate (2.42 mg, 10.78 μmol) and bis(triphenylphosphine)palladium(II) chloride (7.56 mg, 10.78 μmol) were added. The mixture was stirred at 75°C for 2 hours. Additional 001-3 (155.08 mg, 431.01 μmol) was added, and the mixture was stirred at 75°C for 18 hours. The reaction mixture was returned to 20°C and filtered through anhydrous sodium sulfate. The filter cake was washed with 15 mL of ethyl acetate. The filtrate was collected and concentrated to obtain a crude product. The crude product was sequentially subjected to preparative thin-layer chromatography (dichloromethane:methanol = 20:1) and SFC chiral resolution (chromatographic column: DAICEL CHIRALPAK AS (250 mm*30 mm, 10 μm); mobile phase: [supercritical carbon dioxide-ethanol (0.1% ammonia)]; isocratic elution: 40% ethanol (0.1% ammonia)) to obtain 048. $^1$H NMR (400 MHz, $CDCl_3$) δ ppm 8.56 (br s, 2H), 7.72 (d, *J* = 9.29 Hz, 1H), 7.60 - 7.68 (m, 2H), 7.48 (d, *J*= 8.53 Hz, 1H), 6.78 (d, *J*= 9.29 Hz, 1H), 5.78 (br d, *J*= 7.03 Hz, 1H), 5.69 (q, *J* = 7.70 Hz, 1H), 3.78 (s, 3H), 2.98 - 3.12 (m, 2H), 2.63 - 2.74 (m, 1H), 2.32 (q, *J*= 7.53 Hz, 2H), 1.82 - 1.92 (m, 1H), 1.22 - 1.26 (m, 3H); ESI-LCMS: *m*/*z* = 348.2 $[M+H]^+$. SFC analysis was performed (chromatographic column: Chiralpak AD-3 50×4.6 mm I.D., 3 μm; mobile phase: A: supercritical carbon dioxide B: ethanol (0.05% diethylamine); gradient: 5% to 40% B (1.5 min), 40% B (held for 1.0 min), 5% of B (held for 0.5 min); flow rate: 4 mL/min; column temperature: 35°C; pressure: 1500 psi), *ee* = 100%, retention time 1.927 minutes.

## Biological evaluation

### Experimental example 1: *In vitro* activity assay of monoclonal cells overexpressing human CYP11B2

The human renal leiomyoma G402 cell line overexpressing human CYP11B2 was selected to serve as a compound activity screening system. Details are as follows:
1) Information on cells, reagents, and instruments is provided in Tables 1 and 2.

Table 1: Cell and reagent information

| Cells and reagents | Supplier |
|---|---|
| G402 cells | ATCC |
| Aldosterone Detection Kit | Cisbio |
| 11-Deoxycorticosterone | Med Chem Express (MCE) |
| 11-Deoxycortisol | Med Chem Express (MCE) |

Table 2: Instrument information

| Product name | Company | Model/Specification |
|---|---|---|
| Low-speed benchtop centrifuge | Xiangyi Centrifuge Instrument Co., Ltd., Changsha High-tech Industrial Development Zone | TDZ5-WS |
| Thermostat water bath with digital display | Guohua (Changzhou) Instrument Manufacturing Co., Ltd. | HH-6 |
| Cell counter | SelinsBio | EVE™ Plus |
| Microplate reader | BMG LABTECH | PHERAstar FSX |
| Microplate horizontal shaker | Suzhou Jiemei Electronics Co., Ltd. | OS-07U |
| Low-temperature high speed refrigerated centrifuge | eppendorf | Centrifuge 5810 R |
| Mass spectrometry | Waters | XEVO TQ-S Micro |
| Liquid chromatography | Waters | ACQUITY UPLC I-Class Plus |

2) Compound details: The test compound was prepared as a 10 μM working concentration using DMSO.
3) Experimental procedure

Day 1: Cells were digested, and human renal leiomyoma G402 cells overexpressing human CYP11B2 were resuspended to $1\times10^5$ cells/mL, with 100 μL seeded into a 96-well plate.

Day 2: The medium was replaced with DMEM/F12 containing 2.5% charcoal-stripped fetal bovine serum; test compounds were added: 5 μL of test compound was added to the cell culture medium to achieve final concentrations of 10000, 2500, 625, 156.25, 39.06, 9.77, 2.44, 0.61, 0.15, and 0 nM (with a DMSO content of 0.1%).

After one hour, the substrate was added: 30 mM 11-deoxycorticosterone was diluted 1500-fold with culture medium, and 5 μL was dispensed per well to achieve a final concentration of 1 μM, followed by incubation for 16 hours.

Day 3: The cell supernatant was collected and added to 200 μL of a methanol solution containing internal standards (internal standards: 35 ng/mL ketoprofen or 7.5 ng/mL carbamazepine or 5 ng/mL diphenhydramine or 10 ng/mL tolbutamide). The mixture was vortexed and centrifuged (4000×g, 4°C, 5 minutes). Then, 100 μL of the supernatant was taken and mixed with 100 μL of pure water. The mixture was centrifuged (4000 g, 4°C, 15 min), and the supernatant was analyzed by ESI-LCMS/MS.

4) Data analysis:

$$\text{Ratio} = \text{Aldosterone peak area} / \text{Internal standard peak area}$$

$$\text{Control activity\%} = (\text{Ratio test compound} / \text{Ratio DMSO}) \times 100\%$$

"Ratio test compound" represents the Ratio values at different concentrations of the test compound.

"Ratio DMSO" represents the Ratio value without the addition of the test compound.

X = Log (compound concentration)
Y = Control activity%

The $IC_{50}$ of the test compound was determined by fitting to a nonlinear regression equation using XLfit 5.5.0. The test results are presented in Table 3.

Table 3: Results for *in vitro* activity assay of monoclonal cells overexpressing human CYP11B2

| Compound | Human CYP11B2, $IC_{50}$ (nM) | Compound | Human CYP11B2, $IC_{50}$ (nM) |
|---|---|---|---|
| Compound 001 | 11.96 | Compound 025 | 65.49 |
| Compound 005 | 19.47 | Compound 027 | 62.74 |
| Compound 010 | 71.45 | Compound 030 | 48.71 |
| Compound 011 | 59.73 | Compound 031 | 22.59 |
| Compound 014 | 20.65 | Compound 032 | 30.15 |
| Compound 015 | 31.82 | Compound 037 | 14.19 |
| Compound 016 | 63.64 | Compound 041 | 42.17 |
| Compound 017 | 2.09 | Compound 042 | 40.99 |
| Compound 018 | 20.66 | Compound 044 | 32.87 |
| Compound 019 | 17.31 | Compound 045 | 42.70 |
| Compound 020 | 25.48 | Compound 046 | 13.30 |
| Compound 022 | 48.75 | Compound 048 | 7.02 |
| Compound 023 | 12.53 | | |

Experimental conclusion: The compounds of the present disclosure exhibited significant inhibitory effect against human CYP11B2, effectively suppressing aldosterone production. They are clinically applicable for the treatment of primary aldosteronism, resistant hypertension, and other aldosterone-related metabolic syndromes.

**Experimental example 2: *In vitro* activity assay of monoclonal cells overexpressing human CYP11B1**

The human renal leiomyoma G402 cell line overexpressing human CYP11B1 was selected to serve as a compound selectivity screening system. Details are as follows:

1) The cells, reagents, and instrument information were consistent with Experimental Example 1 (activity of monoclonal cells overexpressing human CYP11B2).
2) Compound details: The test compound was prepared as a 50 μM working concentration using DMSO.
3) Experimental procedure

Day 1: Cells were digested, and human renal leiomyoma G402 cells overexpressing CYP11B1 were resuspended to $1\times10^5$ cells/mL, with 100 μL seeded into a 96-well plate.
Day 2: The medium was replaced with DMEM/F12 containing 2.5% charcoal-stripped fetal bovine serum.
Test compounds were added: 5 μL of test compound was added to the cell culture medium to achieve final concentrations of 50000, 12500, 3125, 1526, 781.25, 260.42, 86.81, 28.94, 9.65, and 0 nM (with a DMSO content of 0.5%).
After one hour, the substrate was added: 30 mM 11-deoxycortisol was diluted 1500-fold with culture medium, and 5 μL was dispensed per well to achieve a final concentration of 1 μM, followed by incubation for 16 hours.
Day 3: The cell supernatant was collected and added to 200 μL of a methanol solution containing internal standards (internal standards: 35 ng/mL ketoprofen or 7.5 ng/mL carbamazepine or 5 ng/mL diphenhydramine or 10 ng/mL tolbutamide). The mixture was vortexed and centrifuged (4000×g, 4°C, 5 minutes). Then, 100 μL of the supernatant was taken and mixed with 100 μL of pure water. The mixture was centrifuged (4000 g, 4°C, 15 min), and the supernatant was analyzed by ESI-LCMS/MS.

4) Data analysis:

$$\text{Ratio} = \text{Cortisol peak area} / \text{Internal standard peak area}$$

$$\text{Control activity\%} = (\text{Ratio test compound} / \text{Ratio DMSO}) \times 100\%$$

"Ratio test compound" represents the Ratio values at different concentrations of the test compound.
"Ratio DMSO" represents the Ratio value without the addition of the test compound.

X = Log (compound concentration)
Y = Control activity%

The $IC_{50}$ of the test compound was determined by fitting to a nonlinear regression equation using XLfit 5.5.0. The test results are presented in Table 4.

Table 4: Results for *in vitro* activity assay of monoclonal cells overexpressing human CYP11B1

| Compound | Human CYP11B1, $IC_{50}$ (nM) | Compound | Human CYP11B1, $IC_{50}$ (nM) |
|---|---|---|---|
| Compound 001 | 2472 | Compound 030 | 2181 |
| Compound 005 | 1416 | Compound 031 | 4738 |
| Compound 010 | 6880 | Compound 032 | 5648 |
| Compound 011 | 6912 | Compound 037 | 3472 |
| Compound 017 | 322 | Compound 041 | 6750 |
| Compound 018 | 3811 | Compound 044 | 19474 |
| Compound 019 | 1684 | Compound 045 | 34928 |
| Compound 020 | 1127 | Compound 046 | 1569 |
| Compound 022 | 6894 | Compound 048 | 2486 |
| Compound 023 | 1742 | | |

Experimental conclusion: The compounds of the present disclosure exhibited weak inhibition against human CYP11B1 and minimally affected cortisol synthesis, thereby demonstrating improved clinical safety.

**Experimental example 3: *In vivo* pharmacokinetics study in rats**

Experimental purpose: Male SD rats were used as test animals to determine plasma concentrations of compounds after a single administration and evaluate pharmacokinetic behavior.

Experimental procedure: Two healthy adult male SD rats were selected for the oral administration group. The vehicle in the oral administration group was a mixture of 20% PEG400, 10% solutol, and 70% water. After mixing the compound to be tested with the vehicle, the mixture was vortexed and sonicated to prepare a clear solution of 0.25 mg/mL. After oral administration of 2 mg/kg to rats, whole blood was collected for a certain period of time to prepare plasma, and the drug concentration was analyzed by LC-MS/MS method, then the pharmacokinetic parameters were calculated using Phoenix WinNonlin software (Pharsight, USA). The experimental results are shown in Table 5.

Table 5: PK test results of compounds of the present disclosure in rats

| Compound No. | $C_{max}$ (nM) | $AUC_{0-24h}$ (nM.h) | $T_{1/2}$ (h) | F (%) |
|---|---|---|---|---|
| Compound 001 | 7635 | 59097 | 4.61 | 80 |
| Compound 005 | 1315 | 7802 | 2.77 | 111 |
| Compound 017 | 4165 | 8129 | 1.34 | 115 |
| Compound 023 | 6916 | 44234 | 3.6 | 109 |
| Compound 037 | 8285 | 49894 | 2.36 | 157 |
| Compound 046 | 7855 | 36571 | 3.23 | 71 |

Experimental conclusion: The compounds of the present disclosure exhibited a long half-life, high oral exposure, and good oral bioavailability in oral *in vivo* pharmacokinetic studies in rats, demonstrating favorable pharmacokinetic characteristics suitable for an oral drug.

**Experimental example 4: *In vivo* pharmacokinetic study in cynomolgus monkeys**

Experimental purpose: Cynomolgus monkeys were used as test animals to determine plasma concentrations of compounds after a single administration and evaluate pharmacokinetic behavior.

Experimental procedure: Two healthy male cynomolgus monkeys were selected for the oral administration group. The vehicle used in the oral administration group was a mixture of 20% PEG400, 10% solutol, and 70% water. The test compound was mixed with the vehicle, vortexed, and ultrasonicated to prepare a clear solution of 0.6 mg/mL. The oral administration dose for cynomolgus monkeys was 1 mg/kg or 3 mg/kg. Following oral administration, whole blood was collected for a certain period of time to prepare plasma, and the drug concentration was analyzed by LC-MS/MS method, then the pharmacokinetic parameters were calculated using Phoenix WinNonlin software (Pharsight, USA). The experimental results are shown in Table 6.

Table 6: PK test results of compounds of the present disclosure in cynomolgus monkeys

| Compound No. | Dose (mg/kg) | $C_{max}$ (nM) | $AUC_{0-24h}$ (nM.h) | $T_{1/2}$ (h) | F (%) |
|---|---|---|---|---|---|
| Compound 001 | 3 | 4005 | 23174 | 4.99 | 120 |
| Compound 017 | 1 | 1071 | 7292 | 5.30 | 66 |
| Compound 018 | 1 | 1310 | 7586 | 5.35 | - |
| Compound 019 | 3 | 3795 | 23864 | 4.65 | 113 |
| Compound 023 | 1 | 1320 | 8916 | 4.80 | 76.5 |
| Compound 037 | 1 | 1320 | 10039 | 5.75 | 67 |
| Note: -- indicates that the test was not conducted. | | | | | |

Experimental conclusion: The compounds of the present disclosure exhibited a long half-life, high oral exposure,

and good oral bioavailability in oral *in vivo* pharmacokinetic studies in cynomolgus monkeys, demonstrating favorable pharmacokinetic characteristics suitable for an oral drug.

**Experimental example 5: Permeability test**

Experimental objective: The permeability and efflux ratio of the compound of the present disclosure were determined using the MDR1-MDCKII monolayer cell model to assess its membrane permeability and potential for P-gp-mediated efflux transport.

Experimental protocol: MDR1-MDCKII cells (obtained from the Netherlands Cancer Institute) were seeded in 96-well plates (obtained from Corning) at a cell density of $3.33 \times 10^5$ cells/mL and cultured for 4 to 7 days to form confluent cell monolayers. Hank's balanced salt solution containing 10 mM HEPES (pH 7.40) was used as the transport buffer. The test compound was evaluated for bidirectional transport at a concentration of 2 μM, with the DMSO concentration in the incubation system maintained below 1%. After adding the samples, the cell plate was incubated at 37±1°C, 5% $CO_2$, and saturated humidity for 2.5 hours. All samples were quantitatively analyzed using the LC-MS/MS method. The apparent permeability coefficient ($P_{app}$, cm/s) and efflux ratio (ER) were calculated using the following formula. Apparent permeability coefficient ($P_{app}$, cm/s) was calculated using the following formula: $P_{app} = (dC_r/dt) \times V_r / (A \times C_0)$. Herein, $dC_r/dt$ represents the cumulative concentration of the compound at the receiving end per unit time (μM/s); $V_r$ is the volume of the solution at the receiving end (with solution volumes at the apical and basolateral ends being 0.075 mL and 0.250 mL, respectively); A is the relative surface area of the cell monolayer (0.143 $cm^2$); $C_0$ is the initial concentration of the test substance at the dosing end (μM) or the peak area ratio of the reference substance. The efflux ratio was calculated using the following formula: ER = $P_{app}$ (BA)/ $P_{app}$ (AB). The experimental results are shown in Table 7.

Table 7: Permeability test results of compounds

| Compound | Apparent permeability coefficient ($P_{app}$ (AB), $10^{-6}$ cm/s) | Efflux ratio (ER) |
|---|---|---|
| Compound 001 | 17.2 | 1.76 |
| Compound 005 | 23.1 | 0.70 |
| Compound 011 | 24.3 | 0.89 |
| Compound 017 | 23.2 | 0.95 |
| Compound 018 | 24.6 | 1.32 |
| Compound 019 | 21.5 | 1.55 |
| Compound 020 | 14.0 | 2.47 |
| Compound 023 | 20.8 | 1.28 |
| Compound 031 | 20.0 | 0.89 |
| Compound 032 | 22.6 | 0.86 |
| Compound 037 | 10.7 | 1.88 |
| Compound 044 | 11.0 | 2.46 |
| Compound 045 | 16.7 | 1.30 |
| Compound 046 | 18.0 | 1.17 |

Experimental conclusion: The compounds of the present disclosure exhibited high permeability and low efflux, demonstrating good druggability.

**Example 6: *In vitro* liver microsome metabolic stability study**

Experimental objective: To evaluate the metabolic stability of the test compounds in human liver microsomes.

Experimental materials: Purchased from Corning or Xenotech and stored at -80°C freezer; Reduced Nicotinamide Adenine Dinucleotide Phosphate (NADPH), Supplier: Chemimpex international; Control compounds: testosterone, diclofenac, propafenone.

Experimental procedure:

1. Preparation of the working solution

Stock solution: 10 mM in DMSO. Preparation of working concentration: The stock solution was diluted to 100 μM using 100% acetonitrile (organic phase content: 99% acetonitrile, 1% DMSO).

2. Experimental procedure

Two incubation plates, designated as the T60 incubation plate and the NCF60 incubation plate, were prepared, and the incubation plates were pre-warmed for 10 minutes.

Liver microsomes were diluted to a protein concentration of 0.56 mg/mL using 100 mM potassium phosphate buffer.

445 μL of microsomal working solution (with a liver microsomal protein concentration of 0.56 mg/mL) was transferred to pre-warmed incubation plates T60 and NCF60 and pre-incubated at 37°C in a water bath for 10 minutes.

After pre-incubation, 5 μL of the test compound or control compound working solution was added to both the T60 incubation plate and the NCF60 incubation plate and mixed well. 50 μL of potassium phosphate buffer was added to each well of the NCF60 incubation plate and mixed thoroughly, and the reaction was initiated.

**In** the T0 stop plate, 180 μL of stop solution (acetonitrile solution containing 200 ng/mL tolbutamide and 200 ng/mL labetalol) and 6 μL of NADPH regeneration system working solution were added. 54 μL of sample was transferred from the T60 incubation plate to the T0 stop plate (T0 sample generated). 44 μL of NADPH regeneration system working solution per well was added to the T60 incubation plate to initiate the reaction. Only 54 μL of microsomal working solution, 6 μL of NADPH regeneration system working solution, and 180 μL of stop solution were added to the blank plate. Therefore, in the samples containing the test compounds or control compounds, the final concentrations of the compounds, testosterone, diclofenac, and propafenone in the reaction were 1 μM, the concentration of liver microsomes was 0.5 mg/mL, and the final concentrations of DMSO and acetonitrile in the reaction system were 0.01% (v/v) and 0.99% (v/v), respectively.

After incubation for appropriate durations (5, 15, 30, 45, 60 minutes), 180 μL of stop solution was added to each sample well of the stop plate, followed by transferring 60 μL of the sample from the T60 incubation plate to the stop plate to terminate the reaction.

All sample plates were vortexed and subsequently centrifuged at 4°C for 20 minutes (4000 rpm). 80 μL of supernatant was taken from each well, transferred into 240 μL of pure water for dilution, and mixed for 10 minutes. LC-MS/MS analysis was performed. The experimental results are shown in Table 8.

Table 8: Stability test results of compounds of the present disclosure in human liver microsomes

| Compound No. | Intrinsic hepatic clearance $CL_{int(liver)}$ (mL/min/kg) | Compound No. | Intrinsic hepatic clearance $CL_{int(liver)}$ (mL/min/kg) |
|---|---|---|---|
| Compound 001 | <8.6 | Compound 023 | <8.6 |
| Compound 011 | <8.6 | Compound 031 | <8.6 |
| Compound 017 | <8.6 | Compound 032 | <8.6 |
| Compound 018 | <8.6 | Compound 037 | <8.6 |
| Compound 019 | <8.6 | Compound 046 | <8.6 |
| Compound 020 | 12.9 | | |

Experimental conclusion: The compounds of the present disclosure exhibited low clearance in human liver microsomes, demonstrating relatively good metabolic stability.

**Experimental example 7: Plasma protein binding rate test of compounds**

Experimental objective: To evaluate the protein binding rate of the compounds of the present disclosure in the plasma of SD rat, cynomolgus monkeys, and human using equilibrium dialysis.

Test protocol:

Blank plasma from humans, SD rats, and cynomolgus monkeys (796 μL, purchased from BioreclamationIVT) was spiked with working solutions of the test compound or warfarin to achieve final concentrations of 2 μM for both the test

compound and warfarin in the plasma samples.

The sample was thoroughly mixed. The final concentration of the organic phase DMSO was 0.5%. A volume of 50 μL of the test compound and warfarin plasma sample was transferred to the sample receiving plate, followed by immediate addition of the corresponding volume of blank plasma or buffer solution to achieve a final volume of 100 μL per sample well. The volume ratio of plasma to dialysis buffer was 1:1. A stop solution was then added to these samples, and this sample was designated as the T0 sample for recovery and stability determination.

The test compound and warfarin plasma samples were added to the dosing end of each dialysis well, and blank dialysis buffer was added to the corresponding receiving end. The dialysis plate was then sealed with a gas-permeable membrane and placed in a humidified 5% $CO_2$ incubator, where it was incubated at 37°C with shaking at 100 rpm for 4 hours.

After dialysis, 50 μL of the dialyzed buffer sample and the dialyzed plasma sample were transferred to a new sample receiving plate. Appropriate volumes of corresponding blank plasma or buffer solution were added to the samples, resulting in a final volume of 100 μL per sample well, with a plasma-to-dialysis buffer volume ratio of 1:1.

Data analysis:

All samples were subjected to protein precipitation followed by LC/MS/MS analysis. The free fraction and plasma protein binding rate of the compound were calculated using the following formulas: Free fraction (%) = 100 × Fc/Tc, Plasma protein binding rate of the compound (%) = 100% - Free fraction of the compound (%), where Fc is the concentration of the compound in the buffer compartment of the dialysis plate; Tc is the concentration of the compound in the plasma compartment of the dialysis plate; $T_0$ is the concentration of the compound in the plasma sample at time zero.

The experimental results are shown in Table 9.

Table 9: Results of plasma protein binding rate of compounds of the present disclosure

| Compound No. | Plasma protein binding rate (%) in SD rats | Plasma protein binding rate (%) in cynomolgus monkeys | Human plasma protein binding rate (%) |
|---|---|---|---|
| Compound 001 | 92.1 | 74.6 | 76.1 |
| Compound 005 | 89.8 | 87.3 | 89.4 |
| Compound 011 | 86.1 | 75.5 | 75.5 |
| Compound 017 | 94.9 | 78.1 | 82.7 |
| Compound 018 | 92.0 | 79.5 | 80.9 |
| Compound 019 | 87.1 | 75.2 | 76.2 |
| Compound 020 | 91.7 | 90.3 | 85.6 |
| Compound 023 | 90.4 | 69.0 | 69.6 |
| Compound 031 | 85.9 | 79.7 | 80.7 |
| Compound 032 | 84.9 | 79.5 | 84.9 |
| Compound 037 | 90.8 | 54.9 | 64.6 |
| Compound 044 | 97.6 | 66.4 | 70.5 |
| Compound 045 | 96.9 | 68.0 | 76.1 |
| Compound 046 | 97.6 | 69.6 | 83.4 |

Experimental conclusion: The compounds of the present disclosure exhibited moderate binding to plasma proteins across different species, with an appropriate proportion of free drug in plasma, indicating good druggability.

**Experimental example 8: Human liver microsome CYP inhibition assay**

Experimental objective: To evaluate the inhibitory activity of the test substance on human liver microsomal cytochrome P450 isoenzymes (CYP1A2, CYP2C9, CYP2C19, CYP2D6, CYP3A4) using a 5-in-1 probe substrate for CYP isoenzymes.

Mixed human liver microsomes (HLM) were purchased from Corning Inc. (Steuben, New York, USA) and stored at temperatures below -70°C prior to use.

The working solution of the test substance, which had been diluted to a series of concentrations, was added to an incubation system containing human liver microsomes, probe substrates, and auxiliary factors of the circulatory system. A control containing solvent but no test substance served as an enzyme activity control (100%). The concentration of metabolites produced from probe substrates in the samples was determined using Liquid Chromatography-Tandem Mass Spectrometry (LC-MS/MS) method. Non-linear regression analysis of the average percentage activity of the test substance against its concentration was conducted using SigmaPlot (V.11). The $IC_{50}$ values were calculated using a three-parameter or four-parameter inverse log equation. The experimental results are shown in Table 10.

Table 10: Cytochrome P450 isoenzyme half maximal inhibitory concentration $IC_{50}$ ($\mu$M)

| Compound No. | CYP1A2 | CYP2C9 | CYP2C19 | CYP2D6 | CYP3A4 |
|---|---|---|---|---|---|
| Compound 001 | >50 | >50 | >50 | >50 | >50 |
| Compound 011 | >50 | >50 | >50 | >50 | >50 |
| Compound 017 | >50 | 45.1 | >50 | >50 | >50 |
| Compound 023 | >50 | >50 | >50 | >50 | >50 |
| Compound 037 | >50 | >50 | >50 | >50 | >50 |
| Compound 046 | >50 | >50 | >50 | >50 | >50 |

Experimental conclusion: The compounds of the present disclosure exhibited no inhibitory effect on CYP enzymes CYP1A2, CYP2C9, CYP2C19, CYP2D6, and CYP3A4, indicating a very low risk of drug-drug interactions.

## Claims

1. A compound of formula (II), a stereoisomer thereof, or a pharmaceutically acceptable salt thereof,

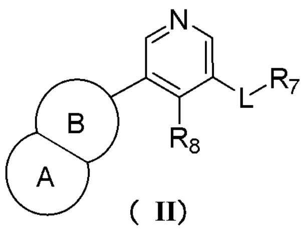

( II) ,

wherein

ring A is selected from the group consisting of ring $A_1$ and ring $A_2$;
ring $A_1$ is

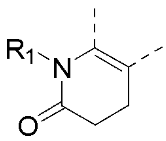

;

ring $A_2$ is selected from the group consisting of

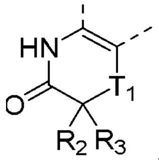 , 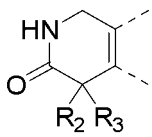 , 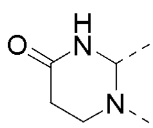 ,

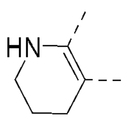, 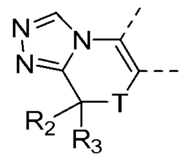, 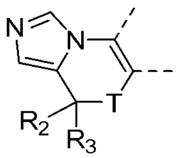, 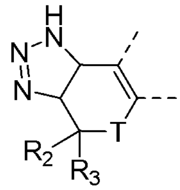, and 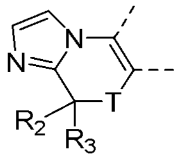,

wherein the

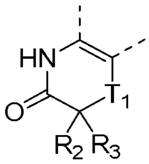, 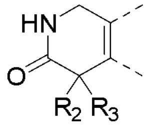, 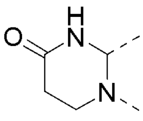, 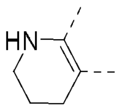, 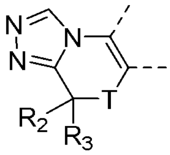, 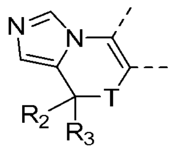,

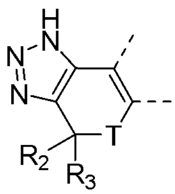, and 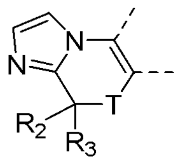

are each independently and optionally substituted by 1, 2, or 3 $R_1$;
ring B is selected from the group consisting of ring $B_1$ and ring $B_2$;
ring $B_1$ is selected from the group consisting of phenyl, pyridyl, and pyridazinyl, wherein the phenyl, pyridyl, and pyridazinyl are each independently and optionally substituted by 1, 2, or 3 $R_6$;
ring $B_2$ is 5-membered heteroaryl, wherein the 5-membered heteroaryl is optionally substituted by 1, 2, or 3 $R_6$;
T is selected from the group consisting of a single bond, $CH_2$, NH, and O;
$T_1$ is selected from the group consisting of O and $CR_4R_5$;
$R_1$ is selected from the group consisting of H, =NR, $C_{1-3}$ alkyl, and $C_{1-3}$ haloalkyl;
each $R_2$, $R_3$, $R_4$, and $R_5$ is independently selected from the group consisting of H, F, Cl, Br, I, $C_{1-3}$ alkyl, $C_{1-3}$ alkoxy, $C_{1-3}$ haloalkyl, and $C_{1-3}$ haloalkoxy, wherein not all four of $R_2$, $R_3$, $R_4$, and $R_5$ are simultaneously H;
alternatively, $R_2$ and $R_3$ together with the carbon atom to which they are attached form a cyclopropyl;
alternatively, $R_4$ and $R_5$ together with the carbon atom to which they are attached form a cyclopropyl;
alternatively, $R_3$ and $R_4$ together with the carbon atom to which they are attached form a double bond or a cyclopropyl;
each $R_6$ is independently selected from the group consisting of H, F, Cl, Br, I, and $C_{1-3}$ alkyl;
the structural moiety

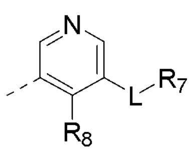

is selected from the group consisting of

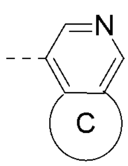

and

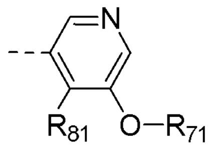;

$R_{71}$ is 4- to 6-membered heterocycloalkyl, wherein the 4- to 6-membered heterocycloalkyl is optionally substituted by 1, 2, or 3 $R_a$;
$R_{81}$ is selected from the group consisting of H, F, Cl, Br, I, and $C_{1-3}$ alkyl;
ring C is selected from the group consisting of ring $C_1$ and ring $C_2$;
ring $C_1$ is $C_{5-6}$ cycloalkyl substituted by 1 $R_9$, wherein the $R_9$ is selected from the group consisting of -NHC(=O)-$C_{1-3}$ alkyl, -NH-C(=O)-5- to 6-membered heteroaryl, -NH-4- to 6-membered heterocycloalkyl, 4- to 6-membered heterocycloalkyl, and

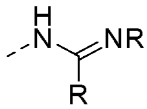,

wherein the -NHC(=O)-$C_{1-3}$ alkyl, -NH-C(=O)-5- to 6-membered heteroaryl, -NH-4- to 6-membered heterocycloalkyl, and 4- to 6-membered heterocycloalkyl are each independently and optionally substituted by 1, 2, or 3 $R_b$;
ring $C_2$ is selected from the group consisting of

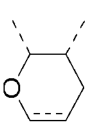, 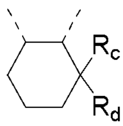, and 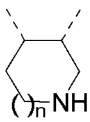,

wherein the

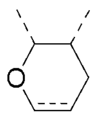, 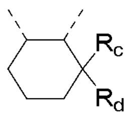, and 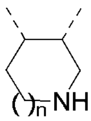

are each independently and optionally substituted by 1, 2, or 3 $R_e$;
each $R_a$ is independently selected from the group consisting of F, Cl, Br, I, $C_{1-3}$ alkyl, and -S(=O)$_2$-$C_{1-3}$ alkyl;
each $R_b$ is independently selected from the group consisting of F, Cl, Br, I, $C_{1-3}$ alkyl, - C(=O)-$C_{1-3}$ alkyl, and -NH-C(=O)-$C_{1-3}$ alkyl;
$R_c$ and $R_d$ together with the carbon atom to which they are attached form a $C_{3-5}$ cycloalkyl or a 5- to 6-membered heterocycloalkyl;
each $R_e$ is independently selected from the group consisting of F, Cl, Br, I, =O, $C_{1-3}$ alkyl, $C_{1-3}$ alkoxy, $C_{1-3}$ alkylamino, -NHC(=O)-$C_{1-3}$ alkyl, -NHC(=O)-$C_{1-3}$ alkoxy, -NHC(=O)-$C_{1-3}$ alkylamino, and =NR;
R is selected from the group consisting of $C_{1-3}$ alkyl and $C_{1-3}$ alkoxy, wherein the $C_{1-3}$ alkyl and $C_{1-3}$ alkoxy are each independently and optionally substituted by 1, 2, or 3 halogens;
n is 0 or 1;
is a single bond or a double bond;
the term "hetero" in the 5-membered heteroaryl, 5- to 6-membered heteroaryl, and 4- to 6-membered heterocycloalkyl represents 1, 2, 3, or 4 heteroatoms or heteroatomic groups, each independently selected from the group consisting of O, S, and N;
provided that
1) when ring A is $A_1$ and ring B is $B_1$, then ring C is $C_2$; or,
2) when ring A is $A_1$, ring B is $B_1$, and ring C is $C_{5-6}$ cycloalkyl substituted by 1 $R_9$, then $R_9$ is

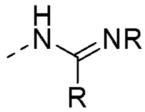

.

2. The compound according to claim 1, a stereoisomer thereof, or a pharmaceutically acceptable salt thereof, wherein R is selected from the group consisting of $CH_3$, $CH_2CH_3$, $CF_3$, $OCH_3$, and $OCF_3$.

3. The compound according to claim 1, a stereoisomer thereof, or a pharmaceutically acceptable salt thereof, wherein $R_a$ is $-S(=O)_2-CH_2CH_3$.

4. The compound according to claim 1, a stereoisomer thereof, or a pharmaceutically acceptable salt thereof, wherein each $R_b$ is independently selected from the group consisting of F, Cl, Br, I, $CH_3$, $-C(=O)-CH_2CH_3$, and $-NHC(=O)-CH_2CH_3$.

5. The compound according to claim 1, a stereoisomer thereof, or a pharmaceutically acceptable salt thereof, wherein $R_c$ and $R_d$ together with the carbon atom to which they are attached form

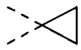

or

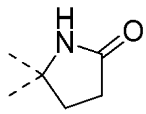

.

6. The compound according to claim 1, a stereoisomer thereof, or a pharmaceutically acceptable salt thereof, wherein $R_e$ is selected from the group consisting of $-NHC(=O)-CH_2CH_3$, $=NCH_3$, and $=N-OCH_3$.

7. The compound according to claim 1, a stereoisomer thereof, or a pharmaceutically acceptable salt thereof, wherein $R_1$ is $CH_3$.

8. The compound according to claim 1, a stereoisomer thereof, or a pharmaceutically acceptable salt thereof, wherein each $R_2$, $R_3$, $R_4$, and $R_5$ is independently selected from the group consisting of H, F, Cl, Br, $CH_3$, $CHF_2$, $CF_3$, $CH_2CH_3$, $OCH_3$, and $OCH_2CH_3$, wherein not all four of $R_2$, $R_3$, $R_4$, and $R_5$ are simultaneously H.

9. The compound according to claim 1, a stereoisomer thereof, or a pharmaceutically acceptable salt thereof, wherein $R_{71}$ is

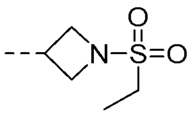

.

10. The compound according to claim 1, a stereoisomer thereof, or a pharmaceutically acceptable salt thereof, wherein $R_9$ is selected from the group consisting of $-NHC(=O)-CH_2CH_3$, -NH-C(=O)-pyridyl, -NH-C(=O)-isoxazolyl, -NH-piperidinyl, -NH-pyrrolidinyl, - NH-azetidinyl, piperidinyl, and

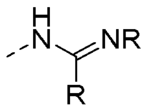

,

wherein the $-NHC(=O)-CH_2CH_3$, -NH-C(=O)-pyridyl, -NH-C(=O)-isoxazolyl, -NH-piperidinyl, -NH-pyrrolidinyl, -NH-azetidinyl, and piperidinyl are each independently and optionally substituted by 1, 2, or 3 $R_b$.

11. The compound according to claim 1, a stereoisomer thereof, or a pharmaceutically acceptable salt thereof, wherein $R_9$ is selected from the group consisting of

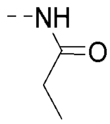,

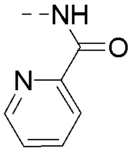, 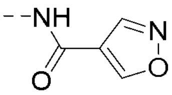, 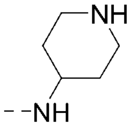, 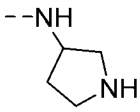, 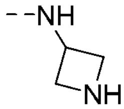, 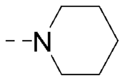, 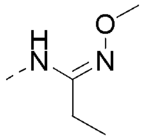,

and

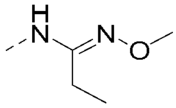,

wherein the

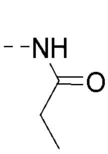, 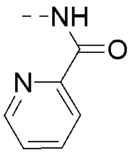, 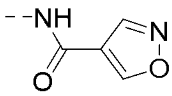, 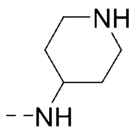, 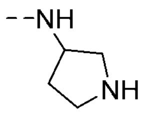,

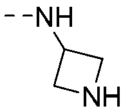, and 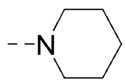

are each independently and optionally substituted by 1, 2, or 3 $R_b$.

12. The compound according to claim 1, a stereoisomer thereof, or a pharmaceutically acceptable salt thereof, wherein $R_9$ is selected from the group consisting of

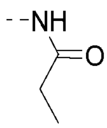,

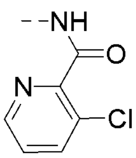, 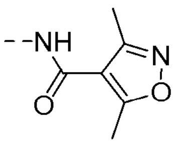, 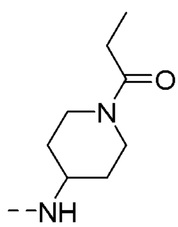, 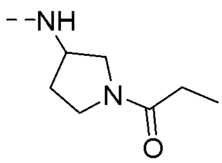, 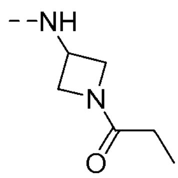,

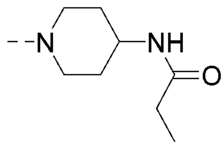, 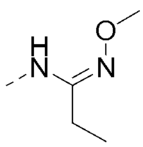, and 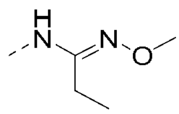.

**13.** The compound according to claim 1, a stereoisomer thereof, or a pharmaceutically acceptable salt thereof, wherein ring $B_1$ is selected from the group consisting of

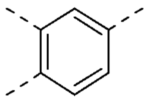,

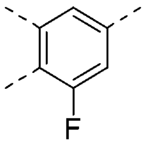, 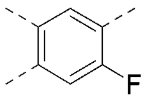, 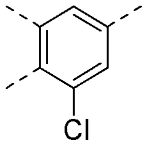, 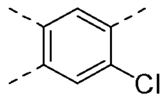, 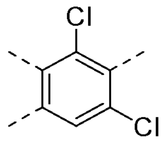, 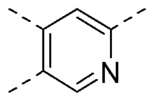,

and

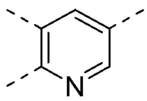.

**14.** The compound according to claim 1, a stereoisomer thereof, or a pharmaceutically acceptable salt thereof, wherein ring $B_2$ is selected from the group consisting of thienyl, thiazolyl, imidazolyl, and pyrazolyl, wherein the thienyl, thiazolyl, imidazolyl, and pyrazolyl are each independently and optionally substituted by 1, 2, or 3 $R_a$.

**15.** The compound according to claim 14, a stereoisomer thereof, or a pharmaceutically acceptable salt thereof, wherein ring $B_2$ is selected from the group consisting of

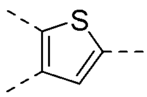,

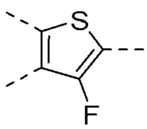, 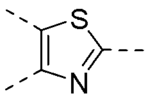, 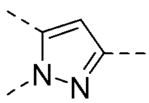, and 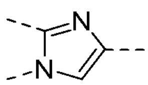.

**16.** The compound according to claim 1, a stereoisomer thereof, or a pharmaceutically acceptable salt thereof, wherein ring $A_2$ is selected from the group consisting of

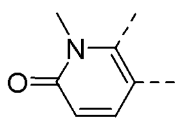,

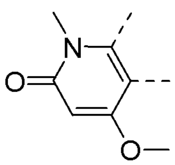, 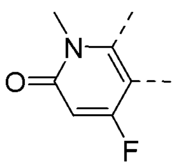, 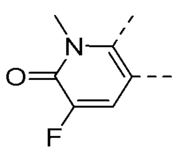, 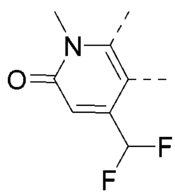, 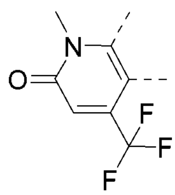, 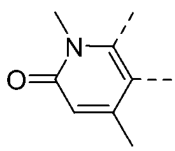,

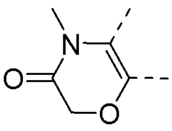, 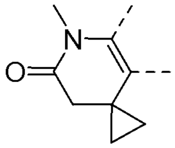, 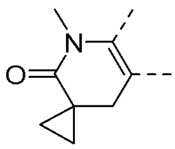, 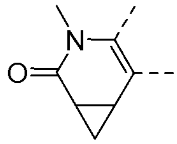, 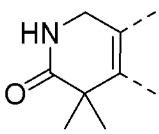, 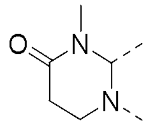,

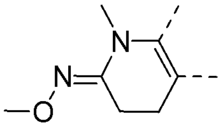, 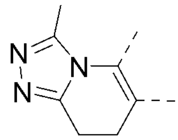, 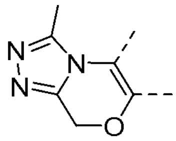, 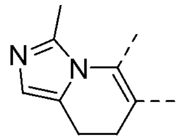, 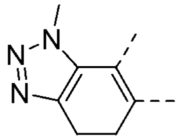,

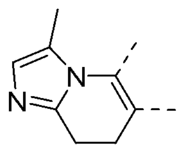, and 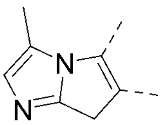.

**17.** The compound according to claim 1, a stereoisomer thereof, or a pharmaceutically acceptable salt thereof, wherein ring $C_1$ is selected from the group consisting of

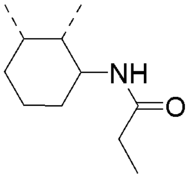, 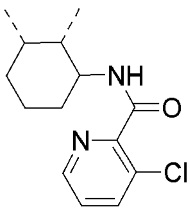, 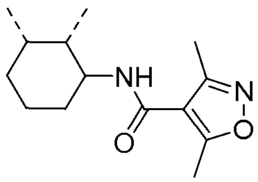, 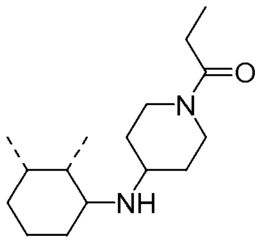,

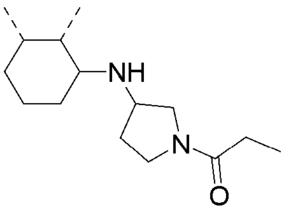, 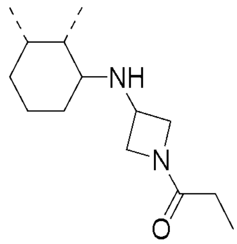, 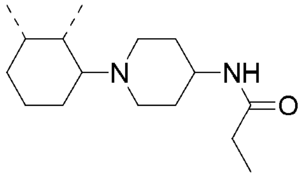, 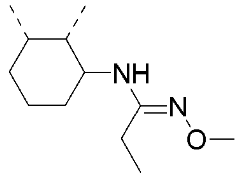,

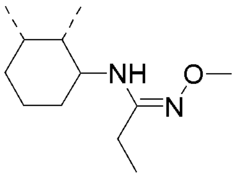, and 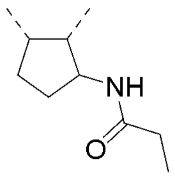.

**18.** The compound according to claim 1, a stereoisomer thereof, or a pharmaceutically acceptable salt thereof, wherein ring $C_2$ is selected from the group consisting of

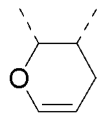,

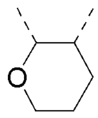, 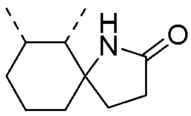, 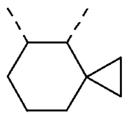, 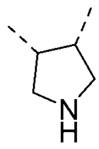, and 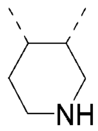,

wherein the

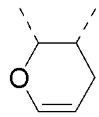, 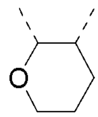,

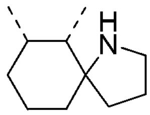, 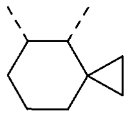, 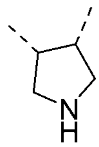, and 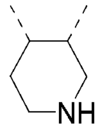

are each independently and optionally substituted by 1, 2, or 3 $R_e$.

**19.** The compound according to claim 18, a stereoisomer thereof, or a pharmaceutically acceptable salt thereof, wherein ring $C_2$ is selected from the group consisting of

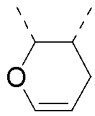,

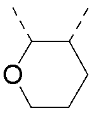, 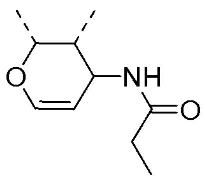, 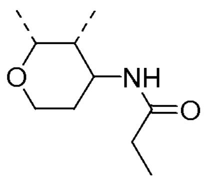, 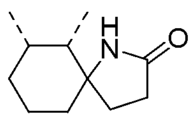, 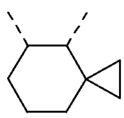, 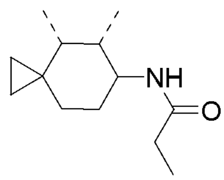,

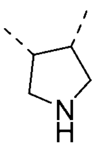, 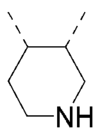, 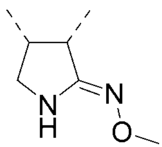, 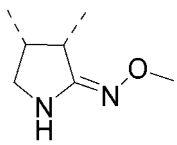, 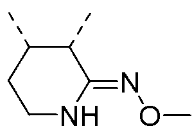, and 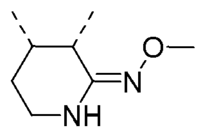.

**20.** The compound according to claim 1, a stereoisomer thereof, or a pharmaceutically acceptable salt thereof, wherein the compound is selected from the group consisting of:

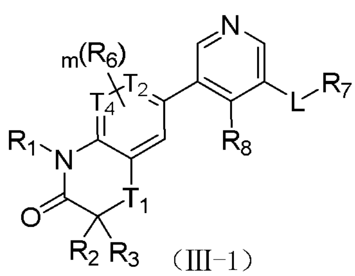 (III-1), 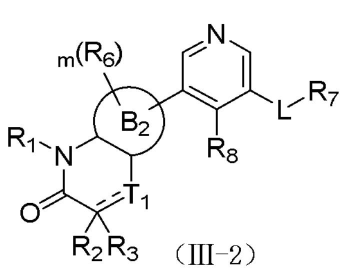 (III-2),

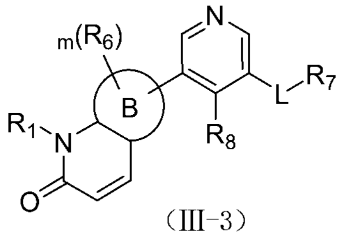
(III-3)

, and

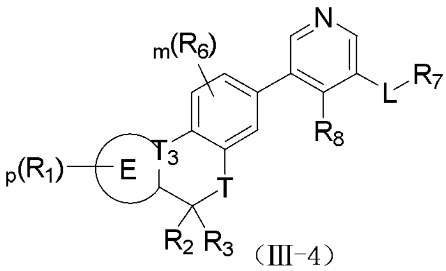
(III-4)

,

wherein

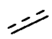

is selected from the group consisting of a single bond and a double bond;
$T_2$ is selected from the group consisting of CH and N;
$T_4$ is selected from the group consisting of CH and N;
$T_3$ is selected from the group consisting of C and N;
ring E is selected from the group consisting of

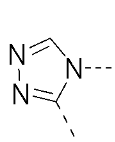
,
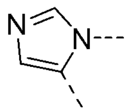
,
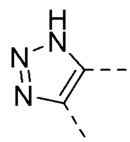
, ;

and

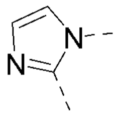
;

m is selected from the group consisting of 0, 1, 2, and 3;
p is selected from the group consisting of 0, 1, and 2;
ring B is selected from the group consisting of ring $B_1$ and ring $B_2$;
ring $B_1$ is selected from the group consisting of phenyl, pyridyl, and pyridazinyl;
ring $B_2$ is 5-membered heteroaryl;
T, Ti, $R_1$, $R_2$, $R_3$, $R_6$, L, $R_7$, and $R_8$ are as defined in claim 1.

**21.** The compound according to claim 1, a stereoisomer thereof, or a pharmaceutically acceptable salt thereof, wherein the compound is selected from the group consisting of:

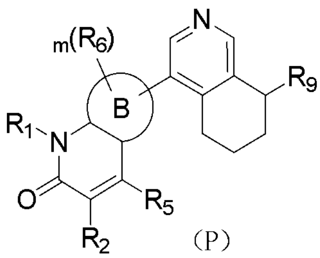
(P)

and

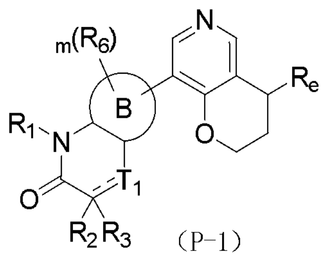
(P-1)

,

wherein

ring B is selected from the group consisting of phenyl, pyridyl, pyridazinyl, and 5-membered heteroaryl;
$T_1$ is selected from the group consisting of O and $CR_4R_5$;

each $R_2$, $R_3$, $R_4$, and $R_5$ is independently selected from the group consisting of H, F, Cl, Br, I, $C_{1-3}$ alkyl, $C_{1-3}$ alkoxy, $C_{1-3}$ haloalkyl, and $C_{1-3}$ haloalkoxy;
alternatively, $R_2$ and $R_3$ together with the carbon atom to which they are attached form a cyclopropyl;
alternatively, $R_4$ and $R_5$ together with the carbon atom to which they are attached form a cyclopropyl;
alternatively, $R_3$ and $R_4$ together with the carbon atom to which they are attached form a double bond or a cyclopropyl;
each $R_6$ is independently selected from the group consisting of H, F, Cl, Br, I, and $C_{1-3}$ alkyl;
$R_9$ is selected from the group consisting of -NHC(=O)-$C_{1-3}$ alkyl, -NH-C(=O)-5- to 6-membered heteroaryl, -NH-4- to 6- membered heterocycloalkyl, 4- to 6- membered heterocycloalkyl, and

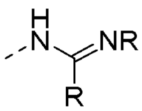,

wherein the -NHC(=O)-$C_{1-3}$ alkyl, -NH-C(=O)-5- to 6-membered heteroaryl, -NH-4- to 6- membered heterocycloalkyl, and 4- to 6- membered heterocycloalkyl are each independently and optionally substituted by 1, 2, or 3 $R_b$;
each $R_b$ is independently selected from the group consisting of F, Cl, Br, I, $C_{1-3}$ alkyl, - C(=O)-$C_{1-3}$ alkyl, and -NH-C(=O)-$C_{1-3}$ alkyl;
each $R_e$ is independently selected from the group consisting of F, Cl, Br, I, =O, $C_{1-3}$ alkyl, $C_{1-3}$ alkoxy, $C_{1-3}$ alkylamino, -NHC(=O)-$C_{1-3}$ alkyl, -NHC(=O)-$C_{1-3}$ alkoxy, -NHC(=O)-$C_{1-3}$ alkylamino, and =NR;
R is selected from the group consisting of $C_{1-3}$ alkyl and $C_{1-3}$ alkoxy, wherein the $C_{1-3}$ alkyl and $C_{1-3}$ alkoxy are each independently and optionally substituted by 1, 2, or 3 halogens;
m is selected from the group consisting of 0, 1, 2, and 3;
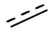 is a single bond or a double bond;
the term "hetero" in the 5-membered heteroaryl, 5- to 6-membered heteroaryl, and 4- to 6-membered heterocycloalkyl represents 1, 2, 3, or 4 heteroatoms or heteroatomic groups, each independently selected from the group consisting of O, S, and N.

**22.** A compound represented by one of the following formulas, a stereoisomer thereof, or a pharmaceutically acceptable salt thereof,

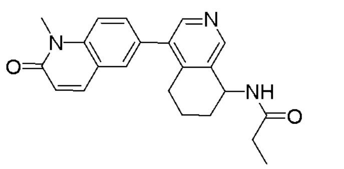, 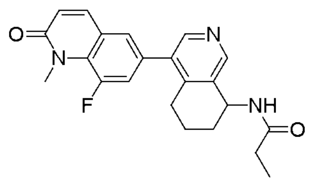, 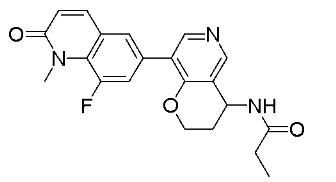,

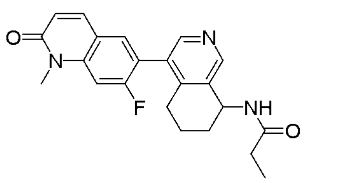, 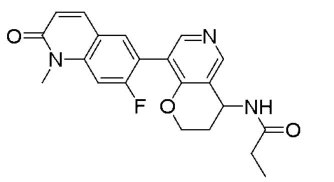, 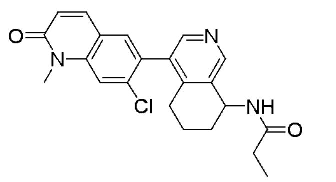,

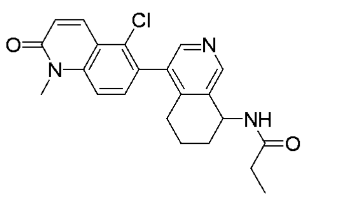, 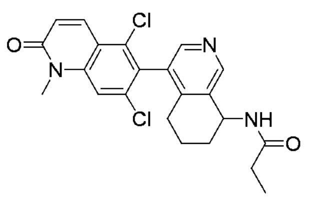, 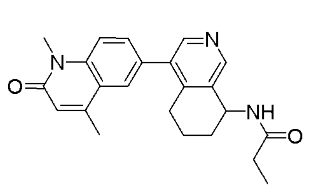,

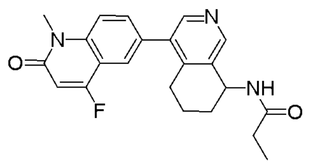,
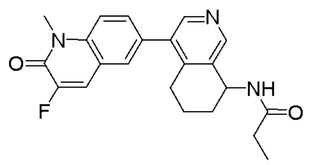,
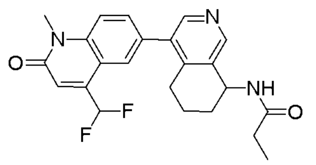,
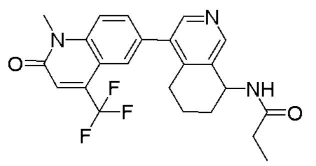,
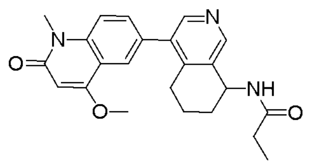,
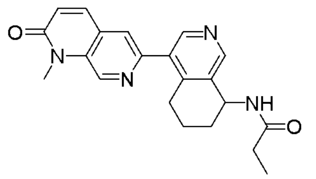,
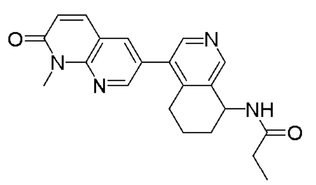,
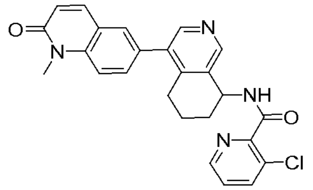,
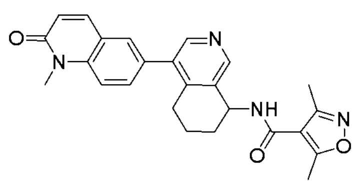,
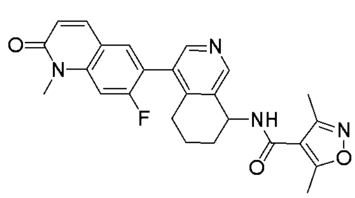,
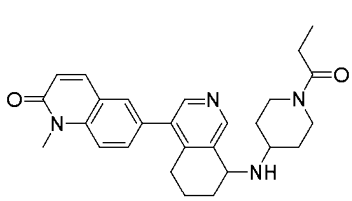,
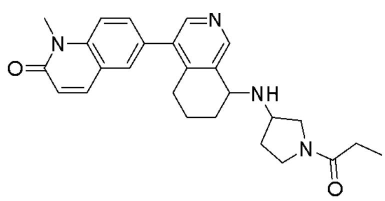,
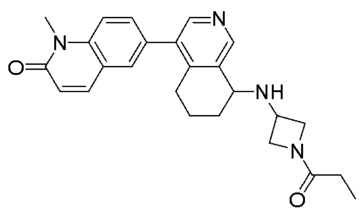,
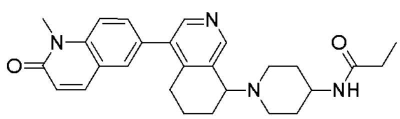,
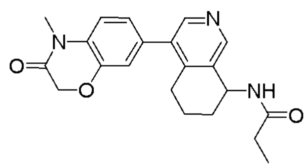,
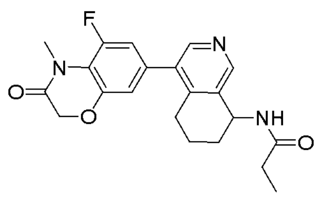,
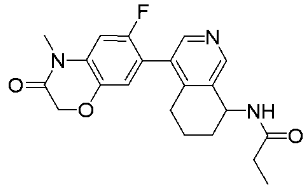,
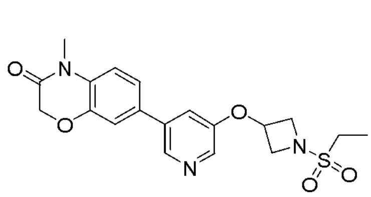,
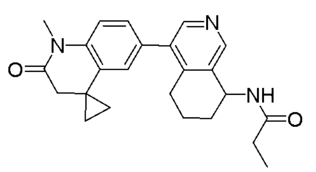,
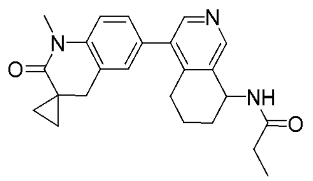,
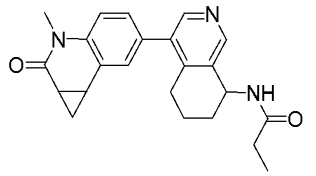,

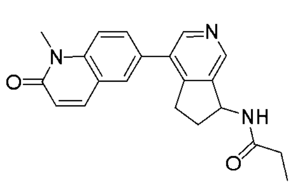 , 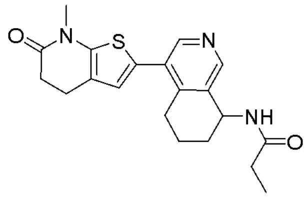 , 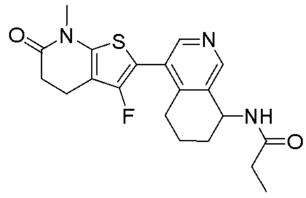 ,

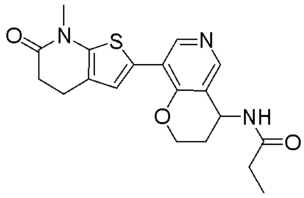 , 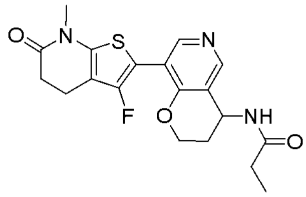 , 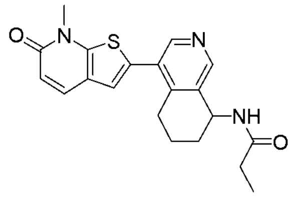 ,

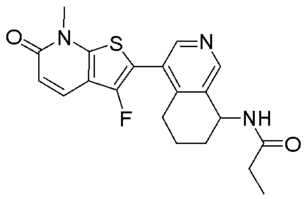 , 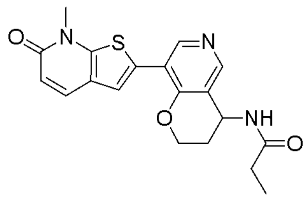 , 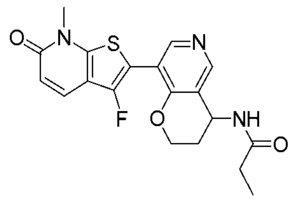 ,

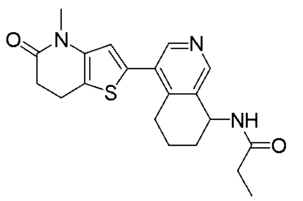 , 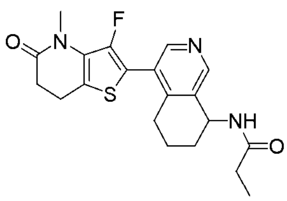 , 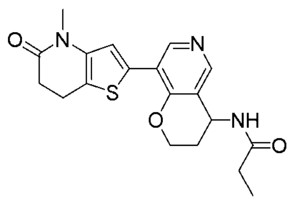 ,

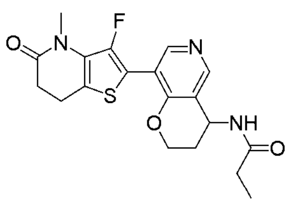 , 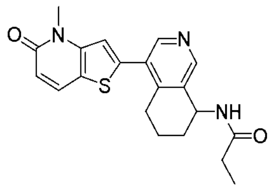 , 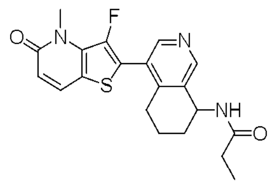 , 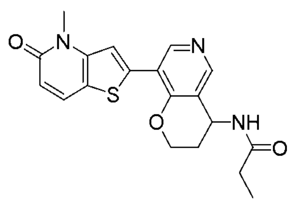 ,

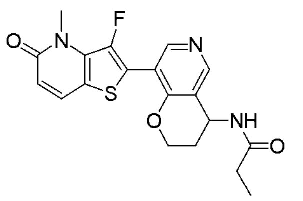 , 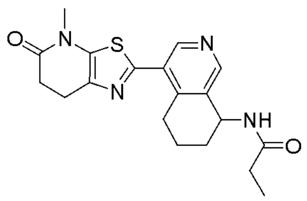 , 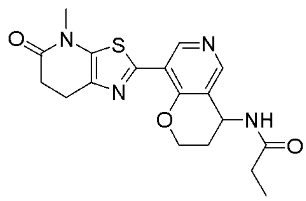 ,

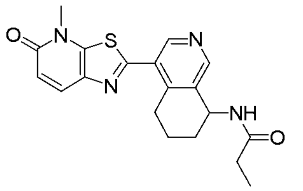 , 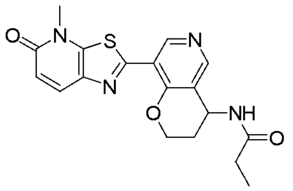 , 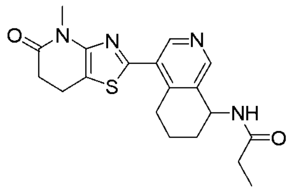 ,

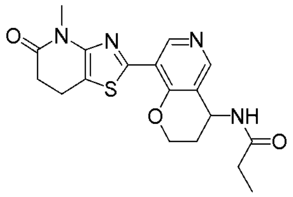,
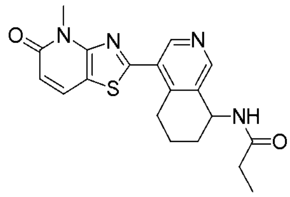,
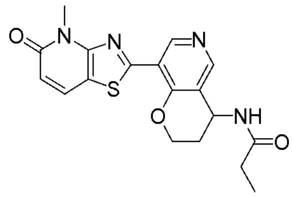,

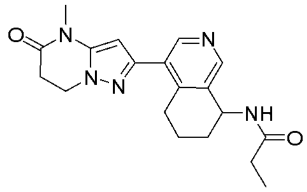,
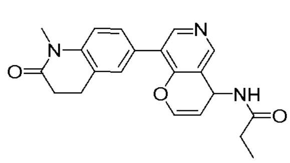,
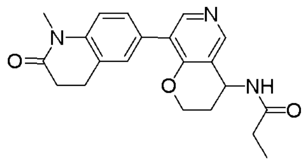,

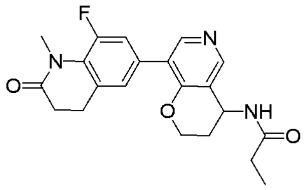,
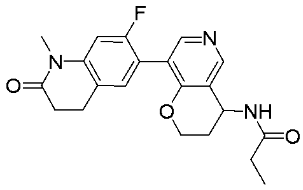,
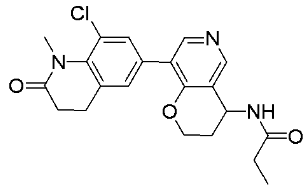,

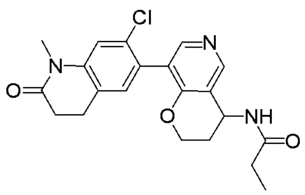,
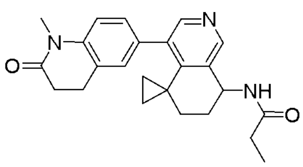,
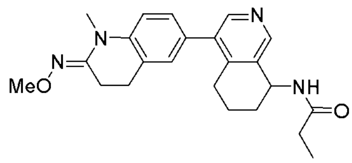,

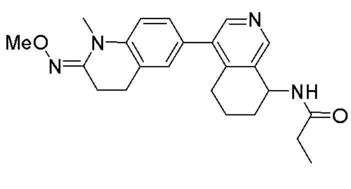,
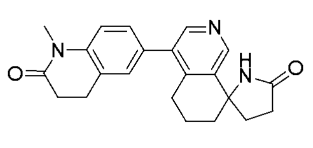,
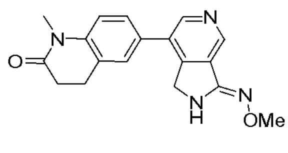,

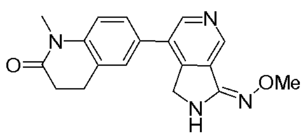,
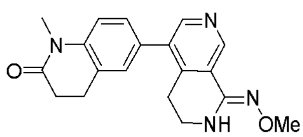,
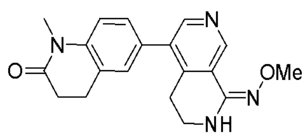,

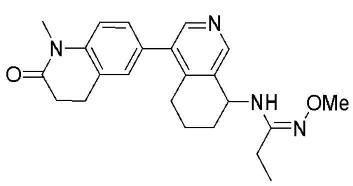,
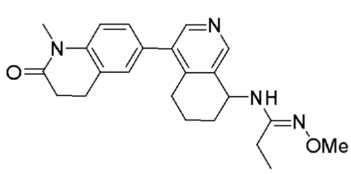,
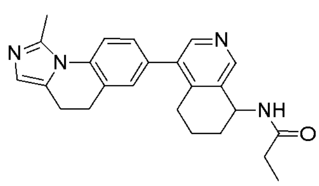,

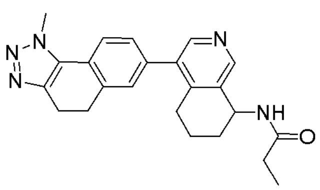,
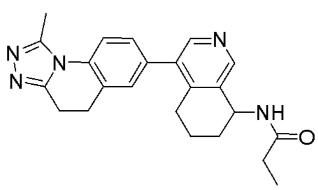,
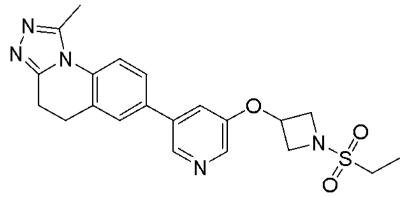,

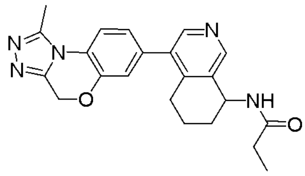,
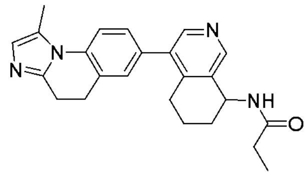,
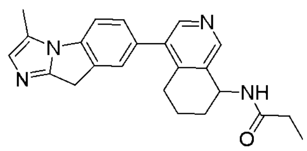,
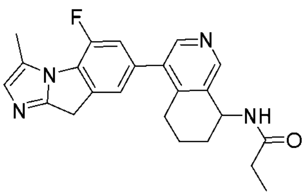, and
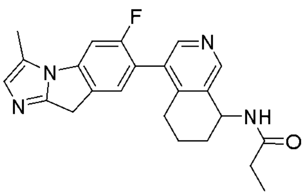.

**23.** The compound according to claim 21, a stereoisomer thereof, or a pharmaceutically acceptable salt thereof, wherein the compound is selected from the group consisting of

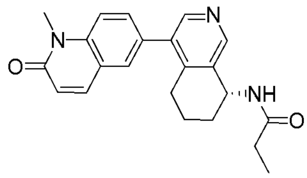,
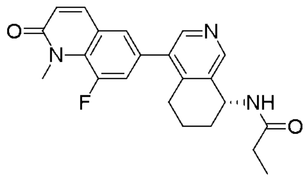,
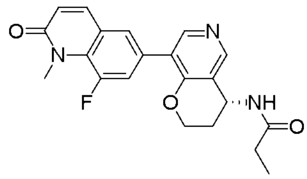,
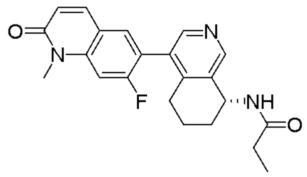,
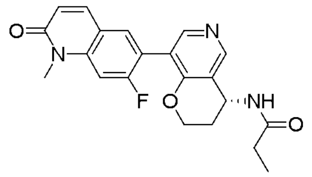,
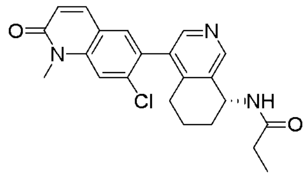,
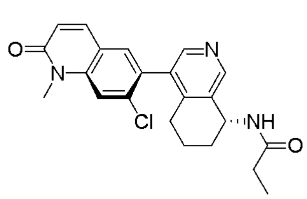,
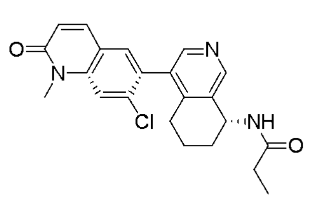,
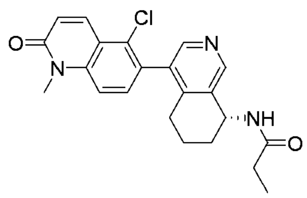,
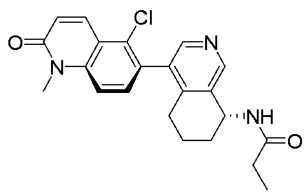,
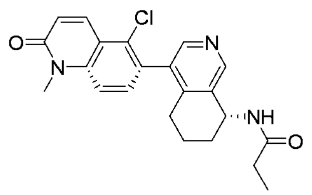,
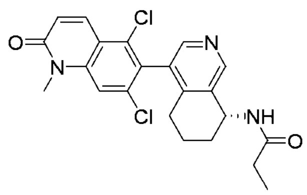,
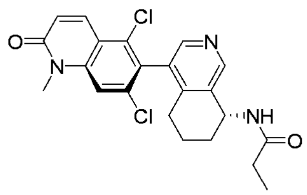,
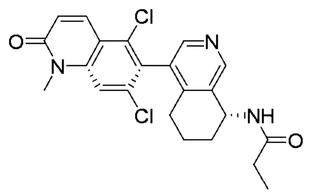,
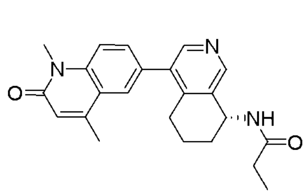,

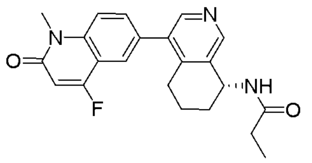 , 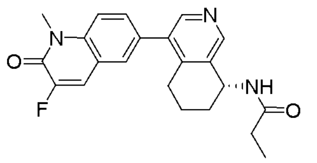 , 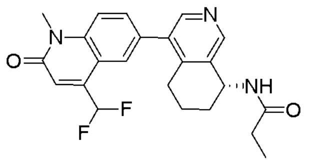 ,

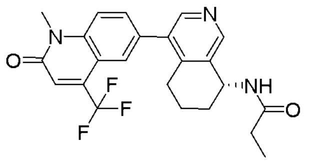 , 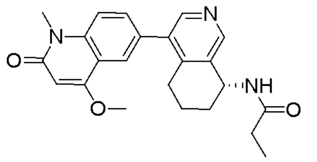 , 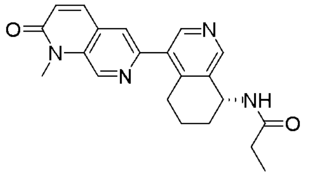 ,

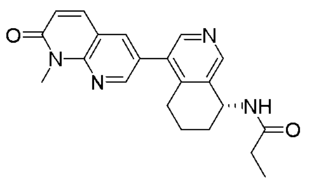 , 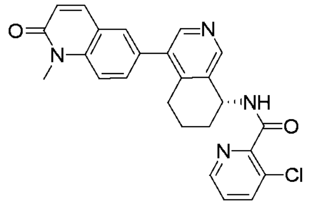 , 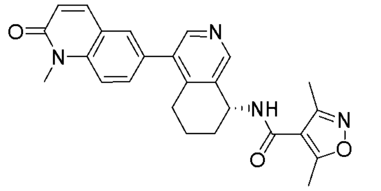 ,

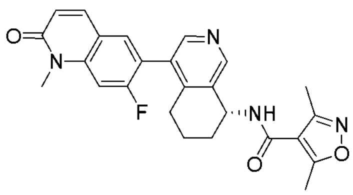 , 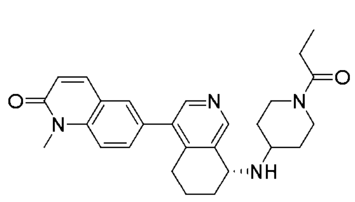 , 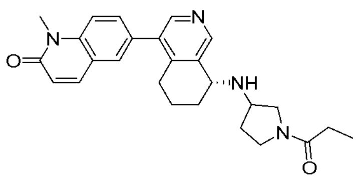 ,

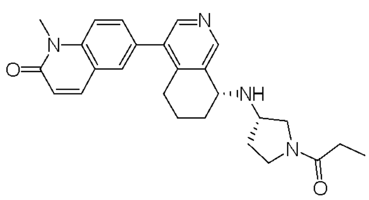 , 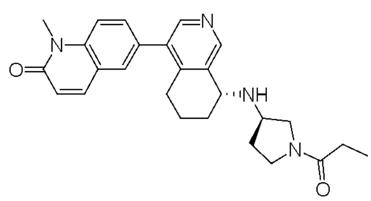 , 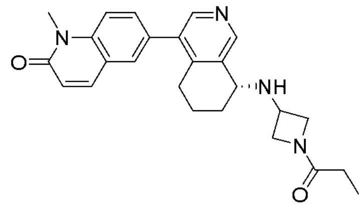 ,

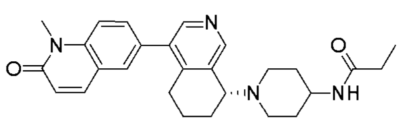 , 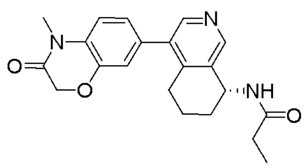 , 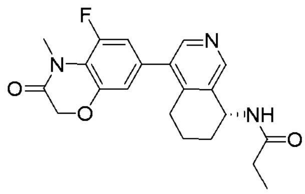 ,

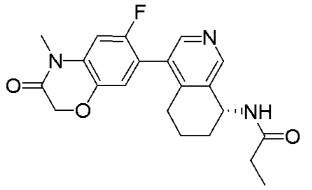 , 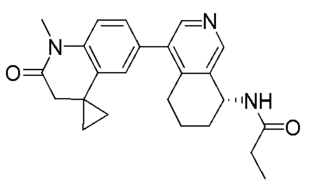 , 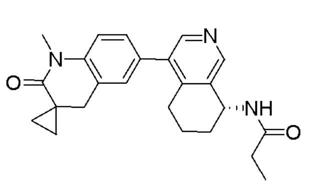 ,

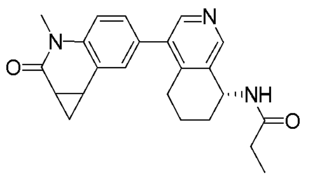 , 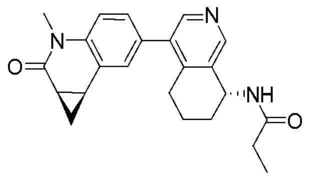 , 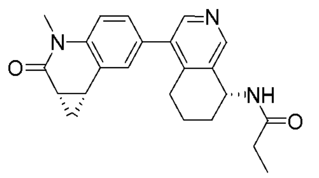 ,

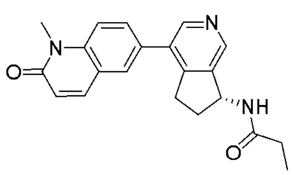 , 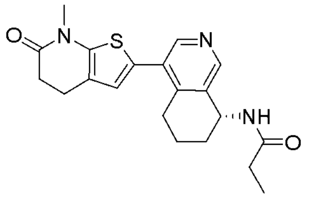 , 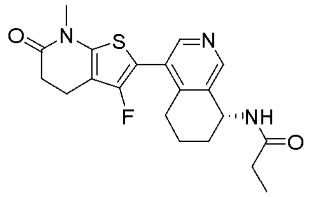 ,

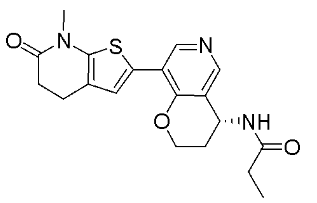 , 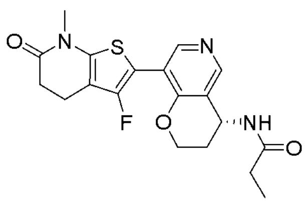 , 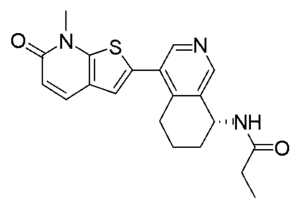 ,

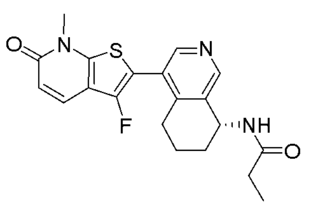 , 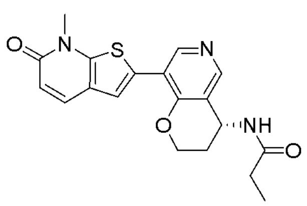 , 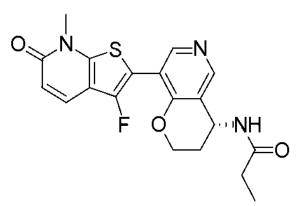 ,

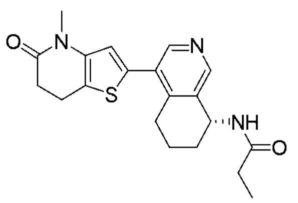 , 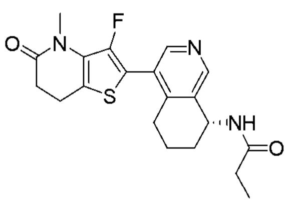 , 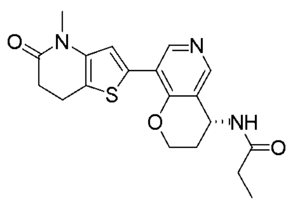 ,

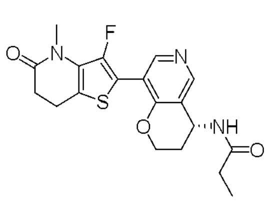 , 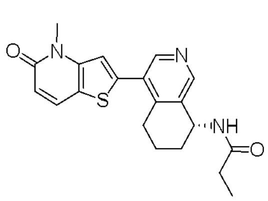 , 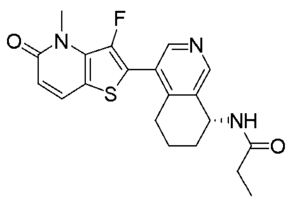 , 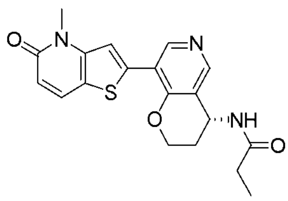 ,

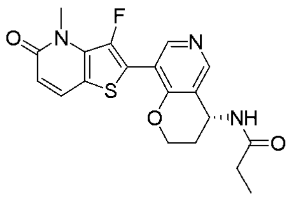 , 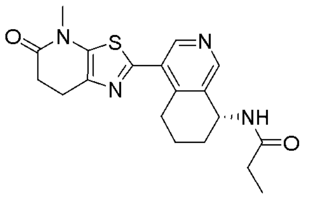 , 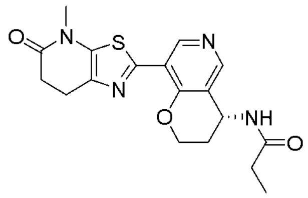 ,

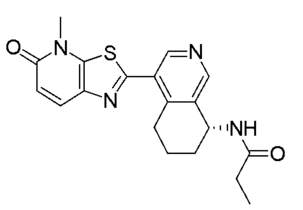,
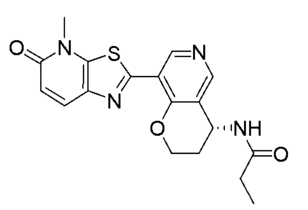,
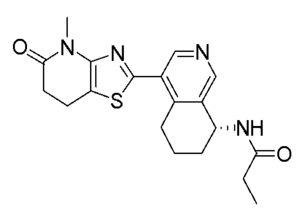,

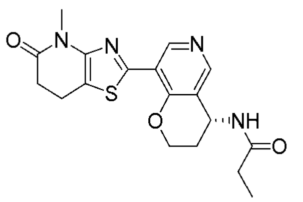,
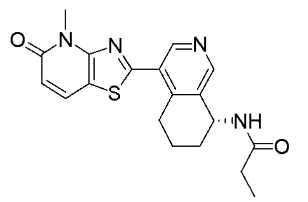,
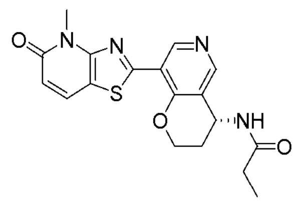,

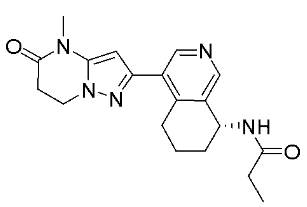,
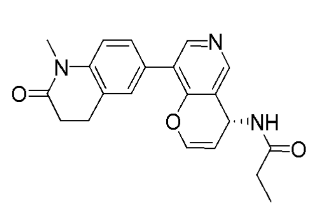,
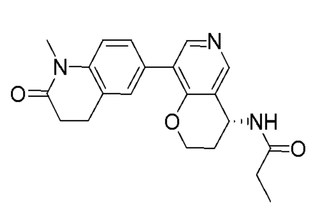,

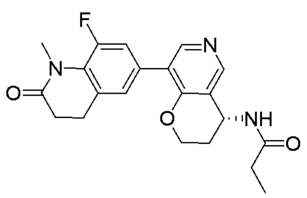,
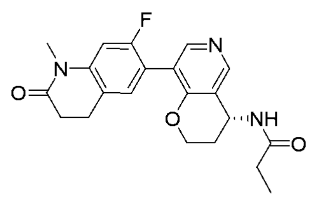,
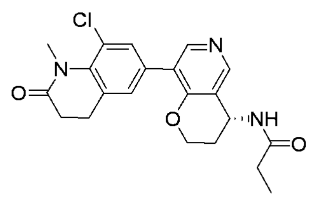,

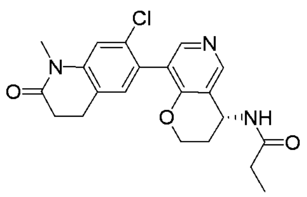,
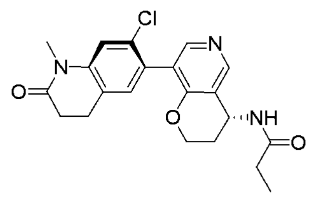,
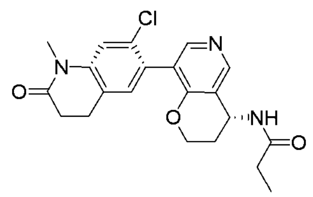,

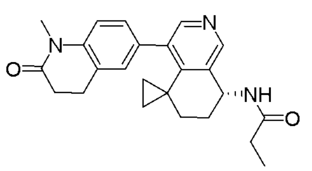,
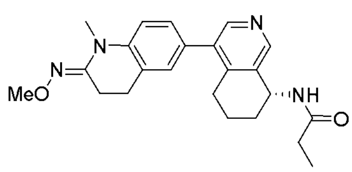,
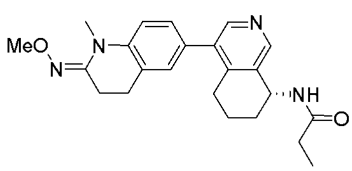,

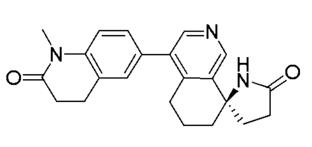,
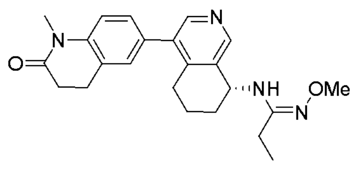,
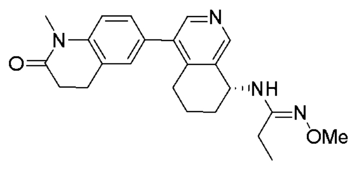,

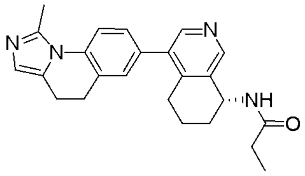,
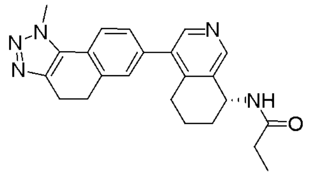,
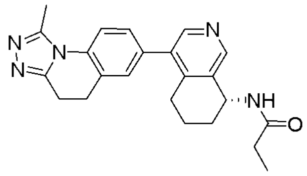,

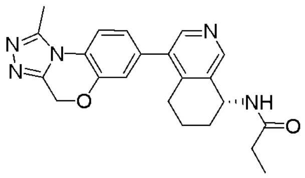,
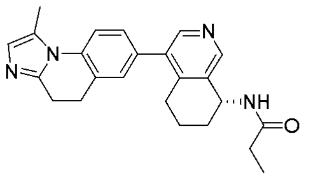,
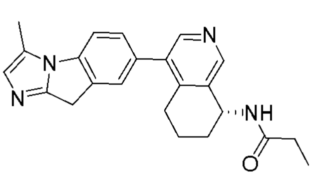,

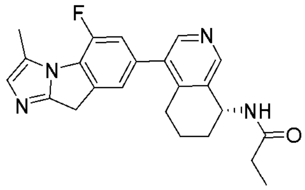,
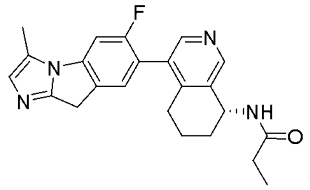,
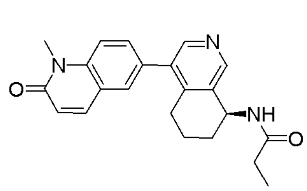,

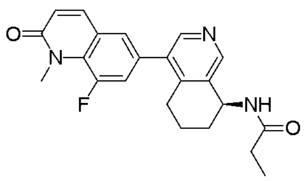,
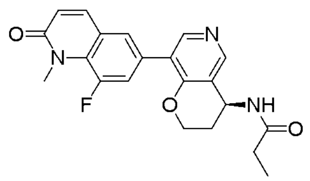,
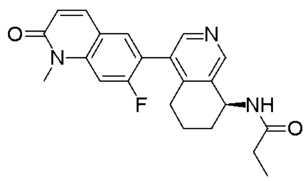,

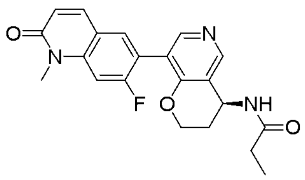,
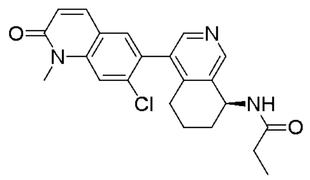,
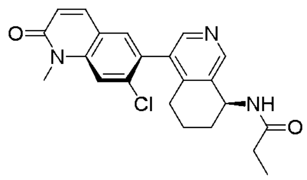,

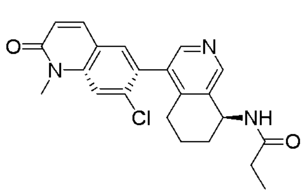,
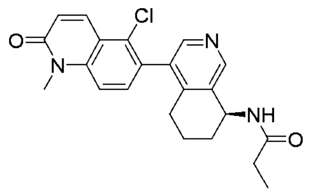,
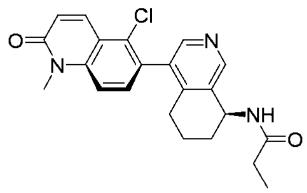,

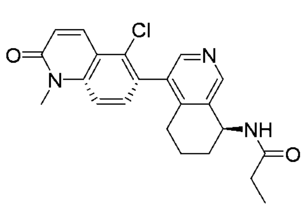,
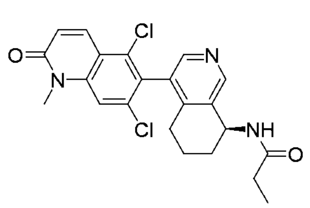,
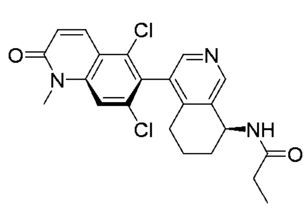,

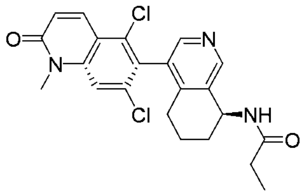
,
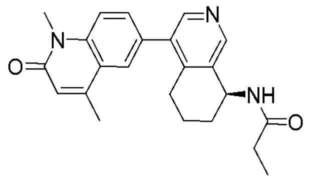
,
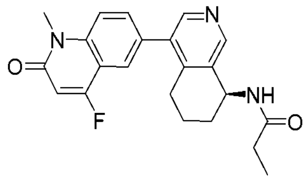
,
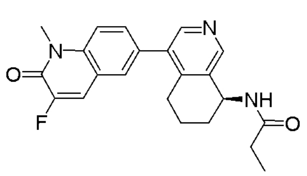
,
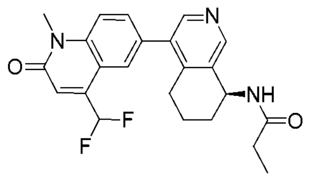
,
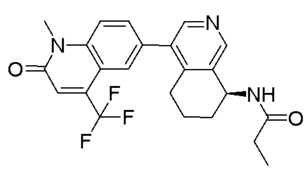
,
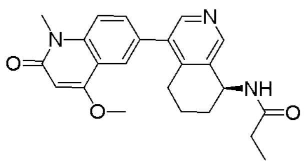
,
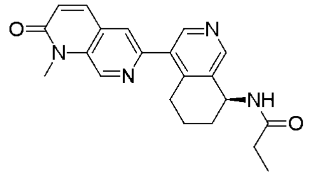
,
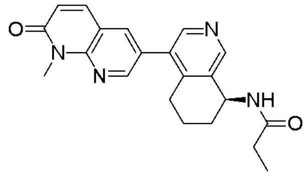
,
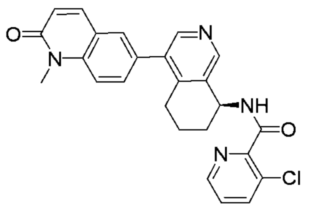
,
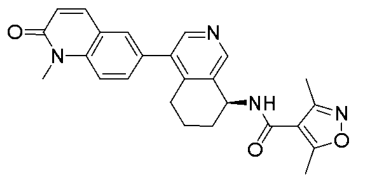
,
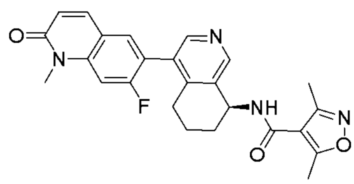
,
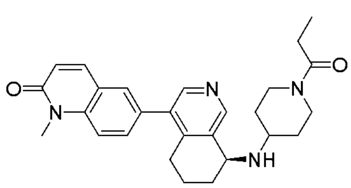
,
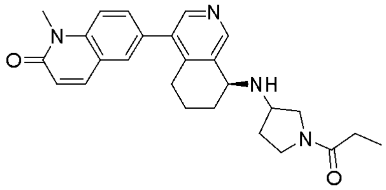
,
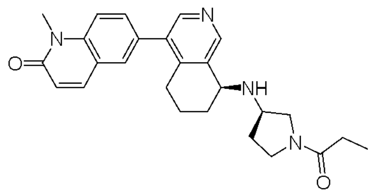
,
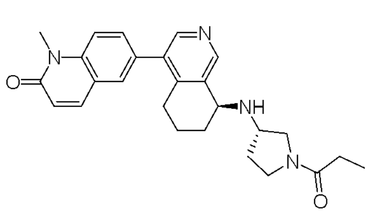
,
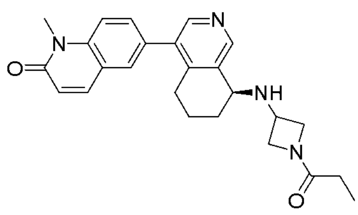
,
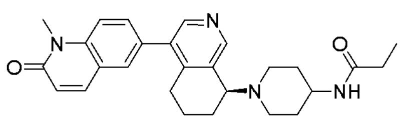
,
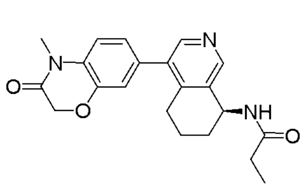
,
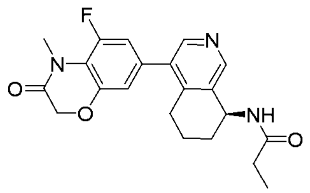
,
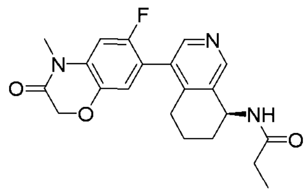
,

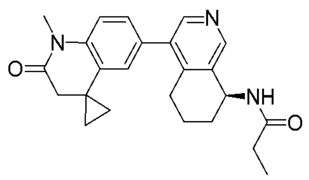 , 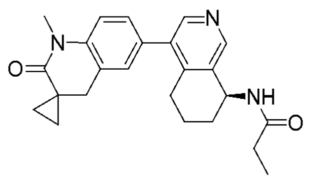 , 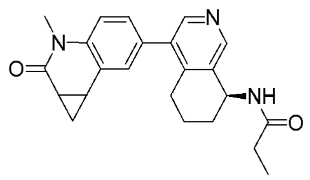 ,

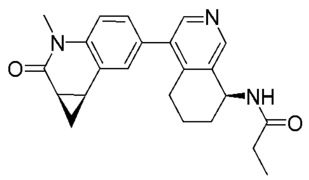 , 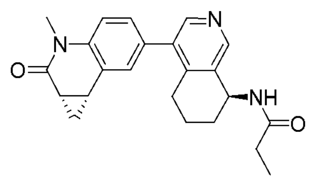 , 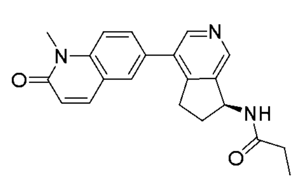 ,

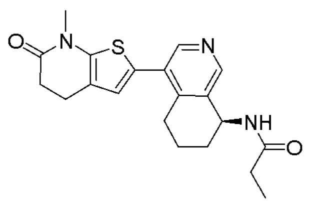 , 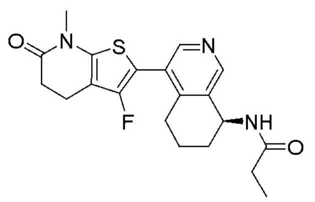 , 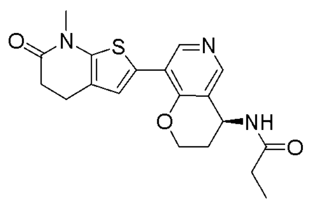 ,

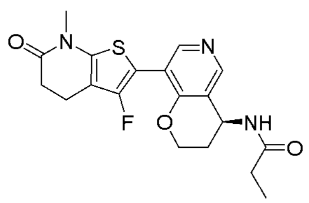 , 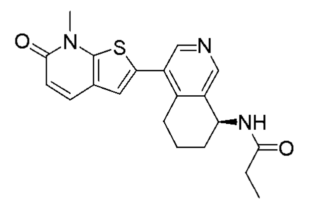 , 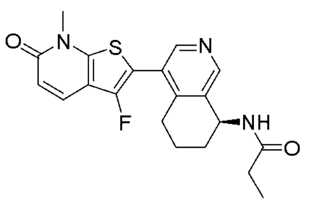 ,

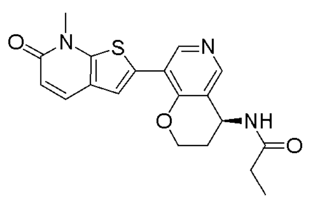 , 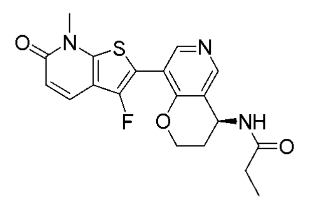 , 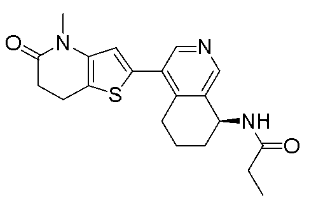 ,

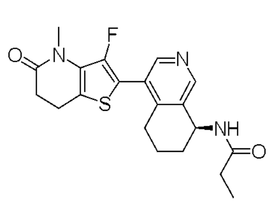 , 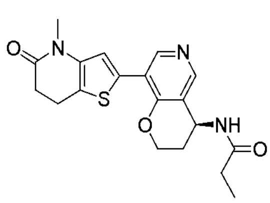 , 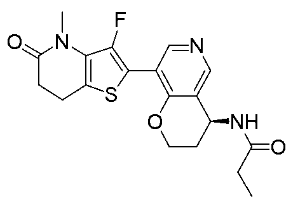 , 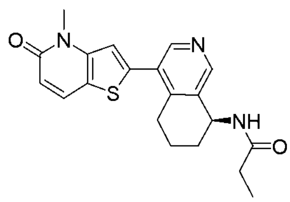 ,

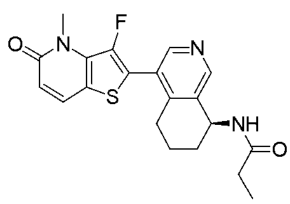 , 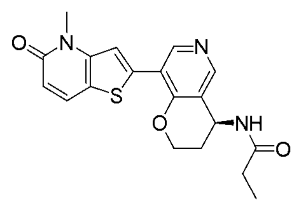 , 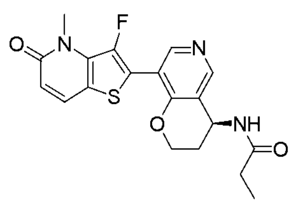 ,

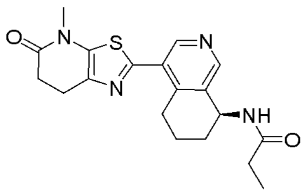 ,

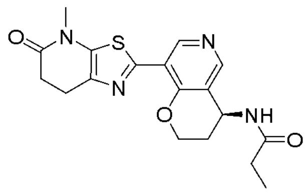 ,

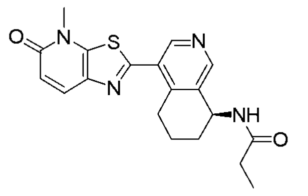 ,

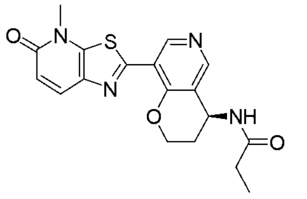 ,

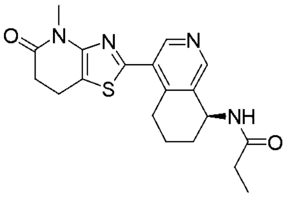 ,

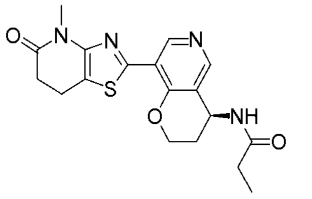 ,

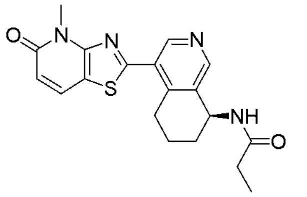 ,

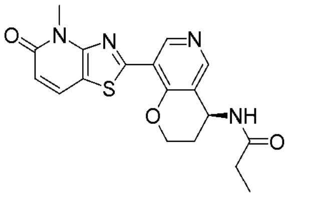 ,

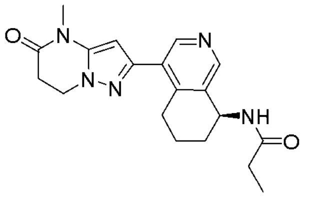 ,

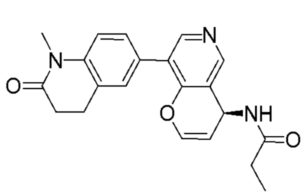 ,

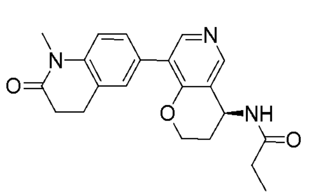 ,

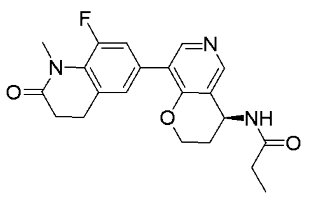 ,

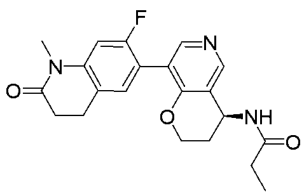 ,

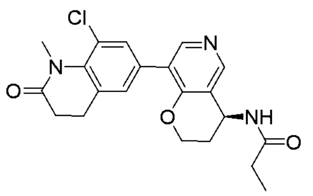 ,

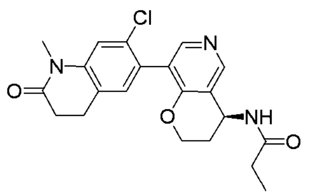 ,

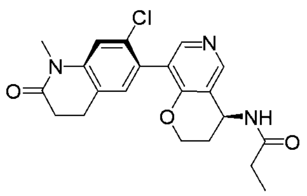 ,

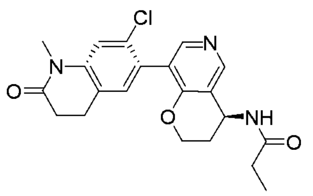 ,

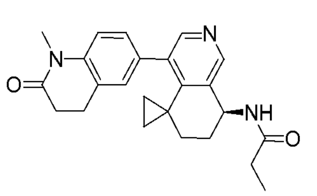 ,

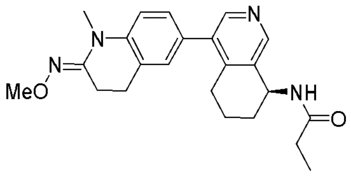 ,

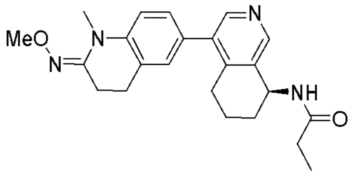 ,

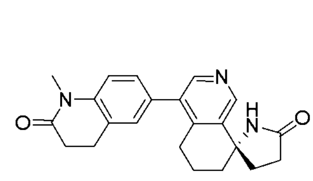 ,

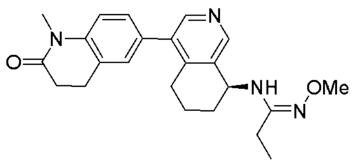 , 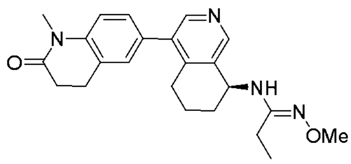 , 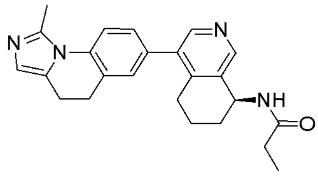 ,

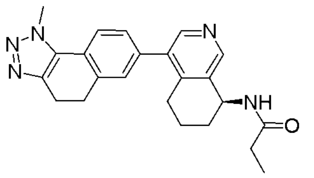 , 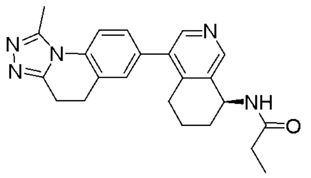 , 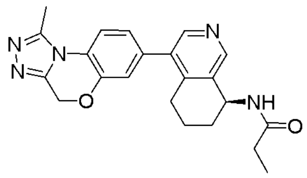 ,

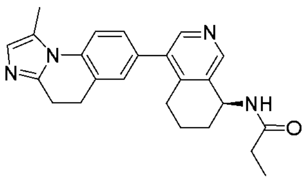 , 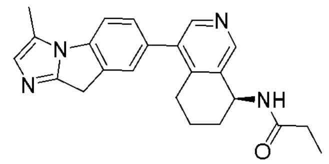 , 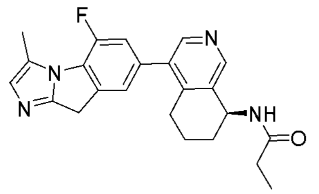 ,

and

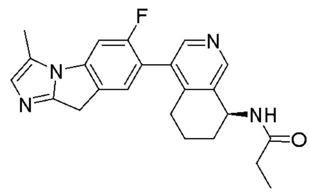 .

**24.** A use of the compound as defined in any one of claims 1 to 23, a stereoisomer thereof, or a pharmaceutically acceptable salt thereof, in the manufacture of a medicament for treating a disease associated with an aldosterone synthase inhibitor.

**25.** A use of the compound as defined in any one of claims 1 to 23, a stereoisomer thereof, or a pharmaceutically acceptable salt thereof, in the manufacture of a medicament for treating resistant hypertension.

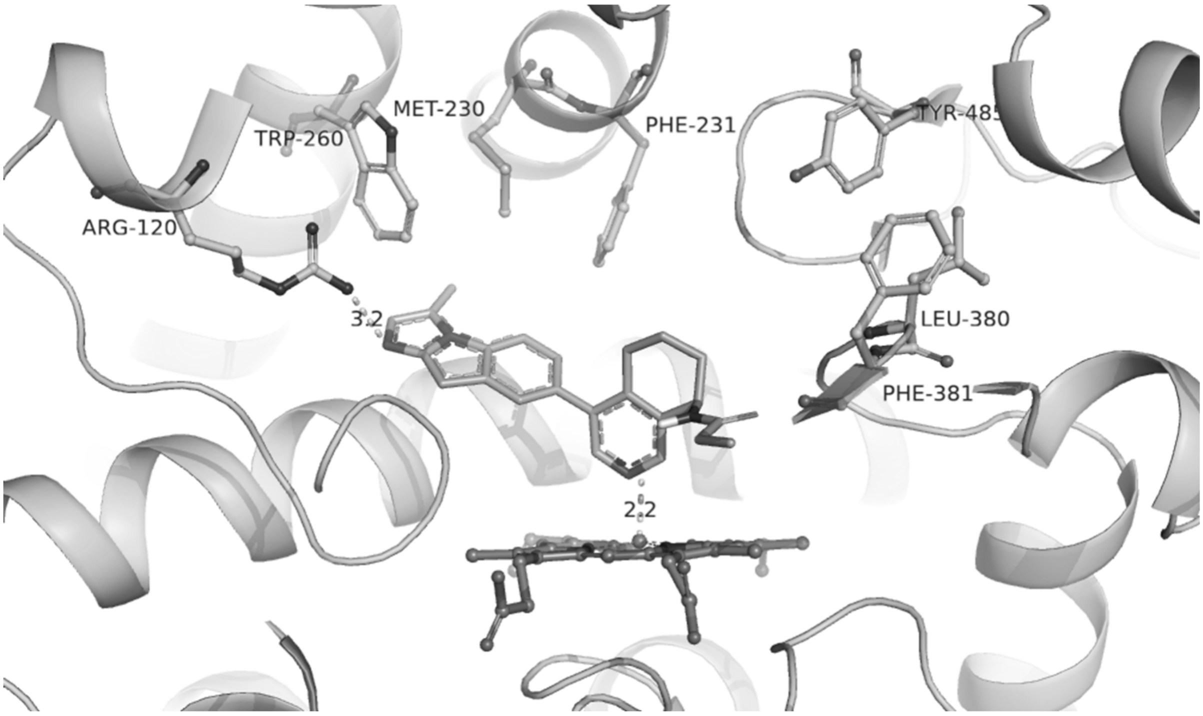
MET-230
TRP-260
PHE-231
TYR-485
ARG-120
3.2
LEU-380
PHE-381
2.2

FIG. 1

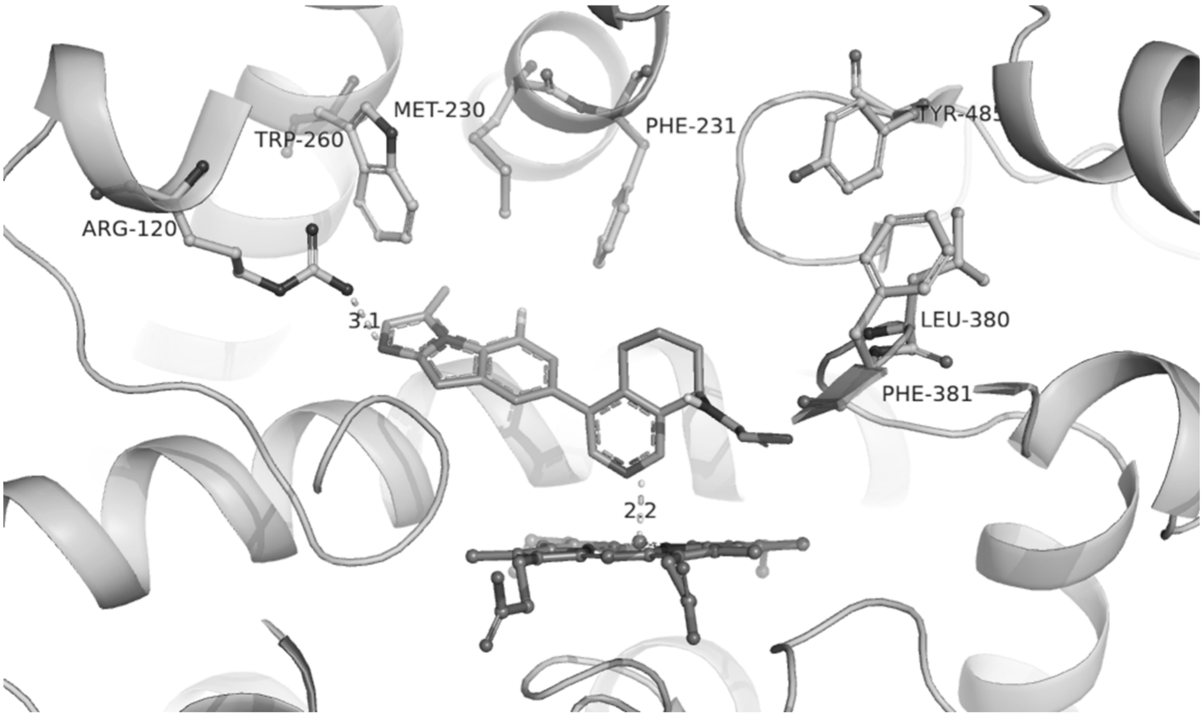
MET-230
TRP-260
PHE-231
TYR-485
ARG-120
3.1
LEU-380
PHE-381
2.2

FIG. 2

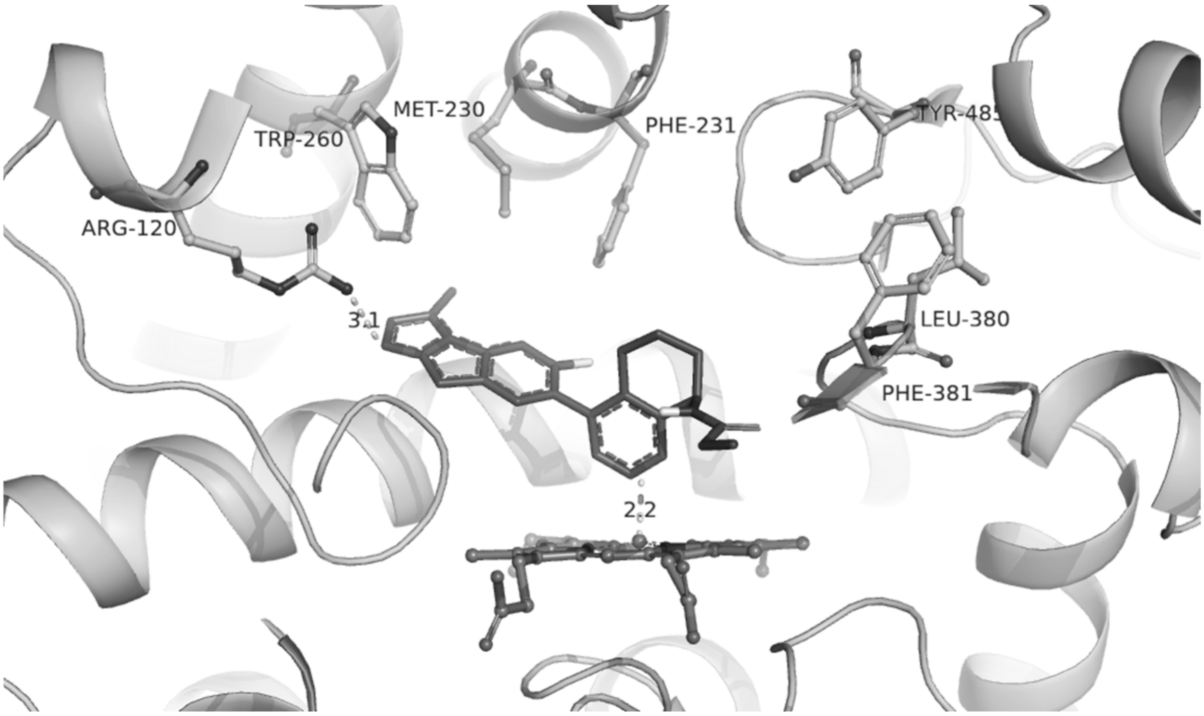


FIG. 3

## INTERNATIONAL SEARCH REPORT

International application No. **PCT/CN2024/092687**

**A. CLASSIFICATION OF SUBJECT MATTER**

C07D401/04(2006.01)i; C07D491/052(2006.01)i; C07D471/04(2006.01)i; C07D413/04(2006.01)i; C07D519/00(2006.01)i; C07D498/04(2006.01)i; A61P9/12(2006.01)i; A61K31/438(2006.01)i; A61K31/538(2006.01)i; A61K31/437(2006.01)i; A61K31/436(2006.01)i; A61K31/4375(2006.01)i; A61K31/4439(2006.01)i

According to International Patent Classification (IPC) or to both national classification and IPC

**B. FIELDS SEARCHED**

Minimum documentation searched (classification system followed by classification symbols)

IPC: C07D,A61P,A61K

Documentation searched other than minimum documentation to the extent that such documents are included in the fields searched

Electronic data base consulted during the international search (name of data base and, where practicable, search terms used)

CNTXT, DWPI, CNKI, 万方, WANFANG, STN(Registry, Caplus): 醛固酮, 酶, aldostrerone, synthase, CYP11B2, CYP11B, 基于式(II)的结构式检索, structural formula search based on formula II

**C. DOCUMENTS CONSIDERED TO BE RELEVANT**

| Category* | Citation of document, with indication, where appropriate, of the relevant passages | Relevant to claim No. |
|---|---|---|
| E | WO 2024109885 A1 (SHANGHAI HANSOH BIOMEDICAL CO., LTD. et al.) 30 May 2024 (2024-05-30)<br>page 60, claim 10, the fourth compound, page 61, the first to second, twelfth to thirteenth, fifteenth, thirty-ninth, and forty-third to forty-fourth compounds, page 62, the first to third and eighth to tenth compounds, page 64, the eleventh compound, and claims 12-13 | 1-25 |
| X | CN 103814021 A (HOFFMANN LA ROCHE) 21 May 2014 (2014-05-21)<br>entire document, in particular description, embodiments 60-63, 67-68, 70, 146, 160-161, 164, 168, and 170 | 1-20, 22-25 |
| Y | CN 103814021 A (HOFFMANN LA ROCHE) 21 May 2014 (2014-05-21)<br>entire document, in particular description, embodiments 60-63, 67-68, 70, 146, 160-161, 164, 168, and 170 | 1-25 |
| X | CN 103827101 A (HOFFMANN LA ROCHE) 28 May 2014 (2014-05-28)<br>entire document, in particular description, embodiments 2-3, 40-41, etc., compounds | 1-25 |
| Y | CN 103827101 A (HOFFMANN LA ROCHE) 28 May 2014 (2014-05-28)<br>entire document, in particular description, embodiments 2-3, 40-41, etc., compounds | 1-25 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 July 2024** | **19 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/092687**

**C. DOCUMENTS CONSIDERED TO BE RELEVANT**

| Category* | Citation of document, with indication, where appropriate, of the relevant passages | Relevant to claim No. |
|---|---|---|
| X | US 2014323468 A1 (BALESTRA MICHAEL et al.) 30 October 2014 (2014-10-30)<br>entire document, in particular table 1, compound 83 | 1-19, 24-25 |
| Y | US 2014323468 A1 (BALESTRA MICHAEL et al.) 30 October 2014 (2014-10-30)<br>entire document, in particular table 1, compound 83 | 1-25 |
| X | US 2014045819 A1 (HOYT SCOTT B et al.) 13 February 2014 (2014-02-13)<br>claims 1-3 and 21 | 1-20, 22-25 |
| Y | US 2014045819 A1 (HOYT SCOTT B et al.) 13 February 2014 (2014-02-13)<br>claims 1-3 and 21 | 1-25 |
| X | WO 2016089800 A1 (BOEHRINGER INGELHEIM INTERNATIONAL GMBH) 09 June 2016 (2016-06-09)<br>page 1, field of the invention, and page 8, table 1 | 1-25 |
| A | CN 101421273 A (SPEEDEL EXPERIMENTA AG) 29 April 2009 (2009-04-29)<br>entire document | 1-25 |
| A | CN 102459247 A (NOVARTIS AG) 16 May 2012 (2012-05-16)<br>entire document | 1-25 |
| A | CN 101506216 A (NOVARTIS AG) 12 August 2009 (2009-08-12)<br>entire document | 1-25 |

Form PCT/ISA/210 (second sheet) (July 2022)

**INTERNATIONAL SEARCH REPORT**
**Information on patent family members**

International application No.
**PCT/CN2024/092687**

| Patent document cited in search report | Publication date (day/month/year) | Patent family member(s) | Publication date (day/month/year) |
|---|---|---|---|
| WO 2024109885 A1 | 30 May 2024 | None | |
| CN 103814021 A | 21 May 2014 | SG 11201400679 WA | 28 April 2014 |
| | | KR 20140067121 A | 03 June 2014 |
| | | KR 101785143 B1 | 12 October 2017 |
| | | US 2013072679 A1 | 21 March 2013 |
| | | US 9260408 B2 | 16 February 2016 |
| | | HRP 20192323 T1 | 20 March 2020 |
| | | MX 2014002770 A | 05 June 2014 |
| | | MX 358376 B | 16 August 2018 |
| | | PE 20141372 A1 | 13 October 2014 |
| | | LT 2755963 T | 27 January 2020 |
| | | CO 6890099 A2 | 10 March 2014 |
| | | RS 59737 B1 | 28 February 2020 |
| | | WO 2013037779 A1 | 21 March 2013 |
| | | IL 231229 A0 | 30 April 2014 |
| | | CL 2014000604 A1 | 17 October 2014 |
| | | JP 2014527077 A | 09 October 2014 |
| | | JP 6012735 B2 | 25 October 2016 |
| | | HUE 047771 T2 | 28 May 2020 |
| | | BR 112014006283 A2 | 11 April 2017 |
| | | BR 112014006283 B1 | 03 November 2021 |
| | | PL 2755963 T3 | 27 July 2020 |
| | | NZ 621599 A | 29 July 2016 |
| | | UA 111626 C2 | 25 May 2016 |
| | | AU 2012307509 A1 | 13 March 2014 |
| | | AU 2012307509 B2 | 29 June 2017 |
| | | ZA 201401884 B | 27 November 2019 |
| | | ES 2763332 T3 | 28 May 2020 |
| | | EA 201490596 A1 | 30 July 2014 |
| | | EA 035757 B1 | 05 August 2020 |
| | | ECSP 14013245 A | 31 July 2014 |
| | | EP 2755963 A1 | 23 July 2014 |
| | | EP 2755963 B1 | 30 October 2019 |
| | | TW 201315724 A | 16 April 2013 |
| | | TWI 462914 B | 01 December 2014 |
| | | SI 2755963 T1 | 31 January 2020 |
| | | CA 2846785 A1 | 21 March 2013 |
| | | CA 2846785 C | 22 September 2020 |
| | | DK 2755963 T3 | 13 January 2020 |
| | | EP 3653618 A1 | 20 May 2020 |
| | | PT 2755963 T | 10 January 2020 |
| | | CR 20140091 A | 03 June 2014 |
| | | MY 169043 A | 07 February 2019 |
| | | AR 087901 A1 | 23 April 2014 |
| CN 103827101 A | 28 May 2014 | KR 20140076591 A | 20 June 2014 |
| | | KR 101723276 B1 | 04 April 2017 |
| | | SI 2758388 T1 | 31 May 2018 |
| | | PE 20141042 A1 | 27 August 2014 |
| | | SG 2014010599 A | 28 April 2014 |
| | | LT 2758388 T | 10 May 2018 |

Form PCT/ISA/210 (patent family annex) (July 2022)

**INTERNATIONAL SEARCH REPORT**
**Information on patent family members**

International application No.
**PCT/CN2024/092687**

| Patent document cited in search report | Publication date (day/month/year) | Patent family member(s) | Publication date (day/month/year) |
|---|---|---|---|
| | | NZ 620652 A | 30 September 2016 |
| | | NO 2758388 T3 | 21 July 2018 |
| | | HUE 038773 T2 | 28 November 2018 |
| | | CL 2014000663 A1 | 03 October 2014 |
| | | ES 2666802 T3 | 07 May 2018 |
| | | EP 2758388 A1 | 30 July 2014 |
| | | EP 2758388 B1 | 21 February 2018 |
| | | IL 230874 A0 | 31 March 2014 |
| | | IL 230874 A | 31 July 2016 |
| | | PL 2758388 T3 | 31 August 2018 |
| | | ECSP 14013264 A | 30 April 2014 |
| | | TW 201319054 A | 16 May 2013 |
| | | TWI 576342 B | 01 April 2017 |
| | | RS 57139 B1 | 31 July 2018 |
| | | WO 2013041591 A1 | 28 March 2013 |
| | | US 2013079365 A1 | 28 March 2013 |
| | | US 9353081 B2 | 31 May 2016 |
| | | DK 2758388 T3 | 07 May 2018 |
| | | ZA 201401577 B | 28 January 2015 |
| | | MX 2014002624 A | 14 April 2014 |
| | | MX 366095 B | 27 June 2019 |
| | | PE 20181378 A1 | 05 September 2018 |
| | | EA 201490567 A1 | 30 July 2014 |
| | | EA 035108 B1 | 28 April 2020 |
| | | UA 115972 C2 | 25 January 2018 |
| | | CA 2845170 A1 | 28 March 2013 |
| | | CA 2845170 C | 13 August 2019 |
| | | JP 2014526539 A | 06 October 2014 |
| | | JP 6012737 B2 | 25 October 2016 |
| | | CO 6870035 A2 | 20 February 2014 |
| | | PT 2758388 T | 17 April 2018 |
| | | AR 087984 A1 | 30 April 2014 |
| | | AU 2012311582 A1 | 20 February 2014 |
| | | AU 2012311582 B2 | 06 July 2017 |
| | | MY 186165 A | 30 June 2021 |
| | | HRP 20180592 T1 | 18 May 2018 |
| | | CR 20140089 A | 21 March 2014 |
| | | BR 112014006660 A2 | 04 April 2017 |
| | | BR 112014006660 B1 | 17 August 2021 |
| US 2014323468 A1 | 30 October 2014 | US 9181272 B2 | 10 November 2015 |
| | | UY 35551 A | 31 October 2014 |
| | | JP 2016518389 A | 23 June 2016 |
| | | JP 6067181 B2 | 25 January 2017 |
| | | EP 2991985 A1 | 09 March 2016 |
| | | EP 2991985 B1 | 13 June 2018 |
| | | WO 2014179186 A1 | 06 November 2014 |
| | | TW 201534606 A | 16 September 2015 |
| US 2014045819 A1 | 13 February 2014 | US 9073929 B2 | 07 July 2015 |
| | | JP 2014513095 A | 29 May 2014 |
| | | JP 5883501 B2 | 15 March 2016 |

Form PCT/ISA/210 (patent family annex) (July 2022)

**INTERNATIONAL SEARCH REPORT**
**Information on patent family members**

International application No.
**PCT/CN2024/092687**

| Patent document cited in search report | Publication date (day/month/year) | Patent family member(s) | Publication date (day/month/year) |
|---|---|---|---|
| | | TW 201309695 A | 01 March 2013 |
| | | AR 086482 A1 | 18 December 2013 |
| | | EP 2701512 A1 | 05 March 2014 |
| | | EP 2701512 A4 | 26 November 2014 |
| | | EP 2701512 B1 | 05 April 2017 |
| | | AU 2012250059 A1 | 10 October 2013 |
| | | WO 2012148808 A1 | 01 November 2012 |
| | | CA 2832996 A1 | 01 November 2012 |
| | | CA 2832996 C | 07 May 2019 |
| WO 2016089800 A1 | 09 June 2016 | JP 2017536362 A | 07 December 2017 |
| | | JP 6707084 B2 | 10 June 2020 |
| | | US 2017267688 A1 | 21 September 2017 |
| | | US 9890171 B2 | 13 February 2018 |
| | | EP 3227300 A1 | 11 October 2017 |
| | | EP 3227300 B1 | 19 June 2019 |
| CN 101421273 A | 29 April 2009 | ES 2398378 T3 | 15 March 2013 |
| | | IL 194578 A0 | 03 August 2009 |
| | | CA 2649210 A1 | 18 October 2007 |
| | | JP 2009533394 A | 17 September 2009 |
| | | JP 5350220 B2 | 27 November 2013 |
| | | BRPI 0710134 A2 | 02 August 2011 |
| | | TW 200813071 A | 16 March 2008 |
| | | WO 2007116098 A1 | 18 October 2007 |
| | | US 2009192145 A1 | 30 July 2009 |
| | | US 8138179 B2 | 20 March 2012 |
| | | AR 060589 A1 | 02 July 2008 |
| | | EP 2010540 A1 | 07 January 2009 |
| | | EP 2010540 B1 | 05 December 2012 |
| CN 102459247 A | 16 May 2012 | KR 20120036850 A | 18 April 2012 |
| | | ES 2430088 T3 | 18 November 2013 |
| | | AU 2010247391 A1 | 01 December 2011 |
| | | CA 2761853 A1 | 18 November 2010 |
| | | JP 2012526769 A | 01 November 2012 |
| | | JP 5654572 B2 | 14 January 2015 |
| | | EA 201101621 A1 | 30 May 2012 |
| | | US 2012071514 A1 | 22 March 2012 |
| | | US 8455522 B2 | 04 June 2013 |
| | | EP 2430018 A2 | 21 March 2012 |
| | | EP 2430018 B1 | 03 July 2013 |
| | | WO 2010130773 A2 | 18 November 2010 |
| | | WO 2010130773 A3 | 06 January 2011 |
| | | MX 2011012199 A | 08 December 2011 |
| | | BRPI 1012852 A2 | 19 June 2018 |
| CN 101506216 A | 12 August 2009 | CA 2660701 A1 | 06 March 2008 |
| | | BRPI 0715938 A2 | 20 May 2014 |
| | | RU 2009110442 A | 27 September 2010 |
| | | WO 2008027284 A1 | 06 March 2008 |
| | | JP 2010501573 A | 21 January 2010 |
| | | MX 2009002040 A | 06 March 2009 |
| | | US 2009264420 A1 | 22 October 2009 |

Form PCT/ISA/210 (patent family annex) (July 2022)

**INTERNATIONAL SEARCH REPORT**
**Information on patent family members**

International application No.
**PCT/CN2024/092687**

| Patent document cited in search report | Publication date (day/month/year) | Patent family member(s) | | | Publication date (day/month/year) |
|---|---|---|---|---|---|
| | | AU | 2007290695 | A1 | 06 March 2008 |
| | | KR | 20090055595 | A | 02 June 2009 |
| | | EP | 2057163 | A1 | 13 May 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310537851
- CN 202311235509X
- CN 202311542512
- CN 202410027233
- CN 202410149461


### Non-patent literature cited in the description


- Glide. Schrödinger, LLC, 2020
- Maestro. Schrödinger, LLC, 2020
- LigPrep. Schrödinger, LLC, 2020
- ConfGen. Schrödinger, LLC, 2020